(19) Europäisches Patentamt
European Patent Office
Office européen des brevets

(11) EP 3 860 994 B1

(12) **EUROPEAN PATENT SPECIFICATION**

(45) Date of publication and mention of the grant of the patent:
**14.08.2024 Bulletin 2024/33**

(21) Application number: **19869723.7**

(22) Date of filing: **03.10.2019**

(51) International Patent Classification (IPC):
***C07D 405/14*** (2006.01) ***C07D 413/14*** (2006.01)
***C07D 417/14*** (2006.01) ***A61K 31/422*** (2006.01)
***A61K 31/427*** (2006.01) ***A61K 31/506*** (2006.01)
***A61P 35/00*** (2006.01)

(52) Cooperative Patent Classification (CPC):
**C07D 405/04; A61P 35/00; C07D 307/80; C07D 413/04; C07D 413/14; C07D 417/04; C07D 417/14; C07D 498/08**

(86) International application number:
**PCT/CA2019/051423**

(87) International publication number:
**WO 2020/069625 (09.04.2020 Gazette 2020/15)**

(54) **TRANSCRIPTION FACTOR BRN2 INHIBITORY COMPOUNDS AS THERAPEUTICS AND METHODS FOR THEIR USE**

TRANSKRIPTIONSFAKTOR-BRN2-HEMMENDE VERBINDUNGEN ALS THERAPEUTIKA UND VERFAHREN ZU DEREN VERWENDUNG

COMPOSÉS INHIBITEURS DU FACTEUR DE TRANSCRIPTION BRN2 UTILISÉS EN TANT QU'AGENTS THÉRAPEUTIQUES ET LEURS PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2018 US 201862740629 P**

(43) Date of publication of application:
**11.08.2021 Bulletin 2021/32**

(73) Proprietor: **The University of British Columbia**
**Vancouver, British Columbia V6T 1Z3 (CA)**

(72) Inventors:
- **ZOUBEIDI, Amina**
  **Vancouver, British Columbia V7W 2J5 (CA)**
- **MUNUGANTI, Ravi Shashi Nayana**
  **Richmond, British Columbia V6X 0A9 (CA)**
- **BISHOP, Jennifer L.**
  **Vancouver, British Columbia V5V 1Z6 (CA)**
- **THAPER, Daksh**
  **Surrey, British Columbia V3S 3M6 (CA)**
- **VAHID, Sepideh**
  **Vancouver, British Columbia V6B 6B8 (CA)**

(74) Representative: **Novitas Patent AB**
**P.O. Box 55557**
**102 04 Stockholm (SE)**

(56) References cited:
**WO-A1-2015/120543**

- **DATABASE CAPLUS [online] -; 1 January 2016 (2016-01-01), - - - ET AL: "CAPLUS logfile", XP055973086, retrieved from - accession no. - Database accession no. -**
- **XU RUIXUE ET AL: "Synthesis and evaluation of novel thiazole-based derivatives as selective inhibitors of DNA-binding domain of the androgen receptor", CHEMICAL BIOLOGY & DRUG DESIGN, vol. 91, no. 1, 3 August 2017 (2017-08-03), pages 172 - 180, XP055923722, ISSN: 1747-0277, Retrieved from the Internet <URL:https://api.wiley.com/onlinelibrary/tdm/v1/articles/10.1111%2Fcbdd.13068> DOI: 10.1111/cbdd.13068**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DONG-DONG LU, XU-XIAN HE, FENG-SHOU LIU: "Bulky Yet Flexible Pd-PEPPSI-IPent for the Synthesis of Sterically Hindered Biaryls in Air", JOURNAL OF ORGANIC CHEMISTRY, vol. 82, no. 20, 20 October 2017 (2017-10-20), pages 10898 - 10911, XP055701431, ISSN: 0022-3263, DOI: 10.1021/acs.joc.7b01711
- HUANFENG JIANG, HUAWEN HUANG, HUA CAO, CHAORONG QI: "TBHP/I2-Mediated Domino Oxidative Cyclization for One-Pot Synthesis of Polysubstituted Oxazoles", ORGANIC LETTERS , vol. 12, no. 23, 3 December 2010 (2010-12-03), pages 5561 - 5563, XP055701434, ISSN: 1523-7060, DOI: 10.1021/ol1023085
- DATABASE CAPLUS 1 January 1979 (1979-01-01), SARDONIC G , KEMPTER G : "Heterocyclic substituted thiazoles as thiabendazole analogs", XP002259643, retrieved from STN Database accession no. 1979-152062
- SOBHI M GOMBA , ABDOU O ABDELHAMID , NADIA A ABDELREHEM , SABRA M KANDEEL : "Efficient Synthesis of New Benzofuran-based Thiazoles and Investigation of their Cytotoxic Activity Against Human Breast Carcinoma Cell Lines", JOURNAL OF HETEROCYCLIC CHEMISTRY, vol. 55, 1 April 2018 (2018-04-01), pages 995 - 1001, XP055701439

## Description

### TECHNICAL FIELD

This invention relates to therapeutics, their uses and methods for the treatment of various indications, including various cancers. In particular, the invention relates to therapies and methods of treatment for cancers such as prostate cancer.

### BACKGROUND

Progression from primary prostate cancer to advanced metastatic disease is heavily dependent on the androgen receptor (AR), which fuels tumor survival. In men whose treatments for localized prostate tumors have failed, or in those who present with metastatic disease, androgen deprivation therapies (ADT) are used to deplete circulating androgens to abrogate AR signaling and prevent disease progression. Eventually, however, prostate cancer recurs after first-line ADT as castration-resistant prostate cancer (CRPC). Despite low levels of serum androgens in men with CRPC, reactivation of the AR occurs; thus, it remains central to tumor cell survival, proliferation, and metastatic spread. Targeting the AR is a cornerstone therapeutic intervention in patients with CRPC, and AR pathway inhibitors (API) that further prevent AR activation, such as enzalutamide (ENZ), have become mainstays in the prostate cancer treatment landscape. Despite its being a potent API, the treatment benefits of ENZ are short-lived in patients with CRPC and resistance rapidly occurs.

ENZ-resistant ($ENZ^R$) CRPC represents a significant clinical challenge not only due to the lack of third-line treatment options to prevent AR-driven tumor progression but also because it can be a precursor to rapidly progressing and lethal neuroendocrine prostate cancer (NEPC). Although NEPC can rarely arise *de novo,* it is increasingly defined as a variant of highly API-resistant CRPC. Aside from the unique small-cell morphology and positive staining for neuroendocrine (NE) markers that characterize NEPC, it is often distinguished from prostatic adenocarcinoma by reduced AR expression or activity. Clinical presentation of NEPC reflects this shift away from reliance on the AR, as patients typically present with low circulating levels of PSA despite high metastatic burden in soft tissues, and are refractory to APIs. Importantly, it has been reported that under the strong selective pressure of potent APIs like ENZ, these "non-AR-driven" prostate cancers, which include NEPC, may constitute up to 25% of advanced, drug-resistant CRPC cases. Not surprisingly, therefore, the incidence of NEPC has significantly increased in recent years, coinciding with the widespread clinical use of APIs.

A number of molecular mechanisms likely facilitate the progression of CRPC to NEPC. These include loss of tumor suppressors, such as RB1 and p53, amplification of MYCN, mitotic deregulation through AURKA and PEG10, epigenetic controls such as REST and EZH2, and splicing factors like SSRM4.

Importantly, the AR plays a crucial, albeit still mechanistically unclear, role in NEPC. Reports over many years have highlighted how ADT or loss of AR promotes the NE differentiation of prostate cancer cells; as such, many genes associated with an NE phenotype, including ARG2, HASH1, and REST are controlled by the AR. Although this evidence underscores an inverse correlation between AR expression and/or activity and molecular events leading to NEPC, the mechanisms by which the AR directly influences the induction of an NEPC phenotype from CRPC under the selective pressure of APIs such as ENZ remain elusive.

Answering such questions requires a model of API-resistant CRPC that recapitulates the trans-differentiation of adenocarcinoma to NEPC that occurs in patients. Herein, we present an *in vivo* -derived model of $ENZ^R$, which, different from others, underscores the emergence of tumors with heterogeneous mechanisms of resistance to ENZ over multiple transplanted generations. These include the natural acquisition of known AR mutations found in $ENZ^R$ patients and the trans-differentiation of NEPC-like tumors through an AR+ state, without the manipulation of oncogenes typically used to establish NEPC in murine prostate cancer models. Bishop et al. (Cancer Discovery (2017) 7(1):54-71) show that a master regulator of neuronal differentiation, the POU-domain transcription factor BRN2 (encoded by POU3F2), is directly transcriptionally repressed by the AR, is required for the expression of terminal NE markers and aggressive growth of ENZ R CRPC, and is highly expressed in human NEPC and metastatic CRPC with low circulating PSA. Beyond suppressing BRN2 expression and activity, Bishop *et al.* show that the AR inhibits BRN2 regulation of SOX2, another transcription factor associated with NEPC, suggesting that relief of AR-mediated suppression of BRN2 is a consequence of ENZ treatment in CRPC that may facilitate the progression of NEPC, especially in men with "non-AR driven" disease.

WO 2015/120543 discloses compounds that modulate androgen receptor (AR) activity and that may be used in the treatment of cancers such as prostate cancer.

Xu et al. (Chemical Biology & Drug Design 2018, vol. 91, p. 172-180) disclose a series of thiazole-based inhibitors that selectively target the DNA-binding domain of the androgen receptor (AR).

## SUMMARY

This invention is based in part on the fortuitous discovery that compounds described herein modulate BRN2 activity. Specifically, some compounds identified herein, also show inhibition of BRN2 and a further subset show specificity for BRN2 expressing cells.

The invention is set out in the appended set of claims. The description may encompass subject-matter extending beyond the scope of the claims. However the scope of the invention and of protection is solely defined by the appended claims and shall not extend beyond their scope. In particular, the scope of protection shall not include any methods of therapeutic treatment of the human or animal body, even if disclosed or implied herein.

In accordance with a first aspect, there is provided a compound having the structure of Formula I:

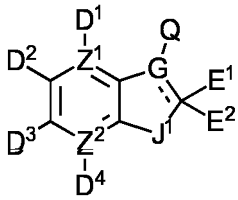

I.

wherein,

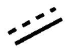

is a double bond: O is absent: G may be selected from N. C(H) and $C(CH_3)$; $J^1$ may be selected from N(H), $N(CH_3)$, $N(CH_2CH_3)$, $N(CF_3)$, $C(H)(CF_3)$, S and O; $Z^1$ may be C or N; $Z^2$ may be C or N; alternatively, $Z^1$ is N, when $D^1$ is absent; alternatively, $Z^2$ is N, when $D^4$ is absent; $E^2$ is absent, $E^1$ is $L^1$-$R^1$; $L^1$ may be selected from

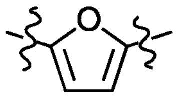,

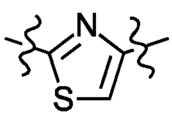, 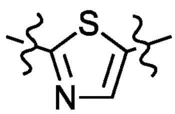, 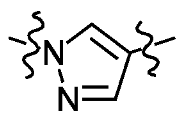, 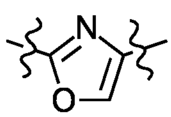, 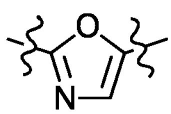, 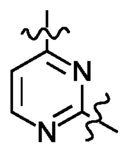,

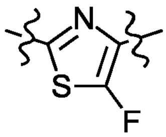, 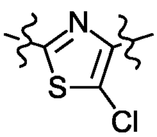, 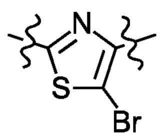, 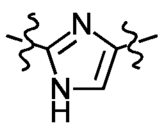, 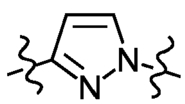, and 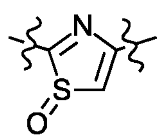;

R1 may be selected from

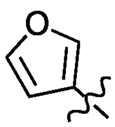, 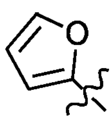, 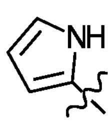, 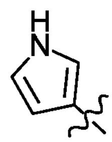, 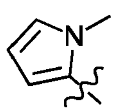, 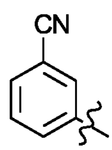,

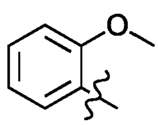, 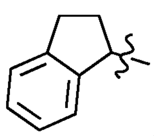, 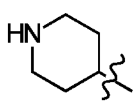, 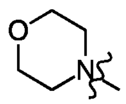, 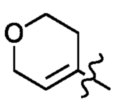, 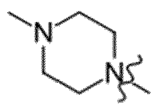, 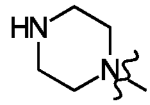, 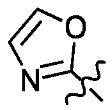.

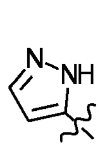, 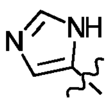, 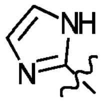, 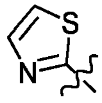, 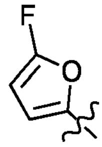, 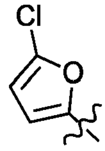, 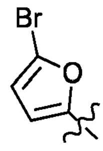, 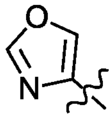, 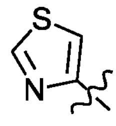, 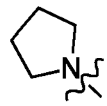, 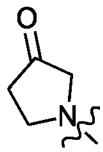, 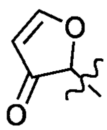, 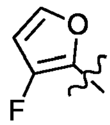, 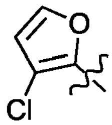, 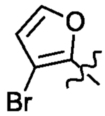, 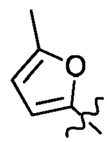, 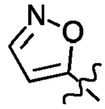, 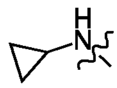, 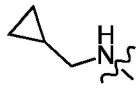

and

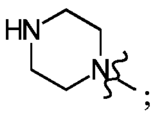;

alternatively, $R^1$ may be

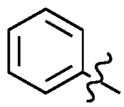

provided that $J^1$ may be selected from N(H), $N(CH_2CH_3)$, $N(CF_3)$, $C(H)(CF_3)$, S and O; alternatively, $R^1$ may be

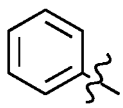

provided that $J^1$ may be $N(CH_3)$, $L^1$ may be selected from

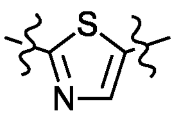, 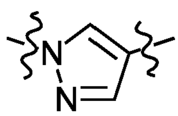, 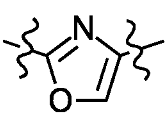,

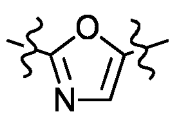

and

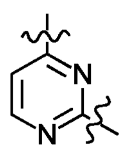

and $D^3$ may be selected from H, Br, F, Cl, $NO_2$, $CF_3$,

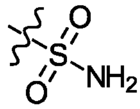,

OMe, $CH_3$ and OH; alternatively, $R^1$ may be

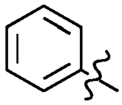

provided that $J^1$ may be $N(CH_3)$, $L^1$ may be

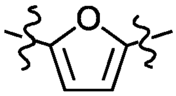

and $D^3$ may be selected from Br, F, Cl,

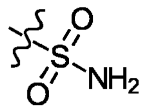,

OMe, $CH_3$ and OH; alternatively, $R^1$ may be

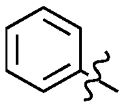

provided that $J^1$ may be $N(CH_3)$, $L^1$ may be

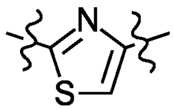

and $D^3$ may be selected from $NO_2$, $CF_3$,

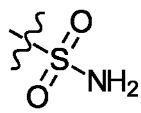,

OMe, $CH_3$ and OH; alternatively, $R^1$ may be

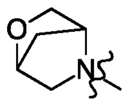

provided that $D^3$ may be selected from Br, F, Cl and $NO_2$; alternatively, $R^1$ may be

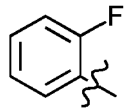

provided that $D^3$ may be selected from H, Br, F, Cl and $NO_2$; alternatively, $R^1$ may be

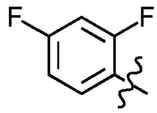

provided that $D^3$ may be H; alternatively, $R^1$ may be

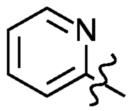,

provided that $J^1$ may be selected from N(H), $N(CH_3)$ and O; alternatively, $R^1$ may be

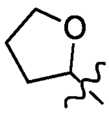

provided that $D^3$ may be selected from H, Br, F, Cl, $CF_3$ and $NO_2$; $D^1$ may be selected from H, Br, F, Cl and OH; $D^2$ may be selected from H, Br, F and Cl; $D^3$ may be selected from H, Br, F, Cl, $CF_3$,

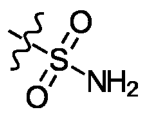,

OMe, $CH_3$ and OH; alternatively, $D^3$ may be $NO_2$, provided that when $R^1$ may be

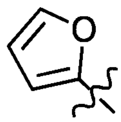

and when $J^1$ may be O, $D^3$ may be selected from H, Br, F, Cl, $CF_3$ and

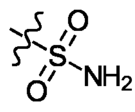;

D4 may be selected from H, Br, F and Cl; provided that the compound is not

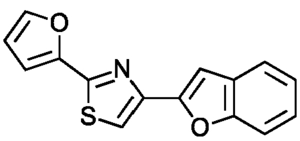, 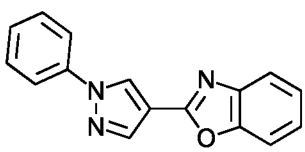, 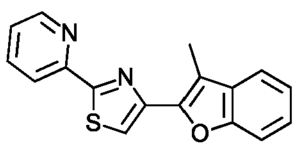,

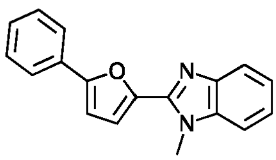 , 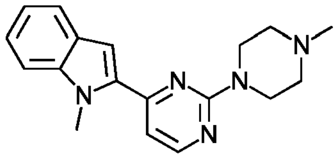 , 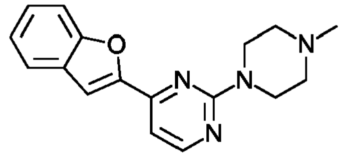 ,

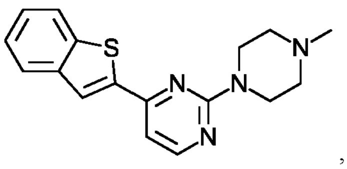 , 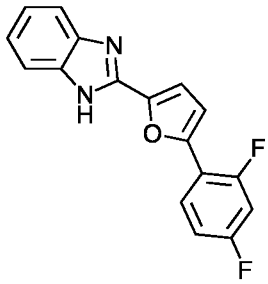 , 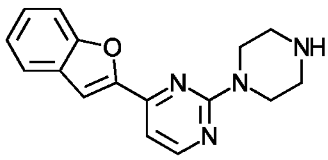 ,

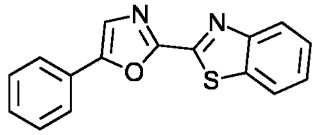 , 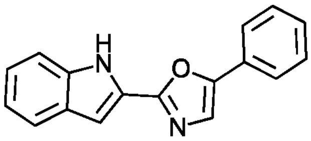 , 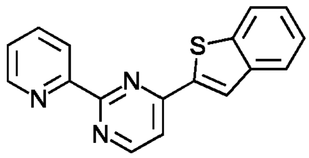 ,

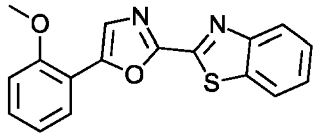 , 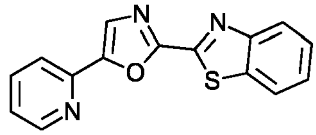 , 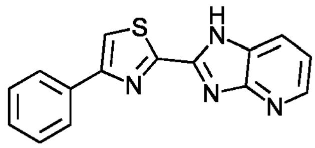 ,

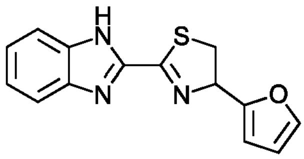 , 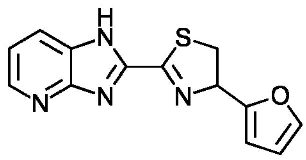 , 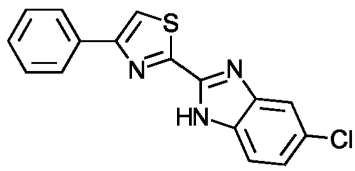 ,

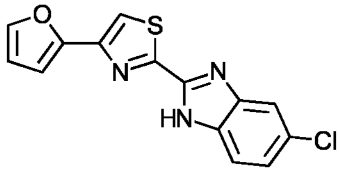 , 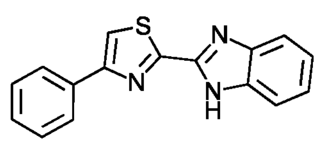 , 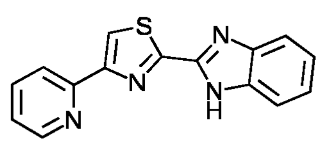 ,

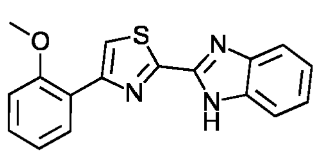 , 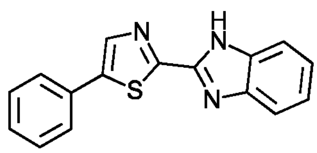 , 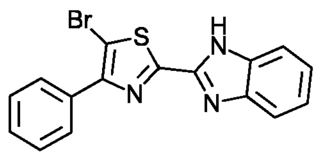 ,

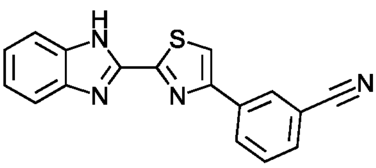 , 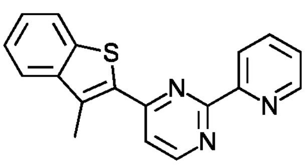 , 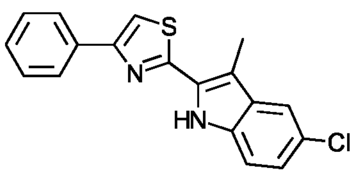 ,

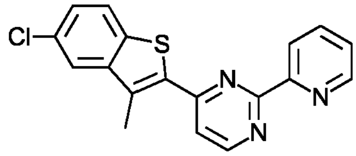, 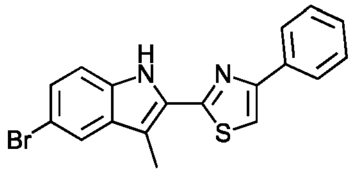, 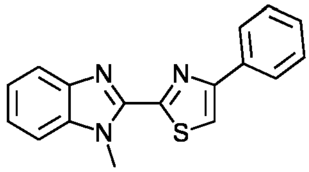,

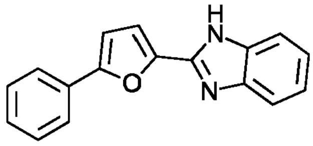 or 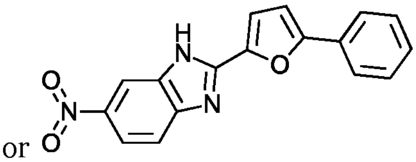.

Alternatively, $R^1$ may be

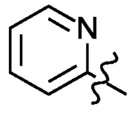,

provided that G is selected from C(H) and $C(CH_3)$.

Alternatively, $R^1$ may be

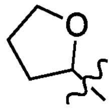

provided that $J^1$ is selected from N(H), $N(CF_3)$, $C(H)(CF_3)$, Sand O. Alternatively, $R^1$ may be

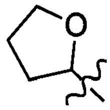

provided that $L^1$ is selected from

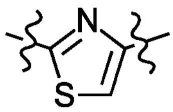,

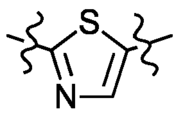, 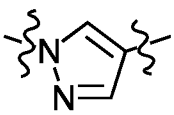, 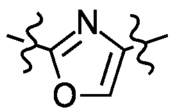, 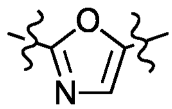, 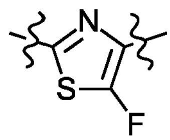, 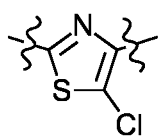,

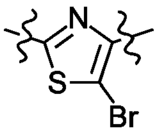, 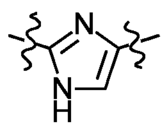, 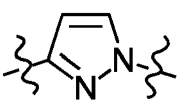, and 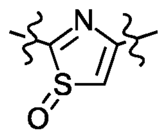.

Alternatively, $D^3$ maybe $NO_2$, provided that $J^1$ is selected from N(H), $N(CH_3)$, $N(CH_2CH_3)$, $N(CF_3)$ and $C(H)(CF_3)$. $L^1$ may be selected from

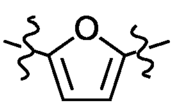, 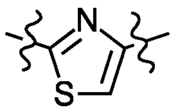, 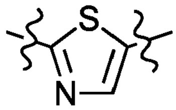, 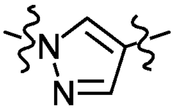,

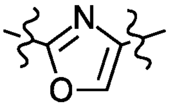, 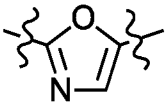, 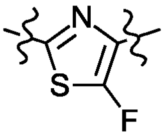, 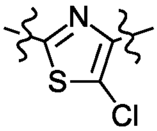, 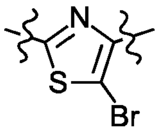, 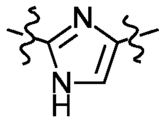,

and

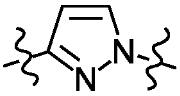.

$R^1$ may be selected from

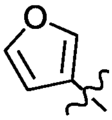, 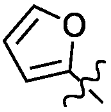, 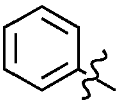, 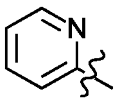, 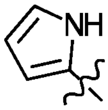,

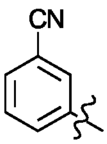, 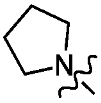, 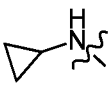, 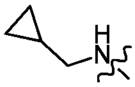, 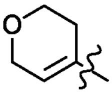, 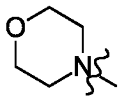, 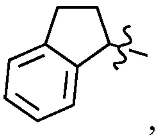, 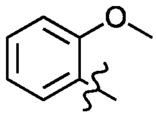,

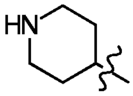, 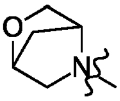, 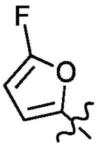, 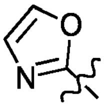, 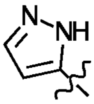, 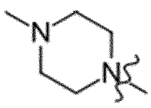 and 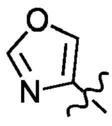.

$D^1$ may be selected from H, Br, F and Cl; $D^2$ may be selected from H, Br, F and Cl. $D^3$ may be selected from H, Br, F, Cl, $NO_2$, $CF_3$, OMe, $CH_3$, OH and

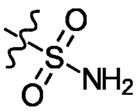.

$D^4$ may be selected from H, Br, F and Cl. $L^1$ may be selected from

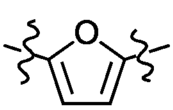, 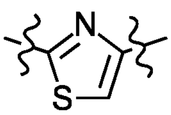, 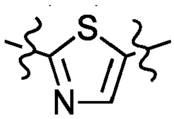 and 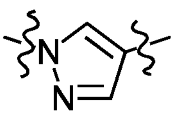.

R1 may be selected from

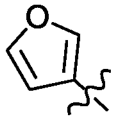, 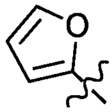, 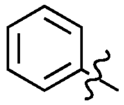, 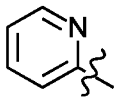 and 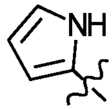.

$D^1$ may be selected from H, Br, F and Cl. $D^2$ may be selected from H, Br, F and Cl. $D^3$ may be selected from H, Br, F, Cl and $CF_3$. $D^4$ may be selected from H, Br, F and Cl. $L^1$ may be selected from

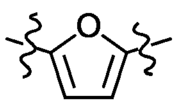, 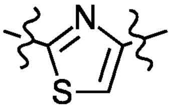, 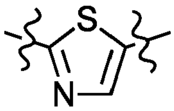

and

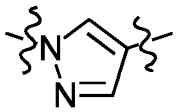.

R1 maybe selected from

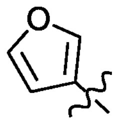, 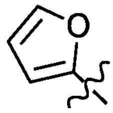, 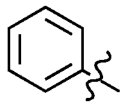, 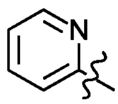 and 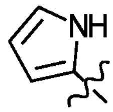.

$D^1$ may be H. $D^2$ may be H. $D^3$ may be selected from H, Br, F, Cl and $CF_3$. $D^4$ may be H. $R^1$ may be

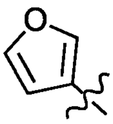, 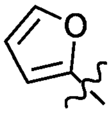, 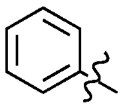, 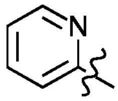, 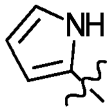, 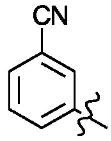, 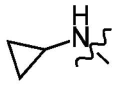,

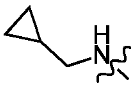, 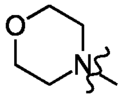, 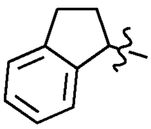, 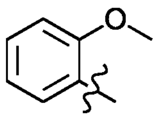, 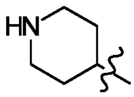, 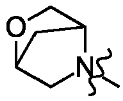, 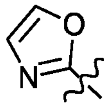, 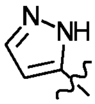

and

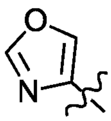.

$D^1$ may be H; $D^2$ may be H; $D^3$ may be selected from H, Br, F, Cl and $CF_3$; and $D^4$ may be H. $R^1$ may be

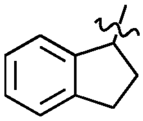

and $L^1$ may be selected from

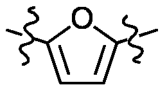

and

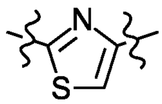

.

G may be selected from N, C(H) and $C(CH_3)$; and $J^1$ may be selected from N(H), $N(CH_3)$, $N(CH_2CH_3)$, S and O. $L^1$ may be selected from

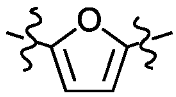

,

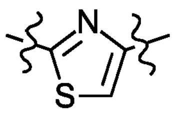

,

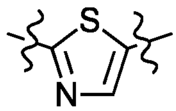

and

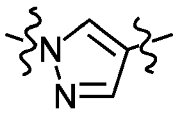

.

$R^1$ may be selected from

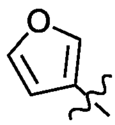

,

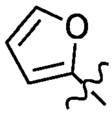

,

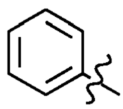

,

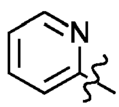

and

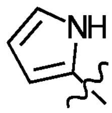

.

$D^1$ may be selected from H, Br, F and Cl. $D^2$ may be selected from H, Br, F and Cl. $D^3$ may be selected from H, Br, F, Cl and $CF_3$. $D^4$ may be selected from H, Br, F and Cl. G may be selected from N, C(H) and $C(CH_3)$. $J^1$ may be selected from N(H), $N(CH_3)$, $N(CH_2CH_3)$, S and O. $L^1$ may be selected from

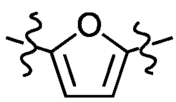

,

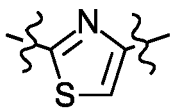

,

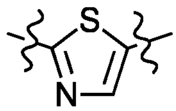

and

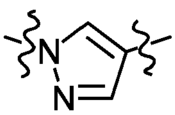

;

$R^1$ may be selected from

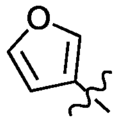, 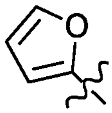, 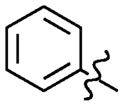, 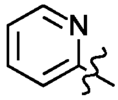

and

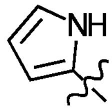;

$D^1$ may be H; $D^2$ may be H; $D^3$ may be selected from H, Br, F, Cl and $CF_3$; and $D^4$ may be H.

The compound may be

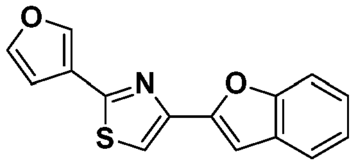, 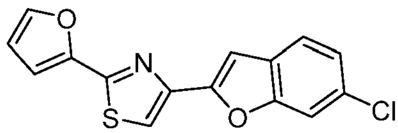,

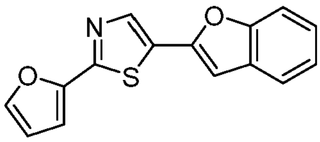, 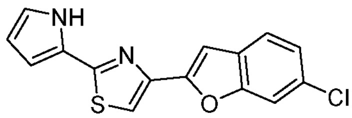, 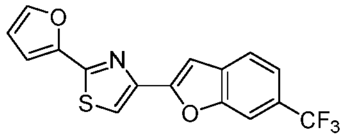

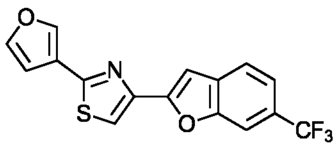, 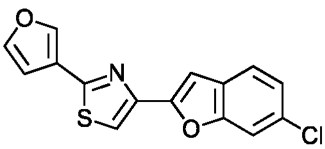, 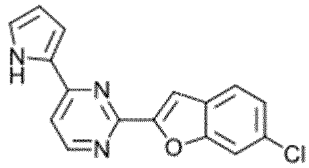,

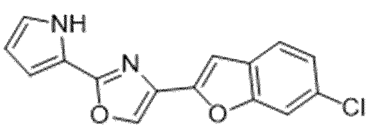, 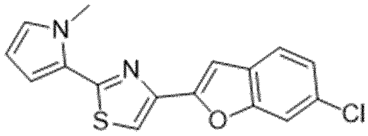, 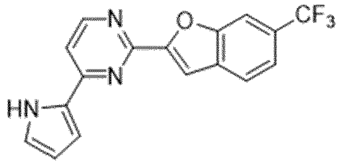,

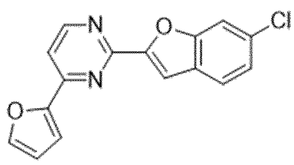, 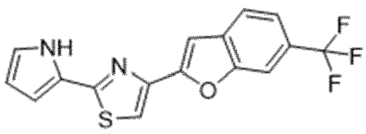, 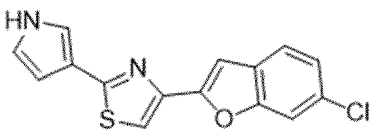,

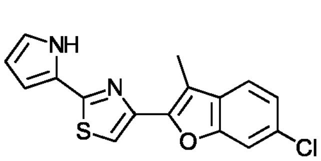, 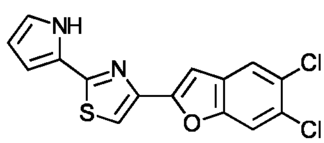, 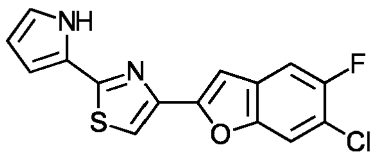

or

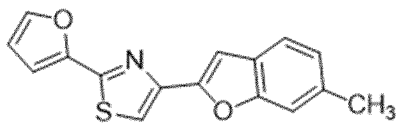.

The compound may have the structure of Formula II:

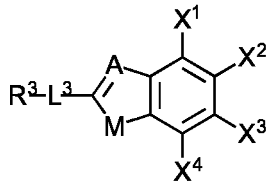 **II,**

wherein, A may be selected from N, C(H) and $C(CH_3)$; M may be selected from N(H), $N(CF_3)C(H)(CF_3)S$ and O; may be selected fro H, Br F and Cl; $X^2$ may be selected from H, Br, F and Cl;$X^3$ may be selected from H, Br, F, Cl, $CF^3$ may be selected from H, Br, F, Cl, $CF_3$,

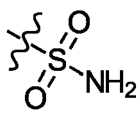

and OH; $X^4$ may be selected from H, Br, F and Cl; $L^3$ may be selected from

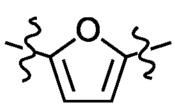, 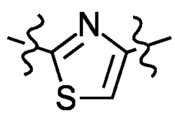, 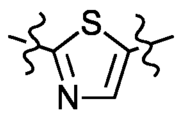, 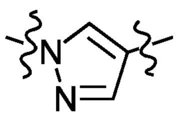,

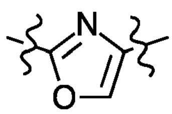, 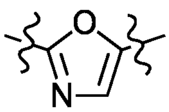 and 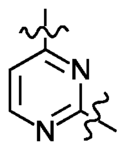;

$R^3$ may be selected from

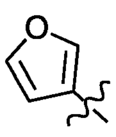, 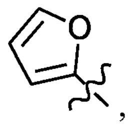,

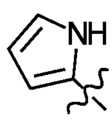, 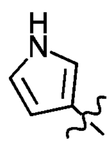 and 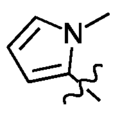.

In accordance with a further aspect, there is provided a compound having the structure of Formulas III:

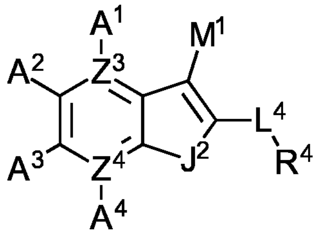

III

wherein, $J^2$ may be selected from $CH_2$, $CH(CH_3)$, N(H), $N(CH_3)$, $N(CH_2CH_3)$, $N(CF_3)$, $C(H)(CF_3)$, S and O; $M^1$ may be selected from H and $CH_3$; $Z^3$ may be C; $Z^4$ may be C; alternatively, $Z^3$ may be N, when $A^1$ is absent; alternatively, $Z^4$ may be N, when $A^4$ is absent; $L^4$ may be selected from

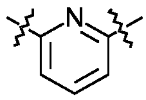 and 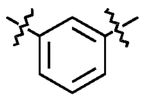 ;

$R^4$ may be selected from

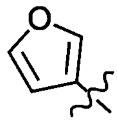 ,

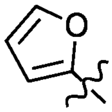 , 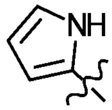 , 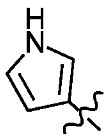 , 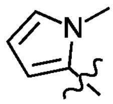 , 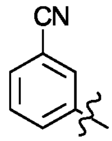 , 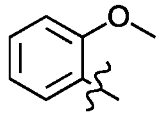 , 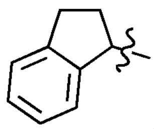 , 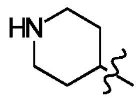 ,

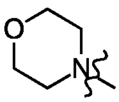 , 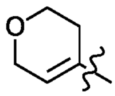 , 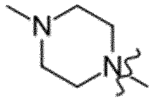 , 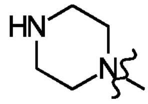 , 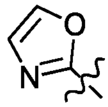 , 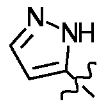 , 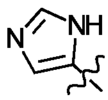 , 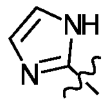 , 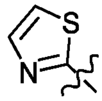 ,

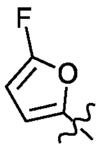 , 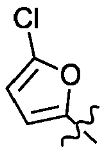 , 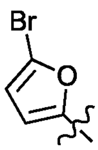 , 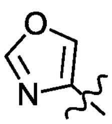 , 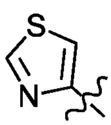 , 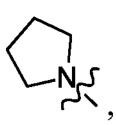 , 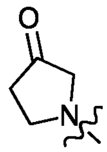 , 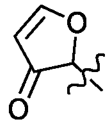 , 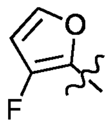 ,

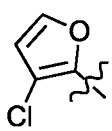 , 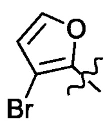 , 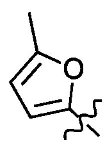 , 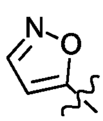 , 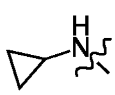 , 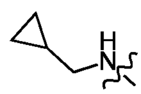 and 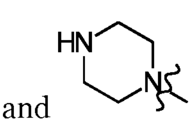 ;

$A^1$ may be selected from H, Br, F, Cl and OH; $A^2$ may be selected from H, Br, F and Cl; $A^3$ may be selected from H, Br, F, Cl, $CF_3$,

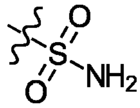 ,

OMe, $CH_3$, $NO_2$ and OH; and $A^4$ may be selected from H, Br, F and Cl.

$R^4$ may be selected from

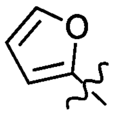, 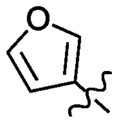, 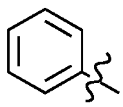, 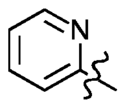, 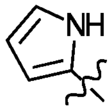, 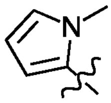

and

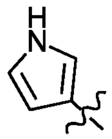.

$R^4$ may be selected from

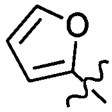, 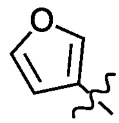, 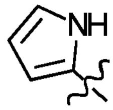, 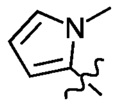 and 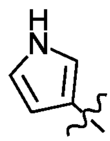.

$A^1$ may be selected from H, Br, F, Cl and OH. $A^2$ may be selected from H, Br, F and Cl. $A^3$ may be selected from H, Br, F, Cl, $CF_3$, and $CH_3$. $A^4$ may be selected from H, Br, F and Cl. $J^2$ may be selected from $CH_2$, $CH(CH_3)$, N(H), $N(CH_3)$, Sand O. $J^2$ may be selected from $CH_2$, $CH(CH_3)$, N(H), Sand O. $J^2$ may be O. $L^4$ may be

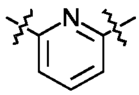.

The compound may be selected from

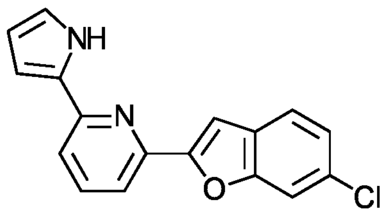 or 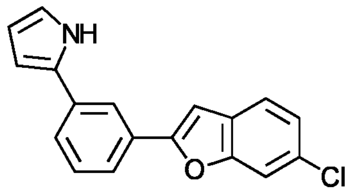.

Also disclosed herein is a method of inhibiting POU domain transcription factor BRN2, the method including administering a compound of any one of claims 1-13.

Also disclosed herein is a compound described herein, for use in inhibiting POU domain transcription factor BRN2.

Also described herein is a pharmaceutical composition for treating cancer, including compound described herein and a pharmaceutically acceptable carrier.

In accordance with a further aspect, there is provided a compound having the structure of Formula I:

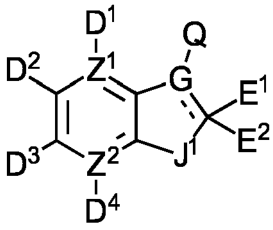

**I**

wherein,

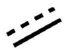

is a double bond;

Q is absent;

G is selected from N, C(H) and $C(CH_3)$;

$J^1$ is selected from N(H), $N(CH_3)$, $N(CH_2CH_3)$, $N(CF_3)$, $C(H)(CF_3)$, S and O;

$Z^1$ is CorN;

$Z^2$ is C or N;

alternatively, $Z^1$ is N, when $D^1$ is absent;

alternatively, $Z^2$ is N, when $D^4$ is absent;

$E^2$ is absent;

$E^1$ is $L^1$-$R^1$;

$L^1$ is selected from

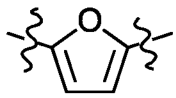, 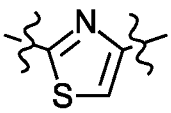, 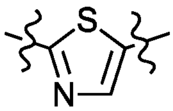, 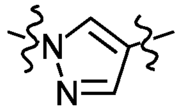, 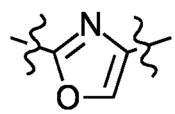, 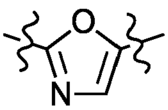, 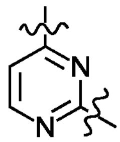, 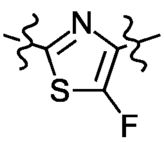, 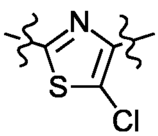, 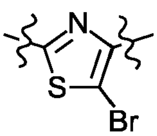, 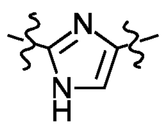,

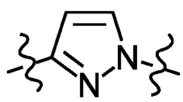, and 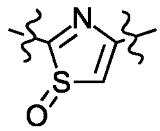;

$R^1$ is selected from

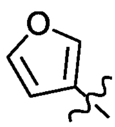, 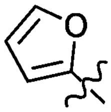, 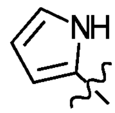, 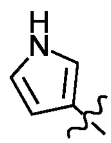, 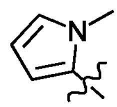, 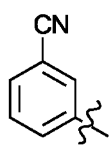, 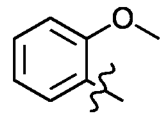,

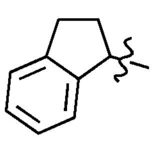, 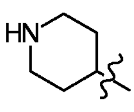, 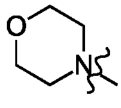, 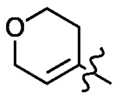, 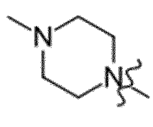, 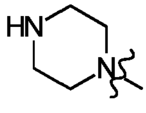, 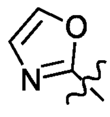, 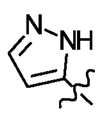,

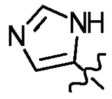, 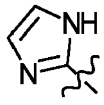, 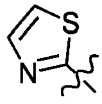, 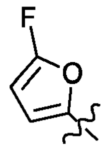, 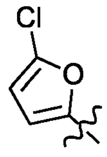, 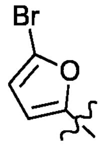, 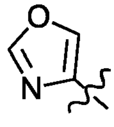, 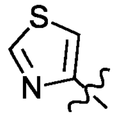, 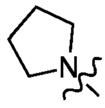,

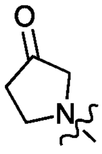, 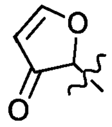, 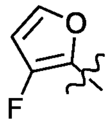, 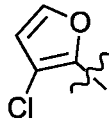, 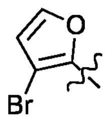, 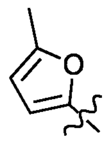, 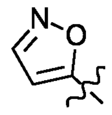, 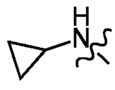, 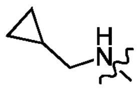

and

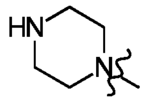;

alternatively, $R^1$ is

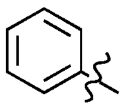

provided that $J^1$ is selected from N(H), $N(CH_2CH_3)$, $N(CF_3)$, $C(H)(CF_3)$, S and O; alternatively, $R^1$ is

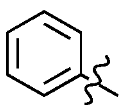

provided that $J^1$ is $N(CH_3)$, $L^1$ is selected from

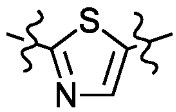,

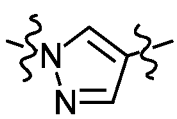, 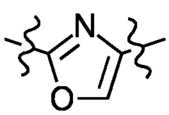, 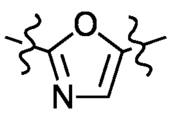 and 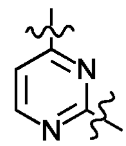

and $D^3$ is selected from H, Br, F, Cl, $NO_2$, $CF_3$,

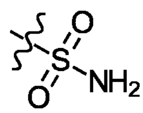,

OMe, $CH_3$ and OH; alternatively, $R^1$ is

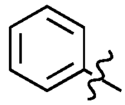

provided that $J^1$ is $N(CH_3)$, $L^1$ is

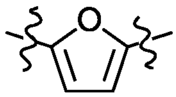

and $D^3$ is selected from Br, F, Cl,

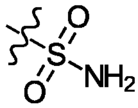,

OMe, $CH_3$ and OH;
alternatively, $R^1$ is

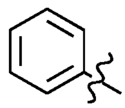

provided that $J^1$ is $N(CH_3)$, $L^1$ is

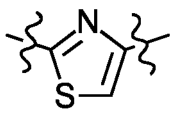

and $D^3$ is selected from $NO_2$, $CF_3$,

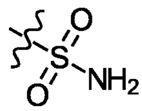,

OMe, $CH_3$ and OH;
alternatively, $R^1$ is

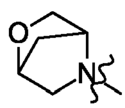

provided that $D^3$ is selected from Br, F, Cl and $NO_2$;
alternatively, $R^1$ is

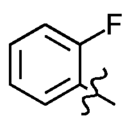

provided that $D^3$ is selected from H, Br, F, Cl and $NO_2$;

alternatively, $R^1$ is

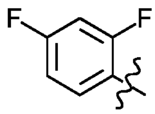

provided that $D^3$ is H;
alternatively, $R^1$ is

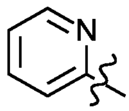,

provided that $J^1$ is selected from N(H), $N(CH_3)$ and O;
alternatively, $R^1$ is

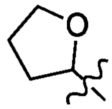

provided that $D^3$ is selected from H, Br, F, Cl, $CF_3$ and $NO_2$;
$D^1$ is selected from H, Br, F, Cl and OH;
$D^2$ is selected from H, Br, F and Cl;
$D^3$ is selected from H, Br, F, Cl, $CF_3$,

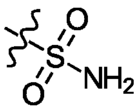,

OMe, $CH_3$ and OH;
alternatively, $D^3$ is $NO_2$, provided that when $R^1$ is

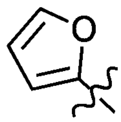

and when $J^1$ is O, $D^3$ is selected from H, Br, F, Cl, $CF_3$ and

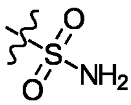;

$D^4$ is selected from H, Br, F and Cl;
or a compound having the structure of Formula II or III described herein; for use in treating cancer.

Also disclosed herein is a commercial package including (a) compound described herein and a pharmaceutically acceptable carrier; and (b) instructions for the use thereof for treating cancer.

Also disclosed herein is a commercial package including (a) a pharmaceutical composition described herein and a pharmaceutically acceptable carrier; and (b) instructions for the use thereof for treating cancer.

The inhibiting of the POU domain transcription factor BRN2, may be for the treatment of cancer. The cancer may be a BRN2 expressing cancer. The cancer may be selected from the following cancers: prostate cancer; lung cancer; bladder cancer; sarcoma; glioma; and melanoma. The prostate cancer may be selected from: Neuroendocrine

Prostate Cancer (NEPC); Prostate Adenocarcinoma; castration resistant prostate cancer (CRPC); androgen receptor pathway inhibitor (ARPI) resistant prostate cancer; enzalutamide (ENZ)-resistant ($ENZ^R$); and Abiraterone (Abi)-resistant ($ABI_R$). The lung cancer may be small cell lung cancer (SCLC) or lung adenocarcinoma. The bladder cancer may be small cell bladder cancer (SCBC). The sarcoma may be Ewing's sarcoma. The glioma may be glioblastoma multiforme. The BRN2 expressing cancer may be selected from the following: bladder cancer; cholangiocarcinoma; colorectal cancer; diffuse large B-cell lymphoma (DLBC); liver cancer; ovarian cancer; thymoma; thyroid cancer; clear cell renal cell carcinoma (CCRCC); chromophobe renal cell carcinoma (ChRCC); prostate cancer; breast cancer; uterine cancer; pancreatic cancer; cervical cancer; uveal melanoma; acute myeloid leukemia (AML); head and neck cancer; small cell lung cancer (SCLC); lung adenocarcinoma sarcoma; mesothelioma; adenoid cystic carcinoma (ACC); sarcoma; testicular germ cell cancer; uterine cancer; pheochromocytoma and paraganglioma (PCPG); melanoma; glioma; glioblastoma multiforme; T-cell Acute Lymphoblastic Leukemia; T-cell Lymphoma, medulloblastoma; and neuroblastoma.

## DETAILED DESCRIPTION

*In silica* computational drug discovery methods were used to conduct a virtual screen of ~4 million purchasable lead-like compounds from the ZINC database (Irwin, J. et al. Abstracts of Papers Am. Chem. Soc. (2005) 230:U1009) to identify potential BRN2 binders. The in silica methods included large-scale docking, in-site rescoring and consensus voting procedures.

It will be understood by a person of skill that COOH and $NR_2$ may include the corresponding ions, for example carboxylate ions and ammonium ions, respectively. Alternatively, where the ions are shown, a person of skill in the art will appreciate that the counter ion may also be present.

Those skilled in the art will appreciate that the point of covalent attachment of the moiety to the compounds as described herein may be, for example, and without limitation, cleaved under specified conditions. Specified conditions may include, for example, and without limitation, *in vivo* enzymatic or non-enzymatic means. Cleavage of the moiety may occur, for example, and without limitation, spontaneously, or it may be catalyzed, induced by another agent, or a change in a physical parameter or environmental parameter, for example, an enzyme, light, acid, temperature or pH. The moiety may be, for example, and without limitation, a protecting group that acts to mask a functional group, a group that acts as a substrate for one or more active or passive transport mechanisms, or a group that acts to impart or enhance a property of the compound, for example, solubility, bioavailability or localization.

In some embodiments, compounds of Formulas I-IV above may be used for systemic treatment of at least one indication selected from the group consisting of: prostate cancer, breast cancer, ovarian cancer, endometrial cancer, hair loss, acne, hirsutism, ovarian cysts, polycystic ovary disease, precocious puberty and age-related macular degeneration. In some embodiments compounds of Formulas I-IV may be used in the preparation of a medicament or a composition for systemic treatment of an indication described herein. In some embodiments, methods of systemically treating any of the indications described herein are also provided.

Compounds as described herein may be in the free form or in the form of a salt thereof. In some embodiment, compounds as described herein may be in the form of a pharmaceutically acceptable salt, which are known in the art (Berge S. M. et al., J. Pharm. Sci. (1977) 66(1):1-19). Pharmaceutically acceptable salt as used herein includes, for example, salts that have the desired pharmacological activity of the parent compound (salts which retain the biological effectiveness and/or properties of the parent compound and which are not biologically and/or otherwise undesirable). Compounds as described herein having one or more functional groups capable of forming a salt may be, for example, formed as a pharmaceutically acceptable salt. Compounds containing one or more basic functional groups may be capable of forming a pharmaceutically acceptable salt with, for example, a pharmaceutically acceptable organic or inorganic acid. Pharmaceutically acceptable salts may be derived from, for example, and without limitation, acetic acid, adipic acid, alginic acid, aspartic acid, ascorbic acid, benzoic acid, benzenesulfonic acid, butyric acid, cinnamic acid, citric acid, camphoric acid, camphorsulfonic acid, cyclopentanepropionic acid, diethylacetic acid, digluconic acid, dodecylsulfonic acid, ethanesulfonic acid, formic acid, fumaric acid, glucoheptanoic acid, gluconic acid, glycerophosphoric acid, glycolic acid, hemisulfonic acid, heptanoic acid, hexanoic acid, hydrochloric acid, hydrobromic acid, hydriodic acid, 2-hydroxyethanesulfonic acid, isonicotinic acid, lactic acid, malic acid, maleic acid, malonic acid, mandelic acid, methanesulfonic acid, 2-napthalenesulfonic acid, naphthalenedisulphonic acid, p-toluenesulfonic acid, nicotinic acid, nitric acid, oxalic acid, pamoic acid, pectinic acid, 3-phenylpropionic acid, phosphoric acid, picric acid, pimelic acid, pivalic acid, propionic acid, pyruvic acid, salicylic acid, succinic acid, sulfuric acid, sulfamic acid, tartaric acid, thiocyanic acid or undecanoic acid. Compounds containing one or more acidic functional groups may be capable of forming pharmaceutically acceptable salts with a pharmaceutically acceptable base, for example, and without limitation, inorganic bases based on alkaline metals or alkaline earth metals or organic bases such as primary amine compounds, secondary amine compounds, tertiary amine compounds, quaternary amine compounds, substituted amines, naturally occurring substituted amines, cyclic amines or basic ion-exchange resins. Pharmaceutically acceptable salts may be derived from, for example, and without limitation, a hydroxide, carbonate, or bicarbonate of a pharmaceutically acceptable metal cation

such as ammonium, sodium, potassium, lithium, calcium, magnesium, iron, zinc, copper, manganese or aluminum, ammonia, benzathine, meglumine, methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, glucamine, methylglucamine, theobromine, purines, piperazine, piperidine, procaine, N-ethylpiperidine, theobromine, tetramethylammonium compounds, tetraethylammonium compounds, pyridine, N,N-dimethylaniline, N-methylpiperidine, morpholine, N-methylmorpholine, N-ethylmorpholine, dicyclohexylamine, dibenzylamine, N,N-dibenzylphenethylamine, 1-ephenamine, N,N'-dibenzylethylenediamine or polyamine resins. In some embodiments, compounds as described herein may contain both acidic and basic groups and may be in the form of inner salts or zwitterions, for example, and without limitation, betaines. Salts as described herein may be prepared by conventional processes known to a person skilled in the art, for example, and without limitation, by reacting the free form with an organic acid or inorganic acid or base, or by anion exchange or cation exchange from other salts. Those skilled in the art will appreciate that preparation of salts may occur *in situ* during isolation and purification of the compounds or preparation of salts may occur by separately reacting an isolated and purified compound.

In some embodiments, compounds and all different forms thereof (e.g. free forms, salts, polymorphs, isomeric forms) as described herein may be in the solvent addition form, for example, solvates. Solvates contain either stoichiometric or non-stoichiometric amounts of a solvent in physical association the compound or salt thereof. The solvent may be, for example, and without limitation, a pharmaceutically acceptable solvent. For example, hydrates are formed when the solvent is water or alcoholates are formed when the solvent is an alcohol.

In some embodiments, compounds and all different forms thereof (e.g. free forms, salts, solvates, isomeric forms) as described herein may include crystalline and amorphous forms, for example, polymorphs, pseudopolymorphs, conformational polymorphs, amorphous forms, or a combination thereof. Polymorphs include different crystal packing arrangements of the same elemental composition of a compound. Polymorphs usually have different X-ray diffraction patterns, infrared spectra, melting points, density, hardness, crystal shape, optical and electrical properties, stability and/or solubility. Those skilled in the art will appreciate that various factors including recrystallization solvent, rate of crystallization and storage temperature may cause a single crystal form to dominate.

In some embodiments, compounds and all different forms thereof (e.g. free forms, salts, solvates, polymorphs) as described herein include isomers such as geometrical isomers, optical isomers based on asymmetric carbon, stereoisomers, tautomers, individual enantiomers, individual diastereomers, racemates, diastereomeric mixtures and combinations thereof, and are not limited by the description of the formula illustrated for the sake of convenience.

In some embodiments, pharmaceutical compositions as described herein may comprise a salt of such a compound, preferably a pharmaceutically or physiologically acceptable salt. Pharmaceutical preparations will typically comprise one or more carriers, excipients or diluents acceptable for the mode of administration of the preparation, be it by injection, inhalation, topical administration, lavage, or other modes suitable for the selected treatment. Suitable carriers, excipients or diluents (used interchangeably herein) are those known in the art for use in such modes of administration.

Suitable pharmaceutical compositions may be formulated by means known in the art and their mode of administration and dose determined by the skilled practitioner. For parenteral administration, a compound may be dissolved in sterile water or saline or a pharmaceutically acceptable vehicle used for administration of non-water soluble compounds such as those used for vitamin K. For enteral administration, the compound may be administered in a tablet, capsule or dissolved in liquid form. The tablet or capsule maybe enteric coated, or in a formulation for sustained release. Many suitable formulations are known, including, polymeric or protein microparticles encapsulating a compound to be released, ointments, pastes, gels, hydrogels, or solutions which can be used topically or locally to administer a compound. A sustained release patch or implant may be employed to provide release over a prolonged period of time. Many techniques known to one of skill in the art are described in Remington: the Science & Practice of Pharmacy by Alfonso Gennaro, 20th ed., Lippencott Williams & Wilkins, (2000). Formulations for parenteral administration may, for example, contain excipients, polyalkylene glycols such as polyethylene glycol, oils of vegetable origin, or hydrogenated naphthalenes. Biocompatible, biodegradable lactide polymer, lactide/glycolide copolymer, or polyoxyethylene-polyoxypropylene copolymers may be used to control the release of the compounds. Other potentially useful parenteral delivery systems for modulatory compounds include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

Compounds or pharmaceutical compositions as described herein or for use as described herein may be administered by means of a medical device or appliance such as an implant, graft, prosthesis, stent, etc. Also, implants may be devised which are intended to contain and release such compounds or compositions. An example would be an implant made of a polymeric material adapted to release the compound over a period of time.

An "effective amount" of a pharmaceutical composition as described herein includes a therapeutically effective amount or a prophylactically effective amount. A "therapeutically effective amount" refers to an amount effective, at

dosages and for periods of time necessary, to achieve the desired therapeutic result, such as reduced tumor size, increased life span or increased life expectancy. A therapeutically effective amount of a compound may vary according to factors such as the disease state, age, sex, and weight of the subject, and the ability of the compound to elicit a desired response in the subject. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as smaller tumors, increased life span, increased life expectancy or prevention of the progression of prostate cancer to an androgen-independent form. Typically, a prophylactic dose is used in subjects prior to or at an earlier stage of disease, so that a prophylactically effective amount may be less than a therapeutically effective amount.

It is to be noted that dosage values may vary with the severity of the condition to be alleviated. For any particular subject, specific dosage regimens may be adjusted over time according to the individual need and the professional judgment of the person administering or supervising the administration of the compositions. Dosage ranges set forth herein are exemplary only and do not limit the dosage ranges that may be selected by medical practitioners. The amount of active compound(s) in the composition may vary according to factors such as the disease state, age, sex, and weight of the subject. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

In some embodiments, compounds and all different forms thereof as described herein may be used, for example, and without limitation, in combination with other treatment methods for at least one indication selected from the group consisting of: prostate cancer, breast cancer, ovarian cancer, endometrial cancer, hair loss, acne, hirsutism, ovarian cysts, polycystic ovary disease, precocious puberty and age-related macular degeneration. For example, compounds and all their different forms as described herein maybe used as neo-adjuvant (prior), adjunctive (during), and/or adjuvant (after) therapy with surgery, radiation (brachytherapy or external beam), or other therapies (for example, HIFU).

In general, compounds as described herein should be used without causing substantial toxicity. Toxicity of the compounds as described herein can be determined using standard techniques, for example, by testing in cell cultures or experimental animals and determining the therapeutic index, i.e., the ratio between the LD50 (the dose lethal to 50% of the population) and the LD100 (the dose lethal to 100% of the population). In some circumstances however, such as in severe disease conditions, it may be appropriate to administer substantial excesses of the compositions. Some compounds as described herein may be toxic at some concentrations. Titration studies may be used to determine toxic and non-toxic concentrations. Toxicity may be evaluated by examining a particular compound's or composition's specificity across cell lines using PC3 cells as a negative control that do not express AR. Animal studies may be used to provide an indication if the compound has any effects on other tissues. Systemic therapy that targets the AR will not likely cause major problems to other tissues since anti-androgens and androgen insensitivity syndrome are not fatal.

Compounds as described herein may be administered to a subject. As used herein, a "subject" may be a human, non-human primate, rat, mouse, cow, horse, pig, sheep, goat, dog, cat, etc. The subject may be suspected of having or at risk for having a cancer, such as prostate cancer, breast cancer, ovarian cancer or endometrial cancer, or suspected of having or at risk for having acne, hirsutism, alopecia, benign prostatic hyperplasia, ovarian cysts, polycystic ovary disease, precocious puberty, or age-related macular degeneration. Diagnostic methods for various cancers, such as prostate cancer, breast cancer, ovarian cancer or endometrial cancer, and diagnostic methods for acne, hirsutism, alopecia, benign prostatic hyperplasia, ovarian cysts, polycystic ovary disease, precocious puberty, or age-related macular degeneration and the clinical delineation of cancer, such as prostate cancer, breast cancer, ovarian cancer or endometrial cancer, diagnoses and the clinical delineation of acne, hirsutism, alopecia, benign prostatic hyperplasia, ovarian cysts, polycystic ovary disease, precocious puberty, or age-related macular degeneration are known to those of ordinary skill in the art.

Definitions used include ligand-dependent activation of the androgen receptor (AR) by androgens such as dihydrotestosterone (DHT) or the synthetic androgen (R1881) used for research purposes. Ligand-independent activation of the AR refers to transactivation of the AR in the absence of androgen (ligand) by, for example, stimulation of the cAMP-dependent protein kinase (PKA) pathway with forskolin (FSK).

Some compounds and compositions as described herein may interfere with a mechanism specific to ligand-dependent activation (e.g., accessibility of the ligand binding domain (LBD) to androgen) or to ligand-independent activation of the AR.

Various alternative embodiments and examples of the invention are described herein. These embodiments and examples are illustrative and should not be construed as limiting the scope of the invention.

## MATERIALS AND METHODS

### *In silico* screening

**Modelling BRN2 protein structure:** Since the crystal structure of human BRN2 has not been resolved, we built its structure using a homology modeling approach. Structurally, POU domain of BRN2 consists of two subdomains, a C-terminal homeodomain ($POU_H$) and N-terminal POU-specific region (POUs) separated by a short non-conserved linker. Based on the sequence homology and crystal structure of other POU-domain proteins namely Pit1, Oct1 and BRN5, we generated a BRN2 structure using Modeller9 (B. Webb, A. Sali. Comparative Protein Structure Modeling Using Modeller. Current Protocols in Bioinformatics 54, John Wiley & Sons, Inc., 5.6.1-5.6.37, 2016). The flexible loop that connects $POU_H$ and $POU_S$ domains was further refined by Loop Modeler module in MOE v2015. The quality of the developed BRN2 structure was assessed using Ramachandran plot and 100ns molecular dynamic (MD) simulations using AMBER (David Case, Thomas Cheatham, iii, Tom Darden, Holger Gohlke, Ray Luo, Kenneth Merz, JR., Alexey Onufriev, Carlos Simmerling, Bing Wang, Robert J. Woods. The Amber Biomolecular Simulation Programs. J Comput Chem. 2005 Dec; 26(16): 1668-1688.). The stability of BRN2 conformations was then assessed by the root mean squared deviation (RMSD) between the initial conformation and each snapshot during MD simulations.

***In silico* modeling for BRN2 Small molecule inhibitors:** 4 millions of small-molecules from ZINC database v15 ((Teague Sterling, John J. Irwin. ZINC 15 - Ligand Discovery for Everyone. J Chem Inf Model. 2015 55 (11): 2324-2337)) were docked into the active site of modeled hBRN2 structure using Glide (Friesner RA1, Banks JL, Murphy RB, Halgren TA, Klicic JJ, Mainz DT, Repasky MP, Knoll EH, Shelley M, Perry JK, Shaw DE, Francis P, Shenkin PS. Glide: a new approach for rapid, accurate docking and scoring. 1. Method and assessment of docking accuracy. J Med Chem. 2004 Mar 25;47(7):1739-49.) and AutoDock (Stefano Forli, Ruth Huey, Michael E. Pique, Michel Sanner, David S. Goodsell, Arthur J. Olson. Computational protein-ligand docking and virtual drug screening with the AutoDock suite. Nat Protoc. 2016, 11(5): 905-919) programs. Next, RMSD values were calculated between the docking poses generated by Glide and AutoDock to identify the most consistent binding orientation of the compounds. Only molecules with poses having RMSD values below 2.0 Å were selected for further analysis. Furthermore, the selected ligands were subjected to additional on-site scoring using the LigX program and the pKi predicting module of the MOE. Finally, 2,000 compounds that consistently demonstrated high predicted binding affinities were selected for visual inspection to filter out compounds containing reactive or toxic groups such as aldehyde and alkyl halide groups. Based on chemical diversity and availability, 72 chemicals were purchased from commercial vendors as a first step of drugs testing.

### General Synthesis and Characterization of Compounds

### LCMS Conditions:

#### LCMS-Condition 01: Method:- LCMS_X-Select (Formic acid)

Column: X-Select CSH C18 (4.6*50) mm 2.5u, Mobile Phase: A.0.1% Formic acid in water B. 0.1% Formic acid in Acetonitrile Inj Volume; 5.0μL, Flow Rate: 1.0. mL/minute, Gradient program: 2% B to 98 % B in 2.8 minute, Hold till 4.8 min, at 5.0 min B conc. is 2 % up to 7.0 min.

#### LCMS-Condition 02: Method:- LCMS_X-Bridge ($NH_3$)

Column: X-Bridge C18 (3.0*50)mm 2.5 μ; Mobile Phase: A. 0.05 % $NH_3$ in water; B. 0.05 % $NH_3$ in Acetonitrile Inj Volume; .2μL, Flow Rate: 1.0 mL/minute; Gradient program:1% B to 90% B in 1.5 minute, 100%B IN2.5 minute, Hold till 2.8 minute, At 3.0 minute B conc. is 1 % up to 4.0 min.

#### LCMS-Condition 03: Method:- LCMS_X-Select (Ammonium Bicarbonate)

Column: X-Select CSH C18 (3.0*50) mm 2.5u; Mobile Phase: A: 5 mM Ammonium Bicarbonate) in water; B: Acetonitrile; Inj Volume: 2μL, Flow Rate: 1.2 mL/minute; Column oven temp. 50 C; Gradient program:0% B to 98 % B in 2.0 minute, hold till 3.0 min, at 3.2 min B conc. is 0 % up to 4.0 min.

**Synthesis of VPCFTE7 (VPC-18-66)** 4-(6-chlorobenzofuran-2-yl)-2-(furan-2-yl)thiazole

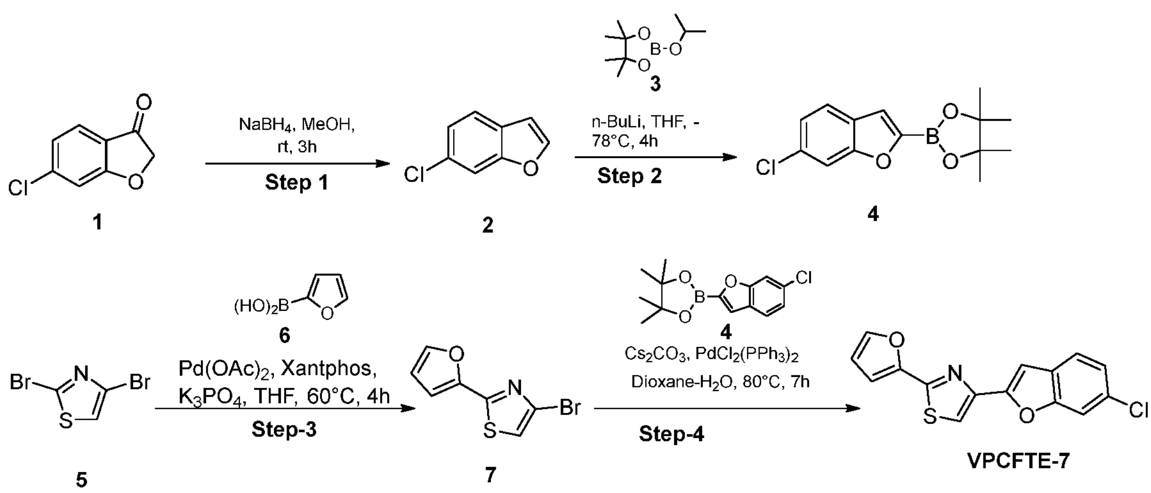


**Step-1: Synthesis of 6-chlorobenzofuran (2):**

To a stirred solution of 6-chlorobenzofuran-3(2H)-one **1** (1.00 g, 5.95 mmol) in MeOH (20 mL) was added $NaBH_4$ (0.57 g, 14.88 mmol) portion wise at room temperature and the reaction mixture was stirred for 3 h. After completion of the reaction (monitored by TLC), the reaction mixture was quenched with acetone (5 mL) followed by 3*N* HCl (10 mL) and the stirred for 1 h. The resulting solution was extracted with ethyl acetate (3 × 25 mL) and washed with water (25 mL). The organic layer was separated, dried over anhydrous $Na_2SO_4$, concentrated under reduced pressure. Crude compound was purified by silica gel column chromatography eluting with 1-2% ethyl acetate in n-hexane to afford 0.4 g (44% yield) of **2** as colourless oil. **$^1$H NMR** (400 MHz, $CDCl_3$) δ: 6.73 - 6.79 (m, 1 H), 7.24 (dd, J=8.31, 1.47 Hz, 1 H), 7.50 - 7.55 (m, 2 H), 7.63 (d, J=1.96 Hz, 1 H).

**Step-2: Synthesis of 2-(6-chlorobenzofuran-2-yl)-4,4,5,5-tetramethyl-1,3,2-dioxaborolane (4):**

To a stirred solution of 6-chlorobenzofuran **2** (0.40 g, 2.63 mmol) in THF (8 mL) was added 1.4M *n*-BuLi in hexane (1.90 mL, 2.63 mmol) at -78°C and stirred for 1.5 h. 2-isopropoxy-4,4,5,5-tetramethyl-1,3,2-dioxaborolane **3** (0.98 mg, 5.26 mmol) was added dropwise at -78 °C and the reaction mixture was stirred at same temperature for 2.5h. After completion of the reaction (monitored by TLC), the reaction mixture was quenched with water (30 mL) extracted with ethyl acetate (3 × 25 mL) and washed with water (25 mL). The organic layer was separated, dried over anhydrous $Na_2SO_4$, concentrated under reduced pressure. Crude compound was purified by silica gel column chromatography eluting with 10-15% ethyl acetate in n-hexane to afford 0.45 g (62% yield) of **4** as colourless oil. **$^1$H NMR** (400 MHz, $CDCl_3$) δ: 7.53 - 7.58 (m, 2 H), 7.37 (s, 1 H), 7.22 - 7.25 (m, 1 H), 1.40 (s, 12 H).

**Step-3: Synthesis of 4-bromo-2-(furan-2-yl)thiazole (7):**

To a stirred solution of 2,4-dibromothiazole **5** (1.0 g, 4.15 mmol) in THF (20 mL) was added furan-2-ylboronic acid **6** (0.42 g, 3.74 mmol) and $K_3PO_4$ (1.76 g, 8.30 mmol) in a sealed tube and degassed with argon for 20 min. To the resulting solution was added $Pd(OAc)_2$ (0.09 g, 0.42 mmol) followed by xantphos (0.24 g, 0.42 mmol) and the reaction mixture was degassed with argon for another 10 min at room temperature. The reaction mixture was heated at 60°C for 4 h. After completion of the reaction (monitored by TLC), the reaction mixture was cooled to room temperature and solvent was removed under reduced pressure. Crude compound was purified by silica gel column chromatography eluting with 0.5-1% ethyl acetate in *n*-hexane to afford 0.60 g (63% yield) of **7** as an off-white solid. **LCMS-Condition-1:** $[M+2+H]^+$ = 231.90; $R_t$ = 1.99 min

**Step-4: Synthesis of 4-(6-chlorobenzofuran-2-yl)-2-(furan-2-yl)thiazole (VPCFTE-7):**

To a stirred solution of 4-bromo-2-(furan-2-yl)thiazole **7** (0.10 g, 0.44 mmol) in 1,4 dioxane:$H_2O$ (10 mL:3mL) in a sealed tube was added 2-(6-chlorobenzofuran-2-yl)-4,4,5,5-tetramethyl-1,3,2-dioxaborolane **4** (0.16 g, 0.57 mmol) followed by $Cs_2CO_3$ (0.28 g, 0.87 mmol) and degassed with argon for 20 min. To the resulting reaction mixture was added $PdCl_2(PPh_3)_2$ (0.015 g, 0.02 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude solid obtained was dissolved in

ethyl acetate (50 mL) and washed with water (2 × 25 mL). Organic layer was separated, dried over anhydrous $Na_2SO_4$ and concentrated under reduced pressure. The crude compound was purified by silica gel column chromatography eluting with 0.5% ethyl acetate in *n*-hexane to afford 0.06 g (46% yield) of **VPCFTE-7** as an off-white solid. HPLC: 98.08%. **LCMS-Condition-1:** $[M+H]^+$ = 301.9; $R_t$ = 2.33 min. **$^1$H NMR** (400 MHz, $CDCl_3$) δ: 7.66 (s, 1H), 7.57-7.53 (m, 3H), 7.27-7.24 (m, 2H), 7.126 (d, *J* = 3.2 Hz, 1H), 6.60-6.59 (m, 1H).

**Synthesis of VPCFTE53 (VPC-18-94)** 4-(6-chlorobenzofuran-2-yl)-2-(1*H*-pyrrol-2-yl)thiazole

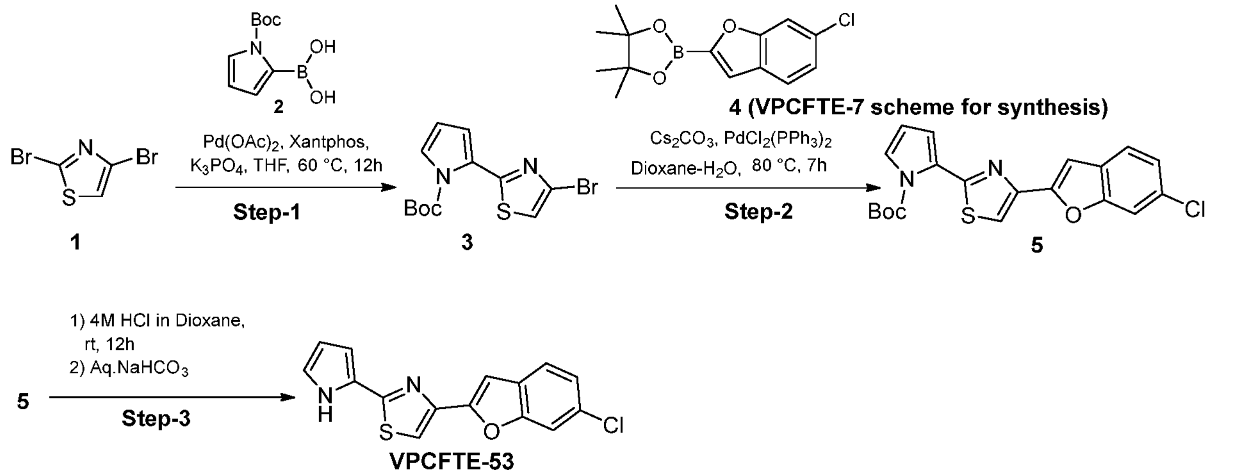


**Note: Synthesis of 4 reported in VPCFTE7 scheme**

**Step-1: Synthesis of *tert*-butyl 2-(4-bromothiazol-2-yl)-1*H*-pyrrole-1-carboxylate (3):**

To a stirred solution of 2,4-dibromothiazole **1** (0.50 g, 2.08 mmol) in THF (20 mL) was added (1-(*tert*-butoxycarbonyl)-1*H*-pyrrol-2-yl)boronic acid **2** (0.52 g, 2.46 mmol) and $K_3PO_4$ (0.87 g, 4.11 mmol) in a sealed tube and degassed with argon for 20 min. To the resulting solution was added $Pd(OAc)_2$ (0.05 g, 0.20 mmol) followed by Xantphos (0.12 g, 0.21 mmol) and the reaction mixture was degassed with argon for another 10 min at room temperature. The reaction mixture was heated at 60 °C for 12 h. After completion of the reaction (monitored by TLC), the reaction mixture was cooled to room temperature and solvent was removed under reduced pressure. Crude compound was purified by silica gel column chromatography eluting with 0.5-2% ethyl acetate in n-hexane to afford 0.45 g (66% yield) of **3** as a pale yellow solid.

**LCMS-Condition-1:** $[M-56+2+H]^+$ = 274.90; $R_t$ = 2.41 min

**Step-2: Synthesis of *tert*-butyl 2-(4-(6-chlorobenzofuran-2-yl)thiazol-2-yl)-1*H*-pyrrole-1-carboxylate (5):**

**Note: Synthesis of 4 reported in VPCFTE7 scheme**

To a stirred solution of *tert*-butyl 2-(4-bromothiazol-2-yl)-1*H*-pyrrole-1-carboxylate **3** (0.40 g, 1.22 mmol) in 1,4 dioxane:$H_2O$ (15 mL:4 mL) in a sealed tube was added 2-(6-chlorobenzofuran-2-yl)-4,4,5,5-tetramethyl-1,3,2-dioxaborolane **4** (0.50 g, 1.80 mmol) followed by $Cs_2CO_3$ (0.79 g, 2.44 mmol) and degassed with argon for 20 min. To the resulting reaction mixture was added $PdCl_2(PPh_3)_2$ (0.085 g, 0.12 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude compound was purified by silica gel column chromatography eluting with 3-4% ethyl acetate in *n*-hexane to afford 0.20 g (41% yield) of **5** as a pale grey solid. **LCMS-Condition-1:** [M+H]+ = 401.05; $R_t$ = 2.52 min

**Step-3: Synthesis of 4-(6-chlorobenzofuran-2-yl)-2-(1*H*-pyrrol-2-yl) thiazole (VPCFTE-53):**

To a stirred solution of tert-butyl 2-(4-(6-chlorobenzofuran-2-yl)thiazol-2-yl)-1*H*-pyrrole-1-carboxylate **5** (0.10

g, 0.25 mmol) in 1,4 dioxane (10 mL) was added 4M HCl in dioxane (5 mL) and the reaction mixture was stirred at room temperature for 12 h. After completion of the reaction (monitored by TLC), solvent was removed under reduced pressure. The crude was washed with *n*-pentane (20 mL) to yield solid compound which was neutralized with aqueous $NaHCO_3$ solution (15 mL). The precipitated solid was filtered, washed with *n*-pentane (2 × 10 mL) and dried to afford 0.05 g (67% yield) of **VPCFTE-53** as a grey solid. **HPLC:** 96.37%.

**LCMS-Condition-1:** $[M+H]^+$ = 301.00; $R_t$ = 2.51 min. **$^1$H NMR** (400 MHz, $CDCl_3$) δ: 9.37 (brs, 1H), 7.53 (d, *J* = 9.2 Hz, 3H), 7.28-7.24 (m, 1H), 7.16 (s, 1H), 6.97 (s, 1H), 6.77 (s, 1H), 6.33-6.22 (m, 1H).

**Synthesis of VPCFTE46 (VPC-18-76)** 4-(benzofuran-2-yl)-2-(furan-3-yl)thiazole

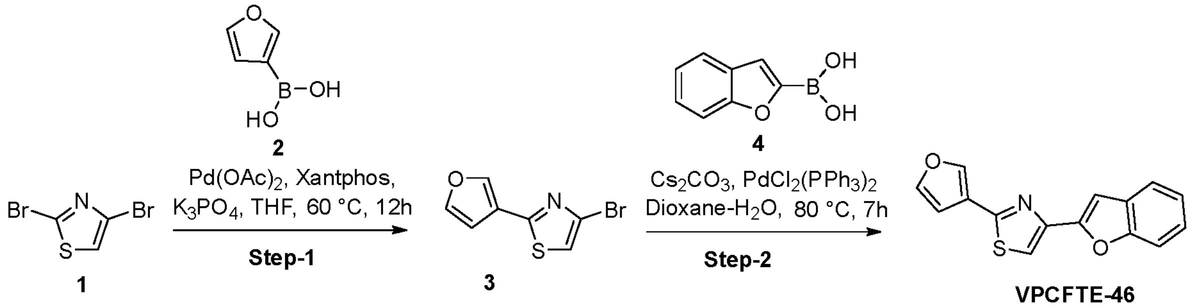


**Step-1: Synthesis of 4-bromo-2-(furan-3-yl)thiazole (3):**

To a stirred solution of 2,4-dibromothiazole **1** (0.5 g, 2.08 mmol) in THF (20 mL) was added furan-3-ylboronic acid **2** (0.23 g, 2.08 mmol) and $K_3PO_4$ (0.88 g, 4.15 mmol) in a sealed tube and degassed with argon for 20 min. To the resulting solution was added $Pd(OAc)_2$ (0.05 mg, 0.21 mmol) followed by xantphos (0.12 g, 0.21 mmol) and the reaction mixture was degassed with argon for another 10 min at room temperature. The reaction mixture was heated at 60 °C for 12 h. After completion of the reaction (monitored by TLC), the reaction mixture was cooled to room temperature and solvent was removed under reduced pressure. Crude compound was purified by silica gel column chromatography eluting with 0.5-1% ethyl acetate in n-hexane to afford 0.33 g (69% yield) of **3** as an off-white solid. **LCMS-Condition-1:** $[M+H]^+$ = 229.8; $R_t$ = 1.74 min

**Step-2: Synthesis of 4-(benzofuran-2-yl)-2-(furan-3-yl)thiazole (VPCFTE-46):**

To a stirred solution of 4-bromo-2-(furan-3-yl)thiazole **3** (0.15 g, 0.66 mmol) in 1,4 dioxane:$H_2O$ (15 mL:4 mL) in a sealed tube was added benzofuran-2-ylboronic acid **4** (0.16 g, 0.98 mmol) followed by $Cs_2CO_3$ (0.43 g, 1.31 mmol) and degassed with argon for 20 min. To the resulting reaction mixture was added $PdCl_2(PPh_3)_2$ (0.046 g, 0.07 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude compound was purified by silica gel column chromatography eluting with 0.1% ethyl acetate in n-hexane to afford 40 mg (23% yield) of **VPCFTE-46** as a white solid. HPLC: 96.39%. **LCMS-Condition-1:** $[M+H]^+$ = 268; $R_t$ = 2.39 min. $^1$H NMR (400 MHz, $CDCl_3$) δ: 8.09 (s, 1H), 7.65-7.63 (m, 2H), 7.55-7.51 (m, 2H), 7.34-7.30 (m, 1H), 7.28-7.25 (m, 2H), 6.92 (s, 1H).

**Synthesis of VPCFTE44 (VPC-18-90) 5-(benzofuran-2-yl)-2-(furan-2-yl)thiazole**

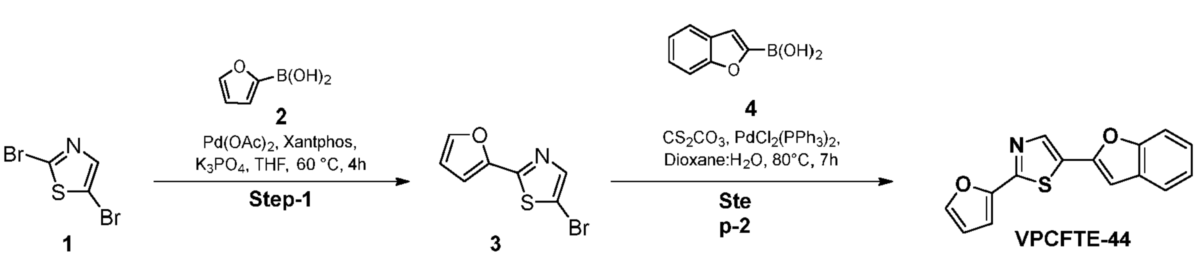

**Step-1: Synthesis of 5-bromo-2-(furan-2-yl)thiazole (3):**

To a degassed solution of 2,5-dibromothiazole**1** (1.00 g, 4.12 mmol) and furan-2-ylboronic acid **2** (0.50 g, 4.47 mmol) in THF (20 mL) was added and $K_3PO_4$ (1.70 g, 8.01 mmol) in a sealed tube and stirred for 5min. $Pd(OAc)_2$ (0.09 mg, 0.41 mmol) was added at room temperature followed by addition of xantphos (0.24 g, 0.41 mmol) and the reaction mixture was heated at 60 °C for 4 h. After completion of the reaction (monitored by TLC), the reaction mixture was cooled to room temperature and solvent was removed under reduced pressure. Crude compound was purified by silica gel column chromatography eluting with 5% ethyl acetate in n-hexane to afford 0.30 g (32% yield) of **3** as an off-white solid. **LCMS-Condition-1:** [M+H]+ = 229.90; $R_t$ = 2.24 min.

**Step-2: Synthesis of 5-(benzofuran-2-yl)-2-(furan-2-yl)thiazole (VPCFTE-44):**

To a degassed solution of 5-bromo-2-(furan-2-yl)thiazole **3** (0.15 g, 0.65 mmol) and benzofuran-2-ylboronic acid **4** (0.16 g, 0.99 mmol) in 1,4 dioxane:$H_2O$ (12 mL:3 mL) was added $Cs_2CO_3$ (0.43 g, 1.31 mmol) followed by addition of $PdCl_2(PPh_3)_2$ (0.05 g, 0.07 mmol) in a sealed tube at room temperature. The reaction mixture was stirred at 80 °C for 7 h. After completion of the reaction (monitored by TLC), the reaction mixture was cooled to room temperature and solvent was removed under reduced pressure. Crude compound was purified by silica gel column chromatography eluting with 25-30% ethyl acetate in *n*-hexane followed by prep HPLC to afford 40 mg (23% yield) of **VPCFTE-44** as an off-white solid.

**HPLC:** 97.66%. **LCMS-Condition-1:** $[M+H]^+$ = 267.90; $R_t$ = 2.19 min. **$^1$H NMR** (400 MHz, $CDCl_3$) δ: 8.16 (s, 1 H), 7.54 - 7.59 (m, 2 H), 7.51 (d, *J*=8.31 Hz, 1H), 7.29 - 7.34 (m, 1 H), 7.23 - 7.28 (m, 1H), 7.07 (d, *J*=3.42 Hz, 1 H), 6.94 (s, 1 H), 6.57-6.59 (m, 1 H).

**Synthesis of VPCFTE45 (VPC-18-81)** 4-(benzofuran-3-yl)-2-(furan-2-yl)thiazole

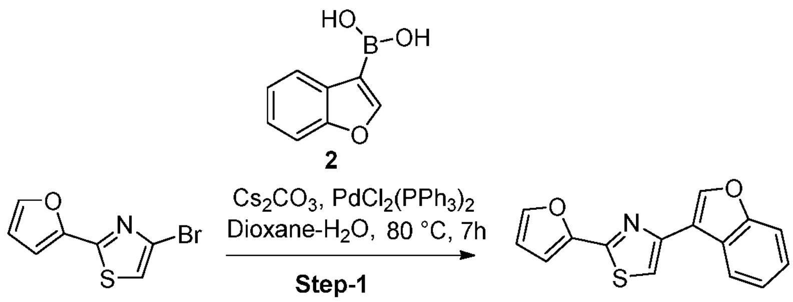


**1 (Int-7 synthesis VPCFTE-7)** **VPCFTE-45**

**Note: Synthesis of 1 reported (as Int-7 synthesis in VPCFTE7 scheme)**

**Step-1: Synthesis of 4-(benzofuran-3-yl)-2-(furan-2-yl)thiazole (VPCFTE-45): Note: Synthesis of 1 reported (as Int-7 synthesis in VPCFTE7 scheme)**

To a stirred solution of 4-bromo-2-(furan-2-yl)thiazole **1** (0.1 g, 0.44 mmol) in 1,4 dioxane:$H_2O$ (10 mL:3 mL) in a sealed tube was added benzofuran-3-ylboronic acid **2** (0.11 g, 0.66 mmol) followed by $Cs_2CO_3$ (0.28 g, 0.87 mmol) and degassed with argon for 20 min. To the resulting reaction mixture was added $PdCl_2(PPh_3)_2$ (0.03 g, 0.04 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude compound was purified by silica gel column chromatography eluting with 1-1.5% ethyl acetate in n-hexane to afford 40 mg (34% yield) of **VPCFTE-45** as a white solid. **HPLC:** 96.78%. **LCMS-Condition-1:** $[M+H]^+$ = 268; $R_t$ = 2.30 min. $^1$H NMR (400 MHz, $CDCl_3$) δ: 8.25 (s, 1H), 8.04-8.01 (m, 1H), 7.59-7.57 (m, 2H), 7.49 (s, 1H), 7.41-7.36 (m, 2H), 7.124 (d, J = 3.2 Hz, 1H), 6.60-6.59 (m, 1H).

**Synthesis of Compound-18(VPC-18-51)** 2-(Furan-2-yl)-4-(6-(trifluoromethyl)benzofuran-2-yl)thiazole

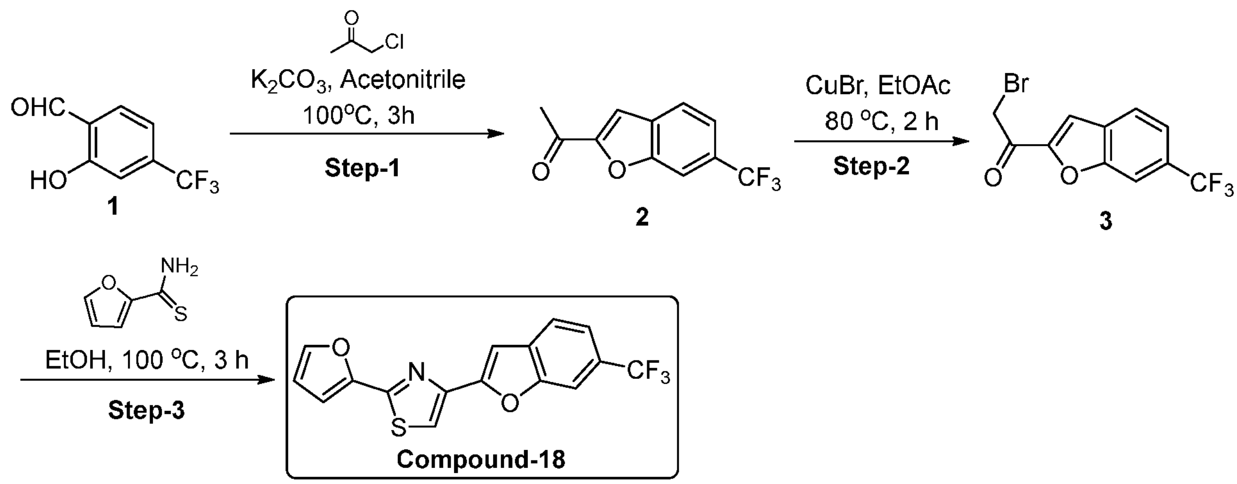


**Step-1: Synthesis of 1-(6-(trifluoromethyl)benzofuran-2-yl)ethan-1-one (2):**

To a solution of 2-hydroxy-4-(trifluoromethyl)benzaldehyde **1** (500 mg, 2.873 mmol) in acetonitrile (10 mL) was added potassium carbonate (396 mg, 2.873 mmol) and 1-chloropropan-2-one (0.22 mL, 2.873 mmol) at room temperature. The reaction mixture was then heated at 100 °C for 3 h. After completion of the reaction, the reaction mixture was cooled to room temperature, diluted with water (25 mL) and extracted with ethyl acetate (3 times). The combined organic layer was washed with water followed by brine; dried over anhydrous $Na_2SO_4$, filtered and concentrated under reduced pressure. The crude compound was purified by silica gel column chromatography eluting using 0-10% ethyl acetate in n-hexane as eluent to afford 200 mg (30% yield) of **Int 2** as pale yellow solid. **LCMS-Condition-1:** [M+H]+ = 228.95; $R_t$ = 1.87 min. **$^1$H NMR** (400 MHz, $CDCl_3$) δ: 7.87 (s, 1H), 7.84 (d, *J* = 8.28 Hz, 1H), 7.58 (d, *J* = 8.28 Hz, 1H), 7.54 (s, 1H), 2.65 (s, 3H).

**Step-2: Synthesis of 2-bromo-1-(6-(trifluoromethyl)benzofuran-2-yl)ethan-1-one (3):**

To a solution of 1-(6-(trifluoromethyl)benzofuran-2-yl)ethan-1-one **2** (200 mg, 0.877 mmol) in ethyl acetate (5 mL) was added copper bromide (391 mg, 1.754 mmol) and heated at 80 °C for 2 h. After completion of the reaction, the reaction mixture was filtered through a pad of Celite and washed with ethyl acetate (10 mL). The filtrate was concentrated under reduced pressure to yield the crude compound which was purified by silica gel column chromatography eluting using 0-10% ethyl acetate in n-hexane as eluent to afford 200 mg (74% yield) of **Int 3** as pale yellow solid.

**$^1$H NMR** (400 MHz, $CDCl_3$) δ: 7.90 (s, 1H), 7.87 (d, *J* = 8.28 Hz, 1H), 7.70 (d, *J* = 0.75 Hz, 1H), 7.61 (d, *J* = 8.28 Hz, 1H), 4.46 (s, 2H).

**Step-2: Synthesis of 2-(furan-2-yl)-4-(6-(trifluoromethyl)benzofuran-2-yl)thiazole (Compound-18):**

To a solution of 2-bromo-1-(6-(trifluoromethyl)benzofuran-2-yl)ethan-1-one **3** (200 mg, 0.651 mmol) in ethanol (5 mL) was added furan-2-carbothioamide (83 mg, 0.651 mmol) at room temperature. The reaction mixture was heated at 100 °C for 3 h. After completion of the reaction, the reaction mixture was cooled to room temperature and diluted with water (25 mL). The precipitated solid was filtered and washed with pentane (10 mL) to afford 30 mg (14% yield) of **Compound 18** as off white solid.

**LCMS-Condition-1:** $[M+H]^+$ = 335.95; $R_t$ = 2.35 min.

**$^1$H NMR** (400 MHz, DMSO-$d_6$) δ: 8.25 (s, 1H), 8.11 (s, 1H), 7.97 (d, *J* = 1.47 Hz, 1H), 7.93 (d, *J* = 8.31 Hz, 1H), 7.64 (d, *J* = 8.31 Hz, 1H), 7.49 (s, 1H), 7.25 (d, *J* = 3.42 Hz, 1H), 6.77 (dd, *J* = 1.47, 3.42 Hz, 1H).

**Synthesis of VPCFTE27 (VPC-18-63)** 3-(5-(1-methyl-1*H*-benzo[*d*]imidazol-2-yl)furan-2-yl)benzonitrile

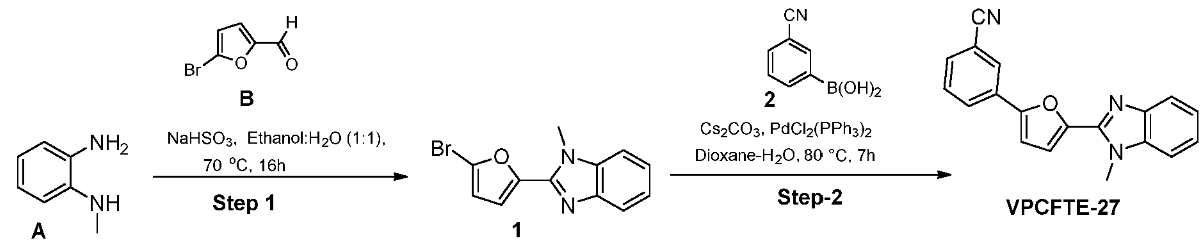


**Step-1: Synthesis of 2-(5-bromofuran-2-yl)-1-methyl-1*H*-benzo[d]imidazole (1):**

To a stirred solution of *N*1-methylbenzene-1,2-diamine **A** (1.0 g, 8.20 mmol) and 5-bromofuran-2-carbaldehyde **B** (2.14 g, 12.29 mmol) in EtOH:$H_2O$ (5:1,12 mL) in a sealed tube was added $NaHSO_3$ (1.7 g, 16.39 mmol) and the reaction mixture was heated at 70°C for 16 h. After completion of the reaction (monitored by TLC), the reaction mixture was cooled to room temperature and solvent was removed under reduced pressure. The solid residue was dissolved in 10% MeOH-DCM (50 mL) and filtered through sintered funnel to remove inorganic impurities. Filtrate obtained was concentrated under reduced pressure. Crude compound was purified by silica gel column chromatography eluting with 12-15% ethyl acetate in n-hexane to afford 1.0 g (44% yield) of **1** as an off-white solid.

**LCMS-Condition-1:** $[M+H]^+$ = 276.9; $R_t$ = 1.44 min.

**Step-1: Synthesis of 3-(5-(1-methyl-1*H*-benzo[*d*]imidazol-2-yl)furan-2-yl)benzonitrile (VPCFTE-27):**

To a stirred solution of 2-(5-bromofuran-2-yl)-1-methyl-1*H*-benzo[*d*]imidazole **1** (0.1 g, 0.36 mmol) in 1,4 dioxane:$H_2O$ (10 mL:3 mL) in a sealed tube was added (3-cyanophenyl)boronic acid **2** (0.08 g, 0.54 mmol) followed by $Cs_2CO_3$ (0.236 g, 0.73 mmol) and degassed with argon for 20 min. To the resulting reaction mixture was added $PdCl_2(PPh_3)_2$ (0.025 g, 0.04 mmol) and degassed with argon for another 10 min at room temperature. The reaction mixture was then heated 80°C for 7 h. After completion of the reaction (monitored by TLC), the reaction mixture was cooled to room temperature and solvent was removed under reduced pressure. Crude solid obtained was dissolved in 10% MeOH: DCM (50 mL) and filtered through celite bed to remove inorganic solid. Filtrate obtained was concentrated under reduced pressure. The crude compound was purified by prep HPLC to afford 23.5 mg (21% yield) of **VPCFTE-27** as an off-white solid. **LCMS-Condition-1:** [M+H]+ = 300.1; $R_t$ = 1.55 min. $^1$H NMR (400 MHz, CDCl3) δ: 8.05 (brs, 1H), 7.99 (d, *J* = 7.6 Hz, 1H), 7.83 (d, *J* = 8.4 Hz, 1H), 7.62-7.58 (m, 2H), 7.46-7.44 (m, 1H), 7.38-7.33 (m, 3H), 6.98 (d, *J* = 4.0 Hz, 1H), 4.19 (s, 3H).

**Synthesis of VPCFTE42 (VPC-18-70)** 1-methyl-2-(5-(1,2,3,6-tetrahydropyridin-4-yl)furan-2-yl)-6-(trifluoromethyl)-1*H*-benzo[*d*]imidazole hydrochloride

**Step-1: Synthesis of *N*-methyl-2-nitro-5-(trifluoromethyl)aniline (2):**

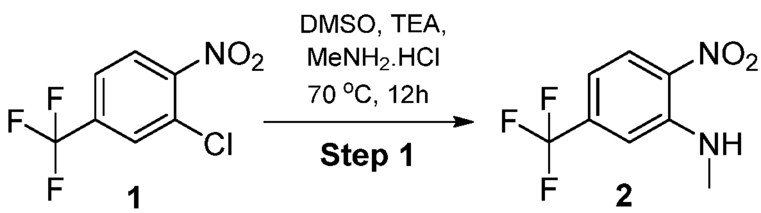


To a degassed mixture of 2-chloro-1-nitro-4-(trifluoromethyl)benzene **1** (2.0 g, 8.89 mmol) and methylamine .HCl (1.48 g, 22.09 mmol) in DMSO (20 mL) was added TEA (3.5 g, 34.65 mmol) in a sealed tube under argon atmosphere and the reaction mixture was heated at 70 °C for 12 h. After completion of the reaction (monitored by TLC), the reaction mixture was cooled to room temperature. Reaction mixture was diluted with water (100 mL). The precipitated solid was filtered and dried to afford 1.7 g (94% yield) of **2** as yellow solid. **LCMS-Condition-1:** $[M+H]^+$ = 221.00; $R_t$ = 2.13 min.

**Step-2: Synthesis of *N*1-methyl-5-(trifluoromethyl)benzene-1,2-diamine (3):**

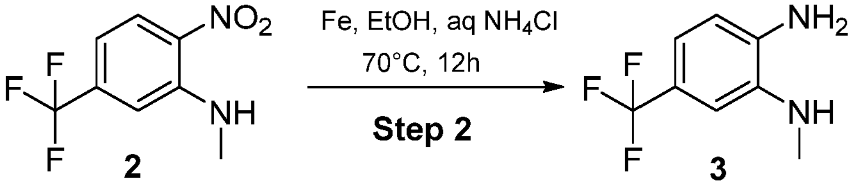


2 3

To a stirred solution of *N*-methyl-2-nitro-5-(trifluoromethyl)aniline **2** (1.7 g, 7.73 mmol) in EtOH (17 mL) and water (5.5 mL) was added Fe powder (2.15 g, 38.53 mmol) and $NH_4Cl$ (0.49 g, 9.16 mmol) in a sealed tube and the reaction mixture was heated at 70°C for 12 h. After completion of the reaction (monitored by TLC), the reaction mixture was cooled to room temperature. Reaction mixture was diluted with water (100 mL). The precipitated solid was filtered, washed with water and dried to afford 1.4 g (100% yield) of **3** as light yellow solid. **LCMS-Condition-1:** $[M+H]^+$ = 190.95; $R_t$ = 1.81 min.

**Step-3: Synthesis of 2-(5-bromofuran-2-yl)-1-methyl-5-(trifluoromethyl)-1*H*-benzo[d]imidazole (5):**

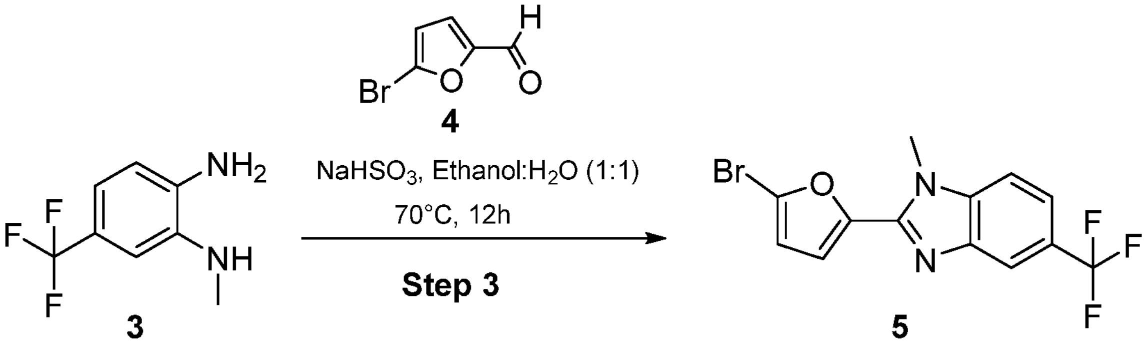


4 3 5

To a stirred solution of $N^1$-methyl-5-(trifluoromethyl)benzene-1,2-diamine **3** (1.4 g, 7.37 mmol) and 5-bromofuran-2-carbaldehyde **4** (1.9 g, 11.05 mmol) in EtOH:$H_2O$ (1:1, 28 mL) in a sealed tube was added $NaHSO_3$ (1.53 g, 14.71 mmol) and the reaction mixture was heated at 70°C for 12 h. After completion of the reaction (monitored by TLC), the reaction mixture was cooled to room temperature and diluted with water (50 mL). The aqueous layer was extracted with ethyl acetate (3 × 50 mL). Combined organic layer was dried over anhydrous $Na_2SO_4$ and concentrated under reduced pressure. Crude compound was purified by silica gel column chromatography eluting with 10-15% ethyl acetate in n-hexane to afford 1.5 g (60% yield) of **5** as an off-white solid. **LCMS-Condition-1:** $[M+H]^+$ = 344.95; $R_t$ = 2.14 min.

**Step-4: Synthesis of *tert*-butyl 4-(5-(1-methyl-6-(trifluoromethyl)-1H-benzo[*d*]imidazol-2-yl)furan-2-yl)-3,6-dihydropyridine-1(2*H*)-carboxylate (7):**

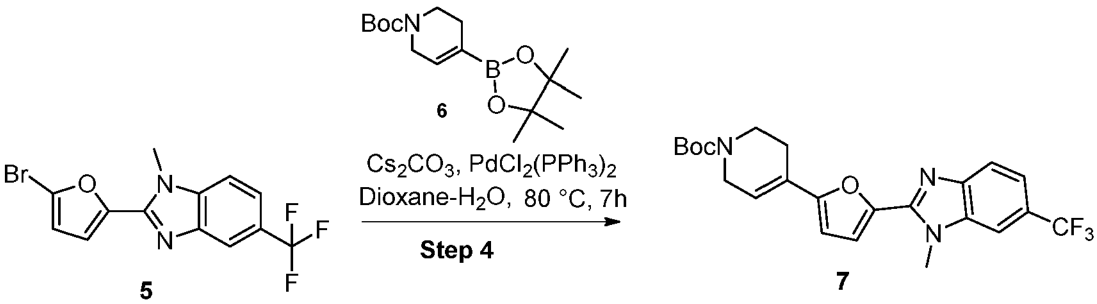


6 5 7

To a stirred solution of 2-(5-bromofuran-2-yl)-1-methyl-5-(trifluoromethyl)-1*H*-benzo[d]imidazole **5** (0.25 g, 0.73 mmol) in 1,4 dioxane:$H_2O$ (4:1, 10 mL) in a sealed tube was added tert-butyl 4-(4,4,5,5-tetramethyl-1,3,2-dioxaborolan-2-yl)-3,6-dihydropyridine-1(2*H*)-carboxylate **6** (0.33 g, 1.07 mmol) followed by $Cs_2CO_3$ (0.47 g, 1.45 mmol) and degassed with argon for 20 min. To the resulting reaction mixture was added $PdCl_2(PPh_3)_2$ (0.05 g, 0.07 mmol) and degassed

with argon for another 10 min at room temperature. The reaction mixture was then heated at 80°C for 7 h. After completion of the reaction (monitored by TLC), the reaction mixture was cooled to room temperature and solvent was removed under reduced pressure. The solid residue was dissolved in 10% MeOH-DCM (50 mL) and filtered through sintered funnel to remove inorganic impurities. Filtrate obtained was concentrated under reduced pressure. Crude compound was purified by silica gel column chromatography eluting with 10-15% ethyl acetate in n-hexane to afford 0.30 g (92% yield) of 7 as an off-white solid. **LCMS-Condition-1:** $[M+H]^+$ = 448.15; $R_t$ = 2.31 min.

**Step-1: Synthesis of 1-methyl-2-(5-(1,2,3,6-tetrahydropyridin-4-yl)furan-2-yl)-6-(trifluoromethyl)-1*H*-benzo[*d*]imidazole hydrochloride (VPCFTE-42):**

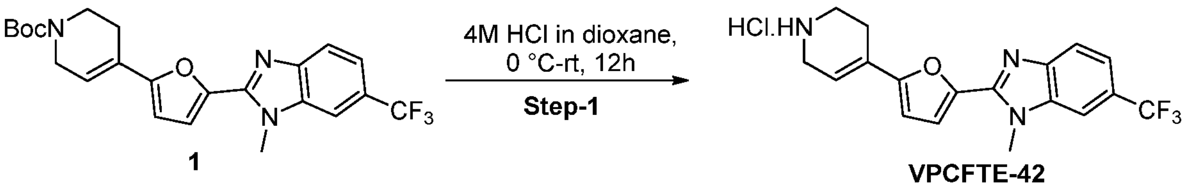


1

VPCFTE-42

To a stirred solution of tert-butyl 4-(5-(1-methyl-6-(trifluoromethyl)-1*H*-benzo[d]imidazol-2-yl)furan-2-yl)-3,6-dihydropyridine-1(2*H*)-carboxylate **1** (0.1 g, 0.22 mmol) in 1,4 dioxane (5 mL) was added 4M HCl in dioxane (10 mL) at 0 °C and the reaction mixture was stirred at room temperature for 12 h. After completion of the reaction (monitored by TLC), the reaction mixture was concentrated under reduced pressure. The crude compound was purified by washing with diethyl ether (20 mL) and *n*-pentane (2 × 10 mL) to afford 25 mg (29% yield) of **VPCFTE-42** as light yellow solid. **LCMS-Condition-1:** $[M+H]^+$ = 347.7; $R_t$ = 1.24 min. (Parent mass). $^1H$ NMR (400 MHz, DMSO-d6) δ: 9.37 (brs, 2H), 8.24 (s, 1H), 7.86 (d, J = 8.8 Hz, 1H), 7.62 (d, J = 8.8 Hz, 1H), 7.545 (d, J = 3.6 Hz, 1H), 6.928 (d, J = 3.2 Hz, 1H), 6.50 (s, 1H), 4.17 (s, 3H), 3.82 (brs, 2H), 3.32 (brs, 2H), 2.70 (brs, 2H).

**General Synthetic Scheme: Synthesis of common intermediate Int-7**

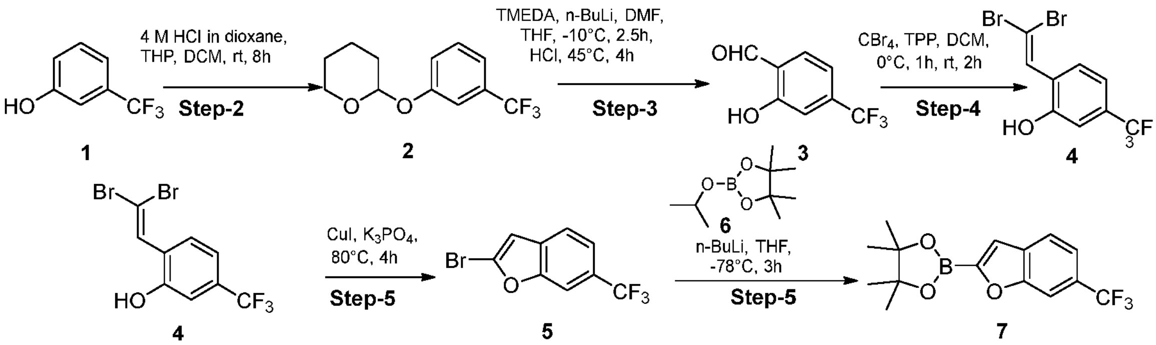


**Step-1: Synthesis of 2-(3-(trifluoromethyl)phenoxy)tetrahydro-2H-pyran (2):**

To a stirred solution of 3-(trifluoromethyl)phenol **1** (5.00 g, 30.9 mmol) and THP (6.48 g, 77.0 mmol) in DCM (15 mL) was added 4 M HCl in dioxane (0.5 mL) at room temperature. The reaction mixture was stirred at room temperature for 8h. After completion of the reaction (monitored by TLC), the reaction mixture was concentrated under reduced pressure. The crude compound was purified by column chromatography (silica, 100-200 mesh, 2 to 3% EtOAc in hexane) to afford 4.50 g (60% yield) of **2** as a colourless oil. **$^1$H NMR** (400 MHz, $CDCl_3$) δ: 7.37 - 7.43 (m, 1 H), 7.31 - 7.35 (m, 1 H), 7.22 - 7.29 (m, 2 H), 5.48 (t, *J*=2.93 Hz, 1 H), 3.84 - 3.97 (m, 1H), 3.59 - 3.70 (m, 1H), 1.98 - 2.09 (m, 1H), 1.87 - 1.94 (m, 2 H), 1.67 - 1.79 (m, 3H).

**Step-2: Synthesis of 2-hydroxy-4-(trifluoromethyl)benzaldehyde (3):**

To a stirred solution of TMEDA (3.18 g, 27.4 mmol) in THF (25 mL) was added n-BuLi (1.4 M in hexane, 1.75 mL, 27.3 mmol) drop wise over a period of 10 min at -10°C. 2-(3-(trifluoromethyl)phenoxy)tetrahydro-2H-pyran **2** (4.50

g, 18.3 mmol) was added drop wise at -10°C and the reaction mixture was stirred at same temperature for 2h. DMF (2.00 g, 27.4 mmol) was added drop wise and the reaction mixture was stirred for 15 min. After completion of the reaction (monitored by TLC), the reaction mixture was added to a Conc.HCl (10 mL) and heated at 45°C for 4h. The reaction mixture was extracted with EtOAc (3 × 30 mL). The organic layer was separated, dried over anhydrous $Na_2SO_4$ and solvent was removed under reduced pressure. The crude compound was purified by column chromatography (silica, 100-200 mesh, 100/1 - 100/5 in hexane/EtOAc) to afford 1.60 g (46% yield) of **3** as a light yellow liquid. **$^1$H NMR** (400 MHz, $CDCl_3$) δ: 11.08 (s, 1 H) 9.99 (s, 1 H) 7.70 - 7.76 (m, 1 H) 7.25 - 7.30 (m, 2 H).

**Step-3: Synthesis of 2-(2,2-dibromovinyl)-5-(trifluoromethyl)phenol (4):**

A mixture of 2-hydroxy-4-(trifluoromethyl)benzaldehyde **3** (1.50 g, 7.89 mmol) and $CBr_4$ (7.85 g, 23.7 mmol) in DCM (20 mL) was stirred for 20 min. The reaction mixture was cooled at 0°C followed by drop wise addition of TPP (6.21 g, 23.7 mmol) solution in DCM (10 mL) over a period of 10 min. The reaction mixture was stirred at 0°C for th and at room temperature for 2h. After completion of the reaction (monitored by TLC), the reaction mixture was quenched with aqueous saturated $NH_4Cl$ solution. The separated aqueous layer was extracted with DCM (3 × 25 mL). The combined organic layer was concentrated under reduced pressure. The crude compound was purified by column chromatography (silica, 100-200 mesh, 100/1 - 100/5 in hexane/EtOAc) to afford 1.40 g (52% yield) of **4. $^1$H NMR** (400 MHz, $CDCl_3$) δ: 7.66 (d, *J*=8.31 Hz, 1H), 7.57 (s, 1 H), 7.22 (d, *J*=7.83 Hz, 1H), 7.11 (s, 1 H), 5.42 (s, 1 H).

**Step-4: Synthesis of 2-bromo-6-(trifluoromethyl)benzofuran (5):**

To a stirred solution of 2-(2,2-dibromovinyl)-5-(trifluoromethyl)phenol **4** (1.30 g, 3.78 mmol) in THF (15 mL) was added $K_3PO_4$ (1.60 g, 7.55 mmol) and CuI (0.07 g, 0.37 mmol) in a sealed tube and the reaction mixture was purged with nitrogen for 5 min. The reaction mixture was heated at 80°C for 4h. After completion of the reaction (monitored by TLC), the reaction mixture was concentrated under reduced pressure. The crude compound was purified by column chromatography (silica, 100-200 mesh, 3 to 4% EtOAc in hexane) to afford 0.55 g (56% yield) of **5** as a brown sticky liquid.

**$^1$H NMR** (400 MHz, $CDCl_3$) δ: 7.74 (s, 1H), 7.63 (d, *J*=8.31 Hz, 1 H), 7.52 (d, *J*=7.83 Hz, 1H), 6.82 (s, 1 H).

**Step-5: Synthesis of 4,4,5,5-tetramethyl-2-(6-(trifluoromethyl)benzofuran-2-yl)-1,3,2-dioxaborolane (7):**

To a stirred solution of 2-bromo-6-(trifluoromethyl)benzofuran **5** (0.40 g, 1.52 mmol) in THF (15 mL) was added n-BuLi (1.4 M in hexane, 1.62 mL) at -78°C and the reaction mixture was stirred at same temperature for 1.5h. 2-isopropoxy-4,4,5,5-tetramethyl-1,3,2-dioxaborolane 6 (0.56 g, 3.02 mmol) was added drop wise at -78°C and the reaction mixture was stirred at same temperature for 1.5h. After completion of the reaction (monitored by TLC), the reaction mixture was quenched with aqueous $NH_4Cl$ (10 mL) solution and extracted with EtOAc (3 × 25 mL). The organic layer was separated, washed with brine, dried over anhydrous $Na_2SO_4$ and concentrated under reduced pressure to afford 0.45 g (crude) of 7 as a brown sticky liquid. The intermediate used without purification for further transformations.

**Synthesis of VPCFTE37 (VPC-18-78)** 4-(4-(6-(trifluoromethyl)benzofuran-2-yl)thiazol-2-yl)morpholine

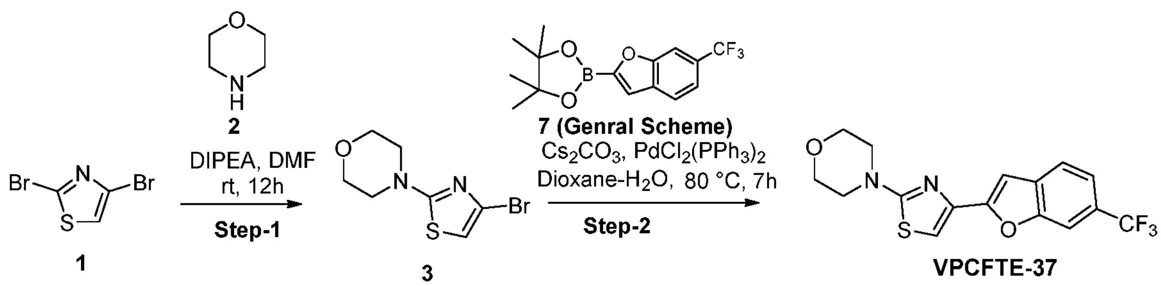


**Note: Int-7 synthesis is mentioned in general Scheme above.**

**Step-1: Synthesis of 4-(4-bromothiazol-2-yl)morpholine (3):**

To a degassed mixture of 2,4-dibromothiazole **1** (0.5 g, 2.08 mmol) and morpholine **2** (0.54 g, 6.17 mmol) in DMF (15 mL) was added DIPEA (0.53 g, 4.16 mmol) and the reaction mixture was stirred at room temperature for 12

h. After completion of the reaction (monitored by TLC), solvent was removed under reduced pressure. Crude solid obtained was dissolved in ethyl acetate (50 mL) and washed with water (2 × 25 mL). Organic layer was separated, dried over anhydrous $Na_2SO_4$ and concentrated under reduced pressure. Crude compound was purified by silica gel column chromatography eluting with 5-7% ethyl acetate in n-hexane to afford 0.40 g (78% yield) of **3** as a brown solid. **LCMS-Condition-1:** $[M+H]^+$ = 248.9; $R_t$ = 1.80 min.

**Step-2: Synthesis of 4-(4-(6-(trifluoromethyl)benzofuran-2-yl)thiazol-2-yl)morpholine (VPCFTE-37):**

**Note: Int-7 synthesis is mentioned in general Scheme above.**

To a stirred solution of 4-(4-bromothiazol-2-yl)morpholine **3** (0.22 g, 0.88 mmol) in 1,4 dioxane:$H_2O$ (5:1, 12 mL) in a sealed tube was added 4,4,5,5-tetramethyl-2-(6-(trifluoromethyl)benzofuran-2-yl)-1,3,2-dioxaborolane **7** (0.25 g, 0.80 mmol) followed by $Cs_2CO_3$ (0.52 g, 1.60 mmol) and degassed with argon for 20 min. To the resulting reaction mixture was added $PdCl_2(PPh_3)_2$ (0.06 g, 0.08 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude solid obtained was dissolved in ethyl acetate (50 mL) and washed with water (2 × 25 mL). Organic layer was separated, dried over anhydrous $Na_2SO_4$ and concentrated under reduced pressure. The crude compound was purified by prep HPLC to afford 48 mg (17% yield) of **VPCFTE-37** as a brown solid.

HPLC: 99.46% **LCMS-Condition-1:** [M+H]+ = 354.9; $R_t$ = 2.23 min
**$^1$H NMR** (400 MHz, $CDCl_3$) δ: 7.76 (s, 1H), 7.67 (d, *J* = 8.0 Hz, 1H), 7.49 (d, *J* = 8.4 Hz, 1H), 7.12 (s, 1H), 7.09 (s, 1H), 3.85-3.88 (t, *J* = 5.0 Hz, 4H), 3.57 (t, *J* = 4.8 Hz, 4H).

**Synthesis** of **VPCFTE17 (VPC-18-72)** 2-(pyrrolidin-1-yl)-4-(6-(trifluoromethyl)benzofuran-2-yl)thiazole

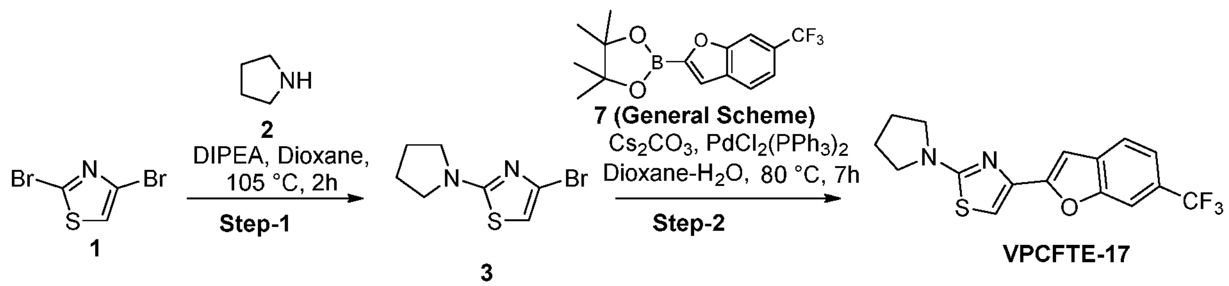


**Step-1: Synthesis of 4-bromo-2-(pyrrolidin-1-yl)thiazole (3):**

To a stirred solution of 2,4-dibromothiazole **1** (1.0 g, 4.12 mmol) in 1,4 dioxane (30 mL) was added DIPEA (1.45 mL, 8.24 mmol) followed by pyrrolidine **2** (1.0 mL, 12.36 mmol) and the reaction mixture was heated at 105 °C for 2 h. After completion of the reaction (monitored by TLC), the reaction mixture was concentrated under reduced pressure. Crude compound was purified by silica gel column chromatography eluting with 10-15% ethyl acetate in n-hexane to afford 0.80 g (83% yield) of **3** as a brown solid. **LCMS-Condition-1:** $[M+H]^+$ = 232.8; $R_t$ = 1.81 min.

**Step-2: Synthesis of 2-(pyrrolidin-1-yl)-4-(6-(trifluoromethyl)benzofuran-2-yl)thiazole (VPCFTE-17):**

**Note: Int-7 synthesis is mentioned in general Scheme above.**

To a stirred solution of 4-bromo-2-(pyrrolidin-1-yl)thiazole **3** (0.18 g, 0.77 mmol) in 1,4 dioxane:$H_2O$ (5:1, 18 mL) in a sealed tube was added 4,4,5,5-tetramethyl-2-(6-(trifluoromethyl)benzofuran-2-yl)-1,3,2-dioxaborolane **7** (0.20 g, 0.64 mmol) followed by $Cs_2CO_3$ (0.42 g, 1.28 mmol) and degassed with argon for 20 min. To the resulting reaction mixture was added $PdCl_2(PPh_3)_2$ (0.044 g, 0.06 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude solid obtained was diluted with water (25 mL) and extracted with ethyl acetate (3 × 25 mL). Combined organic layer was dried over anhydrous $Na_2SO_4$ and concentrated under reduced pressure. The crude compound was purified by prep HPLC to afford 0.025 g (12% yield) of **VPCFTE-17** as a brown solid. HPLC: 99.52%. **LCMS-Condition-1:** [M+H]+ = 339.05; $R_t$ = 2.47 min. **$^1$H**

**NMR** (400 MHz, DMSO-$d_6$) δ: 8.02 (s, 1 H), 7.85 (d, *J*=8.31 Hz, 1 H), 7.59 (d, *J*=7.83 Hz, 1 H), 7.30 (s, 1 H), 7.22 (s, 1 H), 3.43-3.47 (m, 4 H), 1.99-2.03 (m, 4H).

**Synthesis of VPCFTE18 (VPC-18-73)** *N*-cyclopropyl-4-(6-(trifluoromethyl)benzofuran-2-yl)thiazol-2-amine

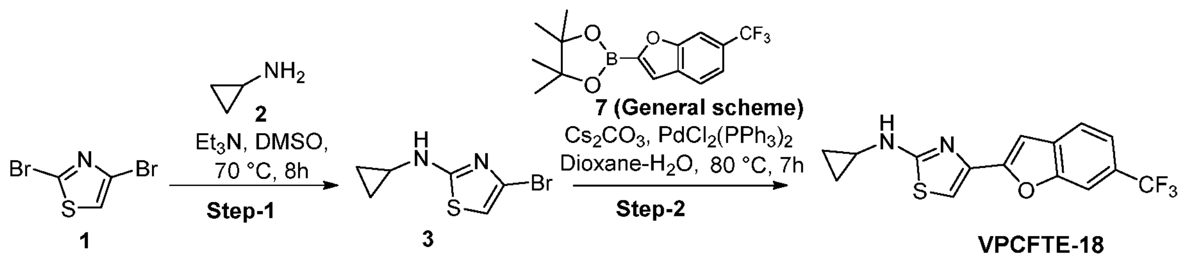


**Step-1: Synthesis of 4-bromo-*N*-cyclopropylthiazol-2-amine (3):**

To a stirred solution of 2,4-dibromothiazole **1** (1.0 g, 4.12 mmol) in DMSO (10 mL) was added $Et_3N$ (2.30 mL, 16.46 mmol) followed by cyclopropanamine **2** (0.70 g, 12.28 mmol) and the reaction mixture was heated at 70 °C for 8 h. After completion of the reaction (monitored by TLC), the reaction mixture was diluted with water and extracted with ethyl acetate. Combined organic layer was dried over anhydrous $Na_2SO_4$ and concentrated under reduced pressure. Crude compound was purified by silica gel column chromatography eluting with 10-15% ethyl acetate in n-hexane to afford 0.60 g (67% yield) of **3** as a brown solid. **LCMS-Condition-1:** [M+2+H]+ = 220.85; $R_t$ = 1.86 min.

**Step-2: Synthesis of *N*-cyclopropyl-4-(6-(tritluoromethyl)benzofuran-2-yl)thiazol-2-amine (VPCFTE-18):**

**Note: Int-7 synthesis is mentioned in general Scheme above.**

To a stirred solution of 4-bromo-*N*-cyclopropylthiazol-2-amine **3** (0.22 g, 0.99 mmol) in 1,4 dioxane:$H_2O$ (4:1, 10 mL) in a sealed tube was added 4,4,5,5-tetramethyl-2-(6-(trifluoromethyl)benzofuran-2-yl)-1,3,2-dioxaborolane **7** (0.28 g, 0.90 mmol) followed by $Cs_2CO_3$ (0.58 g, 1.79 mmol) and degassed with argon for 20 min. To the resulting reaction mixture was added $PdCl_2(PPh_3)_2$ (0.06 g, 0.09 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. Crude solid compound was purified by silica gel column chromatography eluting with 15-20% ethyl acetate in n-hexane followed by re purification by prep HPLC to afford 0.06 g (21% yield) of **VPCFTE-18** as a brown solid.

HPLC: 98.82%. **LCMS-Condition-1:** $[M+H]^+$ = 325.05; $R_t$ = 2.29 min. **$^1$H NMR** (400 MHz, DMSO-$d_6$) δ: 8.31 (s, 1 H), 8.01 (s, 1H), 7.86 (d, *J*=7.83 Hz, 1 H), 7.59 (d, *J*=8.31 Hz, 1 H), 7.29 (s, 1 H) 7.13 (s, 1 H) 2.54 - 2.62 (m, 1 H) 0.72 - 0.81 (m, 2 H) 0.52 - 0.61 (m, 2 H).

**Synthesis of VPCFTE21 (VPC-18-98)** 2-(4-(6-(trifluoromethyl)benzofuran-2-yl)thiazol-2-yl)oxazole

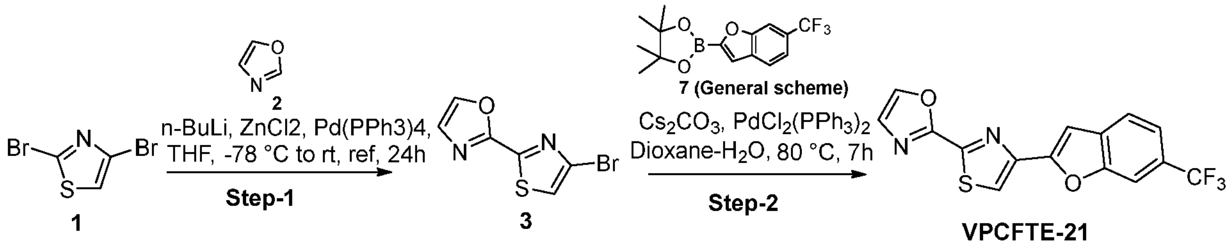

**Note: Int-7 synthesis is mentioned in general Scheme above.**

**Step-1: Synthesis of 2-(4-bromothiazol-2-yl)oxazole (3):**

To a stirred solution of oxazole **2** (1.5 g, 21.72 mmol) in THF (70 mL) was added n-BuLi (1.6 M in n-hexane, 15.5 mL, 24.48 mmol) at -78 °C and the reaction mixture was stirred at that temperature for 30 min. To the resulting reaction mixture was added solid $ZnCl_2$ (8.85 g, 64.94 mmol) and stirred for another 30 min. Then 2,4-dibromothiazole **1** (3.5 g, 14.41 mmol) was added followed by $Pd(PPh_3)_4$ (1.67 g, 1.45 mmol) and the reaction mixture was heated to reflux for 24 h. After completion of the reaction (monitored by TLC), the reaction mixture was cooled to room temperature and poured into saturated $NH_4Cl$ solution. The aqueous layer was extracted with ethyl acetate, dried over anhydrous $Na_2SO_4$ and concentrated under reduced pressure. Crude compound was purified by silica gel column chromatography eluting with 20-25% ethyl acetate in n-hexane to afford 0.50 g (15% yield) of **3** as a white solid. **LCMS-Condition-1:** $[M+2+H]^+$ = 232.90; $R_t$ = 1.85 min.

**Step-2: Synthesis of 2-(4-(6-(trifluoromethyl)benzofuran-2-yl)thiazol-2-yl)oxazole (VPCFTE-21):**

**Note: Int-7 synthesis is mentioned in general Scheme above.**

To a stirred solution of 2-(4-bromothiazol-2-yl)oxazole **3** (0.15 g, 0.65 mmol) in 1,4 dioxane:$H_2O$ (4:1, 15 mL) in a sealed tube was added 4,4,5,5-tetramethyl-2-(6-(trifluoromethyl)benzofuran-2-yl)-1,3,2-dioxaborolane **7** (0.26 g, 0.84 mmol) followed by $Cs_2CO_3$ (0.43 g, 1.31 mmol) and degassed with argon for 20 min. To the resulting reaction mixture was added $PdCl_2(PPh_3)_2$ (0.05 g, 0.07 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. The residue was dissolved in ethyl acetate (50 mL) and washed with water (2 × 25 mL). Combined organic layer was dried over anhydrous $Na_2SO_4$ and concentrated under reduced pressure. Crude compound was purified by silica gel column chromatography eluting with 20-25% ethyl acetate in n-hexane to afford 0.10 g (46% yield) of **VPCFTE-21** as an off-white solid. **HPLC:** 98.52%. **LCMS-Condition-1:** $[M+H]^+$ = 336.9; $R_t$ = 2.12 min

**$^1$H NMR** (400 MHz, DMSO-$d_6$) δ: 8.44 (d, J = 10.0 Hz, 2H), 8.12 (s, 1H), 7.94 (d, J = 7.6 Hz, 1H), 7.65 (d, J = 7.6 Hz, 1H), 7.56 (d, J = 2.0 Hz, 2H).

**Synthesis of VPCFTE41 (VPC-18-89)** 2-(piperidin-4-yl)-4-(6-(trifluoromethyl)benzofuran-2-yl)thiazole

**Note: Int-7 synthesis is mentioned in general Scheme above.**

**Step-1: Synthesis of *tert*-butyl 4-(4-bromothiazol-2-yl)-3,6-dihydropyridine-1(2*H*)-carboxylate (3):**

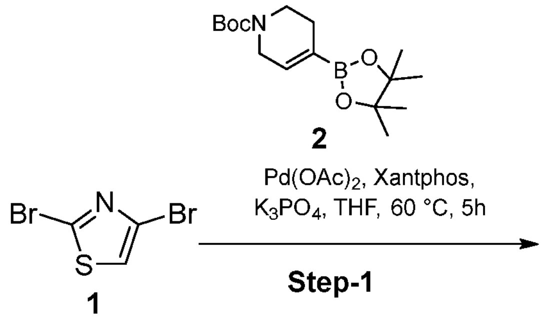


To a stirred solution of 2,4-dibromothiazole **1** (0.7 g, 2.91 mmol) in THF (20 mL) was added tert-butyl 4-(4,4,5,5-tetramethyl-1,3,2-dioxaborolan-2-yl)-3,6-dihydropyridine-1(2*H*)-carboxylate **2** (0.98 g, 3.17 mmol) and $K_3PO_4$ (1.22 g, 5.74 mmol) in a sealed tube and degassed with argon for 20 min. To the resulting solution was added $Pd(OAc)_2$ (0.06 mg, 0.29 mmol) followed by xantphos (0.17 g, 0.29 mmol) and the reaction mixture was degassed with argon for another 10 min at room temperature. The reaction mixture was heated at 60 °C for 5 h. After completion of the reaction (monitored by TLC), the reaction mixture was cooled to room temperature and solvent was removed under reduced pressure. Combined organic layer was dried over anhydrous $Na_2SO_4$ and concentrated under reduced pressure.The crude solid

obtained was dissolved in ethyl acetate (100 mL) and washed with water (2 × 50 mL). Organic layer was separated, dried over anhydrous $Na_2SO_4$ and concentrated under reduced pressure. Crude compound was purified by silica gel column chromatography eluting with 3-4% ethyl acetate in n-hexane to afford 0.9 g (90% yield) of **3** as an off-white solid.

**LCMS-Condition-1:** $[M-56+H]^+$ = 290.7; $R_t$ = 2.10 min

**Step-2: Synthesis of *tert*-butyl 4-(4-(6-(trifluoromethyl)benzofuran-2-yl)thiazol-2-yl)-3,6-dihydropyridine-1(2*H*)-carboxylate (4):**

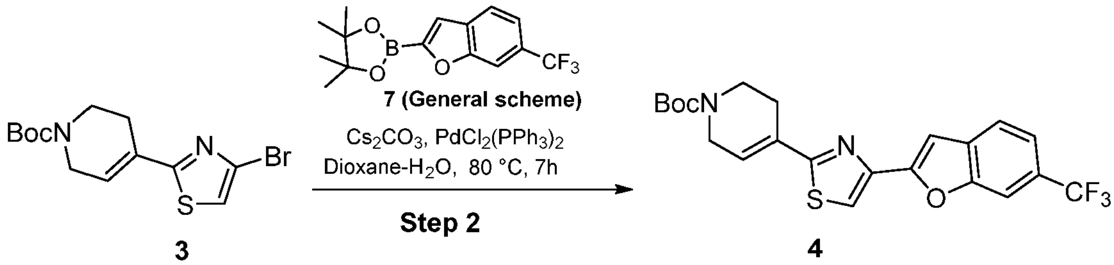


3 4

**Note: Int-7 synthesis is mentioned in general Scheme above.**

To a stirred solution of *tert*-butyl 4-(4-bromothiazol-2-yl)-3,6-dihydropyridine-1(2*H*)-carboxylate **3** (0.89 g, 2.60 mmol) in 1,4 dioxane:$H_2O$ (5:1, 18 mL) in a sealed tube was added 4,4,5,5-tetramethyl-2-(6-(trifluoromethyl)benzofuran-2-yl)-1,3,2-dioxaborolane **4** (0.90 g, 2.88 mmol) followed by $Cs_2CO_3$ (1.87 g, 5.74 mmol) and degassed with argon for 20 min. To the resulting reaction mixture was added $PdCl_2(PPh_3)_2$ (0.20 g, 0.29 mmol) and degassed with argon for another 10 min at room temperature. The reaction mixture was then heated at 80 °C for 7 h. After completion of the reaction (monitored by TLC), the reaction mixture was cooled to room temperature and solvent was removed under reduced pressure. The residue was dissolved in ethyl acetate (100 mL) and washed with water (2 × 50 mL). Combined organic layer was dried over anhydrous $Na_2SO_4$ and concentrated under reduced pressure. Crude compound was purified by silica gel column chromatography eluting with 4-5% ethyl acetate in n-hexane followed by washing with MeOH (2 × 10 mL) to afford 0.65 g (54% yield) of **4** as an off-white solid.

**LCMS-Condition-1:** $[M+H]^+$ = 451.10; $R_t$ = 2.65 min

**Step-3: Synthesis of *tert*-butyl 4-(4-(6-(trifluoromethyl)benzofuran-2-yl)thiazol-2-yl)piperidine-1-carboxylate (5):**

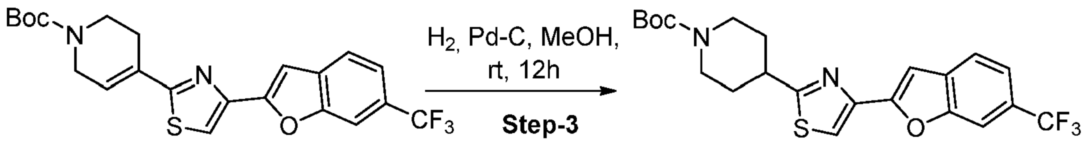


4 5

To a stirred solution of tert-butyl 4-(4-(6-(trifluoromethyl)benzofuran-2-yl)thiazol-2-yl)-3,6-dihydropyridine-1(2*H*)-carboxylate **4** (0.15 g, 0.33 mmol) in MeOH (25 mL) was added 10% Pd/C (0.05 g) and the reaction mixture was stirred under $H_2$ balloon pressure for 12 h. After completion of the reaction (monitored by TLC), the reaction mixture was filtered through celite bed, washed thoroughly with MeOH. Filtrate was concentrated under reduced pressure to yield crude compound which was washed with diethyl ether (20 mL) to afford 0.1 g (67% yield) of **5** as an off-white solid.

**LCMS-Condition-1:** $[M-56+1]^+$ = 397.00; $R_t$ = 2.46 min

**Step-2: Synthesis of 2-(piperidin-4-yl)-4-(6-(trifluoromethyl)benzofuran-2-yl)thiazole (VPCFTE-41):**

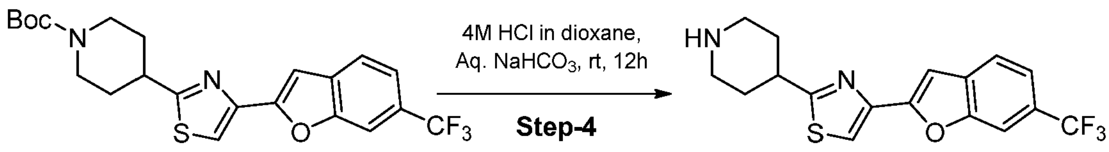


5 VPCFTE-41

To a stirred solution of tert-butyl 4-(4-(6-(trifluoromethyl)benzofuran-2-yl)thiazol-2-yl)piperidine-1-carboxylate **5** (0.1 g, 0.22 mmol) in 1,4 dioxane (10 mL) was added 4M HCl in dioxane (5 mL) and the reaction mixture was stirred at room temperature for 12 h. After completion of the reaction (monitored by TLC), solvent was removed under reduced pressure. The crude was washed with diethyl ether (20 mL) to yield solid compound which was neutralized with aqueous $NaHCO_3$ solution (15 mL). The precipitated solid was filtered, washed with *n*-pentane (2 × 10 mL) and dried to afford 30 mg (39% yield) of **VPCFTE-41** as an off-white solid.

**HPLC:** 99.54%

**LCMS-Condition-1:** $[M+H]^+$ = 353.05; $R_t$ = 1.78 min.

1H NMR (400 MHz, DMSO-d6) δ: 8.10 (s, 1H), 8.07 (s, 1H), 7.89 (d, *J* = 8.4 Hz, 1H), 7.62 (d, *J* = 8.0 Hz, 1H), 7.38 (s, 1H), 3.18-3.14 (m, 1H), 3.02 (d, *J* = 12.4 Hz, 2H), 2.67-2.58 (m, 2H), 2.00 (d, *J* = 12.0 Hz, 2H), 1.66-1.56 (m, 2H) (1H merged in solvent peak).

**Synthesis of VPCFTE55 (VPC-18-95)**_4-(6-chlorobenzofuran-2-yl)-2-(1,2,3,6-tetrahydropyridin-4-yl)thiazole hydrochloride

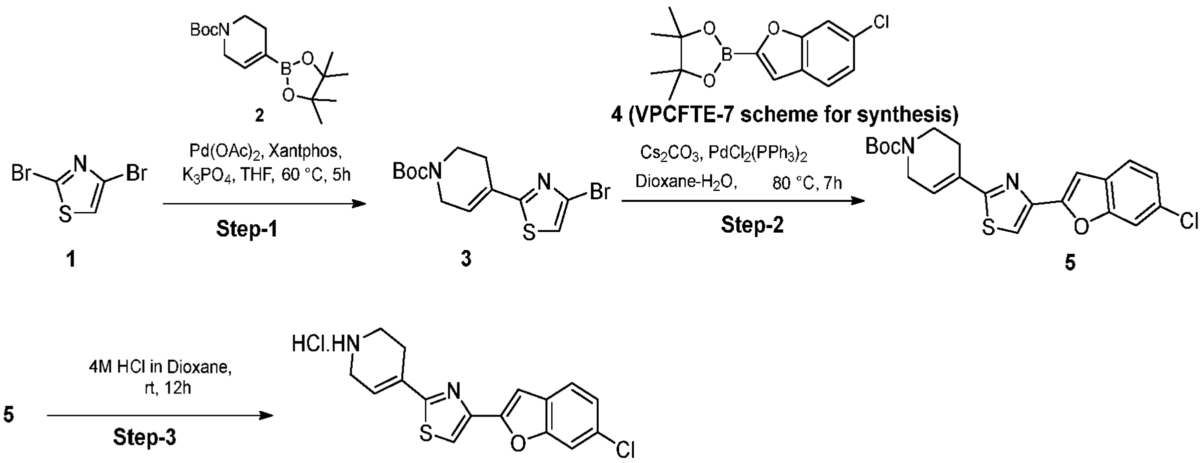


VPCFTE-55

**Step-1: Synthesis of tert-butyl 4-(4-bromothiazol-2-yl)-3,6-dihydropyridine-1(2*H*)-carboxylate (3):**

To a stirred solution of 2,4-dibromothiazole **1** (1.0 g, 4.12 mmol) in THF (20 mL) was added tert-butyl 4-(4,4,5,5-tetramethyl-1,3,2-dioxaborolan-2-yl)-3,6-dihydropyridine-1(2*H*)-carboxylate **2** (0.94 g, 4.12 mmol) and $K_3PO_4$ (1.75 g, 8.24 mmol) in a sealed tube and degassed with argon for 20 min. To the resulting solution was added $Pd(OAc)_2$ (0.09 g, 0.41 mmol) followed by Xantphos (0.24 g, 0.41 mmol) and the reaction mixture was degassed with argon for another 10 min at room temperature. The reaction mixture was heated at 60 °C for 5 h. After completion of the reaction (monitored by TLC), the reaction mixture was cooled to room temperature and solvent was removed under reduced pressure. Crude compound was purified by silica gel column chromatography eluting with 10-15% ethyl acetate in n-hexane to afford 1.2 g (85% yield) of **3** as an off-white solid.

**LCMS-Condition-1:** $[M-56+H]^+$ = 288.8; $R_t$ = 2.09 min.

**Step-2: Synthesis of tert-butyl 4-(4-(6-chlorobenzofuran-2-yl)thiazol-2-yl)-3,6-dihydropyridine-1(2*H*)-carboxylate (5):**

**Note: Synthesis of 4 reported in VPCFTE7 scheme**

To a stirred solution of tert-butyl 4-(4-bromothiazol-2-yl)-3,6-dihydropyridine-1(2*H*)-carboxylate **3** (0.50 g, 1.45 mmol) in 1,4 dioxane:$H_2O$ (4:1, 30 mL) in a sealed tube was added 2-(6-chlorobenzofuran-2-yl)-4,4,5,5-tetramethyl-1,3,2-dioxaborolane **4** (0.53 g, 1.88 mmol) followed by $Cs_2CO_3$ (0.94 g, 2.89 mmol) and degassed with argon for 20 min. To the resulting reaction mixture was added $PdCl_2(PPh_3)_2$ (0.10 g, 0.15 mmol) and degassed with argon for another 10 min at room temperature. The reaction mixture was then heated at 80 ◦C for 7 h. After completion of the reaction (monitored by TLC), the reaction mixture was cooled to room temperature and solvent was removed under reduced pressure. The residue was dissolved in ethyl acetate (50 mL) and washed with water (2 × 25 mL). Combined organic layer was dried over anhydrous $Na_2SO_4$ and concentrated under reduced pressure. Crude compound was purified by silica gel column chromatography eluting with 20-25% ethyl acetate in n-hexane to afford 0.25 g (41% yield) of **5** as an off-white solid. **LCMS-Condition-1:** [M+H]+ = 417.10; $R_t$ = 2.76 min.

**Step-3: Synthesis of 4-(6-chlorobenzofuran-2-yl)-2-(1,2,3,6-tetrahydropyridin-4-yl)thiazole hydrochloride (VPCFTE-55):**

To a stirred solution of *tert*-butyl 4-(4-(6-chlorobenzofuran-2-yl)thiazol-2-yl)-3,6-dihydropyridine-1(2*H*)-carboxylate **5** (0.15 g, 0.36 mmol) in 1,4 dioxane (10 mL) was added 4M HCl in dioxane (5 mL) at 0 °C and the reaction mixture was stirred at room temperature for 12 h. After completion of the reaction (monitored by TLC), solvent was removed under reduced pressure. The crude was washed with diethyl ether (20 mL) and n-pentane (20 mL) to afford 0.115 g (91% yield) of **VPCFTE-55** as an off-white solid.

**HPLC:** 99.21%. **LCMS-Condition-1:** $[M+H]^+$ = 317.05; $R_t$ = 2.51 min.

**$^1$H NMR** (400 MHz, DMSO-$d_6$) δ: 9.40 (brs, 2H), 8.14 (s, 1H), 7.82 (s, 1H), 7.71 (d, *J* = 8.4 Hz, 1H), 7.36-7.32 (m, 2H), 6.72 (brs, 1H), 3.82 (brs, 2H), 3.34 (brs, 2H), 2.86 (brs, 2H).

**Synthesis of VPCFTE71 (VPC-18-97)** 4-(6-chlorobenzofuran-2-yl)-2-(1,2,3,6-tetrahydropyridin-4-yl)thiazole

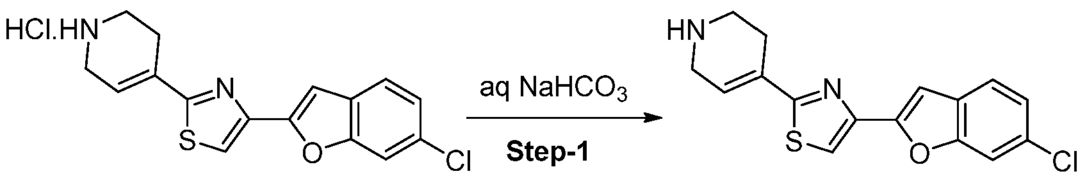


**VPCFTE-55** **VPCFTE-71**

**Step-1: Synthesis of 4-(6-chlorobenzofuran-2-yl)-2-(1,2,3,6-tetrahydropyridin-4-yl)thiazole (VPCFTE-71):**

To solid 4-(6-chlorobenzofuran-2-yl)-2-(1,2,3,6-tetrahydropyridin-4-yl)thiazole hydrochloride **VPCFTE-55** (0.09 g, 0.25 mmol) was added saturated $NaHCO_3$ solution (10 mL) and the reaction mixture was stirred at room temperature for 15 min. The precipitated solid was filtered, washed with ice cold water followed by *n*-pentane to afford 0.07 g (88% yield) of **VPCFTE-71** as an off-white solid.

**HPLC:** 98.50%. **LCMS-Condition-1:** [M+H]+ = 316.95; $R_t$ = 1.42 min.

**$^1$H NMR** (400 MHz, DMSO-d6) δ: 8.02 (s, 1 H), 7.81 (s, 1 H), 7.70 (d, *J*=8.37 Hz, 1 H), 7.31 - 7.37 (m, 1 H) 7.29 (s, 1 H), 6.75 (br s, 1 H), 3.41-3.43 (m, 2 H), 2.90-2.94 (m, 2 H), (3H's merged in solvent peak)

**Synthesis of VPCFTE60 (VPC-18-96)** 4-(6-chlorobenzofuran-2-yl)-2-(1H-pyrazol-5-yl)thiazole

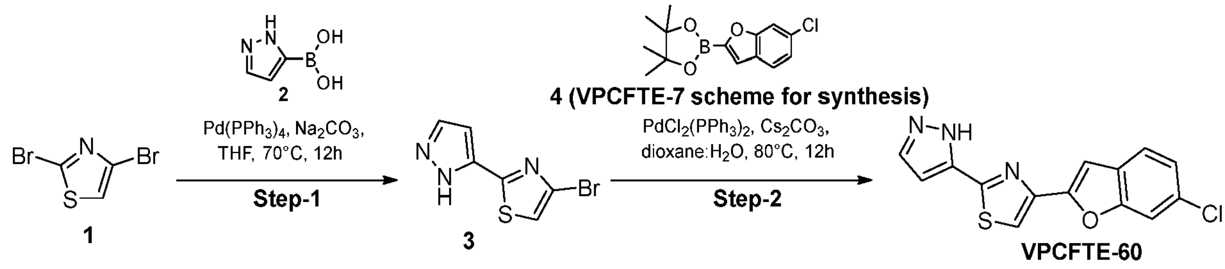


**Step-1: Synthesis of 4-bromo-2-(1*H*-pyrazol-5-yl) thiazole (3):**

To a degassed mixture of 2,4-dibromothiazole **1** (1.00 g, 4.15 mmol) and (1*H*-pyrazol-5-yl)boronic acid **2** (0.51 g, 4.57 mmol) in THF (20 mL) under argon atmosphere was added $Na_2CO_3$ (0.88 g, 8.30 mmol). The reaction mixture was stirred for 5 min followed by addition of $Pd(PPh_3)_4$ (0.24 g, 0.21 mmol) and the reaction mixture was heated in sealed tube at 70°C for 12h. After completion of the reaction (monitored by TLC), the reaction mixture was cooled to room temperature and solvent was removed under reduced pressure. The crude compound was purified by column chromatography (silica, 100-200 mesh, 15 to 20% EtOAc in hexane) to afford 0.12 g (13% yield) of **3** as an off-white solid.

**LCMS-Condition-1:** $[M+H]^+$ = 229.8; $R_t$ = 1.85 min

**Step-2: Synthesis of 4-(6-chlorobenzofuran-2-yl)-2-(1H-pyrazol-5-yl)thiazole (VPCFTE-60):**

**Note: Synthesis of 4 reported in VPCFTE7 scheme**

To a degassed mixture of 4-bromo-2-(1*H*-pyrazol-5-yl)thiazole **3** (0.10 g, 0.44 mmol) and 2-(6-chlorobenzofuran-2-yl)-4,4,5,5-tetramethyl-1,3,2-dioxaborolane **4** (0.16 g, 0.57 mmol) in 1,4 dioxane:$H_2O$ (10 mL:3 mL) was added $Cs_2CO_3$ (0.28 g, 0.87 mmol) followed by addition of $PdCl_2(PPh_3)_2$ (0.03 g, 0.04 mmol) in a sealed tube under argon atmosphere at room temperature. The reaction mixture was heated 80◦C for 12h. After completion of the reaction (monitored by TLC), the reaction mixture was cooled to room temperature and solvent was removed under reduced pressure. The residue was dissolved in EtOAc (50 mL) and washed with $H_2O$ (2 × 25 mL). The organic layer was separated, dried over anhydrous $Na_2SO_4$ and solvent was removed under reduced pressure. The crude compound was purified by column chromatography (silica, 100-200 mesh, 15 to 20% EtOAc in hexane) to afford 0.01 g (7% yield) of **VPCFTE60** as an off-white solid. **HPLC:** 99.39%. **LCMS-Condition-1:** $[M+H]^+$ = 301.85; $R_t$ = 2.20 min.

$^1$**H NMR** (400 MHz, DMSO-d6) δ: 13.36 (brs, 1 H), 8.08 (s, 1 H), 7.94 (s, 1 H), 7.83 (s, 1 H), 7.72 (d, J=8.37 Hz, 1 H), 7.36 (s, 1H), 7.34 (d, J=1.48 Hz, 1 H), 6.85 (d, J=1.97 Hz, 1 H).

**Synthesis of VPCFTE52 (VPC-18-91)** 5-(4-(6-chlorobenzofuran-2-yl)thiazol-2-yl)-2-oxa-5-azabicyclo[2.2.1]heptane

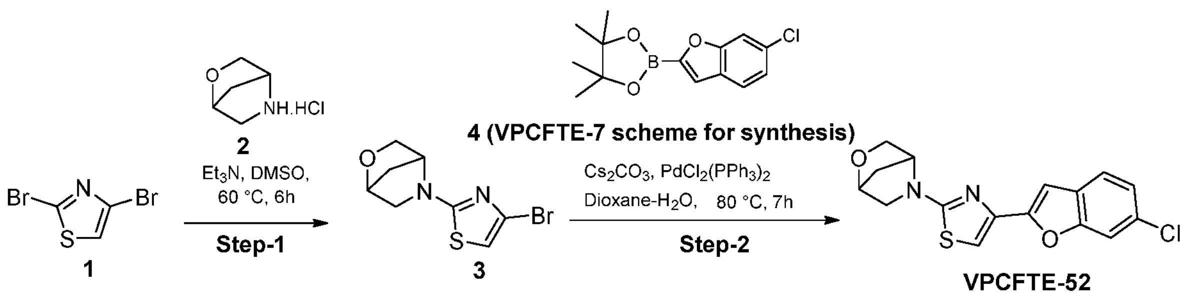


**Note: Synthesis of 4 reported in VPCFTE7 scheme**

**Step-1: Synthesis of 5-(4-bromothiazol-2-yl)-2-oxa-5-azabicyclo[2.2.1]heptane (3):**

To a stirred solution of 2,4-dibromothiazole **1** (0.5 g, 2.06 mmol) in DMSO (10 mL) was added $Et_3N$ (0.86 mL, 6.15 mmol) followed by 2-oxa-5-azabicyclo[2.2.1]heptane hydrochloride **2** (0.42 g, 3.09 mmol) at 0°C and the reaction mixture was heated at 60 °C for 6 h. After completion of the reaction (monitored by TLC), the reaction mixture was diluted

with ice cold water and extracted with ethyl acetate. Combined organic layer was washed with brine, dried over anhydrous $Na_2SO_4$ and concentrated under reduced pressure. Crude compound was purified by silica gel column chromatography eluting with 10% ethyl acetate in *n*-hexane to afford 0.30 g (56% yield) of **3** as an off-white solid. **LCMS-Condition-1:** [M+H]+ = 260.95; $R_t$ = 1.93 min.

**Step-2: Synthesis of 5-(4-(6-chlorobenzofuran-2-yl)thiazol-2-yl)-2-oxa-5-azabicyclo[2.2.1]heptane (VPCFTE-52): Note: Synthesis of 4 reported in VPCFTE7 scheme**

To a stirred solution of 5-(4-bromothiazol-2-yl)-2-oxa-5-azabicyclo[2.2.1]heptane **3** (0.15 g, 0.57 mmol) in 1,4 dioxane:$H_2O$ (4:1, 15 mL) in a sealed tube was added 2-(6-chlorobenzofuran-2-yl)-4,4,5,5-tetramethyl-1,3,2-dioxaborolane **4** (0.21 g, 0.75 mmol) followed by $Cs_2CO_3$ (0.38 g, 1.15 mmol) and degassed with argon for 20 min. To the resulting reaction mixture was added $PdCl_2(PPh_3)_2$ (0.04 g, 0.06 mmol) and degassed with argon for another 10 min at room temperature. The reaction mixture was then heated at 80 ∘C for 7 h. After completion of the reaction (monitored by TLC), the reaction mixture was cooled to room temperature and solvent was removed under reduced pressure. The crude solid obtained was dissolved in ethyl acetate (50 mL) and washed with water (2 × 25 mL). Organic layer was separated, dried over anhydrous $Na_2SO_4$ and concentrated under reduced pressure. Crude compound was purified by prep HPLC to afford 0.04 g (21% yield) of **VPCFTE-52** as an off-white solid. HPLC: 98.94%. **LCMS-Condition-1:** [M+H]+ = 332.9; $R_t$ = 2.13 min. **$^1$H NMR** (400 MHz, $CDCl_3$) δ: 7.50-7.47 (m, 2H), 7.23-7.20 (m, 1H), 7.02 (s, 1H), 6.98 (s, 1H), 4.75 (s, 2H), 4.05 -3.90 (m, 2H), 3.59-3.46(m, 2H), 2.06-2.00 (m, 2H).

**Synthesis of VPCFTE10 (VPC-18-75)** 2-(2-(furan-2-yl)thiazol-4-yl)furo[3,2-b]pyridine

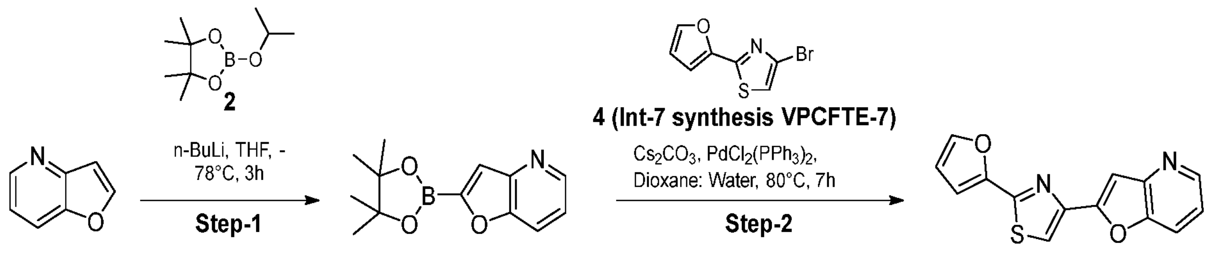


1 3 VPCFTE-10

**Note: Synthesis of 4 reported (as Int-7 synthesis in VPCFTE7 scheme)**

**Step-1: Synthesis of 2-(4,4,5,5-tetramethyl-1,3,2-dioxaborolan-2-yl)furo[3,2-b]pyridine (3):**

To a stirred solution of furo[3,2-b]pyridine **1** (0.25 g, 2.10 mmol) in THF (10 mL) was added 1.4M *n*-BuLi in hexane (0.61 mL, 2.73 mmol) at -78°C and stirred for 1.5 h. 2-isopropoxy-4,4,5,5-tetramethyl-1,3,2-dioxaborolane **2** (0.56 mg, 3.15 mmol) was added dropwise at -78 ∘C and the reaction mixture was stirred at same temperature for 1.5h. After completion of the reaction (monitored by TLC), the reaction mixture was quenched with MeOH (0.6 mL), solvent was removed under reduced pressure. The crude compound was purified by trituration with *n*-pentane (25 mL) to afford 0.5 g (crude) of **3** as brown solid. It is carried to next step without purification.

**Step-2: Synthesis of 2-(2-(furan-2-yl)thiazol-4-yl)furo[3,2-b]pyridine (VPCFTE-10):**

**Note: Synthesis of 4 reported (as Int-7 synthesis in VPCFTE7 scheme)**

To a degassed solution of 2-(4,4,5,5-tetramethyl-1,3,2-dioxaborolan-2-yl)furo[3,2-b]pyridine **3** (0.15 g, 0.66 mmol) and 4-bromo-2-(furan-2-yl)thiazole **4** (0.24 g, 0.98 mmol) in 1,4 dioxane:$H_2O$ (15 mL:4.5 mL) was added $Cs_2CO_3$ (0.43 g, 1.31 mmol) followed by addition of $PdCl_2(PPh_3)_2$ (0.05 g, 0.10 mmol) in a sealed tub. The reaction mixture was stirred at 80 ∘C for 7 h. After completion of the reaction (monitored by TLC), the reaction mixture was cooled to room temperature and solvent was removed under reduced pressure. Crude obtained was dissolved in EtOAc (50 mL) and washed with $H_2O$ (2 × 25 mL). The organic layer was separated, dried over anhydrous $Na_2SO_4$, concentrated under reduced pressure. Crude compound was purified by silica gel column chromatography eluting with 10-13% ethyl acetate in n-hexane to afford 60 mg (34% yield) of **VPCFTE-10** as an off-white solid. **HPLC:** 99.79%. **LCMS-Condition-1:** $[M+H]^+$

= 269.05; $R_t$ = 1.77 min.

**$^1$H NMR** (400 MHz, $CDCl_3$) δ: 8.54 (d, *J*=4.40 Hz, 1 H), 7.76 (d, *J*=8.31 Hz, 1 H), 7.73 (s, 1 H), 7.55 (s, 1 H) 7.43 (s, 1 H), 7.19 - 7.23 (m, 1 H), 7.14 (d, *J*=3.42 Hz, 1 H), 6.56-6.58 (m, 1 H).

**Synthesis of VPCFTE25 (VPC-18-88)** 2-(2-(furan-2-yl)thiazol-4-yl)benzofuran-3-carbonitrile

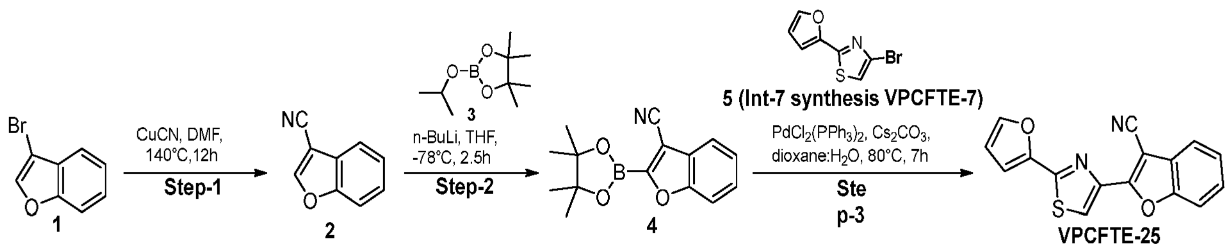


**Note: Synthesis of 5 reported (as Int-7 synthesis in VPCFTE7 scheme)**

**Step-1: Synthesis of benzofuran-3-carbonitrile (2):**

To a degassed mixture of 3-bromobenzofuran **1** (0.75 g, 3.83 mmol) in dry DMF (1.5 mL) was added CuCN (0.69 g, 7.66 mmol) under argon atmosphere at room temperature. The reaction mixture was heated in sealed tube at 140◦C for 12h. After completion of the reaction (monitored by TLC), the reaction mixture was cooled to room temperature, diluted with $Et_2O$ (150 mL) and $H_2O$ (100 mL) and filtered through the pad of Celite®. The organic layer was separated, dried over anhydrous $Na_2SO_4$ and solvent was removed under reduced pressure. The crude compound was purified by column chromatography (silica, 100-200 mesh, 0.5 to 1% EtOAc in hexane) to afford 0.22 g (40% yield) of **2** as a yellow solid.

**$^1$H NMR** (400 MHz, $CDCl_3$) δ: 8.17 (s, 1 H), 7.75 - 7.80 (m, 1 H), 7.60 - 7.64 (m, 1 H), 7.41 - 7.50 (m, 2 H).

**Step-2: Synthesis of 2-(4,4,5,5-tetramethyl-1,3,2-dioxaborolan-2-yl)benzofuran-3-carbonitrile (4):**

To a stirred solution of benzofuran-3-carbonitrile **2** (0.15 g, 1.05 mmol) in THF (6 mL) was added *n*-BuLi (1.4 M in hexane, 0.97 mL, 1.36 mmol) at -78°C and the reaction mixture was stirred at same temperature for 1.5h. 2-isopropoxy-4,4,5,5-tetramethyl-1,3,2-dioxaborolane **3** (2.14 g, 11.4 mmol) was added drop wise at -78°C and the reaction mixture was stirred at same temperature for 2.5h. After completion of the reaction (monitored by TLC), the reaction mixture was quenched with MeOH (0.3 mL) and solvent was removed under reduced pressure. The crude compound was triturated with pentane to afford 0.255 g (97% yield) of **4** as a red solid.

**Step-3: Synthesis of 2-(2-(furan-2-yl)thiazol-4-yl)benzofuran-3-carbonitrile (VPCFTE-25):**

**Note: Synthesis of 5 reported (as Int-7 synthesis in VPCFTE7 scheme)**

To a degassed mixture of 2-(4,4,5,5-tetramethyl-1,3,2-dioxaborolan-2-yl)benzofuran-3-carbonitrile **4** (0.15 g, 0.66 mmol) and 4-bromo-2-(furan-2-yl)thiazole **5** (0.23 g, 0.85 mmol) in 1,4 dioxane:$H_2O$ (15 mL:4.5 mL) was added $Cs_2CO_3$ (0.43 g, 1.31 mmol) followed by addition of $PdCl_2(PPh_3)_2$ (0.05 g, 0.07 mmol) in a sealed tube under argon atmosphere at room temperature. The reaction mixture was heated 80◦C for 7h. After completion of the reaction (monitored by TLC), the reaction mixture was cooled to room temperature and solvent was removed under reduced pressure. The residue was dissolved in EtOAc (50 mL) and washed with $H_2O$ (2 × 25 mL). The organic layer was separated, dried over anhydrous $Na_2SO_4$ and solvent was removed under reduced pressure. The crude compound was purified by column chromatography (silica, 100-200 mesh, 0.5 to 1% EtOAc in hexanes) to afford 0.038 g (19% yield) of **VPCFTE-25** as an off-white solid. **HPLC:** 95.35%. **LCMS-Condition-1:** [M+H]+ = 292.95; $R_t$ = 2.43 min. **$^1$H NMR** (400 MHz, $CDCl_3$) δ: 8.06 (s, 1 H), 7.76 - 7.80 (m, 1 H), 7.63 (d, J=8.31 Hz, 1 H), 7.59 (d, J=0.98 Hz, 1 H), 7.41 - 7.49 (m, 2 H), 6.60-6.62 (m, 1 H), (1H merged in solvent peak).

**Synthesis of VPCFTE23 (VPC-18-74)** 3-methyl-2-(5-phenylfuran-2-yl)benzofuran

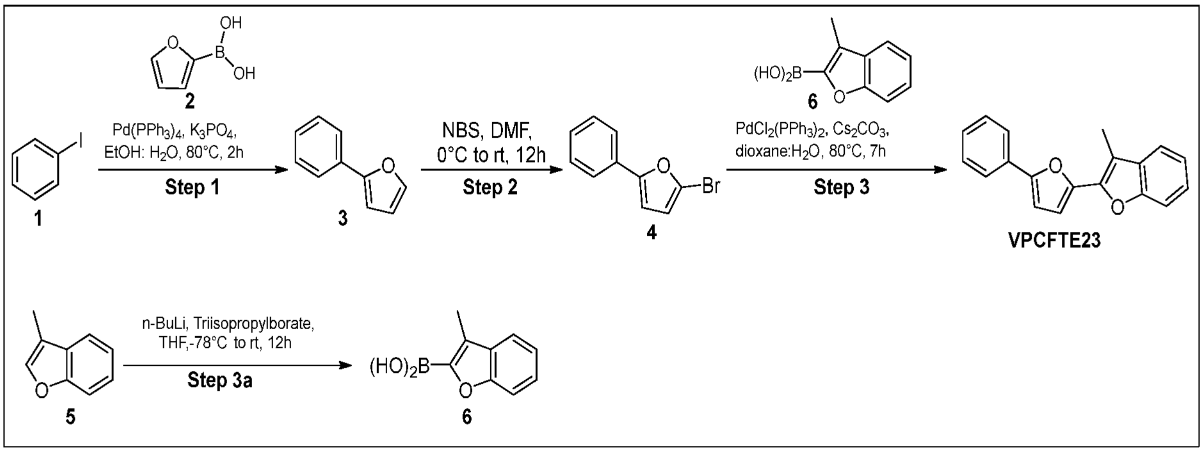


**Step-1: Synthesis of 2-phenylfuran (3):**

To a degassed mixture of iodobenzene **1** (2.00 g, 9.81 mmol) and furan-2-ylboronic acid **2** (1.64 g, 14.7 mmol) in EtOH:$H_2O$ (16 mL:4 mL) under argon atmosphere was added $K_3PO_4$ (4.16 g, 19.6 mmol). The reaction mixture was stirred for 5 min followed by addition of $Pd(PPh_3)_4$ (0.56 g, 0.49 mmol) and the reaction mixture was heated in sealed tube at 80◦C for 2h. After completion of the reaction (monitored by TLC), the reaction mixture was cooled to room temperature and solvent was removed under reduced pressure. The crude compound was purified by column chromatography (silica, 100-200 mesh, 0.5 to 1% EtOAc in hexane) to afford 1.20 g (85% yield) of **3** as a colourless oil. **LCMS-Condition-1:** [M+H]+ = 144.9; $R_t$ = 2.15 min.

**Step-2: Synthesis of 2-bromo-5-phenylfuran (4):**

To a stirred solution of 2-phenylfuran **3** (0.50 g, 3.47 mmol) in DMF (10 mL) was added NBS (0.65 g, 3.64 mmol) portion wise at 0°C. The reaction mixture was stirred at room temperature for 12h. After completion of the reaction (monitored by TLC), the reaction mixture was quenched with ice cold $H_2O$ (25 mL) and extracted with $Et_2O$ (2 × 25 mL). The organic layer was separated, washed with $H_2O$ (25 mL) and solvent was removed under reduced pressure. The crude compound was purified by column chromatography (silica, 100-200 mesh, 0.5 to 1% EtOAc in hexane) to afford 0.64 g (83% yield) of **4** as a light brown oil. $^1$H NMR (400 MHz, $CDCl_3$) δ: 7.64 (d, *J*=7.34 Hz, 2 H), 7.38-7.42 (m, 2 H), 7.26 - 7.33 (m, 1H), 6.62 (d, *J*=3.42 Hz, 1 H), 6.40 (d, *J*=3.42 Hz, 1 H).

**Step-3a: Synthesis of (3-methylbenzofuran-2-yl)boronic acid (6):**

To a stirred solution of 3-methylbenzofuran **5** (0.50 g, 3.79 mmol) in THF (15 mL) was added *n*-BuLi (1.4 M in hexane, 3.20 mL, 4.54 mmol) at -78°C and the reaction mixture was stirred at 0°C for 1h. triisopropyl borate (2.14 g, 11.4 mmol) was added at -78°C. The reaction mixture was stirred at room temperature for 12h. After completion of the reaction (monitored by TLC), the reaction mixture was quenched with 1 *N* HCl and pH adjusted to 5. The reaction mixture then stirred at room temperature for 30 min and extracted with EtOAc (3 × 25 mL). The organic layer was separated, washed with $H_2O$ (25 mL), dried over anhydrous $Na_2SO_4$ and solvent was removed under reduced pressure. The crude compound was triturated with $Et_2O$ (25 mL) and pentane (25 mL) to afford 0.30 g (45% yield) of **6** as an off-white solid.

**Step-3: Synthesis of 3-methyl-2-(5-phenylfuran-2-yl)benzofuran (VPCFTE23):**

To a degassed mixture of 2-bromo-5-phenylfuran **4** (0.10 g, 0.45 mmol) and (3-methylbenzofuran-2-yl)boronic acid **6** (0.12 g, 0.68 mmol) in 1,4 dioxane:$H_2O$ (10 mL:3 mL) in a sealed tube was added $Cs_2CO_3$ (0.39 g, 0.90 mmol) followed by addition of $PdCl_2(PPh_3)_2$ (0.02 g, 0.02 mmol) at room temperature. The reaction mixture was then heated 80◦C for 7h. After completion of the reaction (monitored by TLC), the reaction mixture was cooled to room temperature and solvent was removed under reduced pressure. The crude compound was purified by prep HPLC to afford 0.04 g (32% yield) of **VPCFTE23** as an off-white solid.

**HPLC:** 99.56%. **LCMS-Condition-1:** [M+H]+ = 275.05; $R_t$ = 2.61 min. **$^1$H NMR** (400 MHz, $CDCl_3$) δ: 7.78 (d, *J*=7.6 Hz, 2 H), 7.56 (d, *J*=7.2 Hz, 1 H), 7.42 - 7.51 (m, 3 H), 7.28 - 7.35 (m, 3 H), 6.84 - 6.88 (m, 1 H), 6.81-6.83 (m, 1 H), 2.62 (s, 3 H).

**Synthesis of VPCFTE63 (VPC-18-92)** 6-chloro-2-(5-phenylfuran-2-yl)-1H-benzo[d]imidazole

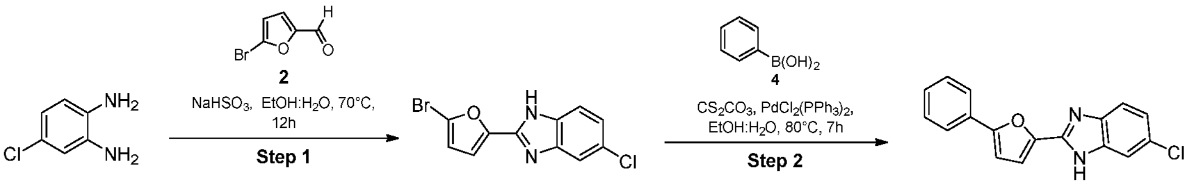


1 3 VPCFTE-63

**Step-1: Synthesis of 2-(5-bromofuran-2-yl)-5-chloro-1*H*-benzo[d]imidazole (3):**

To a stirred solution of 4-chlorobenzene-1,2-diamine **1** (1.00 g, 7.04 mmol) and 5-bromofuran-2-carbaldehyde **2** (1.84 g, 10.56 mmol) in EtOH:$H_2O$ (10 mL:10 mL) was added $NaHSO_3$ (1.47 g, 14.08 mmol) in sealed tube at room temperature. The reaction mixture was stirred at 70 °C for 12 h. After completion of the reaction (monitored by TLC), the reaction mixture was cooled to room temperature and solvent was removed under reduced pressure. Crude compound was purified by silica gel column chromatography eluting with 5-8% ethyl acetate in *n*-hexane to afford 0.72 g (35% yield) of **3** as an off-white solid.

**LCMS-Condition-1:** $[M+2+H]^+$ = 298.8; $R_t$ = 1.76 min.

**Step-2: Synthesis of 6-chloro-2-(5-phenylfuran-2-yl)-1*H*-benzo[d]imidazole (VPCFTE-63):**

To a degassed solution of 2-(5-bromofuran-2-yl)-5-chloro-1*H*-benzo[d]imidazole **3** (0.15 g, 0.51 mmol) and phenylboronic acid **4** (0.90 g, 0.76 mmol) in EtOH:$H_2O$ (15 mL:4.5 mL) was added $Cs_2CO_3$ (0.33 g, 1.01 mmol) followed by addition of $PdCl_2(PPh_3)_2$ (0.04 g, 0.05 mmol) in a sealed tube at room temperature. The reaction mixture was stirred at 80 ◦C for 7 h. After completion of the reaction (monitored by TLC), the reaction mixture was cooled to room temperature and solvent was removed under reduced pressure. Crude obtained was dissolved in ethyl acetate (50 mL) and washed with water (2 × 25 mL). The organic layer was separated, dried over anhydrous $Na_2SO_4$, concentrated under reduced pressure. Crude compound was purified by silica gel column chromatography eluting with 15-20% ethyl acetate in n-hexane to afford 30 mg (20% yield) of **VPCFTE-63** as an off-white solid.

**HPLC:** 99.50%. **LCMS-Condition-1:** $[M+H]^+$ = 294.9; $R_t$ = 1.90 min. **$^1$H NMR** (400 MHz, $CDCl_3$) δ: 9.62 (brs, 1 H), 7.78 (d, *J*=7.82 Hz, 2 H), 7.44-7.48 (m, 3 H), 7.38 (d, J=7.34 Hz, 1 H), 7.33 (d, J=3.91 Hz, 1 H), 6.86 (d, *J*=3.42 Hz, 1 H) (2H's merged in solvent peak).

**Synthesis of VPCFTE10 (VPC-18-75)** 2-(2-(furan-2-yl)thiazol-4-yl)furo[3,2-b]pyridine

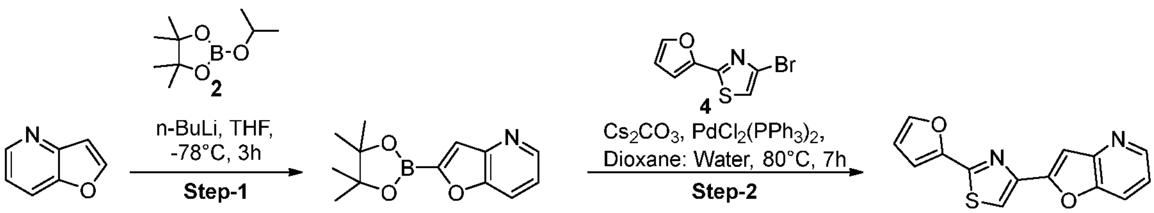


1 3 VPCFTE-10

**Step-1: Synthesis of 2-(4,4,5,5-tetramethyl-1,3,2-dioxaborolan-2-yl)furo[3,2-b]pyridine (3):**

To a stirred solution of furo[3,2-b]pyridine **1** (0.25 g, 2.10 mmol) in THF (10 mL) was added 1.4M n-BuLi in hexane (0.61 mL, 2.73 mmol) at -78°C and stirred for 1.5 h. 2-isopropoxy-4,4,5,5-tetramethyl-1,3,2-dioxaborolane **2** (0.56 mg, 3.15 mmol) was added dropwise at -78 ◦C and the reaction mixture was stirred at same temperature for 1.5h. After completion of the reaction (monitored by TLC), the reaction mixture was quenched with MeOH (0.6 mL), solvent was removed under reduced pressure. The crude compound was purified by trituration with *n*-pentane (25 mL) to afford 0.5 g (crude) of **3** as brown solid. It is carried to next step without purification.

**Step-2: Synthesis of 2-(2-(furan-2-yl)thiazol-4-yl)furo[3,2-b]pyridine (VPCFTE-10):**

To a degassed solution of 2-(4,4,5,5-tetramethyl-1,3,2-dioxaborolan-2-yl)furo[3,2-b]pyridine **3** (0.15 g, 0.66 mmol) and 4-bromo-2-(furan-2-yl)thiazole **4** (0.24 g, 0.98 mmol) in 1,4 dioxane:$H_2O$ (15 mL:4.5 mL) was added $Cs_2CO_3$ (0.43 g, 1.31 mmol) followed by addition of $PdCl_2(PPh_3)_2$ (0.05 g, 0.10 mmol) in a sealed tub. The reaction mixture was stirred at 80 ∘C for 7 h. After completion of the reaction (monitored by TLC), the reaction mixture was cooled to room temperature and solvent was removed under reduced pressure. Crude obtained was dissolved in EtOAc (50 mL) and washed with $H_2O$ (2 × 25 mL). The organic layer was separated, dried over anhydrous $Na_2SO_4$, concentrated under reduced pressure. Crude compound was purified by silica gel column chromatography eluting with 10-13% ethyl acetate in n-hexane to afford 60 mg (34% yield) of **VPCFTE-10** as an off-white solid. **HPLC:** 99.79%. **LCMS-Condition-1:** [M+H]+ = 269.05; $R_t$ = 1.77 min. **$^1$H NMR** (400 MHz, $CDCl_3$) δ: 8.54 (d, *J*=4.40 Hz, 1H), 7.76 (d, *J*=8.31 Hz, 1 H), 7.73 (s, 1 H), 7.55 (s, 1 H) 7.43 (s, 1 H), 7.19 - 7.23 (m, 1 H), 7.14 (d, *J*=3.42 Hz, 1 H), 6.56-6.58 (m, 1 H).

**Synthesis of VPCFTE23 (VPC-18-74)** 3-methyl-2-(5-phenylfuran-2-yl)benzofuran

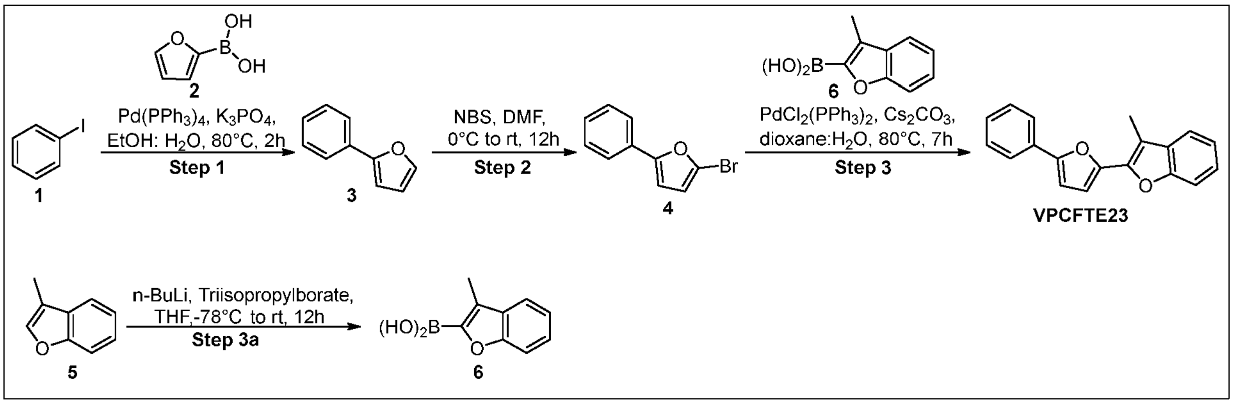


**Step-1: Synthesis of 2-phenylfuran (3):**

To a degassed mixture of iodobenzene **1** (2.00 g, 9.81 mmol) and furan-2-ylboronic acid **2** (1.64 g, 14.7 mmol) in EtOH:$H_2O$ (16 mL:4 mL) under argon atmosphere was added $K_3PO_4$ (4.16 g, 19.6 mmol). The reaction mixture was stirred for 5 min followed by addition of $Pd(PPh_3)_4$ (0.56 g, 0.49 mmol) and the reaction mixture was heated in sealed tube at 80∘C for 2h. After completion of the reaction (monitored by TLC), the reaction mixture was cooled to room temperature and solvent was removed under reduced pressure. The crude compound was purified by column chromatography (silica, 100-200 mesh, 0.5 to 1% EtOAc in hexane) to afford 1.20 g (85% yield) of **3** as a colourless oil. **LCMS-Condition-1:** [M+H]+ = 144.9; $R_t$ = 2.15 min.

**Step-2: Synthesis of 2-bromo-5-phenylfuran (4):**

To a stirred solution of 2-phenylfuran **3** (0.50 g, 3.47 mmol) in DMF (10 mL) was added NBS (0.65 g, 3.64 mmol) portion wise at 0°C. The reaction mixture was stirred at room temperature for 12h. After completion of the reaction (monitored by TLC), the reaction mixture was quenched with ice cold $H_2O$ (25 mL) and extracted with $Et_2O$ (2 × 25 mL). The organic layer was separated, washed with $H_2O$ (25 mL) and solvent was removed under reduced pressure. The crude compound was purified by column chromatography (silica, 100-200 mesh, 0.5 to 1% EtOAc in hexane) to afford 0.64 g (83% yield) of **4** as a light brown oil. $^1$H NMR (400 MHz, $CDCl_3$) δ: 6.40 (d, *J*=3.42 Hz, 1 H), 6.62 (d, *J*=3.42 Hz, 1 H), 7.26 - 7.33 (m, 1H), 7.38-7.42 (m, 2 H), 7.64 (d, *J*=7.34 Hz, 2 H).

**Step-3a: Synthesis of (3-methylbenzofuran-2-yl)boronic acid (6):**

To a stirred solution of 3-methylbenzofuran **5** (0.50 g, 3.79 mmol) in THF (15 mL) was added n-BuLi (1.4 M in hexane, 3.20 mL, 4.54 mmol) at -78°C and the reaction mixture was stirred at 0°C for 1h. Triisopropyl borate (2.14 g, 11.4 mmol) was added at -78°C. The reaction mixture was stirred at room temperature for 12h. After completion of the reaction (monitored by TLC), the reaction mixture was quenched with 1 *N* HCl and pH adjusted to 5. The reaction mixture then stirred at room temperature for 30 min and extracted with EtOAc (3 × 25 mL). The organic layer was separated,

washed with $H_2O$ (25 mL), dried over anhydrous $Na_2SO_4$ and solvent was removed under reduced pressure. The crude compound was triturated with $Et_2O$ (25 mL) and pentane (25 mL) to afford 0.30 g (45% yield) of **6** as an off-white solid.

**Step-3: Synthesis of 3-methyl-2-(5-phenylfuran-2-yl)benzofuran (VPCFTE23):**

To a degassed mixture of 2-bromo-5-phenylfuran **4** (0.10 g, 0.45 mmol) and (3-methylbenzofuran-2-yl)boronic acid **6** (0.12 g, 0.68 mmol) in 1,4 dioxane:$H_2O$ (10 mL:3 mL) in a sealed tube was added $Cs_2CO_3$ (0.39 g, 0.90 mmol) followed by addition of $PdCl_2(PPh_3)_2$ (0.02 g, 0.02 mmol) at room temperature. The reaction mixture was then heated 80◦C for 7h. After completion of the reaction (monitored by TLC), the reaction mixture was cooled to room temperature and solvent was removed under reduced pressure. The crude compound was purified by prep HPLC to afford 0.04 g (32% yield) of **VPCFTE23** as an off-white solid.

**HPLC:** 99.56%. **LCMS-Condition-1:** [M+H]+ = 275.05; $R_t$ = 2.61 min. $^1$**H NMR** (400 MHz, $CDCl_3$) δ: 7.78 (d, *J*=7.6 Hz, 2 H), 7.56 (d, *J*=7.2 Hz, 1 H), 7.42 - 7.51 (m, 3 H), 7.28 - 7.35 (m, 3 H), 6.84 - 6.88 (m, 1 H), 6.81-6.83 (m, 1 H), 2.62 (s, 3 H).

**Synthesis of VPCFTE25 (VPC-18-88)** 2-(2-(furan-2-yl)thiazol-4-yl)benzofuran-3-carbonitrile

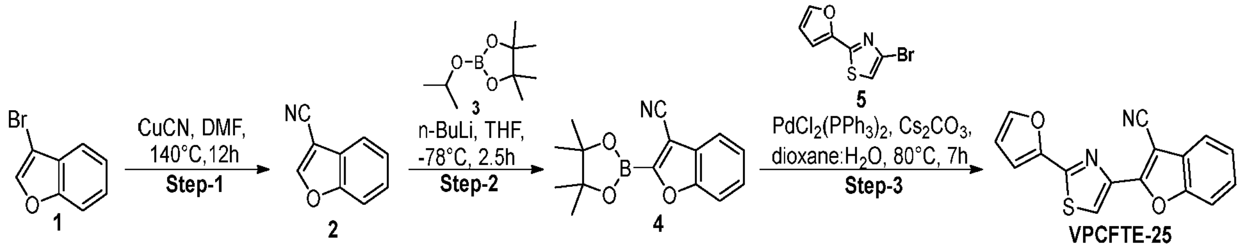


**Step-1: Synthesis of benzofuran-3-carbonitrile (2):**

To a degassed mixture of 3-bromobenzofuran **1** (0.75 g, 3.83 mmol) in dry DMF (1.5 mL) was added CuCN (0.69 g, 7.66 mmol) under argon atmosphere at room temperature. The reaction mixture was heated in sealed tube at 140°C for 12h. After completion of the reaction (monitored by TLC), the reaction mixture was cooled to room temperature, diluted with $Et_2O$ (150 mL) and $H_2O$ (100 mL) and filtered through the pad of Celite®. The organic layer was separated, dried over anhydrous $Na_2SO_4$ and solvent was removed under reduced pressure. The crude compound was purified by column chromatography (silica, 100-200 mesh, 0.5 to 1% EtOAc in hexane) to afford 0.22 g (40% yield) of **2** as a yellow solid.

$^1$**H NMR** (400 MHz, $CDCl_3$) δ: 7.41 - 7.50 (m, 2 H), 7.60 - 7.64 (m, 1 H), 7.75 - 7.80 (m, 1 H), 8.17 (s, 1 H).

**Step-2: Synthesis of 2-(4,4,5,5-tetramethyl-1,3,2-dioxaborolan-2-yl)benzofuran-3-carbonitrile (4):**

To a stirred solution of benzofuran-3-carbonitrile **2** (0.15 g, 1.05 mmol) in THF (6 mL) was added n-BuLi (1.4 M in hexane, 0.97 mL, 1.36 mmol) at -78°C and the reaction mixture was stirred at same temperature for 1.5h. 2-isopropoxy-4,4,5,5-tetramethyl-1,3,2-dioxaborolane **3** (2.14 g, 11.4 mmol) was added drop wise at -78°C and the reaction mixture was stirred at same temperature for 2.5h. After completion of the reaction (monitored by TLC), the reaction mixture was quenched with MeOH (0.3 mL) and solvent was removed under reduced pressure. The crude compound was triturated with pentane to afford 0.255 g (97% yield) of **4** as a red solid.

**Step-3: Synthesis of 2-(2-(furan-2-yl)thiazol-4-yl)benzofuran-3-carbonitrile (V-PCFTE-25):**

To a degassed mixture of 2-(4,4,5,5-tetramethyl-1,3,2-dioxaborolan-2-yl)benzofuran-3-carbonitrile **4** (0.15 g, 0.66 mmol) and 4-bromo-2-(furan-2-yl)thiazole **5** (0.23 g, 0.85 mmol) in 1,4 dioxane:$H_2O$ (15 mL:4.5 mL) was added $Cs_2CO_3$ (0.43 g, 1.31 mmol) followed by addition of $PdCl_2(PPh_3)_2$ (0.05 g, 0.07 mmol) in a sealed tube under argon atmosphere at room temperature. The reaction mixture was heated 80°C for 7h. After completion of the reaction (monitored by TLC), the reaction mixture was cooled to room temperature and solvent was removed under reduced pressure. The residue was dissolved in EtOAc (50 mL) and washed with $H_2O$ (2 × 25 mL). The organic layer was separated, dried over anhydrous $Na_2SO_4$ and solvent was removed under reduced pressure. The crude compound was purified by column chromatography (silica, 100-200 mesh, 0.5 to 1% EtOAc in hexanes) to afford 0.038 g (19% yield) of **VPCFTE-25** as

an off-white solid. **HPLC:** 95.35%. **LCMS-Condition-1:** [M+H]+ = 292.95; $R_t$ = 2.43 min.

**$^1$H NMR** (400 MHz, $CDCl_3$) δ: 8.06 (s, 1 H) 7.76 - 7.80 (m, 1 H) 7.63 (d, *J*=8.31 Hz, 1 H) 7.59 (d, *J*=0.98 Hz, 1H) 7.41 - 7.49 (m, 2 H) 6.60-6.62 (m, 1 H) (1H merged in solvent peak).

**Synthesis of VPCFTE44 (VPC-18-90) 5-(benzofuran-2-yl)-2-(furan-2-yl)thiazole**

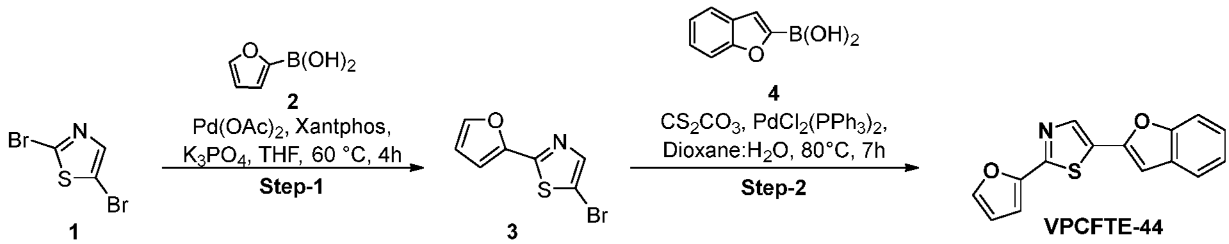


1 3 VPCFTE-44

**Step-1: Synthesis of 5-bromo-2-(furan-2-yl)thiazole (3):**

To a degassed solution of 2,5-dibromothiazole**1** (1.00 g, 4.12 mmol) and furan-2-ylboronic acid **2** (0.50 g, 4.47 mmol) in THF (20 mL) was added and $K_3PO_4$ (1.70 g, 8.01 mmol) in a sealed tube and stirred for 5min. $Pd(OAc)_2$ (0.09 mg, 0.41 mmol) was added at room temperature followed by addition of xantphos (0.24 g, 0.41 mmol) and the reaction mixture was heated at 60 °C for 4 h. After completion of the reaction (monitored by TLC), the reaction mixture was cooled to room temperature and solvent was removed under reduced pressure. Crude compound was purified by silica gel column chromatography eluting with 5% ethyl acetate in n-hexane to afford 0.30 g (32% yield) of **3** as an off-white solid.

**LCMS-Condition-1:** $[M+H]^+$ = 229.90; $R_t$ = 2.24 min.

**Step-2: Synthesis of 5-(benzofuran-2-yl)-2-(furan-2-yl)thiazole (VPCFTE-44):**

To a degassed solution of 5-bromo-2-(furan-2-yl)thiazole **3** (0.15 g, 0.65 mmol) and benzofuran-2-ylboronic acid **4** (0.16 g, 0.99 mmol) in 1,4 dioxane:$H_2O$ (12 mL:3 mL) was added $Cs_2CO_3$ (0.43 g, 1.31 mmol) followed by addition of $PdCl_2(PPh_3)_2$ (0.05 g, 0.07 mmol) in a sealed tube at room temperature. The reaction mixture was stirred at 80 °C for 7 h. After completion of the reaction (monitored by TLC), the reaction mixture was cooled to room temperature and solvent was removed under reduced pressure. Crude compound was purified by silica gel column chromatography eluting with 25-30% ethyl acetate in n-hexane followed by prep HPLC to afford 40 mg (23% yield) of **VPCFTE-44** as an off-white solid.

**HPLC:** 97.66%. **LCMS-Condition-1:** [M+H]+ = 267.90; $R_t$ = 2.19 min. **$^1$H NMR** (400 MHz, $CDCl_3$) δ: 8.16 (s, 1 H), 7.54 - 7.59 (m, 2 H), 7.51 (d, *J*=8.31 Hz, 1H), 7.29 - 7.34 (m, 1 H), 7.23 - 7.28 (m, 1H), 7.07 (d, *J*=3.42 Hz, 1H), 6.94 (s, 1H), 6.57-6.59 (m, 1H).

**Synthesis of VPCFTE60 (VPC-18-96)** 4-(6-chlorobenzofuran-2-yl)-2-(1H-pyrazol-5-yl)thiazole

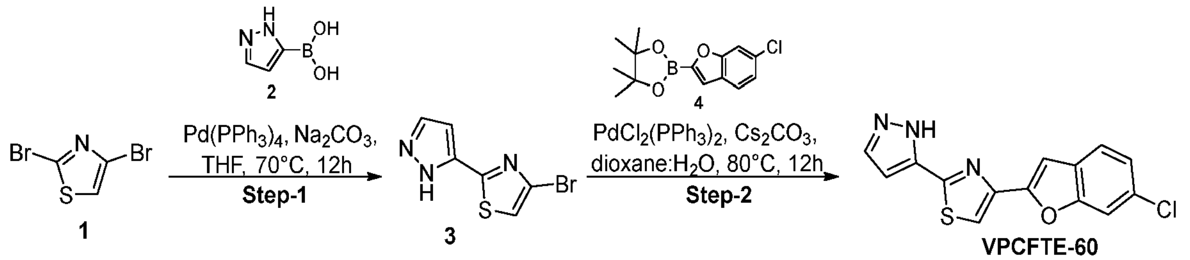


1 3 VPCFTE-60

**Step-1: Synthesis of 4-bromo-2-(1H-pyrazol-5-yl)thiazole (3):**

To a degassed mixture of 2,4-dibromothiazole **1** (1.00 g, 4.15 mmol) and (1*H*-pyrazol-5-yl)boronic acid **2** (0.51 g, 4.57 mmol) in THF (20 mL) under argon atmosphere was added $Na_2CO_3$ (0.88 g, 8.30 mmol). The reaction mixture

was stirred for 5 min followed by addition of $Pd(PPh_3)_4$ (0.24 g, 0.21 mmol) and the reaction mixture was heated in sealed tube at 70°C for 12h. After completion of the reaction (monitored by TLC), the reaction mixture was cooled to room temperature and solvent was removed under reduced pressure. The crude compound was purified by column chromatography (silica, 100-200 mesh, 15 to 20% EtOAc in hexane) to afford 0.12 g (13% yield) of **3** as an off-white solid. **LCMS-Condition-1:** [M+H]+ = 229.8; $R_t$ = 1.85 min.

**Step-2: Synthesis of 4-(6-chlorobenzofuran-2-yl)-2-(1H-pyrazol-5-yl)thiazole (VPCFTE-60):**

To a degassed mixture of 4-bromo-2-(1*H*-pyrazol-5-yl)thiazole **3** (0.10 g, 0.44 mmol) and 2-(6-chlorobenzofuran-2-yl)-4,4,5,5-tetramethyl-1,3,2-dioxaborolane **4** (0.16 g, 0.57 mmol) in 1,4 dioxane:$H_2O$ (10 mL:3 mL) was added $Cs_2CO_3$ (0.28 g, 0.87 mmol) followed by addition of $PdCl_2(PPh_3)_2$ (0.03 g, 0.04 mmol) in a sealed tube under argon atmosphere at room temperature. The reaction mixture was heated 80°C for 12h. After completion of the reaction (monitored by TLC), the reaction mixture was cooled to room temperature and solvent was removed under reduced pressure. The residue was dissolved in EtOAc (50 mL) and washed with $H_2O$ (2 × 25 mL). The organic layer was separated, dried over anhydrous $Na_2SO_4$ and solvent was removed under reduced pressure. The crude compound was purified by column chromatography (silica, 100-200 mesh, 15 to 20% EtOAc in hexane) to afford 0.01 g (7% yield) of **VPCFTE60** as an off-white solid. **HPLC:** 99.39%. **LCMS-Condition-1:** [M+H]+ = 301.85; $R_t$ = 2.20 min. **$^1$H NMR** (400 MHz, DMSO-d6) δ: 13.36 (brs, 1 H), 8.08 (s, 1 H), 7.94 (s, 1 H), 7.83 (s, 1 H), 7.72 (d, J=8.37 Hz, 1 H), 7.36 (s, 1H), 7.34 (d, J=1.48 Hz, 1 H), 6.85 (d, J=1.97 Hz, 1H).

**Synthesis of VPCFTE63 (VPC-18-92)** 6-chloro-2-(5-phenylfuran-2-yl)-1H-benzo[d]imidazole

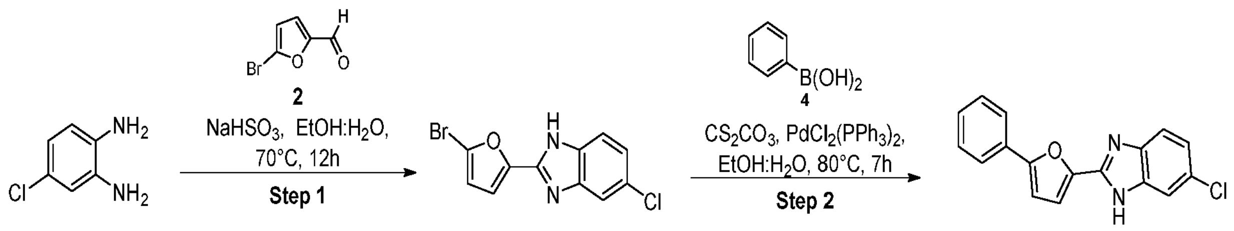


1 3 VPCFTE-63

**Step-1: Synthesis of 2-(5-bromofuran-a-yl)-5-chloro-1*H*-benzo[d]imidazole (3):**

To a stirred solution of 4-chlorobenzene-1,2-diamine **1** (1.00 g, 7.04 mmol) and 5-bromofuran-2-carbaldehyde **2** (1.84 g, 10.56 mmol) in EtOH:$H_2O$ (10 mL:10 mL) was added $NaHSO_3$ (1.47 g, 14.08 mmol) in sealed tube at room temperature. The reaction mixture was stirred at 70 °C for 12 h. After completion of the reaction (monitored by TLC), the reaction mixture was cooled to room temperature and solvent was removed under reduced pressure. Crude compound was purified by silica gel column chromatography eluting with 5-8% ethyl acetate in *n*-hexane to afford 0.72 g (35% yield) of **3** as an off-white solid.

**LCMS-Condition-1:** $[M+2+H]^+$ = 298.8; $R_t$ = 1.76 min

**Step-2: Synthesis of 6-chloro-2-(5-phenylfuran-2-yl)-1*H*-benzo[d]imidazole (VPCFTE-63):**

To a degassed solution of 2-(5-bromofuran-2-yl)-5-chloro-1*H*-benzo[d]imidazole **3** (0.15 g, 0.51 mmol) and phenylboronic acid **4** (0.90 g, 0.76 mmol) in EtOH:$H_2O$ (15 mL:4.5 mL) was added $Cs_2CO_3$ (0.33 g, 1.01 mmol) followed by addition of $PdCl_2(PPh_3)_2$ (0.04 g, 0.05 mmol) in a sealed tube at room temperature. The reaction mixture was stirred at 80 °C for 7 h. After completion of the reaction (monitored by TLC), the reaction mixture was cooled to room temperature and solvent was removed under reduced pressure. Crude obtained was dissolved in ethyl acetate (50 mL) and washed with water (2 × 25 mL). The organic layer was separated, dried over anhydrous $Na_2SO_4$, concentrated under reduced pressure. Crude compound was purified by silica gel column chromatography eluting with 15-20% ethyl acetate in n-hexane to afford 30 mg (20% yield) of **VPCFTE-63** as an off-white solid. **HPLC:** 99.50%. **LCMS-Condition-1:** $[M+H]^+$ = 294.9; $R_t$ = 1.90 min. **$^1$H NMR** (400 MHz, $CDCl_3$) δ: 9.62 (brs, 1H), 7.78 (d, *J*=7.82 Hz, **2** H), 7.44-7.48 (m, **3** H), 7.38 (d, *J*=7.34 Hz, 1 H), 7.33 (d, *J*=3.91 Hz, 1H), 6.86 (d, *J*=3.42 Hz, 1 H) (2H's merged in solvent peak).

**Synthesis of Compound-01 (VPC-18-30)** 1-Methyl-6-nitro-2-(5-phenylfuran-2-yl)-1*H*-benzo[*d*]imidazole

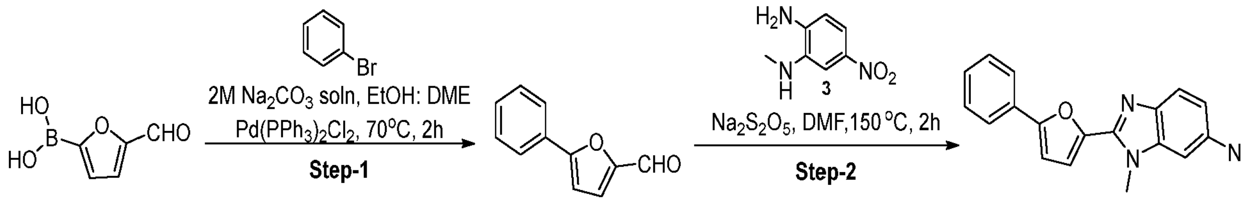


1 2 Compound-01

**Step-1: Synthesis of 5-phenylfuran-2-carbaldehyde (2):**

To a solution of (5-formylfuran-2-yl)boronic acid **1** (1 g, 7.147 mmol) in EtOH:DME (2:1, 18 mL) was added bromobenzene (897 mg, 5.718 mmol) and 2M $Na_2CO_3$ solution (7.08 mL, 14.29 mmol) and degassed with argon for 20 min. To the resulting solution was added $PdC1_2(PPh_3)_2$ (250 mg, 0.357 mmol) and degassed with argon for another 10 min at room temperature. The reaction mixture was then heated 70 °C for **2** h. After completion of the reaction (monitored by TCL and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure and the crude compound was purified by silica gel column chromatography eluting with 0-5% ethyl acetate in *n*-hexane to afford 600 mg (50% yield) of **Int 2** as pale yellow oil. **LCMS-Condition-1:** [M+H]+ = 173.15; $R_t$ = 1.70 min. **$^1$H NMR** (400 MHz, $CDCl_3$) δ: 9.66 (s, 1H), 7.83 (d, *J* = 7.34 Hz, 2H), 7.37 - 7.48 (m, 3H), 7.33 (d, *J* = 3.91 Hz, 1H), 6.85 (d, *J* = 3.42 Hz, 1H).

**Step-2: Synthesis of 1-methyl-6-nitro-2-(5-phenylfuran-2-yl)-1*H*-benzo[d]imidazole (Compound-1):**

To a solution of 5-phenylfuran-2-carbaldehyde **2** (150 mg, 0.872 mmol) in DMF (2 mL) was added $Na_2S_2O_5$ (198 mg, 1.042 mmol) and *N*1-methyl-5-nitrobenzene-1,2-diamine **3** (145 mg, 0.868 mmol) at room temperature. The reaction mixture was heated at 150 °C for 2 h. After completion of the reaction (monitored by TCL and LCMS), the reaction mixture was cooled to room temperature and diluted with water (20 mL). The precipitated solid was filtered and washed with water (10 mL) and n-hexane (10 mL) resulting in the crude compound. The crude compound was purified by silica gel column chromatography eluting with 0-5% methanol in DCM to afford 50 mg (18% yield) of **Compound 1** as pale yellow solid.

**LCMS-Condition-1:** [M+H]+ = 320.10; $R_t$ = 2.18 min.

**$^1$H NMR** (400 MHz, DMSO-$d_6$) δ: 8.71 (d, *J* = 2.45 Hz, 1H), 8.13 - 8.18 (m, 1H), 7.91 (d, *J* = 7.34 Hz, 2H), 7.83 (d, *J* = 8.80 Hz, 1H), 7.57 (d, *J* = 3.42 Hz, 1H), 7.53 (t, *J* = 7.83 Hz, 2H), 7.38 - 7.45 (m, 1H), 7.33 (d, *J* = 3.91 Hz, 1H), 4.27 (s, 3H).

**Synthesis of Compound-02(VPC-18-39)** 2-(5-(2,4-Difluorophenyl)furan-2-yl)-1-methyl-6-nitro-1*H*-benzo [d]imidazole

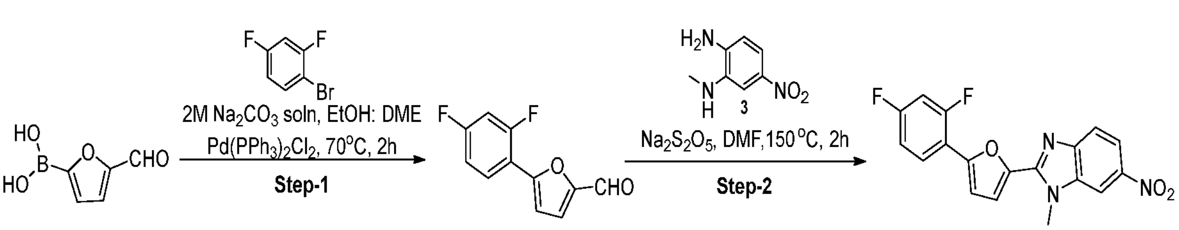


1 2 Compound-02

**Step-1: Synthesis of 5-(2,4-difluorophenyl)furan-2-carbaldehyde (2):**

To a solution of (5-formylfuran-2-yl)boronic acid **1** (1 g, 7.147 mmol) in EtOH:DME (2:1, 18 mL) was added 1-bromo-2,4-difluorobenzene (1.1 g, 5.718 mmol) and 2M $Na_2CO_3$ solution (7.1 mL, 14.29 mmol) and degassed with argon for 20 min. To the resulting solution was added $Pd(PPh_3)_2Cl_2$ (250 mg, 0.357 mmol) and degassed with argon for another

10 min at room temperature. The reaction mixture was then heated at 70 °C for 2 h. After completion of the reaction (monitored by TLC and LCMS), the reaction mixture was cooled to room temperature and diluted with water (50 mL) and extracted with ethyl acetate (3 times). The combined organic layer was dried over anhydrous $Na_2SO_4$, filtered and concentrated under reduced pressure; the crude compound was purified by silica gel column chromatography eluting with 0-5% ethyl acetate in *n*-hexane to afford 600 mg (40% yield) **of Int 2** as off white solid. **LCMS-Condition-1:** [M+H]+ = 209.15; $R_t$ = 1.80 min. **$^1$H NMR** (400 MHz, $CDCl_3$) δ: 9.67 (s, 1H), 8.01 (dt, *J* = 6.36, 8.56 Hz, 1H), 7.34 (d, *J* = 3.91 Hz, 1H), 6.90-7.04 (m, 3H).

**Step-2: Synthesis of 2-(5-(2,4-difluorophenyl)furan-2-yl)-1-methyl-6-nitro-1*H*-benzo[d]imidazole (Compound-2):**

To a solution of **5-(2,4-difluorophenyl)furan-2-carbaldehyde 2** (200 mg, 0.961 mmol) in DMF (4 mL) was added $Na_2S_2O_5$ (219 mg, 1.153 mmol) and *N*1-methyl-5-nitrobenzene-1,2-diamine **3** (160 mg, 0.961 mmol) at room temperature. The reaction mixture was heated at 150 °C for 2 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by preparative HPLC to afford 30 mg (9% yield) of **Compound 02** as white solid.

**LCMS-Condition-1:** [M+H]+ = 356.05; $R_t$ = 2.03 min. **$^1$H NMR** (400 MHz, DMSO-*d*$_6$) δ: 8.72 (d, *J* = 2.45 Hz, 1H), 8.15 (dd, *J* = 1.96, 8.80 Hz, 1H), 8.04 (dt, *J* = 6.60, 8.93 Hz, 1H), 7.83 (d, *J* = 8.80 Hz, 1H), 7.59 (d, *J* = 3.42 Hz, 1H), 7.50 (ddd, *J* = 2.45, 9.29, 11.74 Hz, 1H), 7.31 (dt, *J* = 2.45, 8.56 Hz, 1H), 7.15 (t, *J* = 3.42 Hz, 1H), 4.26 (s, 3H).

**Synthesis of Compound-03 (VPC-18-34)** 2-(5-(4-Bromo-2-chlorophenyl)furan-2-yl)-1-methyl-6-(trifluoromethyl)-1*H*-benzo[d]imidazole

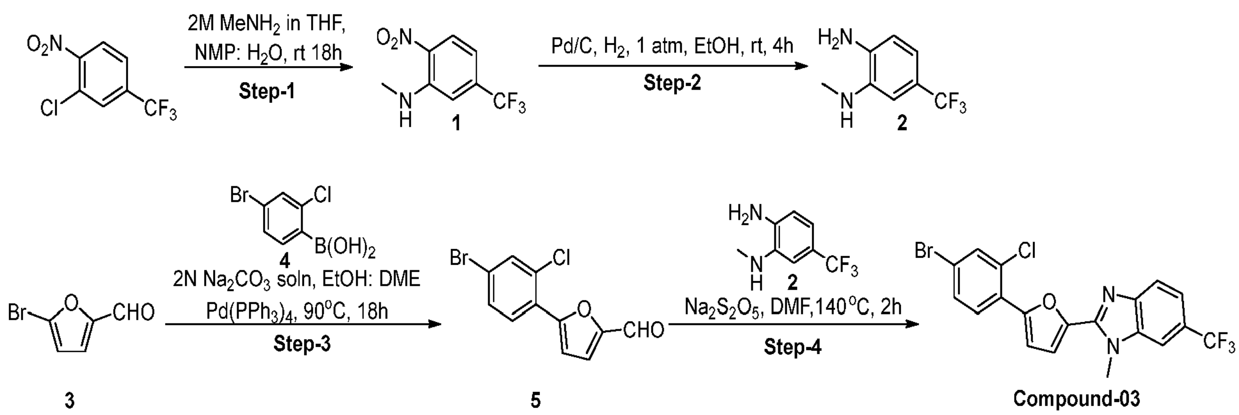


**Step-1: Synthesis of *N*1-methyl-5-(trifluoromethyl)benzene-1,2-diamine (1):**

To a solution of 2-chloro-1-nitro-4-(trifluoromethyl)benzene (1 g, 4.433 mmol) in NMP (5 mL) was added 2M solution of methyl amine in THF (2.8 mL, 5.763 mmol) and water (2 mL) at room temperature in a sealed tube. The reaction mixture was sealed properly and stirred at room temperature for 18 h. After completion of the reaction (monitored by TLC and LCMS), the reaction mixture was diluted with water (50 mL) and extracted with ethyl acetate (3 × 50 mL). The combined organic layer was washed with brine, dried over anhydrous $Na_2SO_4$, filtered and concentrated under reduced pressure and the crude compound was purified by Combiflash column chromatography eluting with 10-15% ethyl acetate in *n*-hexane to afford 400 mg (41% yield) of **Int 1** as brown solid.

**Step-2: Synthesis of *N*1-methyl-5-(trifluoromethyl)benzene-1,2-diamine (2):**

To a solution of N-methyl-2-nitro-5-(trifluoromethyl)aniline **1** (400 mg, 1.817 mmol) in ethanol (10 mL) was added 10% palladium on carbon (40 mg, 50% moisture) at room temperature. The reaction mixture was then stirred under hydrogen atmosphere under balloon pressure for 4 h. After completion of the reaction, the reaction mixture was filtered through a pad of Celite and washed with ethanol. The filtrate was concentrated under reduced pressure to afford 280 mg (81% yield) of **Int 2** as brown oil.

**LCMS-Condition-1:** [M+H]+ = 191.00; $R_t$ = 1.84 min. **$^1$H NMR** (400 MHz, $CDCl_3$) δ: 6.95 (d, *J* = 8.31 Hz, 1H),

6.83 (s, 1H), 6.72 (d, *J* = 7.82 Hz, 1H), 3.55 (br. s, 2H), 3.39 (br. s, 1H), 2.89 (s, 3H).

**Step-3: Synthesis of 5-(4-bromo-2-chlorophenyl)furan-2-carbaldehyde (5):**

To a solution of 5-bromofuran-2-carbaldehyde **3** (2 g, 14.29 mmol) in EtOH:DME (2:1, 30 mL) was added (4-bromo-2-chlorophenyl)boronic acid **4** (4.5 g, 14.29 mmol) and 2N $Na_2CO_3$ solution (5 mL, 10.00 mmol) and degassed with argon for 20 min. To the resulting solution was added $Pd(PPh_3)_4$ (826 mg, 0.714 mmol) and degassing was continued for another 10 min at room temperature. The reaction mixture was further heated at 90 °C for 18 h. After completion of the reaction (monitored by TLC and LCMS), the reaction mixture was cooled to room temperature, diluted with water (50 mL) and extracted with ethyl acetate (3 times). The combined organic layer was washed with brine, dried over anhydrous $Na_2SO_4$, filtered and concentrated under reduced pressure and the crude compound was purified by Combiflash column chromatography eluting with 0-3% ethyl acetate in n-hexane to afford 240 mg (6% yield) of **5** as pale yellow solid. **LCMS-Condition-1:** [M+H]+ = 286.90; $R_t$ = 2.28 min. **$^1$H NMR** (400 MHz, $CDCl_3$) δ: 9.70 (s, 1H), 7.90 (d, *J* = 8.80 Hz, 1H), 7.67 (d, *J* = 1.96 Hz, 1H), 7.52 (dd, *J* = 1.96, 8.31 Hz, 1H), 7.31-7.36 (m, 2H).

**Step-4: Synthesis of 2-(5-(4-bromo-2-chlorophenyl)furan-2-yl)-1-methyl-6-(trifluoromethyl)-1*H*-benzo[*d*]imidazole (Compound-03):**

To a solution of **5-(4-bromo-2-chlorophenyl)furan-2-carbaldehyde 5** (100 mg, 0.350 mmol) in DMF (2 mL) was added $Na_2S_2O_5$ (66.5 mg, 0.350 mmol) and *N*1-methyl-5-(trifluoromethyl)benzene-1,2-diamine **2** (66.5 mg, 0.350 mmol) at room temperature. The reaction mixture was heated at 140 °C for 2 h. After completion of the reaction (monitored by TLC and LCMS), the reaction mixture was cooled to room temperature, quenched with water (25 mL) and extracted with ethyl acetate (3 times). The combined organic layer was washed with brine, dried over anhydrous $Na_2SO_4$, filtered and concentrated under reduced pressure and the crude compound was purified by Combiflash column chromatography eluting with 10-15% ethyl acetate in n-hexane to afford 21 mg (13% yield) of **Compound 03** as off white solid.

**LCMS-Condition-1:** $[M+H]^+$ = 457.05; $R_t$ = 2.57 min.

**$^1$H NMR** (400 MHz, $CDCl_3$) δ: 7.87 (d, *J* = 8.80 Hz, 1H), 7.80 (d, *J* = 8.31 Hz, 1H), 7.69 (d, *J* = 1.96 Hz, 2H), 7.57 (d, *J* = 8.31 Hz, 1H), 7.53 (dd, *J* = 1.96, 8.80 Hz, 1H), 7.40 (d, *J* = 3.91 Hz, 1H), 7.33 (d, *J* = 3.91 Hz, 1H), 4.19 (s, 3H).

**Synthesis of Compound-04 (VPC-18-40)** 2-(5-(4-Bromo-2-chlorophenyl)furan-2-yl)-6-nitro-1*H*-benzo[*d*]imidazole

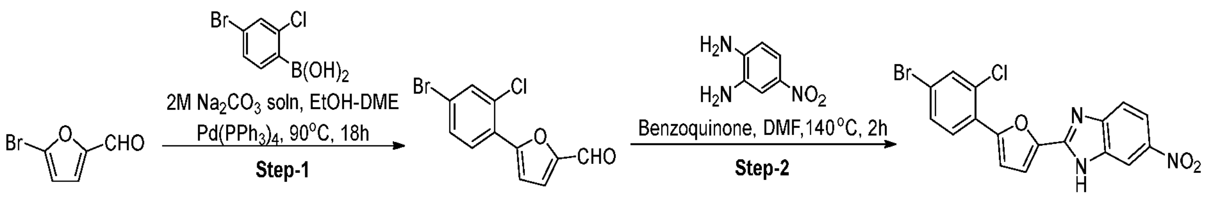


1

2

Compound-04

**Step-1: Synthesis of 5-(4-bromo-2-chlorophenyl)furan-2-carbaldehyde (2):**

To a solution of 5-bromofuran-2-carbaldehyde **1** (2 g, 14.29 mmol) in EtOH:DME (2:1, 30 mL) was added (4-bromo-2-chlorophenyl)boronic acid (4.53 g, 14.29 mmol) and 2M $Na_2CO_3$ solution (14 mL, 28.59 mmol) and degassed with argon for 15 min. To the resulting solution was added $Pd(PPh_3)_4$ (826 mg, 0.714 mmol) and degassed with argon for another 10 min at room temperature. The reaction mixture was heated at 90 °C for 18 h. After completion of the reaction (monitored by TLC and LCMS), the reaction mixture was cooled to room temperature, quenched with water (50 mL) and extracted with ethyl acetate (3 times). The combined organic layer was washed with water and brine, dried over anhydrous $Na_2SO_4$, filtered and concentrated under reduced pressure; the crude compound was purified by Combiflash column chromatography eluting with 0-3% ethyl acetate in n-hexane to afford 240 mg (6% yield) of **Int 2** as pale yellow solid. **LCMS-Condition-1:** $[M+H]^+$ = 284.95; $R_t$ = 2.29 min. **$^1$H NMR** (400 MHz, $CDCl_3$) δ: 9.70 (s, 1H), 7.90 (d, *J* = 8.80 Hz, 1H), 7.67 (d, *J* = 1.96 Hz, 1H), 7.52 (dd, *J* = 1.96, 8.31 Hz, 1H), 7.31-7.37 (m, 2H).

**Step-2: Synthesis of 2-(5-(4-bromo-2-chlorophenyl)furan-2-yl)-6-nitro-1*H*-benzo[d]imidazole (Compound-4):**

To a solution of **5-(4-bromo-2-chlorophenyl)furan-2-carbaldehyde 2** (100 mg, 0.351 mmol) and 4-nitrobenzene-1,2-diamine **3** (53.6 mg, 0.351 mmol) in DMF (2 mL) was added benzoquinone (37.8 mg, 0.351 mmol) at room temperature. The reaction mixture was heated at 140 °C for **2** h. After completion of the reaction, the reaction mixture was cooled to room temperature, quenched with water (20 mL) and extracted with ethyl acetate (3 times). The combined organic layer was washed with water and brine, dried over anhydrous $Na_2SO_4$, filtered and concentrated under reduced pressure; the crude compound was purified by Combiflash column chromatography eluting with 0-1% methanol in DCM to afford 27 mg (18% yield) of **Compound 4** as yellow solid. **LCMS-Condition-1:** $[M+H]^+$ = 419.95; $R_t$ = 2.18 min. **$^1$H NMR** (400 MHz, DMSO-*d*$_6$) δ: 13.81 (br. s, 1H), 8.49 (br. s, 1H), 8.15-8.18 (m, 1H), 8.13 (d, *J* = 8.31 Hz, 1H), 7.94 (d, *J* = 1.96 Hz, 1H), 7.81 (dd, *J* = 1.96, 8.31 Hz, 2H), 7.46-7.53 (m, 2H).

**Synthesis of Compound-05 (VPC-18-41)** 2-(5-(2,4-Difluorophenyl)furan-2-yl)-1-methyl-1*H*-benzo[d]imidazole

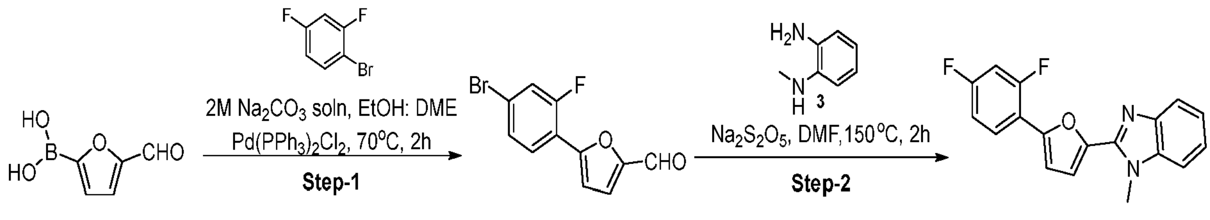


1 2 Compound-05

**Step-1: Synthesis of 5-(2,4-difluorophenyl)furan-2-carbaldehyde (2):**

To a solution of (5-formylfuran-2-yl)boronic acid **1** (1 g, 7.147 mmol) in EtOH:DME (2:1, 18 mL) was added 1-bromo-2,4-difluorobenzene (1.1 g, 5.718 mmol) and 2M $Na_2CO_3$ solution (7.1 mL, 14.29 mmol) and degassed with argon for 20 min. To the resulting solution was added $Pd(PPh_3)_2Cl_2$ (250 mg, 0.357 mmol) and degassed with argon for another 10 min at room temperature. The reaction mixture was then heated at 70 °C for 2 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by silica gel column chromatography eluting with 0-5% ethyl acetate in n-hexane to afford 600 mg (40% yield) of **Int 2** as off white solid. **LCMS-Condition-1:** $[M+H]^+$ = 209.15; $R_t$ = 1.80 min. **$^1$H NMR** (400 MHz, $CDCl_3$) δ: 9.67 (s, 1H), 8.01 (dt, *J* = 6.36, 8.56 Hz, 1H), 7.34 (d, *J* = 3.91 Hz, 1H), 6.90-7.04 (m, 3H).

**Step-2: Synthesis of 2-(5-(2,4-difluorophenyl)furan-2-yl)-1-methyl-1*H*-benzo[d]imidazole (Compound-5):**

To a solution of 5-(4-bromo-2-fluorophenyl)furan-2-carbaldehyde **2** (200 mg, 0.961 mmol) in DMF (4 mL) was added $Na_2S_2O_5$ (219 mg, 1.153 mmol) and *N*1-methylbenzene-1,2-diamine **3** (117 mg, 0.961 mmol) at room temperature. The reaction mixture was heated at 150 °C for 2 h. After completion of the reaction (monitored by TLC and LCMS), the reaction mixture was cooled to room temperature and diluted with water (20 mL). The precipitated solid was filtered, washed with water (10 mL) and n-hexane (10 mL) which was purified by silica gel column chromatography eluting with 0-5% methanol in DCM to afford 50 mg (17% yield) of **Compound 5** as pale yellow solid. **LCMS-Condition-1:** $[M+H]^+$ = 311.05; $R_t$ = 1.70 min. **$^1$H NMR** (400 MHz, DMSO-*d*$_6$) δ: 8.02 (dt, *J* = 6.36, 8.80 Hz, 1H), 7.67 (dd, *J* = 4.89, 7.34 Hz, 2H), 7.48 (ddd, *J* = 2.69, 9.17,11.62 Hz, 1H), 7.40 (d, *J* = 3.42 Hz, 1H), 7.23-7.34 (m, 3H), 7.10 (t, *J* = 3.42 Hz, 1H), 4.12 (s, 3H).

**Synthesis of Compound-06 (VPC-18-31)** 4-Fluoro-2-(5-(2-fluorophenyl)furan-2-yl)-1-methyl-1*H*-benzo[*d*]imidazole

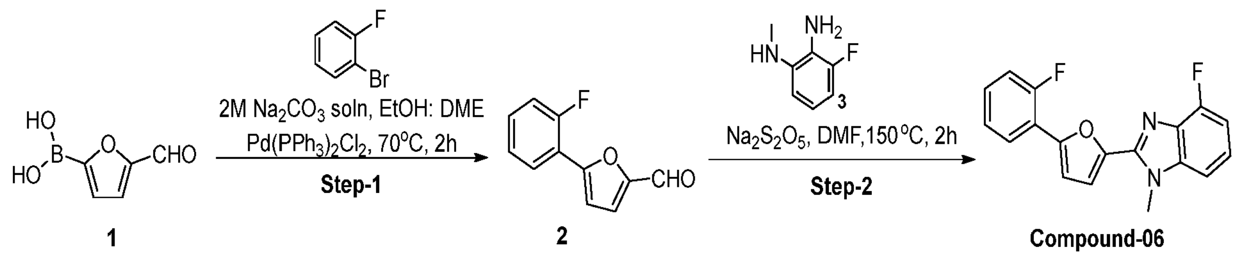


**Step-1: Synthesis of 5-(2-fluorophenyl)furan-2-carbaldehyde (2):**

To a solution of (5-formylfuran-2-yl)boronic acid **1** (1 g, 7.147 mmol) in EtOH:DME (2:1, 18 mL) was added 1-bromo-2-fluorobenzene (999 mg, 5.708 mmol) and 2M $Na_2CO_3$ solution (7.08 mL, 14.29 mmol) and degassed with argon for 20 min. To the resulting solution was added $PdCl_2(PPh_3)_2$ (250 mg, 0.357 mmol) and degassed with argon for another 10 min at room temperature. The reaction mixture was then heated at 70 °C for 2 h. After completion of the reaction (monitored by TCL and LCMS), the reaction mixture was cooled to room temperature, diluted with water (25 mL) and extracted with ethyl acetate (3 times). The combined organic layer was dried over anhydrous $Na_2SO_4$, filtered and concentrated under reduced pressure and the crude compound was purified by silica gel column chromatography eluting with 0-5% ethyl acetate in n-hexane to afford 350 mg (26% yield) of **Int 2** as pale yellow solid. **LCMS-Condition-1:** [M+H]+ = 190.95; $R_t$ = 1.76 min. **$^1$H NMR** (400 MHz, $CDCl_3$) δ: 9.69 (s, 1H), 8.02 (dt, *J* = 1.71, 7.70 Hz, 1H), 7.37 - 7.41 (m, 1H), 7.35 (d, *J* = 3.42 Hz, 1H), 7.23 - 7.29 (m, 1H), 7.13 - 7.21 (m, 1H), 7.03 (t, *J* = 3.67 Hz, 1H).

**Step-2: Synthesis of 4-fluoro-2-(5-(2-fluorophenyl)furan-2-yl)-1-methyl-1*H*-benzo[d]imidazole (Compound-06):**

To a solution of 5-(2-fluorophenyl)furan-2-carbaldehyde **2** (100 mg, 0.526 mmol) in DMF (2 mL) was added $Na_2S_2O_5$ (119 mg, 0.626 mmol) and 3-fluoro-*N*1-methylbenzene-1,2-diamine **3** (73 mg, 0.521 mmol) at room temperature. The reaction mixture was heated at 150 °C for 2 h. After completion of the reaction (monitored by TCL and LCMS), the reaction mixture was cooled to room temperature and diluted with water (20 mL). The precipitated solid was filtered, washed with water (10 mL) and *n*-hexane (10 mL) resulting in the crude compound which was purified by preparative HPLC to afford 50 mg (30% yield) of **Compound 6** as pale yellow solid. **LCMS-Condition-1:** [M+H]+ = 311.15; $R_t$ = 2.11 min. **$^1$H NMR** (400 MHz, DMSO-$d_6$) δ: 7.95 - 8.01 (m, 1H), 7.68 (dd, *J* = 4.89, 8.80 Hz, 1H), 7.61 (dd, *J* = 2.45, 9.29 Hz, 1H), 7.35 - 7.48 (m, $4^H$), 7.13 (d, *J* = 3.91 Hz, 1H), 7.11 (dd, *J* = 1.47, 2.45 Hz, 1H), 4.11 (s, 3H).

**Synthesis of Compound-07(VPC-18-58)** 1-Methyl-2-(5-(tetrahydrofuran-2-yl)furan-2-yl)-1*H*-benzo[*d*]imidazole

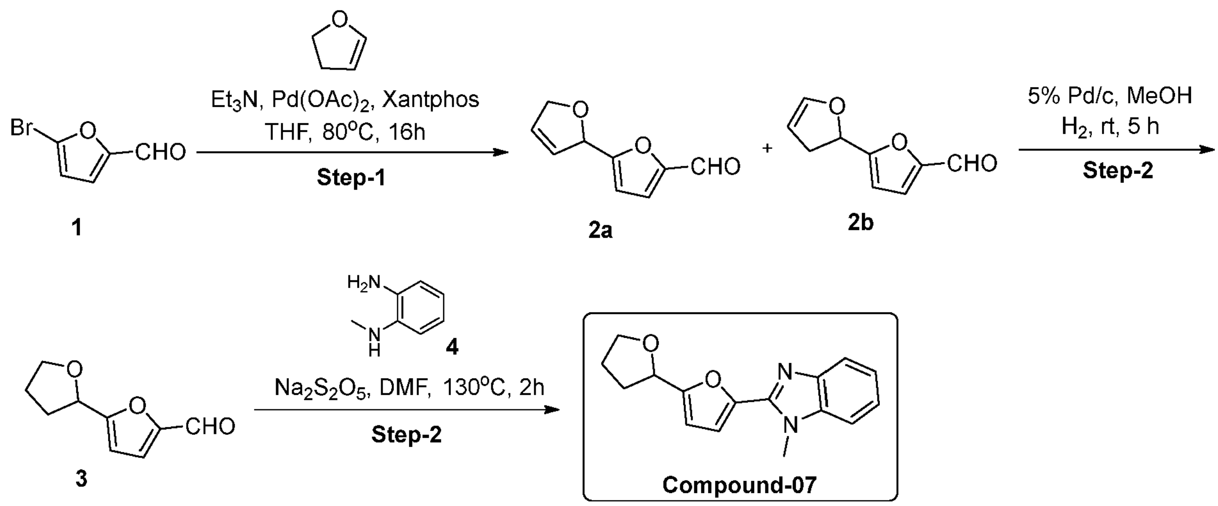


**Step-1: Synthesis of 2',5'-dihydro-[2,2'-bifuran]-5-carbaldehyde (2a):**

To a solution of 5-bromofuran-2-carbaldehyde **1** (2 g, 11.49 mmol) in THF (20 mL) was added 2,3-dihydrofuran

(4 g, 57.47 mmol) and triethyl amine (3.2 mL, 22.98 mmol) and degassed with argon for 15 min. To the resulting solution was added Xantphos (664 mg, 1.149 mmol) and $Pd(OAc)_2$ (128 mg, 0.574 mmol) and degassing was continued for another 5 min at room temperature. The reaction mixture was then heated at 80 °C for 16 h. After completion of the reaction, the reaction mixture was cooled to room temperature, diluted with water (25 mL) and extracted with ethyl acetate (3 x 25 mL). The combined organic layer was dried over anhydrous $Na_2SO_4$, filtered and concentrated under reduced pressure. The crude material was purified by silica gel column chromatography eluting with 0-5% ethyl acetate in *n*-hexane to afford 200 mg (10% yield) of the **Int 2a** and 60 mg (4% yield) **of Int 2b** as colourless oil.

**Int-2a. LCMS-Condition-1:** [M+H]+ = 164.95; $R_t$ = 1.60 min. **$^1$H NMR** (400 MHz, $CDCl_3$) δ: 9.61 (s, 1H), 7.20 (d, *J* = 3.51 Hz, 1H), 6.46 (d, *J* = 3.51 Hz, 1H), 6.13-6.18 (m, 1H), 5.92-5.96 (m, 1H), 5.86 (td, *J* = 1.79, 3.70 Hz, 1H), 4.81-4.88 (m, 1H), 4.72-4.79 (m, 1H). **Int-2b. LCMS-Condition-1:** $[M+H]^+$ = 165.05; $R_t$ = 1.63 min

**$^1$H NMR** (400 MHz, $CDCl_3$) δ: 9.62 (s, 1H), 7.22 (d, *J* = 3.51 Hz, 1H), 6.54 (d, *J* = 3.51 Hz, 1H), 6.37 (q, *J* = 2.34 Hz, 1H), 5.57 (dd, *J* = 7.53, 10.79 Hz, 1H), 5.02 (q, *J* = 2.26 Hz, 1H), 2.98-3.10 (m, 1H), 2.81-2.92 (m, 1H).

**Step-2: Synthesis of 5-(tetrahydrofuran-2-yl)furan-2-carbaldehyde (3):**

To a solution of 2',5'-dihydro-[2,2'-bifuran]-5-carbaldehyde **2a** (200 mg, 1.219 mmol) in methanol (10 mL) was added 5% Pd/C (20 mg) under nitrogen atmosphere at room temperature. The reaction mixture was stirred under hydrogen atmosphere at room temperature for 5 h. After completion of the reaction, the reaction mixture was filtered through a pad of Celite, washed with methanol and the filtrate was concentrated under reduced pressure. The crude compound was purified by silica gel column chromatography eluting with 0-20% ethyl acetate in n-hexane to afford 70 mg (35% yield) **of Int 3** as colourless oil. **LCMS-Condition-1:** $[M+H]^+$ = 167.05; $R_t$ = 1.75 min.

**Step-3: Synthesis of 1-methyl-2-(5-(tetrahydrofuran-2-yl)furan-2-yl)-1*H*-benzo[*d*]imidazole (Compound-07):**

To a solution of 5-(tetrahydrofuran-2-yl)furan-2-carbaldehyde **3** (70 mg, 0.421 mmol) in DMF (1 mL) was added $Na_2S_2O_5$ (96 mg, 0.506 mmol) and *N*1-methylbenzene-1,2-diamine **4** (51 mg, 0.421 mmol) at room temperature. The reaction mixture was heated at 130 °C for 2 h. After completion of the reaction, the reaction mixture was cooled to room temperature, diluted with water (20 mL) and extracted with ethyl acetate (3 x 20 mL). The combined organic layer was dried over anhydrous $Na_2SO_4$, filtered and concentrated under reduced pressure. The crude compound was purified by silica gel column chromatography eluting with 0-10% methanol in DCM to afford 25 mg (22% yield) of **Compound 07** as pale yellow sticky solid. **LCMS-Condition-1:** [M+H]+ = 269.05; $R_t$ = 1.20 min. **$^1$H NMR** (400 MHz, $CDCl_3$) δ: 7.78 (dd, *J* = 2.69, 6.11 Hz, 1H), 7.35-7.40 (m, 1H), 7.28-7.32 (m, 2H), 7.11-7.15 (m, 1H), 6.47 (d, *J* = 2.93 Hz, 1H), 5.06 (t, *J* = 6.60 Hz, 1H), 4.03-4.10 (m, 1H), 4.06 (s, 3H), 3.92-3.96 (m, 1H), 1.99-2.37 (m, 4H).

**Synthesis of Compound-08(VPC-18-47)** 1-Methyl-2-(5-phenylfuran-2-yl)-1*H*-benzo[d]imidazole-6-sulfonamide

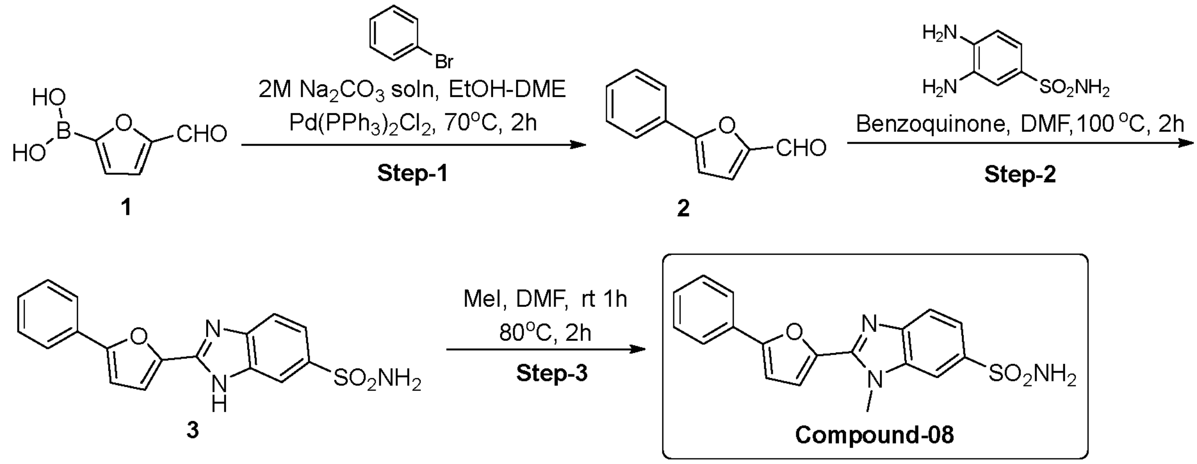


1 2 3 Compound-08

**Step-1: Synthesis of 5-phenylfuran-2-carbaldehyde (2):**

To a solution of (5-formylfuran-2-yl)boronic acid **1** (1 g, 7.147 mmol) in EtOH:DME (2:1, 18 mL) was added bromobenzene (897 mg, 5.718 mmol) and 2M $Na_2CO_3$ solution (7.1 mL, 14.29 mmol) and degassed with argon for 20

min. To the resulting solution was added $Pd(PPh_3)_2Cl_2$ (250 mg, 0.357 mmol) and degassed with argon for another 10 min at room temperature. The reaction mixture was then heated at 70 °C for 2 h. After completion of the reaction (monitored by TLC and LCMS), the reaction mixture was cooled to room temperature, diluted with water (50 mL) and extracted with ethyl acetate (3 times). The combined dried over anhydrous $Na_2SO_4$, filtered and concentrated under reduced pressure; the crude compound was purified by silica gel column chromatography with 0-5% ethyl acetate in *n*-hexane as eluent to afford 600 mg (50% yield) of **Int 2** as pale yellow oil. **LCMS-Condition-1:** $[M+H]^+$ = 173.15; $R_t$ = 1.70 min. **$^1$H NMR** (400 MHz, $CDCl_3$) δ: 9.66 (s, 1H), 7.83 (d, *J* = 7.34 Hz, 2H), 7.37 - 7.48 (m, 3H), 7.33 (d, *J* = 3.91 Hz, 1H), 6.85 (d, *J* = 3.42 Hz, 1H).

**Step-2: Synthesis of 2-(5-phenylfuran-2-yl)-1*H*-benzo[*d*]imidazole-6-sulfonamide (3):**

To a solution of 5-phenylfuran-2-carbaldehyde **2** (200 mg, 1.162 mmol) in ethanol (4 mL) was added benzoquinone (125 mg, 1.157 mmol) followed by 3,4-diaminobenzenesulfonamide (217 mg, 1.162 mmol) at room temperature. The reaction mixture was heated at 100 °C for 2 h. After completion of the reaction (monitored by TLC and LCMS), the reaction mixture was cooled to room temperature and diluted with water (20 mL). The precipitated solid was filtered and washed with water followed by n-hexane; the crude compound was purified by recrystallization using ethanol to afford 400 mg (98% yield) of **Int 3** as grey solid. **LCMS-Condition-1:** [M+H]+ = 340.05; $R_t$ = 2.40 min. **$^1$H NMR** (400 MHz, DMSO-$d_6$) δ: 13.38 (br. s, 1H), 7.98 - 8.11 (m, 1H), 7.95 (d, *J* = 7.34 Hz, 2H), 7.64 - 7.83 (m, 2H), 7.52 (t, *J* = 7.83 Hz, 2H), 7.37 - 7.43 (m, 2H), 7.31 (br. s, 2H), 7.25 (d, *J* = 3.42 Hz, 1H).

**Step-3: Synthesis of 1-methyl-2-(5-phenylfuran-2-yl)-1*H*-benzo[*d*]imidazole-6-sulfonamide (Compound-08):**

To a solution of 2-(5-phenylfuran-2-yl)-1*H*-benzo[d]imidazole-6-sulfonamide **3** (100 mg, 0.295 mmol) in DMF (2 mL) was added sodium hydride (60% dispersion in oil, 23.5 mg, 0.589 mmol) and stirred at room temperature for 20 min. To the resulting solution was added methyl iodide (0.03 mL, 0.451 mmol) at room temperature and stirred for 1 h. The reaction mixture was then heated at 80 °C for 2 h. After completion of the reaction (monitored by TLC and LCMS), the reaction mixture was cooled to room temperature and diluted with water (20 mL). The precipitated solid was filtered and the crude compound was purified by preparative HPLC to afford 25 mg (12% yield) of **Compound-08** as white solid which was characterized by $^1$H NMR and NOE experiment. **LCMS-Condition-1:** $[M+H]^+$ = 354.04; $R_t$ = 1.57 min. **$^1$H NMR** (400 MHz, DMSO-$d_6$) δ: 8.12 (d, *J* = 1.25 Hz, 1H), 7.88 - 7.93 (m, 2H), 7.79 - 7.84 (m, 1H), 7.71 - 7.75 (m, 1H), 7.5$^2$ (t, *J* = 7.65 Hz, 2H), 7.48 (d, *J* = 3.51 Hz, 1H), 7.38 - 7.43 (m, 1H), 7.30 - 7.34 (m, 3H), 4.21 (s, 3H).

**Synthesis of Compound-09(VPC-18-32)**_2-(5-(2-Fluorophenyl)furan-2-yl)-6-nitro-1*H*-benzo[d]imidazole

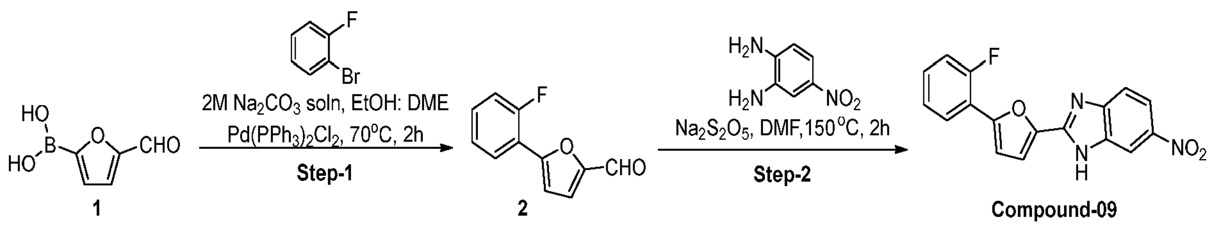

1
2
Compound-09

**Step-1: Synthesis of 5-(2-fluorophenyl)furan-2-carbaldehyde (2):**

To a solution of (5-formylfuran-2-yl)boronic acid **1** (1 g, 7.147 mmol) in EtOH:DME (2:1, 18 mL) was added 1-bromo-2-fluorobenzene (999 mg, 5.708 mmol) and 2M $Na_2CO_3$ solution (7.08 mL, 14.29 mmol) and degassed with argon for 20 min. To the resulting solution was added $PdCl_2(PPh_3)_2$ (250 mg, 0.357 mmol) and degassed with argon for another 10 min at room temperature. The reaction mixture was then heated at 70 °C for 2 h. After completion of the reaction (monitored by TCL and LCMS), the reaction mixture was cooled to room temperature, diluted with water (50 mL) and extracted with ethyl acetate (3 times). The combined dried over anhydrous $Na_2SO_4$, filtered and concentrated under reduced pressure to and the crude compound was purified by silica gel column chromatography eluting using 0-5% ethyl acetate in n-hexane as eluent to afford 350 mg (26% yield) of **Int 2** as pale yellow solid. **LCMS-Condition-1:** [M+H]+ = 190.95; $R_t$ = 1.76 min. **$^1$H NMR** (400 MHz, $CDCl_3$) δ: 9.69 (s, 1H), 8.02 (dt, *J* = 1.71, 7.70 Hz, 1H), 7.37 - 7.41 (m, 1H), 7.35 (d, *J* = 3.42 Hz, 1H), 7.23 - 7.29 (m, 1H), 7.13 - 7.21 (m, 1H), 7.03 (t, *J* = 3.67 Hz, 1H).

**Step-2: Synthesis of 2-(5-(2-fluorophenyl)furan-2-yl)-6-nitro-1*H*-benzo[*d*]imidazole (Compound-09):**

To a solution of 5-(2-fluorophenyl)furan-2-carbaldehyde 2 (200 mg, 1.052 mmol) in ethanol (4 mL) was added benzoquinone (113.6 mg, 1.052 mmol) and 4-nitrobenzene-1,2-diamine (161 mg, 1.052 mmol) at room temperature. The reaction mixture was heated at 100 °C for 2 h. After completion of the reaction, the reaction mixture was cooled to room temperature and diluted with water (20 mL). The precipitated solid was filtered and washed with water (10 mL) and n-hexane (10 mL). The crude compound was purified by silica gel column chromatography eluting with 0-5% ethyl acetate in n-hexane to afford 30 mg (8% yield) of **Compound 09** as off white solid. **LCMS-Condition-1:** $[M+H]^+$ = 324.10; $R_t$ = 1.92 min. **$^1$H NMR** (400 MHz, DMSO-$d_6$) δ: 13.75 (br. s, 1H), 8.48 (br. s, 1H), 8.10 - 8.19 (m, 2H), 7.78 (d, *J* = 9.29 Hz, 1H), 7.49 - 7.52 (m, 1H), 7.36 - 7.48 (m, 3H), 7.12 (t, *J* = 3.42 Hz, 1H).

**Synthesis of Compound-10(VPC-18-35)** 2-(5-(2-Methoxyphenyl)furan-2-yl)-1-methyl-1*H*-benzo[*d*]imidazole

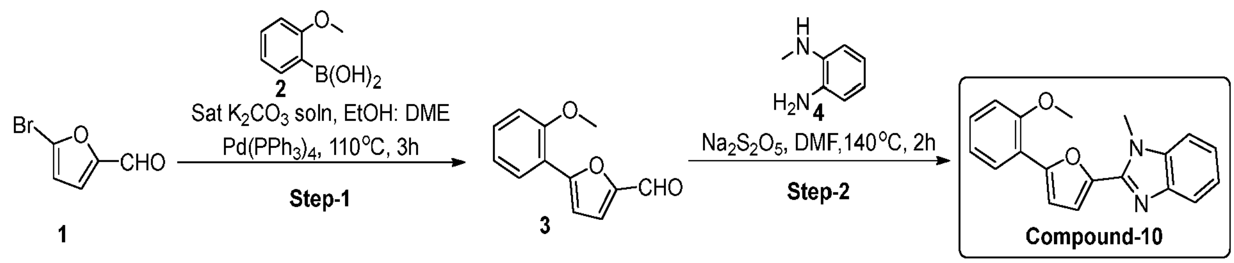


**Step-1: Synthesis of 5-(2-methoxyphenyl)furan-2-carbaldehyde (3):**

To a solution of 5-bromofuran-2-carbaldehyde **1** (1 g, 5.714 mmol) in Toluene:Ethanol (5:3, 16 mL) was added (2-methoxyphenyl)boronic acid **2** (868 mg, 5.714 mmol) and saturated aqueous $K_2CO_3$ solution (3 mL) and degassed with argon for 20 min. To the resulting solution was added $Pd(PPh_3)_4$ (329 mg, 0.285 mmol) and degassed with argon for another 10 min at room temperature. The reaction mixture was further heated at 110 °C for 3 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure and the crude compound was purified by Combiflash column chromatography eluting with 0-8% ethyl acetate in n-hexane to afford 1 g (86% yield) of **Int 3** as brown oil. **LCMS-Condition-1:** $[M+H]^+$ = 203.00; $R_t$ = 1.75 min. **$^1$H NMR** (400 MHz, $CDCl_3$) δ: 9.65 (s, 1H), 8.05 (dd, *J* = 1.47, 7.83 Hz, 1H), 7.34-7.40 (m, 1H), 7.33 (d, *J* = 3.91 Hz, 1H), 7.14 (d, *J* = 3.91 Hz, 1H), 7.07 (t, *J* = 7.58 Hz, 1H), 7.00 (d, *J* = 8.31 Hz, 1H), 3.97 (s, 3H).

**Step-2: Synthesis of 2-(5-(2-methoxyphenyl)furan-2-yl)-1-methyl-1H-benzo[d]imidazole (Compound-10):**

To a solution of 5-(2-methoxyphenyl)furan-2-carbaldehyde **3** (300 mg, 1.483 mmol) in DMF (5 mL) was added $Na_2S_2O_5$ (338 mg, 1.780 mmol) and *N*1-methylbenzene-1,2-diamine **4** (181 mg, 1.483 mmol) at room temperature. The reaction mixture was heated at 140 °C for 2 h. After completion of the reaction (monitored by TLC and LCMS), the reaction mixture was cooled to room temperature, quenched with water (25 mL) and extracted with ethyl acetate (3 times). The combined organic layer was washed with brine, dried over anhydrous $Na_2SO_4$, filtered and concentrated under reduced pressure and the crude compound was purified by Combiflash column chromatography eluting with 10-15% ethyl acetate in n-hexane to afford 340 mg (63% yield) of **Compound 10** as light brown solid. **LCMS-Condition-1:** [M+H]+ = 305.10; $R_t$ = 1.53 min. **$^1$H NMR** (400 MHz, $CDCl_3$) δ: 7.94 (dd, *J* = 1.47, 7.82 Hz, 1H), 7.77-7.82 (m, 1H), 7.37-7.42 (m, 1H), 7.34 (d, *J* = 3.91 Hz, 1H), 7.32 (d, *J* = 1.47 Hz, 1H), 7.30 (d, *J* = 3.91 Hz, 2H), 7.16 (d, *J* = 3.91 Hz, 1H), 7.08 (t, *J* = 7.58 Hz, 1H), 7.01 (d, *J* = 8.31 Hz, 1H), 4.18 (s, 3H), 3.99 (s, 3H).

**Synthesis of Compound-11(VPC-18-42)** 6-Chloro-1-methyl-2-(5-phenylfuran-2-yl)-1*H*-benzo[*d*]imidazole

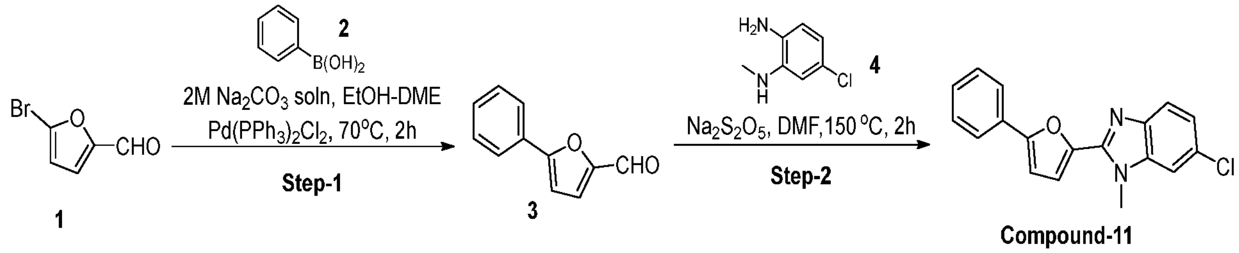


**Step-1: Synthesis of 5-phenylfuran-2-carbaldehyde (3):**

To a solution of 5-bromofuran-2-carbaldehyde **1** (1 g, 7.147 mmol) in EtOH:DME (2:1, 18 mL) was added phenyl boronic acid **2** (897 mg, 5.718 mmol) and 2M $Na_2CO_3$ solution (7.1 mL, 14.29 mmol) and degassed with argon for 20 min. To the resulting solution was added $Pd(PPh_3)_2Cl_2$ (250 mg, 0.357 mmol) and degassed with argon for another 10 min at room temperature. The reaction mixture was then heated at 70 °C for 2 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by silica gel column chromatography eluting with 0-5% ethyl acetate in n-hexane to afford 600 mg (49% yield) of **Int 3** as pale yellow oil. **LCMS-Condition-1:** [M+H]+ = 173.15; $R_t$ = 1.70 min. **$^1$H NMR** (400 MHz, $CDCl_3$) δ: 9.66 (s, 1H), 7.83 (d, *J* = 7.34 Hz, 2H), 7.37-7.48 (m, 3H), 7.33 (d, *J* = 3.91 Hz, 1H), 6.85 (d, *J* = 3.42 Hz, 1H).

**Step-2: Synthesis of 6-chloro-1-methyl-2-(5-phenylfuran-2-yl)-1*H*-benzo[*d*]imidazole (Compound-11):**

To a solution of **5-phenylfuran-2-carbaldehyde 3** (100 mg, 0.581 mmol) in DMF (3 mL) was added $Na_2S_2O_5$ (132 mg, 0.697 mmol) and 5-chloro-*N*1-methylbenzene-1,2-diamine **4** (91 mg, 0.581 mmol) at room temperature. The reaction mixture was heated at 150 °C for 2 h. After completion of the reaction (monitored by TLC and LCMS), the reaction mixture was cooled to room temperature, diluted with water (50 mL) and extracted with ethyl acetate (3 times). The combined organic layer was washed with water and brine, dried over anhydrous $Na_2SO_4$, filtered and concentrated under reduced pressure; the crude compound was purified by silica gel column chromatography eluting with 0-5% methanol in DCM to afford 40 mg (22% yield) of **Compound 11** as light brown solid. **LCMS-Condition-1:** $[M+H]^+$ = 309.05; $R_t$ = 2.20 min. **$^1$H NMR** (400 MHz, DMSO-$d_6$) δ: 7.88 (d, *J* = 7.34 Hz, 2H), 7.84 (s, 1H), 7.66 (d, *J* = 8.80 Hz, 1H), 7.51 (t, *J* = 7.34 Hz, 2H), 7.36-7.43 (m, 2H), 7.23-7.30 (m, 2H), 4.12 (s, 3H).

**Synthesis of Compound-12(VPC-18-36)** 1-Ethyl-2-(5-phenylfuran-2-yl)-1H-benzo[d]imidazole

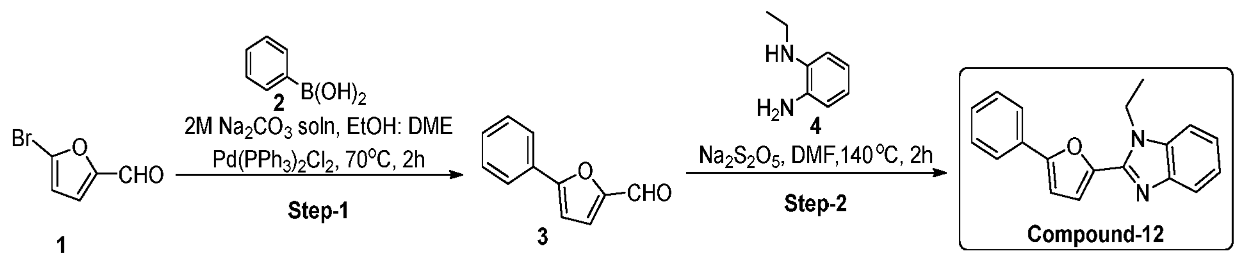


**Step-1: Synthesis of 5-phenylfuran-2-carbaldehyde (3):**

To a solution of 5-bromofuran-2-carbaldehyde **1** (1 g, 7.147 mmol) in ethanol: DME (2:1, 18 mL) was added phenylboronic acid **2** (897 mg, 5.718 mmol) and 2M $Na_2CO_3$ solution (7 mL, 14.29 mmol) and degassed with argon for 20 min. To the resulting solution was added $Pd(PPh_3)_2Cl_2$ (250 mg, 0.357 mmol) and degassed with argon for another 10 min at room temperature. The reaction mixture was then heated at 70 °C for 2 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by silica gel column chromatography eluting

with 0-5% ethyl acetate in n-hexane to afford 600 mg (49% yield) of **Int 3** as pale yellow oil. **LCMS-Condition-1:** [M+H]+ = 173.15; $R_t$ = 1.70 min. **$^1$H NMR** (400 MHz, $CDCl_3$) δ: 9.66 (s, 1H), 7.83 (d, *J* = 7.34 Hz, 2H), 7.37-7.48 (m, 3H), 7.33 (d, *J* = 3.91 Hz, 1H), 6.85 (d, *J* = 3.42 Hz, 1H).

**Step-2: Synthesis of 1-ethyl-2-(5-phenylfuran-2-yl)-1H-benzo[d]imidazole (Compound-12):**

To a solution of 5-phenylfuran-2-carbaldehyde **3** (150 mg, 0.872 mmol) in DMF (3 mL) was added $Na_2S_2O_5$ (198 mg, 1.042 mmol) and *N*1-ethylbenzene-1,2-diamine **4** (118 mg, 0.872 mmol) at room temperature. The reaction mixture was heated at 150 °C for 2 h. After completion of the reaction (monitored by TLC and LCMS), the reaction mixture was cooled to room temperature, diluted with water (50 mL) and extracted with ethyl acetate (3 times). The combined organic layer was washed with brine, dried over anhydrous $Na_2SO_4$, filtered and concentrated under reduced pressure; the crude compound was purified by silica gel column chromatography eluting with 10-15% methanol in DCM to afford 50 mg (20% yield) of **Compound 12** as light brown solid.

**LCMS-Condition-1:** $[M+H]^+$ = 289.10; $R_t$ = 1.69 min

**$^1$H NMR** (400 MHz, DMSO-*d*$_6$) δ: 7.85 (d, *J* = 8.31 Hz, 2H), 7.66 (d, *J* = 8.31 Hz, 2H), 7.51 (t, *J* = 7.58 Hz, 2H), 7.39 (d, *J* = 7.34 Hz, 1H), 7.35 (d, *J* = 3.91 Hz, 1H), 7.22-7.32 (m, 3H), 4.64 (q, *J* = 6.85 Hz, 2H), 1.48 (t, *J* = 7.09 Hz, 3H).

**Synthesis of Compound-13(VPC-18-43)** 1-Methyl-2-(5-(pyrimidin-5-yl)furan-2-yl)-1*H*-benzo[d]imidazole

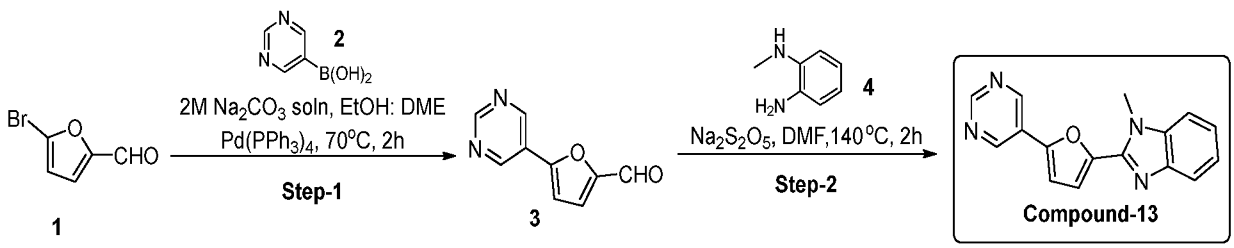


**Step-1: Synthesis of 5-(pyrimidin-5-yl)furan-2-carbaldehyde (3):**

To a solution of 5-bromofuran-2-carbaldehyde **1** (1 g, 5.714 mmol) in Toluene:Ethanol (5:3, 16 mL) was added pyrimidin-5-ylboronic acid **2** (708 mg, 5.714 mmol) and $K_2CO_3$ (1.6 g, 11.42 mmol) and degassed with argon for 20 min. To the resulting solution was added $Pd(PPh_3)_4$ (329 mg, 0.285 mmol) and degassed with argon for another 10 min at room temperature. The reaction mixture was then heated at 110 °C for 3 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by Combiflash column chromatography eluting with 0-10% ethyl acetate in n-hexane to afford 750 mg (75% yield) of **Int 3** as brown oil. **LCMS-Condition-1:** [M+H]+ = 175.00; $R_t$ = 1.13 min.

**Step-2: Synthesis of 1-methyl-2-(5-(pyrimidin-5-yl)furan-2-yl)-1*H*-benzo[d]imidazole (Compound-13):**

To a solution of 5-(pyrimidin-5-yl)furan-2-carbaldehyde **3** (200 mg, 1.135 mmol) and *N*1-methylbenzene-1,2-diamine **4** (138.7 mg, 1.135 mmol) in DMF (5 mL) was added $Na_2S_2O_5$ (216 mg, 1.135 mmol) at room temperature. The reaction mixture was heated at 140 °C for 2 h. After completion of the reaction (monitored by TLC and LCMS), the reaction mixture was cooled to room temperature, diluted with water (50 mL) and extracted with ethyl acetate (3 times). The combined organic layer was washed with brine, dried over anhydrous $Na_2SO_4$, filtered and concentrated under reduced pressure; the crude compound was purified by Combiflash column chromatography eluting with 10-15% ethyl acetate in n-hexane to afford 52 mg (16% yield) of **Compound 13** as pale yellow solid. **LCMS-Condition-1:** [M+H]+ = 277.10; $R_t$ = 1.44 min. **$^1$H NMR** (400 MHz, DMSO-*d*$_6$) δ: 9.30 (s, 2H), 9.18 (s, 1H), 7.68 (d, *J* = 8.31 Hz, 2H), 7.55 (d, *J* = 3.91 Hz, 1H), 7.45 (d, *J* = 3.42 Hz, 1H), 7.23-7.34 (m, 2H), 4.15 (s, 3H).

**Synthesis of Compound-16(VPC-18-48)** 2-(Furan-2-yl)-4-(6-nitro-1*H*-benzo[d]imidazol-2-yl)thiazole

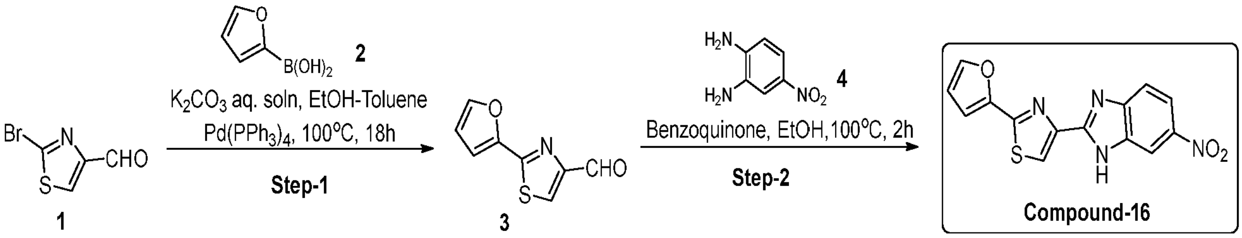


**Step-1: Synthesis of 2-(furan-2-yl)thiazole-4-carbaldehyde (3):**

To a solution of 2-bromothiazole-4-carbaldehyde **1** (1 g, 5.208 mmol) and furan-2-ylboronic acid **2** (699 mg, 6.241 mmol) in toluene:ethanol (2:1, 25 mL) was added a solution of $K_2CO_3$ (1.43 g, 10.36 mmol) in water (8 mL) and degassed with argon for 20 min. To the resulting solution was added $Pd(PPh_3)_4$ (300 mg, 0.259 mmol) and degassed with argon for another 10 min at room temperature. The reaction mixture was then heated at 100 °C for 18 h. After completion of the reaction (monitored by TLC and LCMS), the reaction mixture was cooled to room temperature, quenched with water (100 mL) and extracted with ethyl acetate (3 times). The combined organic layer was washed with water (100 mL) and brine (100 mL) dried over anhydrous $Na_2SO_4$, filtered and concentrated under reduced pressure; the crude compound was purified by silica gel column chromatography eluting with 0-25% ethyl acetate in n-hexane to afford 600 mg (64% yield) of **Int 3** as white solid. **LCMS-Condition-1:** [M+H]+ = 179.95; $R_t$ = 1.39 min. **$^1$H NMR** (400 MHz, DMSO-$d_6$) δ: 9.94 (s, 1H), 8.72 (s, 1H), 7.96 (d, *J* = 1.96 Hz, 1H), 7.25 (d, *J* = 3.91 Hz, 1H), 6.75 (dd, *J* = 1.96, 3.42 Hz, 1H).

**Step-2: Synthesis of 2-(furan-2-yl)-4-(6-nitro-1*H*-benzo[*d*]imidazol-2-yl)thiazole (Compound-16):**

To a solution of 2-(furan-2-yl)thiazole-4-carbaldehyde **3** (100 mg, 0.558 mmol) in ethanol (2 mL) was added benzoquinone (60 mg, 0.558 mmol) followed by 4-nitrobenzene-1,2-diamine **4** (85 mg, 0.558 mmol) at room temperature. The reaction mixture was heated at 100 °C for 2 h. After completion of the reaction (monitored by TLC and LCMS), the reaction mixture was cooled to room temperature and diluted with water (20 mL). The precipitated solid was filtered and washed with water and n-hexane; and the crude compound was purified by recrystallization using ethanol to afford 30 mg (17% yield) of **Compound 16** as gray solid. **LCMS-Condition-1:** [M+H]+ = 312.95; $R_t$ = 1.77 min. **$^1$H NMR** (400 MHz, DMSO-$d_6$) δ: 13.39 (br. s, 1H), 8.46 - 8.54 (m, 2H), 8.14 (dd, *J* = 1.96, 8.80 Hz, 1H), 7.92 (d, *J* = 0.98 Hz, 1H), 7.77 (d, *J* = 8.80 Hz, 1H), 7.23 (d, *J* = 2.93 Hz, 1H), 6.76 (dd, *J* = 1.47, 3.42 Hz, 1H).

**Synthesis of Compound-19(VPC-18-52)** 2-(Furan-2-yl)-4-(6-nitrobenzofuran-2-yl)thiazole

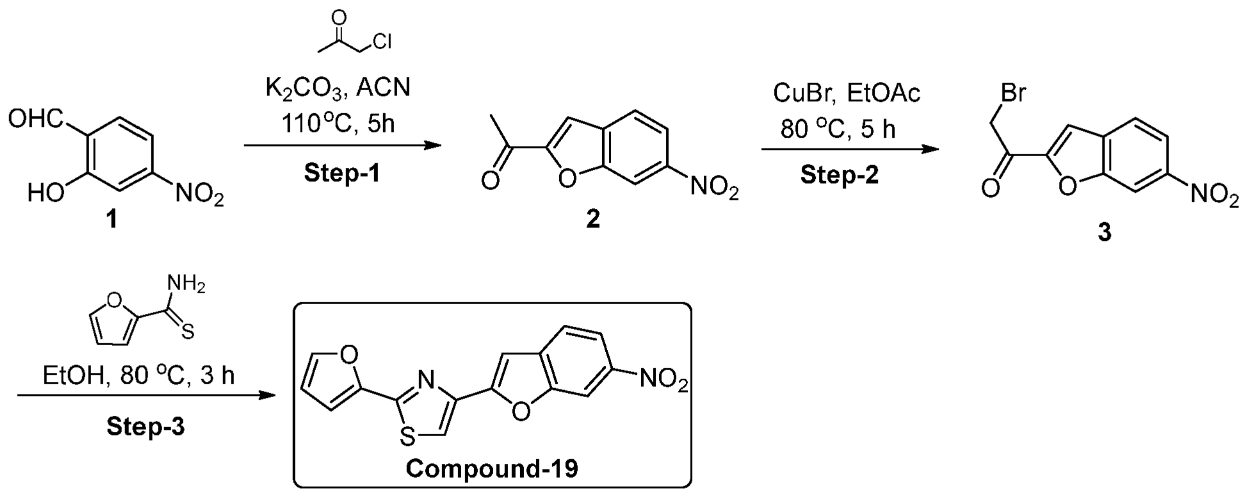


**Step-1: Synthesis of 1-(6-nitrobenzofuran-2-yl)ethan-1-one (2):**

To a solution of 2-hydroxy-4-nitrobenzaldehyde **1** (2 g, 11.96 mmol) in acetonitrile (15 mL) was added potassium carbonate (3.3 g, 23.91 mmol) and 1-chloropropan-2-one (1.1 g, 11.88 mmol) at room temperature. The reaction mixture

was then heated at 110 °C for 5 h. After completion of the reaction, the reaction mixture was cooled to room temperature, diluted with water (100 mL) and extracted with ethyl acetate (3 x 100 mL). The combined organic layer was dried over anhydrous $Na_2SO_4$, filtered and concentrated under reduced pressure. The crude compound was purified by silica gel column chromatography eluting with 0-6% ethyl acetate in n-hexane to afford 1.2 g (49% yield) of **Int 2** as pale yellow solid. **$^1$H NMR** (400 MHz, $CDCl_3$) δ: 8.50 (d, *J* = 0.98 Hz, 1H), 8.25 (dd, *J* = 1.96, 8.80 Hz, 1H), 7.86 (d, *J* = 8.80 Hz, 1H), 7.57 (s, 1H), 2.68 (s, 3H).

**Step-2: Synthesis of 2-bromo-1-(6-nitrobenzofuran-2-yl)ethan-1-one (3):**

To a solution of 1-(6-nitrobenzofuran-2-yl)ethan-1-one **2** (1.2 g, 5.853 mmol) in ethyl acetate (8 mL) was added copper bromide (2.61 g, 11.68 mmol) and heated at 80 °C for 5 h. After completion of the reaction, the reaction mixture was filtered through a pad of Celite and washed with ethyl acetate (10 mL). The filtrate was concentrated under reduced pressure to yield the crude compound which was purified by Combiflash column chromatography eluting with 0-5% ethyl acetate in n-hexane to afford 340 mg (20% yield) of **Int 3** as yellow solid. **$^1$H NMR** (400 MHz, $CDCl_3$) δ: 8.53 (s, 1H), 8.27 (dd, *J* = 1.88, 8.66 Hz, 1H), 7.89 (d, *J* = 8.53 Hz, 1H), 7.73 (d, *J* = 1.00 Hz, 1H), 4.47 (s, 2H).

**Step-3: Synthesis of 2-(furan-2-yl)-4-(6-nitrobenzofuran-2-yl)thiazole (Compound-19):**

To a solution of 2-bromo-1-(6-nitrobenzofuran-2-yl)ethan-1-one **3** (170 mg, 0.598 mmol) in ethanol (3 mL) was added furan-2-carbothioamide (76 mg, 0.598 mmol) at room temperature. The reaction mixture was then heated at 80 °C for 3 h. After completion of the reaction, the reaction mixture was filtered hot, and obtained solid residue was washed with hot methanol (10 mL) to afford 50 mg (26% yield) of **Compound 19** as yellow solid. **LCMS-Condition-1:** [M+H]+ = 313.00; $R_t$ = 2.15 min. **$^1$H NMR** (400 MHz, DMSO-$d_6$) δ: 8.57 (br. s, 1H), 8.33 (s, 1H), 8.20 (dd, *J* = 1.76, 8.53 Hz, 1H), 7.95 (d, *J* = 8.28 Hz, 2H), 7.55 (s, 1H), 7.25 (d, *J* = 2.76 Hz, 1H), 6.77 (br. s, 1H).

**Synthesis of Compound-21(VPC-18-33)** 4-(Benzofuran-2-yl)-2-phenylthiazole

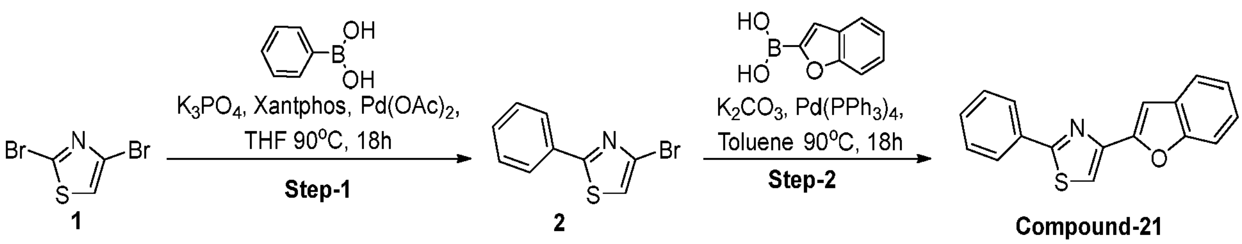


1 2 Compound-21

**Step-1: Synthesis of 4-bromo-2-phenylthiazole (2):**

To a solution of 2,4-dibromothiazole **1** (1 g, 4.132 mmol) and phenylboronic acid (547 mg, 4.486 mmol) in THF (10 mL) was added $K_3PO_4$ (2.62 g, 12.34 mmol) and degassed with argon for 15 min. To the resulting solution was added $Pd(OAc)_2$ (46 mg, 0.205 mmol) and Xantphos (237 mg, 0.410 mmol) and degassed with argon for another 10 min at room temperature. The reaction mixture was then heated at 90 °C for 18 h. After completion of the reaction (monitored by TCL and LCMS), the reaction mixture was cooled to room temperature, quenched with water (25 mL) and extracted with ethyl acetate (3 times). The combined organic layer was washed with water followed by brine, dried over anhydrous $Na_2SO_4$, filtered and concentrated under reduced pressure and the crude compound was purified by silica gel column chromatography eluting using 0-10% ethyl acetate in n-hexane as eluent to afford 975 mg (98% yield) of **Int 2** as white solid. **LCMS-Condition-1:** $[M+H]^+$ = 239.85; $R_t$ = 2.24 min. **$^1$H NMR** (400 MHz, $CDCl_3$) δ: 7.90 - 7.96 (m, 2H), 7.42 - 7.46 (m, 3H), 7.21 (s, 1H).

**Step-2: Synthesis of 4-(benzofuran-2-yl)-2-phenylthiazole (Compound-21):**

To a solution of 4-bromo-2-phenylthiazole **2** (250 mg, 1.041 mmol) and benzofuran-2-ylboronic acid **3** (253 mg, 1.562 mmol) in toluene:ethanol:water (2:1:1. 8 mL) was added potassium carbonate (287 mg, 2.079 mmol) and degassed with argon for 15 min. To the resulting solution was added $Pd(PPh_3)_4$ (60 mg, 0.0519 mmol) and degassed with argon for another 10 min at room temperature. The reaction mixture was then heated at 90 °C for 18 h. After completion of the reaction (monitored by TCL and LCMS), the reaction mixture was cooled to room temperature, quenched with water (25

mL) and extracted with ethyl acetate (3 times). The combined organic layer was dried over anhydrous $Na_2SO_4$, filtered and concentrated under reduced pressure and the crude compound was purified by silica gel column chromatography eluting using 0-8% ethyl acetate in *n*-hexane as eluent to afford 75 mg (26% yield) of **Compound 21** as off white solid. **LCMS-Condition-1:** $[M+H]^+$ = 278.05; $R_t$ = 2.38 min. **$^1$H NMR** (400 MHz, $CDCl_3$) δ: 8.05 (dd, *J* = 2.45, 7.34 Hz, 2H), 7.69 (s, 1H), 7.61 - 7.66 (m, 1H), 7.53 (d, *J* = 8.31 Hz, 1H), 7.46 - 7.51 (m, 3H), 7.26 - 7.36 (m, 3H).

**Synthesis of Compound-22(VPC-18-53)** 2-(Furan-2-yl)-4-(3-methylbenzofuran-2-yl)thiazole

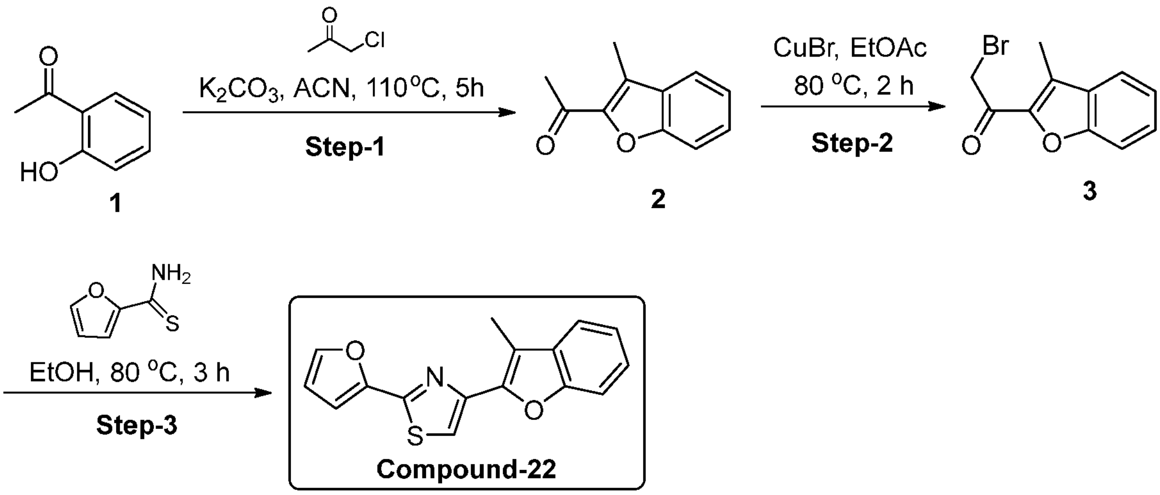


1

2

3

Compound-22

**Step-1: Synthesis of 1-(3-methylbenzofuran-2-yl)ethan-1-one (2):**

To a solution of 1-(2-hydroxyphenyl)ethan-1-one **1** (1 g, 7.344 mmol) in acetonitrile (8 mL) was added potassium carbonate (2.02 g, 14.68 mmol) followed by 1-chloropropan-2-one (679 mg, 7.344 mmol) at room temperature. The reaction mixture was then heated at 110 °C for 5 h. After completion of the reaction, the reaction mixture was cooled to room temperature, diluted with water (100 mL) and extracted with ethyl acetate (3 × 100 mL). The combined organic layer was washed with water and brine, dried over anhydrous $Na_2SO_4$, filtered and concentrated under reduced pressure. The crude compound was purified by silica gel column chromatography eluting with 0-1% ethyl acetate in n-hexane to afford 340 mg (26% yield) of **Int 2** as white solid. **LCMS-Condition-1:** $[M+H]^+$ = 175.05; $R_t$ = 1.86 min. **$^1$H NMR** (400 MHz, $CDCl_3$) δ: 7.66 (d, *J* = 7.78 Hz, 1H), 7.45-7.54 (m, 2H), 7.28-7.34 (m, 1H), 2.62 (s, 3H), 2.61 (s, 3H).

**Step-2: Synthesis of 2-bromo-1-(3-methylbenzofuran-2-yl)ethan-1-one (3):**

To a solution of 1-(3-methylbenzofuran-2-yl)ethan-1-one **2** (340 mg, 1.954 mmol) in diethyl ether (8 mL) was added bromine (0.1 mL, 1.954 mmol) at room temperature and stirred for 2 h. After completion of the reaction, the reaction mixture was quenched with saturated sodium thiosulfate solution and extracted with ethyl acetate (3 × 100 mL). The combined organic layer was washed with water and brine, dried over anhydrous $Na_2SO_4$, filtered and concentrated under reduced pressure to yield the crude compound. The crude compound was purified by silica gel column chromatography eluting with 0-1% ethyl acetate in n-hexane to afford 100 mg (20% yield) of **Int 3** as off white solid. **LCMS-Condition-1:** $[M+H]^+$ = 253.00; $R_t$ = 2.16 min. **$^1$H NMR** (400 MHz, $CDCl_3$) δ: 7.66-7.73 (m, 1H), 7.50-7.58 (m, 2H), 7.29-7.40 (m, 1H), 4.52 (s, 2H), 2.68 (s, 3H).

**Step-3: Synthesis of 2-(furan-2-yl)-4-(3-methylbenzofuran-2-yl)thiazole (Compound-22):**

To a solution of 2-bromo-1-(3-methylbenzofuran-2-yl)ethan-1-one **3** (100 mg, 0.395 mmol) in ethanol (3 mL) was added furan-2-carbothioamide (50 mg, 0.395 mmol) at room temperature. The reaction mixture was then heated at 80 °C for 3 h. After completion of the reaction, the reaction mixture was filtered hot, and the obtained solid residue was washed with hot methanol (10 mL) to afford 50 mg (45% yield) of **Compound 22** as off white solid. **LCMS-Condition-1:** [M+H]+ = 282.05; $R_t$ = 2.51 min. **$^1$H NMR** (400 MHz, $CDCl_3$) δ: 7.60-7.63 (m, 1H), 7.53-7.59 (m, 2H), 7.48 (d, *J* = 8.03 Hz, 1H), 7.27-7.34 (m, 2H), 7.11 (d, *J* = 3.26 Hz, 1H), 6.57 (dd, *J* = 1.76, 3.26 Hz, 1H), 2.68 (s, 3H).

**Synthesis of Compound-24(VPC-18-49)** 4-(Benzofuran-2-yl)-2-(5-nitrofuran-2-yl)thiazole

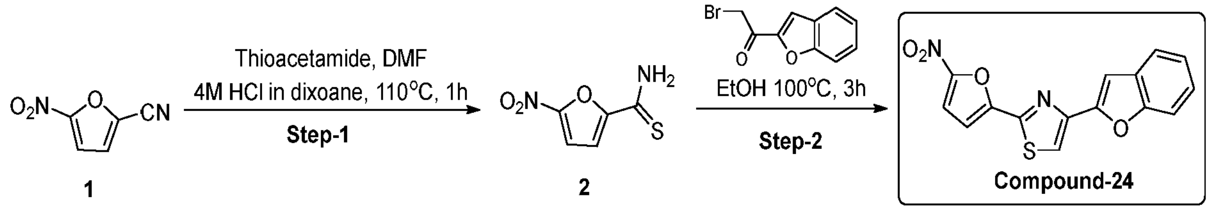


**Step-1: Synthesis of 5-nitrofuran-2-carbothioamide (2):**

To a solution of 5-nitrofuran-2-carbonitrile **1** (1 g, 7.299 mmol) in DMF (3 mL) was added 4M HCl in dioxane (7 mL) and thioacetamide (547 mg, 7.299 mmol) at room temperature. The reaction mixture was then heated at 110 °C for 1 h. After completion of the reaction (monitored by TLC and LCMS), the reaction mixture was concentrated under reduced pressure, diluted with water (25 mL) and extracted with ethyl acetate (3 times). The combined organic layer was washed with water followed by brine, dried over anhydrous $Na_2SO_4$, filtered and concentrated under reduced pressure; the crude compound was purified by Combiflash column chromatography eluting using 20-30% ethyl acetate in n-hexane as eluent to afford 400 mg (32% yield) of **Int 2** as brown solid. **LCMS-Condition-1:** $[M+H]^+$ = 172.90; $R_t$ = 1.53 min.

$^1$**H NMR** (400 MHz, DMSO-$d_6$) δ: 10.82 (br. s, 1H), 9.77 (br. s, 1H), 7.72 (d, *J* = 4.40 Hz, 1H), 7.45 (d, *J* = 3.91 Hz, 1H).

**Step-2: Synthesis of 4-(benzofuran-2-yl)-2-(5-nitrofuran-2-yl)thiazole (Compound-24):**

To a solution of 5-nitrofuran-2-carbothioamide 2 (200 mg, 1.162 mmol) in ethanol (5 mL) was added 1-(benzofuran-2-yl)-2-bromoethan-1-one (277 mg, 1.162 mmol) at room temperature. The reaction mixture was then heated at 100 °C for 3 h. After completion of the reaction, the reaction mixture was concentrated under reduced pressure and diluted with water (25 mL). Obtained solid was filtered and washed with ethyl acetate (10 mL) and DCM (10 mL) to afford 170 mg (47% yield) of **Compound 24** as pale yellow solid. **LCMS-Condition-1:** [M+H]+ = 312.85; $R_t$ = 2.11 min. $^1$**H NMR** (400 MHz, DMSO-$d_6$) δ: 8.40 (s, 1H), 7.92 (d, *J* = 3.91 Hz, 1H), 7.73 (d, *J* = 7.83 Hz, 1H), 7.68 (d, *J* = 8.31 Hz, 1H), 7.59 (d, *J* = 3.91 Hz, 1H), 7.44 (s, 1H), 7.36 - 7.41 (m, 1H), 7.28 - 7.34 (m, 1H).

**Synthesis of Compound-25(VPC-18-54)** 4-(6-Fluorobenzofuran-2-yl)-2-(5-nitrofuran-2-yl)thiazole

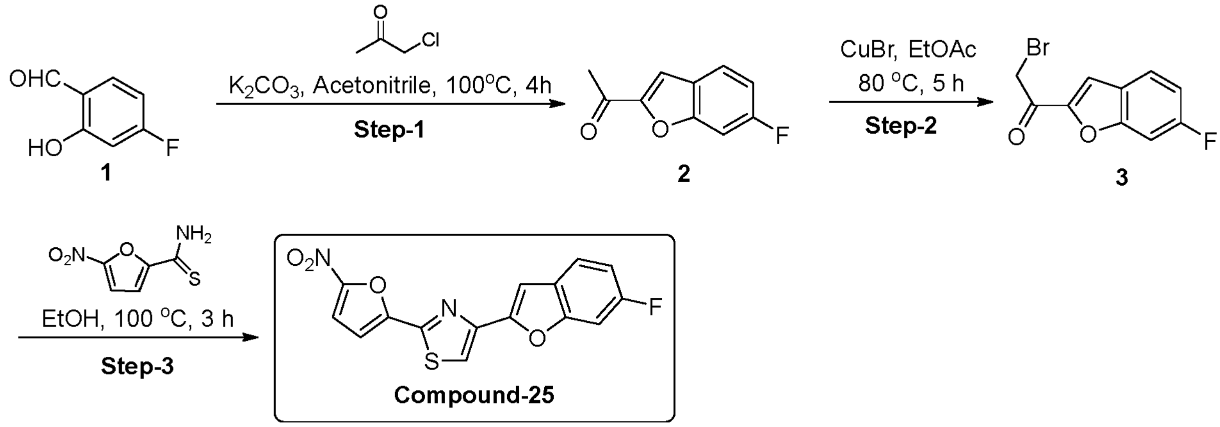


**Step-1: Synthesis of 1-(6-fluorobenzofuran-2-yl)ethan-1-one (2):**

To a solution of 4-fluoro-2-hydroxybenzaldehyde **1** (400 mg, 2.857 mmol) in acetonitrile (10 mL) was added potassium carbonate (788 mg, 5.714 mmol) and 1-chloropropan-2-one (0.21 mL, 2.857 mmol) at room temperature.

The reaction mixture was then heated to 100°C for 4 h. After completion of the reaction, the reaction mixture was concentrated under reduced pressure, diluted with water (25 mL) and extracted with ethyl acetate (3 × 25 mL). The combined organic layer was dried over anhydrous $Na_2SO_4$, filtered and concentrated under reduced pressure. The crude compound was purified by Combiflash column chromatography eluting with 10-15% ethyl acetate in n-hexane to afford 230 mg (45% yield) **of Int** 2 as off white solid. **LCMS-Condition-1:** [M+H]+ = 178.95; $R_t$ = 1.63 min. **$^1$H NMR** (400 MHz, $CDCl_3$) δ: 7.66 (dd, *J* = 5.40, 8.66 Hz, 1H), 7.49 (d, *J* = 0.75 Hz, 1H), 7.27-7.31 (m, 1H), 7.10 (dt, *J* = 2.26, 9.03 Hz, 1H), 2.60 (s, 3H).

**Step-2: Synthesis of 2-bromo-1-(6-fluorobenzofuran-2-yl)ethan-1-one (3):**

To a solution of 1-(6-fluorobenzofuran-2-yl)ethan-1-one **2** (230 mg, 1.292 mmol) in ethyl acetate (10 mL) was added copper bromide (559 mg, 2.506 mmol) and heated at 80 °C for 5 h. After completion of the reaction, the reaction mixture was filtered through a pad of Celite and washed with ethyl acetate (10 mL). The filtrate was concentrated under reduced pressure and the crude compound was purified by Combiflash column chromatography eluting with 0-5% ethyl acetate in n-hexane to afford 260 mg (81% yield) of **Int 3** as yellow solid. **LCMS-Condition-1:** $[M+H]^+$ = 256.90; $R_t$ = 1.79 min. **$^1$H NMR** (400 MHz, $CDCl_3$) δ: 7.70 (dd, *J* = 5.40, 8.66 Hz, 1H), 7.65 (s, 1H), 7.31 (dd, *J* = 1.25, 8.53 Hz, 1H), 7.13 (dt, *J* = 2.26, 9.03 Hz, 1H), 4.42 (s, 2H).

**Step-3: Synthesis of 4-(6-fluorobenzofuran-2-yl)-2-(5-nitrofuran-2-yl)thiazole (Compound-25):**

To a solution of 2-bromo-1-(6-fluorobenzofuran-2-yl)ethan-1-one **3** (180 mg, 1.046 mmol) in ethanol (10 mL) was added 5-nitrofuran-2-carbothioamide (269 mg, 1.046 mmol) at room temperature. The reaction mixture was then heated at 100 °C for 3 h. After completion of the reaction, the reaction mixture was concentrated under reduced pressure and diluted with water (50 mL). The precipitated solid was filtered, washed with ethyl acetate (10 mL) and DCM (10 mL) and dried to afford 195mg (56% yield) of **Compound 25** as yellow solid. **LCMS-Condition-1:** [M+H]+ = 331.10; $R_t$ = 2.20 min. **$^1$H NMR** (400 MHz, DMSO-$d_6$) δ: 8.38 (s, 1H), 7.92 (d, *J* = 3.76 Hz, 1H), 7.75 (dd, *J* = 5.52, 8.53 Hz, 1H), 7.65 (d, *J* = 9.03 Hz, 1H), 7.59 (d, *J* = 4.02 Hz, 1H), 7.46 (s, 1H), 7.18-7.25 (m, 1H).

**Synthesis of Compound-26(VPC-18-44)** 4-(Benzofuran-2-yl)-2-(4-nitrophenyl)thiazole

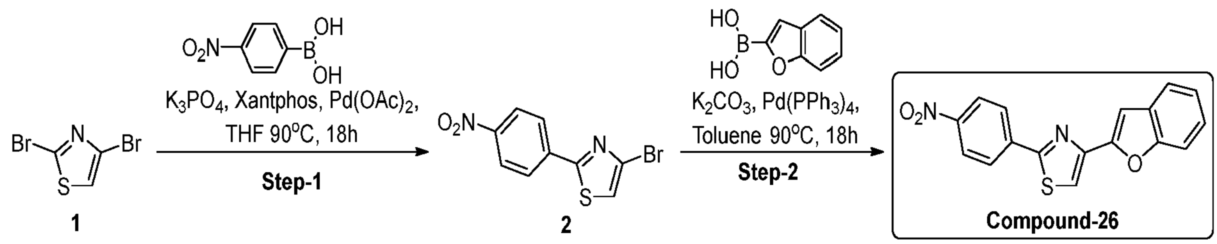


**Step-1: Synthesis of 4-bromo-2-(4-nitrophenyl)thiazole (2):**

To a solution of 2,4-dibromothiazole **1** (1 g, 4.166 mmol) and (4-nitrophenyl)boronic acid (755 mg, 4.528 mmol) in THF (10 mL) was added $K_3PO_4$ (2.6 g, 12.35 mmol) and degassed with argon for 15 min. To the resulting solution was added $Pd(OAc)_2$ (46 mg, 0.206 mmol) and Xantphos (237 mg, 0.4116 mmol) and degassed with argon for another 10 min at room temperature. The reaction mixture was heated at 90 °C for 18 h. After completion of the reaction (monitored by TLC and LCMS), the reaction mixture was cooled to room temperature, quenched with water (25 mL) and extracted with ethyl acetate (3 times). The combined organic layer was washed with water followed by brine, dried over anhydrous $Na_2SO_4$, filtered and concentrated under reduced pressure; the crude compound was purified by silica gel column chromatography eluting with 0-15% ethyl acetate in n-hexane to afford 1 g (85% yield) of **Int 2** as pale yellow solid. **LCMS-Condition-1:** $[M+H]^+$ = 284.90; $R_t$ = 2.23 min. **$^1$H NMR** (400 MHz, $CDCl_3$) δ: 8.31 (d, *J* = 8.8 Hz, 2H), 8.12 (*J* = 8.8 Hz, 2H), 7.39 (s, 1H).

**Step-2: Synthesis of 4-(benzofuran-2-yl)-2-(4-nitrophenyl)thiazole (Compound-26):**

To a solution of 4-bromo-2-(4-nitrophenyl)thiazole **2** (250 mg, 0.876 mmol) and benzofuran-2-ylboronic acid **3** (213 mg, 1.315 mmol) in toluene:ethanol:water (2:1:1, 8 mL) was added potassium carbonate (242 mg, 1.753 mmol)

and degassed with argon for 15 min. To the resulting solution was added $Pd(PPh_3)_4$ (50 mg, 0.0432 mmol) and degassed with argon for another 10 min at room temperature. The reaction mixture was then heated at 90 °C for 18 h. After completion of the reaction (monitored by TLC and LCMS), the reaction mixture was cooled to room temperature, quenched with water (25 mL) and extracted with ethyl acetate (3 times). The combined organic layer was dried over anhydrous $Na_2SO_4$, filtered and concentrated under reduced pressure; the crude compound was purified by silica gel column chromatography eluting using 0-4% ethyl acetate in n-hexane, followed by recrystallization in ethanol to afford 24 mg (8.5% yield) of **Compound 26** as yellow solid. **LCMS-Condition-1:** $[M+H]^+$ = 323.00; $R_t$ = 2.31 min. **$^1$H NMR** (400 MHz, DMSO-$d_6$) δ: 8.35 (d, *J* = 8.4 Hz, 2H), 8.23 (d, *J* = 8.4 Hz, 2H), 7.83 (s, 1H), 7.66 (d, *J* = 7.6 Hz, 1H), 7.55 (d, *J* = 8.0 Hz, 1H), 7.28-7.38 (m, 3H).

**Synthesis of Compound-27(VPC-18-55)** 2-(2,4-Difluorophenyl)-4-(6-nitrobenzofuran-2-yl)thiazole

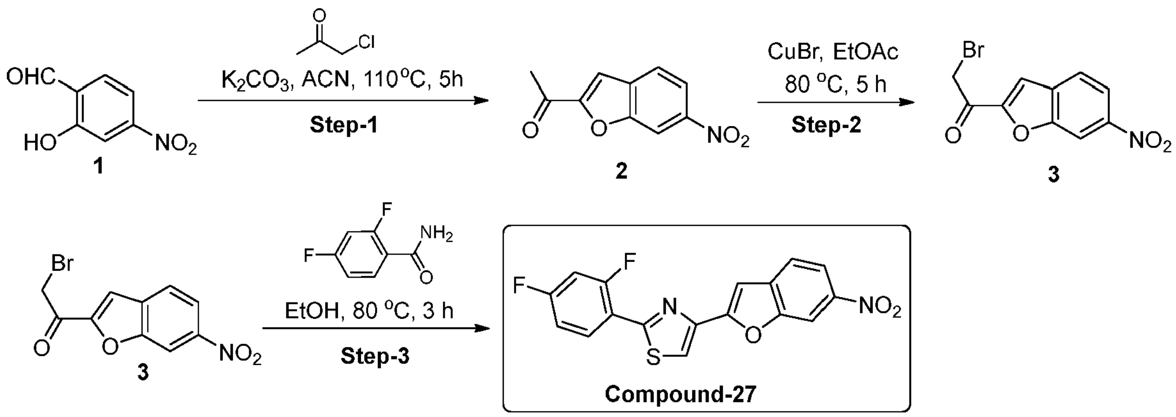

1
2
3
3
Compound-27

**Step-1: Synthesis of 1-(6-nitrobenzofuran-2-yl)ethan-1-one (2):**

To a solution of 2-hydroxy-4-nitrobenzaldehyde **1** (2 g, 11.96 mmol) in acetonitrile (15 mL) was added potassium carbonate (3.3 g, 23.91 mmol) and 1-chloropropan-2-one (1.1 g, 11.88 mmol) at room temperature. The reaction mixture was then heated at 110 °C for 5 h. After completion of the reaction, the reaction mixture was cooled to room temperature, diluted with water (100 mL) and extracted with ethyl acetate (3 × 100 mL). The combined organic layer was dried over anhydrous $Na_2SO_4$, filtered and concentrated under reduced pressure. The crude compound was purified by silica gel column chromatography eluting with 0-6% ethyl acetate in n-hexane to afford 1.2 g (49% yield) of **Int 2** as pale yellow solid. **$^1$H NMR** (400 MHz, $CDCl_3$) δ: 8.50 (d, *J* = 0.98 Hz, 1H), 8.25 (dd, *J* = 1.96, 8.80 Hz, 1H), 7.86 (d, *J* = 8.80 Hz, 1H), 7.57 (s, 1H), 2.68 (s, 3H).

**Step-2: Synthesis of 2-bromo-1-(6-nitrobenzofuran-2-yl)ethan-1-one (3):**

To a solution of 1-(6-nitrobenzofuran-2-yl)ethan-1-one **2** (1.2 g, 5.853 mmol) in ethyl acetate (8 mL) was added copper bromide (2.61 g, 11.68 mmol) and heated at 80 °C for 5 h. After completion of the reaction, the reaction mixture was filtered through a pad of Celite and washed with ethyl acetate (30 mL). The filtrate was concentrated under reduced pressure and the crude compound was purified by Combiflash column chromatography eluting with 0-5% ethyl acetate in n-hexane to afford 340 mg (20% yield) of **Int 3** as yellow solid. $^1$H **NMR** (400 MHz, $CDCl_3$) δ: 8.53 (s, 1H), 8.27 (dd, *J* = 1.88, 8.66 Hz, 1H), 7.89 (d, *J* = 8.53 Hz, 1H), 7.73 (d, *J* = 1.00 Hz, 1H), 4.47 (s, 2H).

**Step-3: Synthesis of 2-(2,4-difluorophenyl)-4-(6-nitrobenzofuran-2-yl)thiazole (Compound-27):**

To a solution of 2-bromo-1-(6-nitrobenzofuran-2-yl)ethan-1-one **3** (170 mg, 0.598 mmol) in ethanol (3 mL) was added 2,4-difluorobenzamide (103 mg, 0.598 mmol) at room temperature. The reaction mixture was then heated at 80 °C for 3 h. After completion of the reaction, the reaction mixture was hot filtered, the solid residue obtained and washed with hot methanol (10 mL) to afford 50 mg (23% yield) of **Compound 27** as yellow solid. **LCMS-Condition-1:** $[M+H]^+$ = 359.05; $R_t$ = 2.38 min. **$^1$H NMR** (400 MHz, DMSO-$d_6$) δ: 8.55 (s, 1H), 8.46 (s, 1H), 8.31-8.41 (m, 1H), 8.19 (dd, *J* = 1.88, 8.66 Hz, 1H), 7.92 (d, *J* = 8.53 Hz, 1H), 7.59 (s, 1H), 7.52-7.57 (m, 1H), 7.30-7.39 (m, 1H).

**Synthesis of Compound-28(VPC-18-59)** 4-(Benzofuran-2-yl)-2-(4-bromo-2-chlorophenyl)thiazole

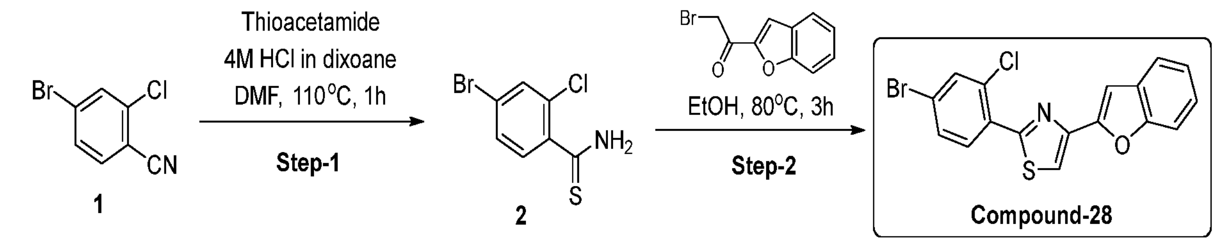


**Step-1: Synthesis of 4-bromo-2-chlorobenzothioamide (2):**

To a solution of 4-bromo-2-chlorobenzonitrile **1** (250 mg, 1.154 mmol) in DMF (4 mL) was added 4M HCl in dioxane (3 mL) and thioacetamide (87 mg, 1.154 mmol) at room temperature. The reaction mixture was then heated at 110 °C for 1 h. After completion of the reaction, the reaction mixture was concentrated under reduced pressure, diluted with water (15 mL) and extracted with ethyl acetate (3 × 15 mL). The combined organic layer was washed with water followed by brine, dried over anhydrous $Na_2SO_4$, filtered and concentrated under reduced pressure. The crude compound was purified by Combiflash column chromatography eluting with 20-30% ethyl acetate in n-hexane to afford 150 mg (52% yield) of **Int 2** as brown solid. **LCMS-Condition-1:** [M+H]+ = 251.85; $R_t$ = 1.86 min. **$^1$H NMR** (400 MHz, $CDCl_3$) δ: 8.01 (br. s, 1H), 7.53-7.62 (m, 2H), 7.44 (dd, *J* = 1.76, 8.28 Hz, 1H), 7.20 (br. s, 1H).

**Step-2: Synthesis of 4-(benzofuran-2-yl)-2-(4-bromo-2-chlorophenyl)thiazole (Compound-28):**

To a solution of 4-bromo-2-chlorobenzothioamide **2** (150 mg, 0.598 mmol) in ethanol (4 mL) was added 1-(benzofuran-2-yl)-2-bromoethan-1-one (143 mg, 0.598 mmol) at room temperature. The reaction mixture was then heated at 80 °C for 3 h. After completion of the reaction, the reaction mixture was filtered hot, and the obtained solid residue was washed with hot methanol (10 mL) and dried to afford 70 mg (30% yield) of **Compound 28** as off white solid. **LCMS-Condition-3:** $[M+H]^+$ = 392.15; $R_t$ = 2.76 min. **$^1$H NMR** (400 MHz, DMSO-$d_6$) δ: 8.38 (br. s, 1H), 8.28 (d, *J* = 7.34 Hz, 1H), 8.02 (br. s, 1H), 7.80 (d, *J* = 7.82 Hz, 1H), 7.64-7.75 (m, 2H), 7.43 (br. s, 1H), 7.26-7.39 (m, 2H).

**Synthesis of Compound-29(VPC-18-45)** 4-(6-Fluoro-1-methyl-1*H*-benzo[*d*]imidazol-2-yl)-2-(2-fluorophenyl)thiazole

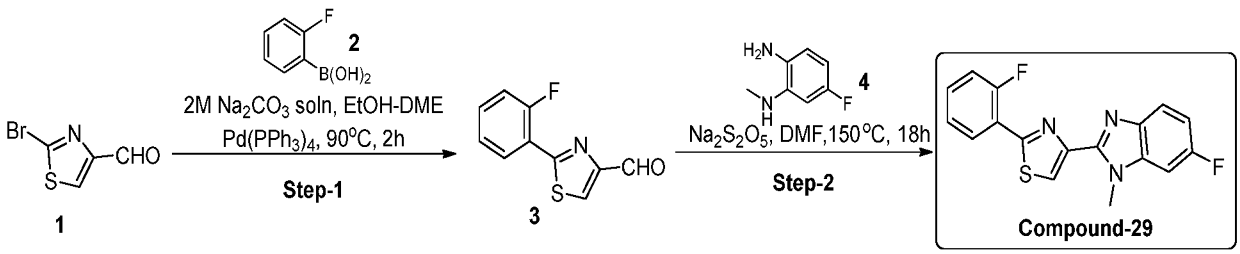


**Step-1: Synthesis of 2-(2-fluorophenyl)thiazole-4-carbaldehyde (3):**

To a solution of 2-bromothiazole-4-carbaldehyde **1** (1 g, 5.208 mmol) in ethanol: DME (2:1, 30 mL) was added (2-fluorophenyl)boronic acid **2** (723 mg, 5.208 mmol) followed by 2M $Na_2CO_3$ solution (5.2 mL, 10.41 mmol) and degassed with argon for 20 min. To the resulting solution was added $Pd(PPh_3)_4$ (300 mg, 0.260 mmol) and degassed with argon for another 10 min at room temperature. The reaction mixture was heated at 90 °C for 2 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by silica gel column chromatography eluting with 0-6% ethyl acetate in n-hexane to afford 386 mg (36% yield) of **Int 3** as off white solid. **LCMS-Condition-1:** $[M+H]^+$ = 207.90; $R_t$ = 1.70 min. **$^1$H NMR** (400 MHz, DMSO-$d_6$) δ: 10.03 (s, 1H), 8.88 (s, 1H), 8.27 (t, *J* = 7.58 Hz, 1H), 7.41-7.68

(m, 3H).

**Step-2: Synthesis of 4-(6-fluoro-1-methyl-1*H*-benzo[*d*]imidazol-2-yl)-2-(2-fluorophenyl)thiazole (Compound-29):**

To a solution of 2-(2-fluorophenyl)thiazole-4-carbaldehyde **3** (150 mg, 0.723 mmol) in DMF (3 mL) was added $Na_2S_2O_5$ (55 mg, 0.289 mmol) and 5-fluoro-*N*1-methylbenzene-1,2-diamine **4** (101 mg, 0.723 mmol) at room temperature. The reaction mixture was heated at 150 °C for 18 h. After completion of the reaction (monitored by TLC and LCMS), the reaction mixture was cooled to room temperature, diluted with water (50 mL) and extracted with ethyl acetate (3 times). The combined organic layer was washed with brine, dried over anhydrous $Na_2SO_4$, filtered and concentrated under reduced pressure; the crude compound was purified by silica gel column chromatography eluting with 10-15% ethyl acetate in n-hexane, followed by preparative TLC purification to afford 60 mg (25% yield) of **Compound 29** as off white solid. **LCMS-Condition-1:** $[M+H]^+$ = 328.00; $R_t$ = 1.97 min. **$^1$H NMR** (400 MHz, $CDCl_3$) δ: 8.34-8.39 (m, 1H), 8.33 (s, 1H), 7.72 (dd, *J* = 4.65, 8.56 Hz, 1H), 7.42-7.50 (m, 1H), 7.29-7.35 (m, 1H), 7.22-7.25 (m, 1H), 7.12 (dd, *J* = 1.96, 8.80 Hz, 1H), 7.02-7.09 (m, 1H), 4.29 (s, 3H).

**Synthesis of Compound-30(VPC-18-56)** 2-(4-Bromo-2-fluorophenyl)-4-(6-fluoro-1-methyl-1*H*-benzo[*d*]imidazol-2-yl)thiazole

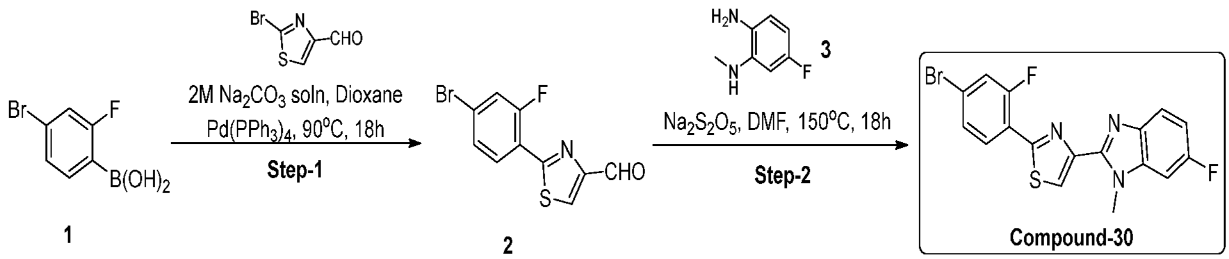


**Step-1: Synthesis of 2-(4-bromo-2-fluorophenyl)thiazole-4-carbaldehyde (2):**

To a solution of 2-bromothiazole-4-carbaldehyde (500 mg, 2.604 mmol) in dioxane (10 mL) was added 2M $Na_2CO_3$ solution (2.6 mL, 552 mg, 5.207 mmol) and (4-bromo-2-fluorophenyl)boronic acid **1** (500 g, 2.28 mmol) and degassed with argon for 20 min. To the resulting solution was added $Pd(PPh_3)_4$ (150 mg, 0.129 mmol) and degassing was continued for another 10 min at room temperature. The reaction mixture was further heated at 90 °C for 18 h. After completion of the reaction, the reaction mixture was cooled to room temperature, diluted with water (25 mL) and extracted with ethyl acetate (3 × 25 mL). The combined organic layer was dried over anhydrous $Na_2SO_4$, filtered and concentrated under reduced pressure. The crude compound was purified by silica gel column chromatography eluting with 0-5% ethyl acetate in n-hexane to afford 500 mg (63% yield) of **Int 2** as white solid. **LCMS-Condition-1:** [M+H]+ = 285.80; $R_t$ = 1.92 min **$^1$H NMR** (400 MHz, $CDCl_3$) δ: 10.13 (s, 1H), 8.30 (s, 1H), 8.27 (d, *J* = 8.31 Hz, 1H), 7.42-7.49 (m, 2H).

**Step-2: Synthesis of 2-(4-bromo-2-fluorophenyl)-4-(6-fluoro-1-methyl-1H-benzo[d]imidazol-2-yl)thiazole (Compound-go):**

To a solution of 2-(4-bromo-2-fluorophenyl)thiazole-4-carbaldehyde **2** (200 mg, 0.699 mmol) in DMF (6 mL) was added $Na_2S_2O_5$ (159 mg, 0.836 mmol) and 5-fluoro-N1-methylbenzene-1,2-diamine **3** (98 mg, 0.699 mmol) at room temperature. The reaction mixture was heated at 150 °C for 18 h. After completion of the reaction, the reaction mixture was cooled to room temperature and diluted with water (20 mL) and extracted with ethyl acetate (3 × 20 mL). The combined organic layer was dried over anhydrous $Na_2SO_4$, filtered and concentrated under reduced pressure. The crude compound was purified by preparative HPLC to afford 50 mg (17% yield) of **Compound 30** as white solid. **LCMS-Condition-1:** $[M+H]^+$ = 407.95; $R_t$ = 2.20 min. **$^1$H NMR** (400 MHz, $CDCl_3$) δ: 8.34 (s, 1H), 8.23 (t, *J* = 8.28 Hz, 1H), 7.72 (dd, *J* = 4.77, 8.78 Hz, 1H), 7.43-7.49 (m, 2H), 7.12 (dd, *J* = 2.26, 8.53 Hz, 1H), 7.06 (dt, *J* = 2.38, 9.22 Hz, 1H), 4.27 (s, 3H).

**Synthesis of Compound-31(VPC-18-57)** 2-(4-Bromo-2-fluorophenyl)-4-(6-fluoro-3-methylbenzofuran-2-yl)thiazole

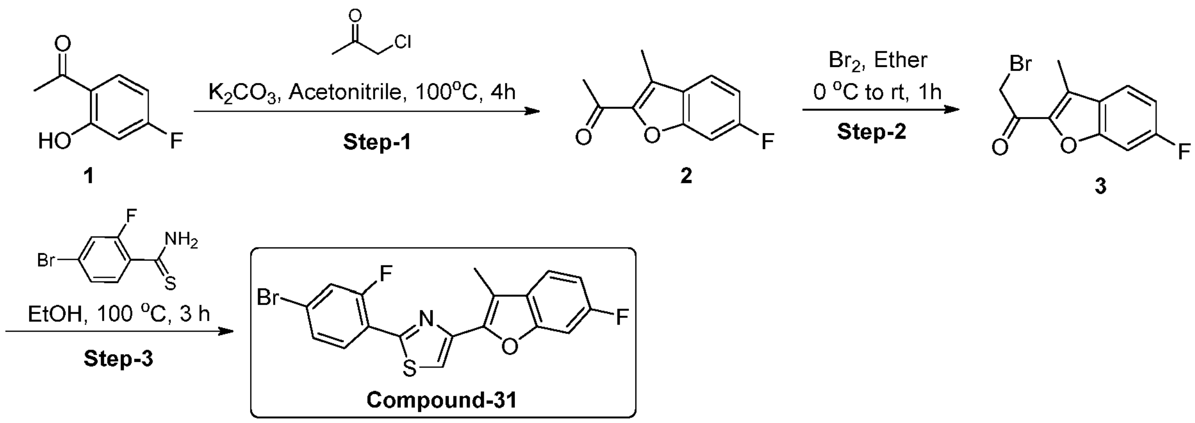


**Step-1: Synthesis of 1-(6-fluoro-3-methylbenzofuran-2-yl)ethan-1-one (2):**

To a solution of 1-(4-fluoro-2-hydroxyphenyl)ethan-1-one **1** (500 mg, 3.246 mmol) in acetonitrile (10 mL) was added potassium carbonate (896 mg, 6.492 mmol) and 1-chloropropan-2-one (302 mg, 3.246 mmol) at room temperature. The reaction mixture was then heated at 100 °C for 4 h. After completion of the reaction, the reaction mixture was concentrated under reduced pressure, diluted with water (20 mL) and extracted with ethyl acetate (3 × 20 mL). The combined organic layer was washed with water followed by brine, dried over anhydrous $Na_2SO_4$, filtered and concentrated under reduced pressure. The crude compound was purified by Combiflash column chromatography eluting with 10-15% ethyl acetate in *n*-hexane to afford 200 mg (32% yield) of **Int** 2 as off white solid. **LCMS-Condition-1:** $[M+H]^+$ = 192.85; $R_t$ = 2.12 min. **$^1$H NMR** (400 MHz, $CDCl_3$) δ: 7.59 (dd, *J* = 5.38, 8.80 Hz, 1H), 7.22 (dd, *J* = 2.45, 8.80 Hz, 1H), 7.08 (dt, *J* = 2.45, 9.05 Hz, 1H), 2.60 (s, 3H), 2.59 (s, 3H).

**Step-2: Synthesis of 2-bromo-1-(6-fluoro-3-methylbenzofuran-2-yl)ethan-1-one (3):**

To a solution of 1-(6-fluoro-3-methylbenzofuran-2-yl)ethan-1-one 2 (200 mg, 1.041 mmol) in diethyl ether (10 mL) at 0 °C was added bromine (0.05 mL, 1.041 mmol) and stirred at room temperature for 1 h. After completion of the reaction, the reaction mixture was concentrated under reduced pressure, diluted with water (20 mL) and extracted with ethyl acetate (3 × 20 mL). The combined organic layer was washed with water followed by brine, dried over anhydrous $Na_2SO_4$, filtered and concentrated under reduced pressure. The crude compound was purified by Combiflash column chromatography eluting with 0-4% ethyl acetate in n-hexane to afford 228 mg (81% yield) of **Int 3** as yellow solid. 1H NMR of **Int 3** shows some impurities which was forwarded for the next step without further purification. **LCMS-Condition-1:** $[M+H]^+$ = 273.10; $R_t$ = 2.18 min. **$^1$H NMR** (400 MHz, DMSO-*d*$_6$) δ: 7.85-7.97 (m, 1H), 7.63-7.72 (m, 1H), 7.33-7.36 (m, 1H), 4.75 (s, 2H), 2.54 (s, 3H).

**Step-3: Synthesis of 2-(4-bromo-2-fluorophenyl)-4-(6-fluoro-3-methylbenzofuran-2-yl)thiazole (Compound-31):**

To a solution of 2-bromo-1-(6-fluoro-3-methylbenzofuran-2-yl)ethan-1-one **3** (225 mg, 0.833 mmol) in ethanol (10 mL) was added 4-bromo-2-fluorobenzothioamide (195 mg, 0.833 mmol) at room temperature. The reaction mixture was then heated at 100 °C and stirred for 3 h. After completion of the reaction (monitored by TLC and LCMS), the reaction mixture was concentrated under reduced pressure and diluted with water (50 mL). The precipitated solid was filtered, washed with ethyl acetate (10 mL) and dried to afford 210 mg (62% yield) of **Compound 31** as white solid. **LCMS-Condition-1:** $[M+H]^+$ = 406.00; $R_t$ = 2.90 min. **$^1$H NMR** (400 MHz, $CDCl_3$) δ: 8.26-8.33 (m, 1H), 7.77 (s, 1H), 7.41-7.51 (m, 3H), 7.20 (dd, *J* = 2.13, 8.91 Hz, 1H), 7.00-7.07 (m, 1H), 2.70 (s, 3H).

**Synthesis of Compound-32(VPC-18-37)** 4-(6-Chloro-1-methyl-1*H*-benzo[*d*]imidazol-2-yl)-2-phenylthiazole

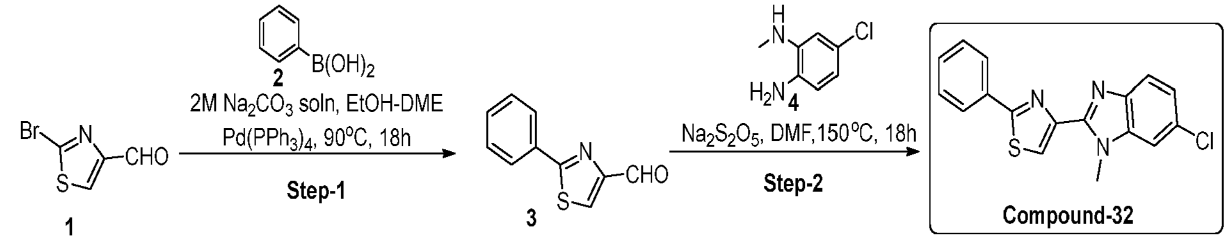


**Step-1: Synthesis of 2-phenylthiazole-4-carbaldehyde (3):**

To a solution of 2-bromothiazole-4-carbaldehyde **1** (1 g, 5.208 mmol) in EtOH:DME (2:1, 30 mL) was added phenylboronic acid **2** (635 mg, 5.208 mmol) and 2N $Na_2CO_3$ solution (5 mL, 10.00 mmol) and degassed with argon for 20 min. To the resulting solution was added $Pd(PPh_3)_4$ (300 mg, 0.260 mmol) and degassed with argon for another 10 min at room temperature. The reaction mixture was then heated at 90 °C for 18 h. After completion of the reaction (monitored by TLC and LCMS), the reaction mixture was quenched with water (50 mL) and extracted with ethyl acetate (3 times). The combined organic layer was dried over anhydrous $Na_2SO_4$, filtered and concentrated under reduced pressure; the crude compound was purified by silica gel column chromatography eluting with 0-8% ethyl acetate in n-hexane to afford 380 mg (38% yield) of **Int 3** as off white solid. **LCMS-Condition-1:** [M+H]+ = 189.95; $R_t$ = 1.63 min **$^1$H NMR** (400 MHz, DMSO-*d*$_6$) δ: 9.99 (s, 1H), 8.78 (s, 1H), 8.00-8.04 (m, 2H), 7.54-7.58 (m, 3H).

**Step-2: Synthesis of 4-(6-chloro-1-methyl-1H-benzo[d]imidazol-2-yl)-2-phenylthiazole (Compound-32):**

To a solution of 2-phenylthiazole-4-carbaldehyde **3** (150 mg, 0.792 mmol) in DMF (30 mL) was added $Na_2S_2O_5$ (180 mg, 0.951 mmol) and 5-chloro-N1-methylbenzene-1,2-diamine **4** (124 mg, 0.792 mmol) at room temperature. The reaction mixture was heated at 150 °C for 18 h. After completion of the reaction, the reaction mixture was cooled to room temperature and quenched with water (25 mL) and extracted with ethyl acetate (3 times). The combined organic layer was washed with brine, dried over anhydrous $Na_2SO_4$, filtered and concentrated under reduced pressure; the crude compound was purified by silica gel column chromatography eluting with 0-15% ethyl acetate in n-hexane to afford 60 mg (23% yield) of **Compound 32** as off white solid. **LCMS-Condition-1:** $[M+H]^+$ = 326.05; $R_t$ = 2.30 min. **$^1$H NMR** (400 MHz, $CDCl_3$) δ: 8.24 (s, 1H), 8.01-8.07 (m, 2H), 7.70 (d, *J* = 8.31 Hz, 1H), 7.47-7.54 (m, 3H), 7.44 (d, *J* = 1.96 Hz, 1H), 7.28 (d, *J* = 1.96 Hz, 1H), 4.31 (s, 3H).

**Synthesis of Compound-33(VPC-18-46)** 2-(2-(2-Fluorophenyl)thiazol-4-yl)-1*H*-benzo[*d*]imidazole-6-sulfonamide

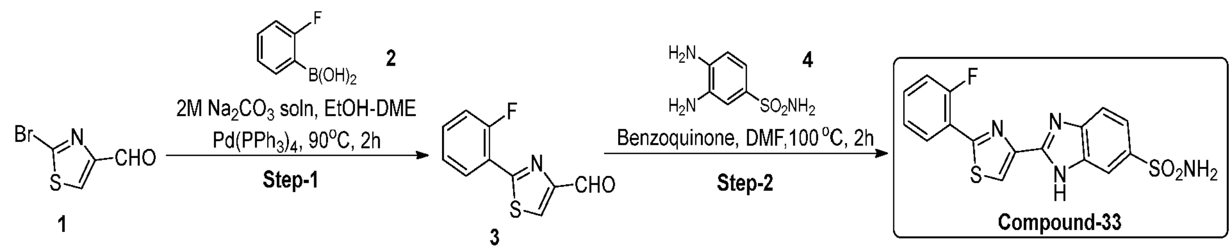


**Step-1: Synthesis of 2-(2-fluorophenyl)thiazole-4-carbaldehyde (3):**

To a solution of 2-bromothiazole-4-carbaldehyde **1** (1 g, 5.208 mmol) in EtOH:DME (2:1, 30 mL) was added (2-fluorophenyl)boronic acid **2** (723 mg, 5.208 mmol) and 2M $Na_2CO_3$ solution (5.2 mL, 10.41 mmol) and degassed with argon for 20 min. To the resulting solution was added $Pd(PPh_3)_4$ (300 mg, 0.260 mmol) and degassed with argon for another 10 min at room temperature. The reaction mixture was heated at 90 °C for 2 h. After completion of the reaction (monitored by TLC and LCMS), the reaction mixture was cooled to room temperature, diluted with water (50 mL) and extracted with ethyl acetate (3 times). The combined organic layer was dried over anhydrous $Na_2SO_4$, filtered and concentrated under reduced pressure; the crude compound was purified by silica gel column chromatography eluting with 0-6% ethyl acetate in n-hexane to afford 386 mg (36% yield) of **Int 3** as off white solid. **LCMS-Condition-1:** [M+H]+ = 207.90; $R_t$ = 1.70 min. **$^1$H NMR** (400 MHz, DMSO-*d*$_6$) δ: 10.03 (s, 1H), 8.88 (s, 1H), 8.27 (t, *J* = 7.58 Hz, 1H), 7.41-7.68 (m, 3H).

**Step-2: Synthesis of 2-(2-(2-fluorophenyl)thiazol-4-yl)-1*H*-benzo[*d*]imidazole-6-sulfonamide (Compound-33):**

To a solution of 2-(2-fluorophenyl)thiazole-4-carbaldehyde **3** (100 mg, 0.483 mmol) in DMF (2 mL) was added benzoquinone (26 mg, 0.240 mmol) and 3,4-diaminobenzenesulfonamide **4** (90 mg, 0.483 mmol) at room temperature. The reaction mixture was heated at 100 °C for 2 h. After completion of the reaction (monitored by TLC and LCMS), the reaction mixture was cooled to room temperature and quenched with water (20 mL) and extracted with ethyl acetate (3 times). The combined organic layer was washed with water and brine, dried over anhydrous $Na_2SO_4$, filtered and concentrated under reduced pressure; the crude compound was purified by recrystallization in ethanol to afford 25 mg (14% yield) of **Compound 33** as pinkish white solid. **LCMS-Condition-1:** [M+H]+ = 375.0; $R_t$ = 1.53 min. **$^1$H NMR** (400 MHz, DMSO-$d_6$) δ: 13.37 (br. s, 1H), 8.67 (s, 1H), 8.49 (t, *J* = 7.34 Hz, 1H), 8.09 (br. s, 1H), 7.70-7.81 (m, 2H), 7.59-7.67 (m, 1H), 7.46-7.55 (m, 2H), 7.33 (br. s, 2H).

**Synthesis of Compound-37(VPC-18-38)** 5-(4-(Benzofuran-2-yl)thiazol-2-yl)-2-oxa-5-azabicyclo[2.2.1]heptane

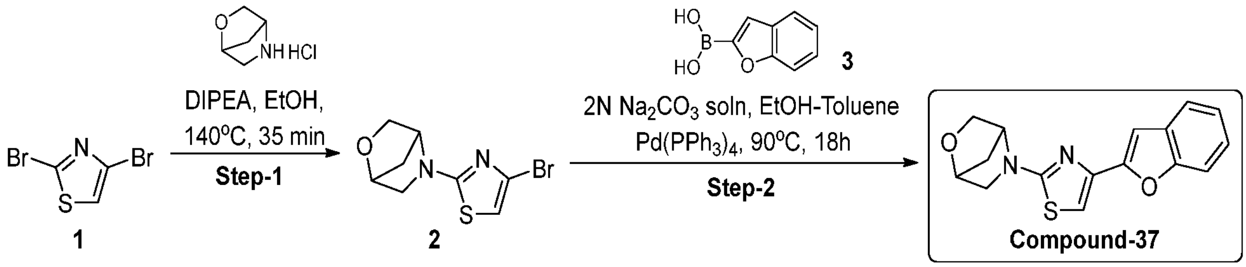


**Step-1: Synthesis of 5-(4-bromothiazol-2-yl)-2-oxa-5-azabicyclo[2.2.1]heptane (2):**

To a solution of 2,4-dibromothiazole **1** (300 mg, 1.234 mmol) in ethanol (15 mL) was added DIPEA (0.64 mL, 3.702 mmol) and 2-oxa-5-azabicyclo[2.2.1]heptane hydrochloride (291 mg, 1.234 mmol) at room temperature. The reaction mixture was irradiated at 140 °C for 35 min in microwave. After completion of the reaction (monitored by TLC and LCMS), the reaction mixture was concentrated under reduced pressure, quenched with water (25 mL) and extracted with ethyl acetate (3 times). The combined organic layer was dried over anhydrous $Na_2SO_4$, filtered and concentrated under reduced pressure; the crude compound was purified by Combiflash column chromatography eluting with 0-1% methanol in DCM to afford 130 mg (41% yield) of **Int 2** as colourless oil. **LCMS-Condition-1:** $[M+H]^+$ = 261.00; $R_t$ = 1.74 min. **$^1$H NMR** (400 MHz, $CDCl_3$) δ: 6.38 (s, 1H), 4.69 (d, *J* = 8.80 Hz, 2H), 3.97 (d, *J* = 7.82 Hz, 1H), 3.85 (d, *J* = 8.31 Hz, 1H), 3.49 (d, *J* = 9.29 Hz, 1H), 3.35 (d, *J* = 9.29 Hz, 1H), 1.99 (s, 2H).

**Step-2: Synthesis of 5-(4-(benzofuran-2-yl)thiazol-2-yl)-2-oxa-5-azabicyclo[2.2.1]heptane (Compound-37):**

To a solution of 5-(4-bromothiazol-2-yl)-2-oxa-5-azabicyclo[2.2.1]heptane **2** (130 g, 0.50 mmol) in ethanol:toluene (1:2, 3 mL) was added benzofuran-2-ylboronic acid **3** (162 mg, 1.00 mmol) and 2N potassium carbonate aqueous solution (1 mL, 276 mg, 2.00 mmol) and degassed with argon for 20 min. To the resulting solution was added $Pd(PPh_3)_4$ (57.8 mg, 0.05 mmol) and degassed with argon for another 10 min at room temperature. The reaction mixture was then heated at 90°C for 18 h. After completion of the reaction (monitored by TLC and LCMS), the reaction mixture was cooled to room temperature, quenched with water (50 mL) and extracted with ethyl acetate (3 times). The combined organic layer was dried over anhydrous $Na_2SO_4$, filtered and concentrated under reduced pressure, the crude compound was purified by silica gel column chromatography eluting with 0-1% methanol in DCM to afford 94 mg (63% yield) of **Compound 37** as light brown solid. **LCMS-Condition-1:** [M+H]+ = 299.10; $R_t$ = 2.12 min. **$^1$H NMR** (400 MHz, $CDCl_3$) δ: 7.57 (d, *J* = 7.34 Hz, 1H), 7.45-7.50 (m, 1H), 7.26-7.30 (m, 1H), 7.17-7.24 (m, 1H), 7.05 (s, 1H), 6.97 (s, 1H), 4.74 (d, *J* = 9.78 Hz, 2H), 4.04 (d, *J* = 7.83 Hz, 1H), 3.90 (d, *J* = 7.34 Hz, 1H), 3.55-3.60 (m, 1H), 3.44-3.49 (m, 1H), 1.96-2.08 (m, 2H).

**Synthesis of Compound-38 (VPC-18-50)** 5-(5-(1-Methyl-1*H*-benzo[*d*]imidazol-2-yl)furan-2-yl)-2-oxa-5-azabicyclo[2.2.1]heptane

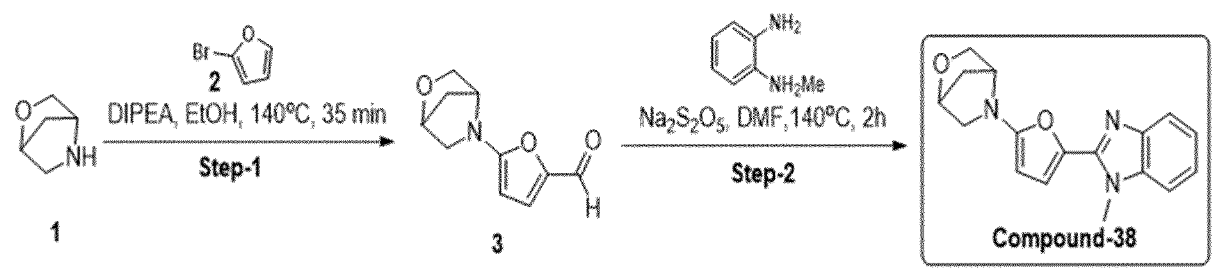


**Step-1: Synthesis of 5-(2-oxa-5-azabicyclo[2.2.1]heptan-5-yl)furan-2-carbaldehyde (2):**

To a solution of 2-oxa-5-azabicyclo[2.2.1]heptane **1** (250 mg, 1.843 mmol) in ethanol (15 mL) was added DIPEA (0.95 mL, 5.511 mmol) and 2-bromofuran (354 mg, 2.022 mmol) at room temperature. The reaction mixture was irradiated at 140 °C for 35 min in microwave. After completion of the reaction (monitored by TLC and LCMS), the reaction mixture was concentrated under reduced pressure, quenched with water (50 mL) and extracted with ethyl acetate (3 times). The combined organic layer was dried over anhydrous $Na_2SO_4$, filtered and concentrated under reduced pressure; the crude compound was purified by Combiflash column chromatography eluting with 0-1% methanol in DCM to afford 170 mg (48% yield) of **Int 3** as colourless oil. **LCMS-Condition-1:** $[M+H]^+$ = 194.00; $R_t$ = 1.09 min. $^1$**H NMR** (400 MHz, $CDCl_3$) δ: 9.03 (s, 1H), 7.21 (br. s, 1H), 5.24 (d, *J* = 3.94 Hz, 1H), 4.63 - 4.74 (m, 2H), 3.83 - 3.93 (m, 2H), 3.38 - 3.55 (m, 2H), 1.94 - 2.05 (m, 2H).

**Step-2: Synthesis of 5-(5-(1-methyl-1*H*-benzo[d]imidazol-2-yl)furan-2-yl)-2-oxa-5-azabicyclo[2.2.1]heptane (Compound-38):**

To a solution of 5-(2-oxa-5-azabicyclo[2.2.1]heptan-5-yl)furan-2-carbaldehyde **2** (160 mg, 0.829 mmol) and 2-(methyl-l4-azaneyl)aniline (101 mg, 0.829 mmol) in DMF (3 mL) was added $Na_2S_2O_5$ (196 mg, 1.031 mmol) at room temperature. The reaction mixture was heated at 140 °C for 2 h. After completion of the reaction (monitored by TLC and LCMS), the reaction mixture was cooled to room temperature and diluted with water (20 mL) and extracted with ethyl acetate (3 times). The combined organic layer was dried over anhydrous $Na_2SO_4$, filtered and concentrated under reduced pressure; the crude compound was purified by preparative HPLC to afford 70 mg (28% yield) of **Compound 38** as yellow solid. **LCMS-Condition-1:** $[M+H]^+$ = 296.05; $R_t$ = 1.08 min. $^1$**H NMR** (400 MHz, $CDCl_3$) δ: 7.71 - 7.76 (m, 1H), 7.29-7.33 (m, 1H), 7.21 - 7.26 (m, 3H), 7.07 (d, *J* = 3.42 Hz, 1H), 5.25 (d, *J* = 3.42 Hz, 1H), 4.65-4.68 (m, 1H), 4.50 - 4.53 (m, 1H), 3.96 (s, 3H), 3.84 (d, *J* = 7.82 Hz, 1H), 3.51 (d, *J* = 9.29 Hz, 1H), 3.35 (d, *J* = 9.78 Hz, 1H), 1.94 - 2.05 (m, 2H).

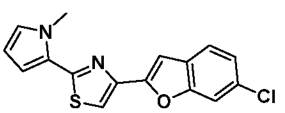
VPCFTE-111

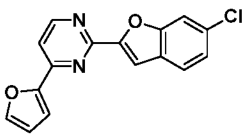
VPCFTE-120

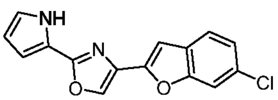
VPCFTE-95

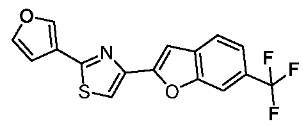
VPCFTE-72

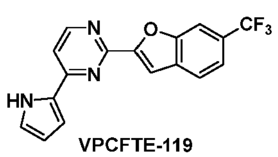
VPCFTE-119

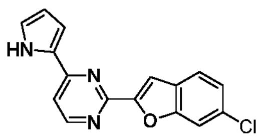
VPCFTE-105

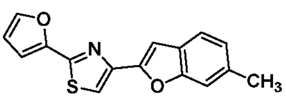
VPCFTE-68

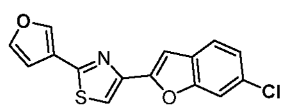
VPCFTE-73

**VPCFTE111 (VPC-18-139)**

**4-(6-chlorobenzofuran-2-yl)-2-(1-methyl-1H-pyrrol-2-yl)thiazole**

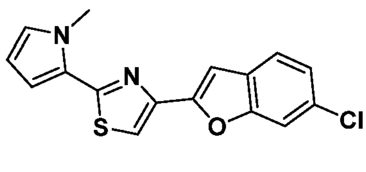

**VPCFTE-111**

**General Synthetic Scheme:**

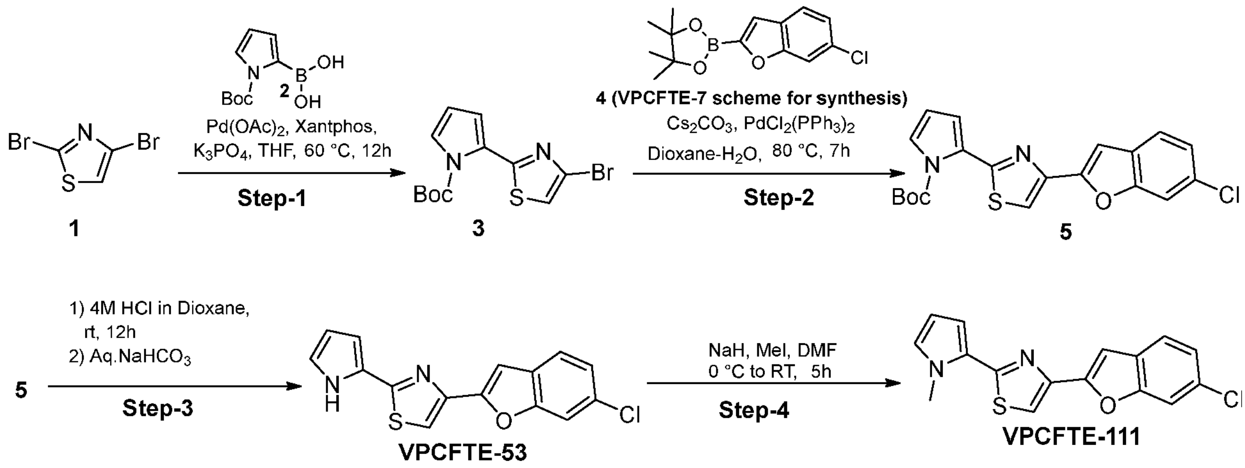


**Note:** Synthesis of **3,5** and **VPCFTE-53** reported in **VPCFTE-53** scheme
Synthesis of **4** reported in **VPCFTE-7** scheme

**Note:** Synthesis of **3, 5** and **VPCFTE-53 (Steps 1-3)** reported in VPCFTE-53 scheme Synthesis of **4** reported in **VPCFTE-7** scheme

**Step 4: 4-(6-chlorobenzofuran-2-yl)-2-(1-methyl-1H-pyrrol-2-yl)thiazole (VPCFET-111)**

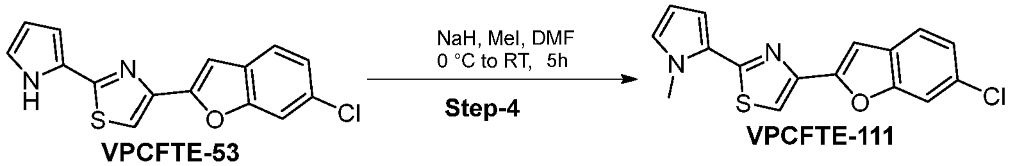


To a stirred solution of 4-(6-chlorobenzofuran-2-yl)-2-(1H-pyrrol-2-yl)thiazole **VPCFTE-53** (0.15 g, 0.5 mmol) in DMF (10 mL) was added NaH (0.024 g, 2 mmol) at 0°C, resulting RM was stirred for 15 min at RT, and MeI (0.11 g, 0.75 mmol) was added to it at 0°C, resulting reaction mixture (RM) was allowed to stirred at RT for 5h. Reaction was monitored by TLC for disappearance of starting material (SM). RM was quenched (SM was consumed as indicated by TLC) with ice cold water and extracted with ethyl acetate, dried over sodium sulphate and concentrated under reduced pressure to afford crude product. Crude was purified by column chromatography using silica gel, followed by prep-HPLC to afford 4-(6-chlorobenzofuran-2-yl)-2-(1-methyl-1H-pyrrol-2-yl)thiazole **VPCFTE-111** as a pale yellow solid. (Yield = 0.06 gm, 38.21%).

**HPLC:** 94.73% **LCMS-Condition-1:** [M+H]+ = 314.9; $R_t$ = 2.47 min.

**$^1$H NMR** (400 MHz, DMSO-$d_6$) δ 7.96 (s, 1H), 7.82 (s, 1H), 7.72 (d, *J*=8.31 Hz, 1H), 7.34-7.35 (m, 2H), 7.06 (s, 1H), 6.75-6.77 (m, 1H), 6.14 - 6.18 (m, 1H), 4.06 (s, 3H).

<u>**VPCFTE119 (VPC-18-140)**</u>

**4-(1H-pyrrol-2-yl)-2-(6-(trifluoromethyl)benzofuran-2-yl)pyrimidine**

**General Synthetic Scheme:**

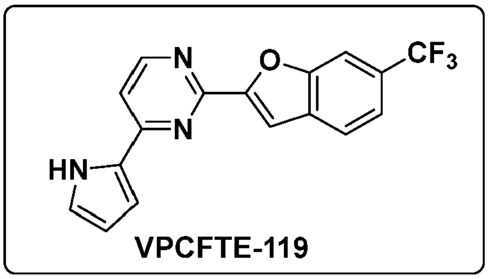


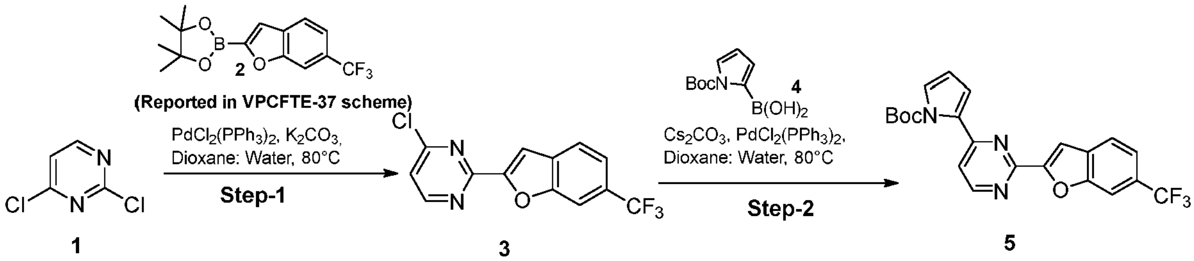


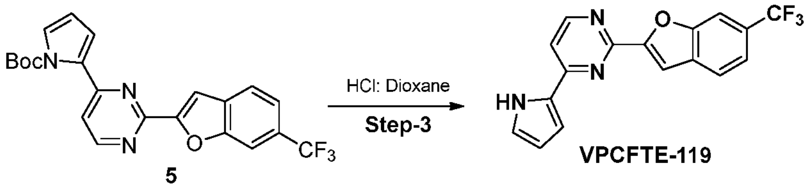


**Note:** Synthesis of **2** reported in **VPCFTE-37** scheme

**Note:** Synthesis of **2** reported in **VPCFTE-37** scheme

**Step -1 Synthesis of 4-chloro-2-(6-(trifluoromethyl)benzofuran-2-yl)pyrimidine (3)**

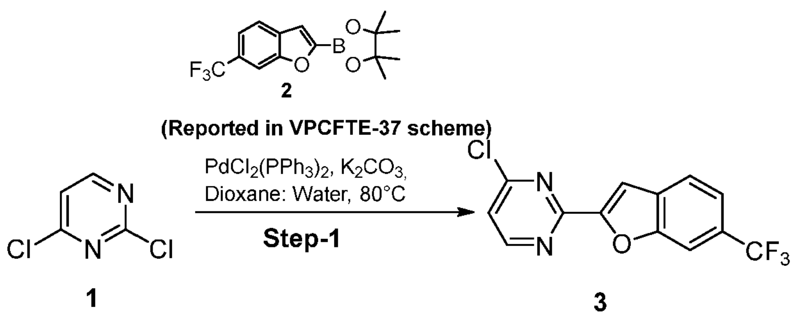


To a degassed mixture of 2,4-dichloropyrimidine **1** (500 mg, 1.6 mmol) and **Int- 2** (262 mg, 1.76 mmol) in Dioxane: Water (25mL : 8mL) was added $K_2CO_3$ (442 mg, 3.21 mmol) followed by $PdCl_2(PPh_3)_2$ (112 mg, 0.16 mmol) in sealed tube under argon atmosphere at room temperature. Stirred the reaction mixture (RM) at 80 °C for 7 h. After completion of reaction (checked by TLC 10% EtOAc :Hexane) RM was cooled to room temperature, solvent was removed under reduced pressure. Crude solid obtained was dissolved in ethyl acetate (50 mL) and washed with water (2×25

mL), organic layer was separated, dried over anhydrous $Na_2SO_4$ and conc. under reduced pressure. Crude product obtained was purified by column chromatography using 100-200 mesh silica using 1-2 % EtOAc: Hexane as an eluent to afford 4-chloro-2-(6-(trifluoromethyl)benzofuran-2-yl)pyrimidine as off white solid (Yield- 300 mg, 65.20%).

**LCMS-Condition:** [M+H] + =299.20 $R_t$ = 2.11 min

**Step 2: Synthesis of tert-butyl 2-(2-(6-(trifluoromethyl)benzofuran-2-yl)pyrimidin-4-yl)-1H-pyrrole-1-carboxylate (5)**

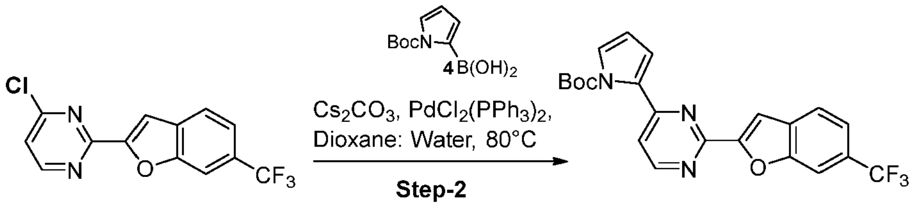


3 5

To a degassed mixture of 4-chloro-2-(6-(trifluoromethyl)benzofuran-2-yl)pyrimidine **3** (150 mg,1.0 mmol) and (1-(tert-butoxycarbonyl)-1H-pyrrol-2-yl)boronic acid **Int 4** (127 mg,1.1 mmol) in Dioxane: Water (20mL : 3 mL) was added $Cs_2CO_3$ (327 mg, 2.0 mmol) followed by $PdCl_2(PPh_3)_2$ (35 mg, 0.1 mmol) in sealed tube under argon atmosphere at room temperature. Stirred the reaction mixture at 80°C for 7h. After completion of reaction (checked by TLC 10% EtOAc: Hexane) cooled to room temperature, solvent was removed under reduced pressure. Crude solid obtained was dissolved ethyl acetate (50 mL) and washed with water (2×25 mL) organic layer was separated, dried over anhydrous $Na_2SO_4$ and conc. under reduced pressure. Crude product obtained was purified by column chromatography using 100-200 mesh silica using 2 % EtOAc: Hexane as an eluent to afford **VPCFTE119** as an off white solid (Yield- 100 mg, 47.6%).

**LCMS-Condition:** [M-Boc+1]$^+$=330.20 $R_t$ = 2.39 min

**Step 3: 4-(1H-pyrrol-2-yl)-2-(6-(trifluoromethyl)benzofuran-2-yl)pyrimidine VPCFTE-119**

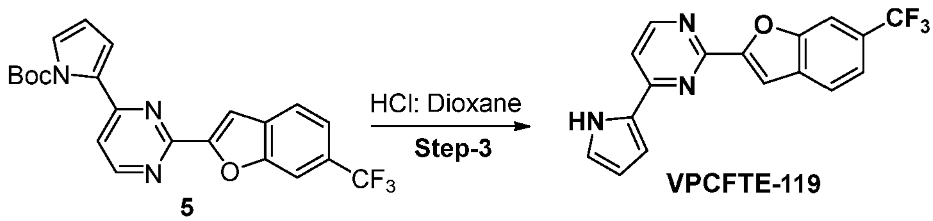


5

**VPCFTE-119**

To a stirred solution of tert-butyl 2-(2-(6-(trifluoromethyl)benzofuran-2-yl)pyrimidin-4-yl)-1H-pyrrole-1-carboxylate **5** (100 mg, 1 mmol) in Dioxane (10 mL) was added 4 M HCl in Dioxane (10 ml) at 0°C, resulting RM was stirred for 12 hr. at RT, After complete consumption of SM (Checked by TLC, 20% EA in Hex), Reaction mixture was concentrate under reduced pressure to get semi solid was neutralized by using aqueous solution of sodium bicarbonate to get solid was filter and crude was purified by column chromatography using silica gel, followed by prep-HPLC to afford 4-(1H-pyrrol-2-yl)-2-(6-(trifluoromethyl)benzofuran-2-yl)pyrimidine **VPCFTE-119** 0.03 gm as an off white solid. (Yield = 30 mg, 39%)

**HPLC:** 97.72% **LCMS-Condition-1:** [M+H]+ = 329.90; $R_t$ = 2.15 min.

**$^1$H NMR** (400 MHz, DMSO-$d_6$) δ 11.80 (brs, 1H), 8.88 (d, *J*=5.38 Hz, 1H), 8.21 (s, 1H), 8.16 (s, 1H), 8.07 (d, *J*=8.31 Hz, 1H), 7.71 (d, *J*=8.31 Hz, 1H), 7.64 (d, *J*=4.89 Hz, 1H), 7.02 - 7.08 (m, 2H), 6.25 (d, *J*=1.96 Hz, 1H).

**VPCFTE120 (VPC-18-141)**

**2-(6-chlorobenzofuran-2-yl)-4-(furan-2-yl)pyrimidine**

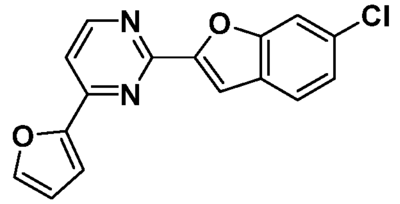

**VPCFTE-120**

**General Synthetic Scheme:**

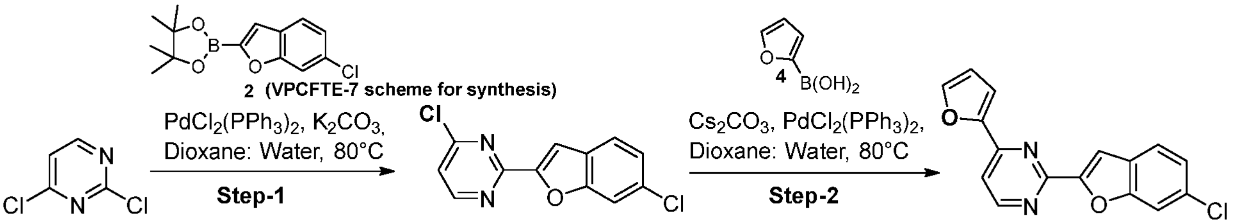


**1** **3** **VPCFTE-120**

**Note: Synthesis of Int-2 reported in VPCFTE-7 scheme.**

**Note:** Synthesis of **Int-2** reported in **VPCFTE-7 scheme.**

**Step Synthesis of 1 4-chloro-2-(6-chlorobenzofuran-2-yl)pyrimidine (3)**

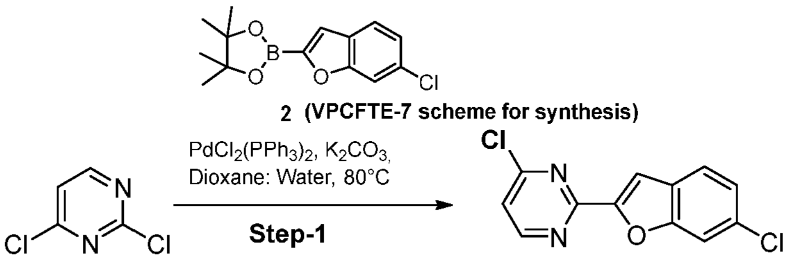


**1** **3**

**Note: Synthesis of Int-2 reported in VPCFTE-7 scheme.**

To a degassed mixture of 2-(6-chlorobenzofuran-2-yl)-4,4,5,5-tetramethyl-1,3,2-dioxaborolane **2** (0.5 g, 3.38 mmol) and **Int-1** (1.4 g, 5.40 mmol.) in Dioxane: Water (30mL : 10mL) was added $K_2CO_3$ (0.93 g, 6,76 mmol) followed by $PdCl_2(PPh_3)_2$ (0.24g, 0.34 mmol) in sealed tube under argon atmosphere at room temperature. Stirred the reaction mixture at 80° C for 7h. After completion of reaction (checked by TLC 20% EtOAc :Hexane) cooled to room temperature, solvent was removed under reduced pressure. Crude solid obtained was purified by column chromatography using 100-200 mesh silica using 15-20% EtOAc: Hexane as an eluent to afford product as a brown solid 4-chloro-2-(6-chlorobenzofuran-2-yl)pyrimidine (Yield- 0.3g, 33.67%)

**LCMS-Condition-1:** [M+H]+ = 265.20; $R_t$ = 2.08min.

**Step 2: Synthsesis of 2-(6-chlorobenzofuran-2-yl)-4-(furan-2-yl)pyrimidine (VPCFTE-120)**

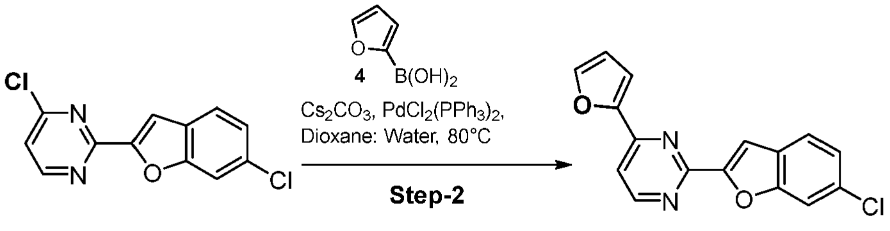


3 VPCFTE-120

To a degassed mixture of 4-chloro-2-(6-chlorobenzofuran-2-yl)pyrimidine **3** (0.3 g, 1.14 mmol) and **Int-4** (0.19 g, 1.7 mmol) in Dioxane: Water (16mL: 4mL) was added $Cs_2CO_3$ (0.74 g, 2.27 mmol) followed by PdCl2 $(PPh_3)_2$ (0.08 g, 0.11 mmol) in sealed tube under argon atmosphere at room temperature and stirred at 8o°C for 7h. After completion of reaction (checked by TLC 20% EtOAc : Hexane); RM was cooled to room temperature, solvent was removed under reduced pressure. Crude solid obtained was purified by column chromatography using 100-200 mesh silica using 15-20% EtOAc: Hexane as an eluent to afford 2-(6-chlorobenzofuran-2-yl)-4-(furan-2-yl)pyrimidine **VPCFTE-120** as white solid (Yield- 0.05g, 14.88%).

**HPLC:** 96.86% **LCMS-Condition-1:** $[M+H]^+$ = 297.10; $R_t$ = 2.20 min.

**$^1$H NMR** (400 MHz, DMSO-$d_6$) δ 8.94 (d, *J*=5.38 Hz, 1H), 7.95 - 8.00 (m, 3H), 7.83 (d, *J*=8.31 Hz, 1H), 7.80 (d, *J*=5.38 Hz, 1H), 7.40 - 7.45 (m, 2H), 6.74-6.76 (m, 1H).

<u>**VPCFTE73 (VPC-18-105)**</u>

**4-(6-chlorobenzofuran-2-yl)-2-(furan-3-yl)thiazole**

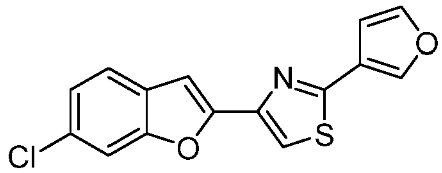

**VPCFTE-73**

**General Synthetic Scheme:**

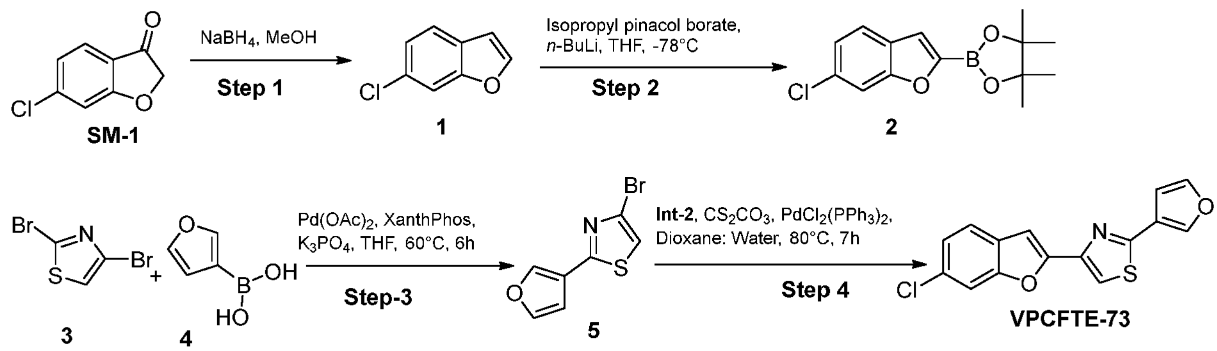


SM-1 1 2

3 4 5 VPCFTE-73

**Note: Synthesis of Int-1 and Int-2 reported in VPCFTE-7 scheme.**

**Note:** Synthesis of **Int-1** and **Int-2 (Step 1 and 2**) reported in **VPCFTE-7** scheme.

**Step 3: Synthesis of 4-bromo-2-(furan-3-yl)thiazole (5)**

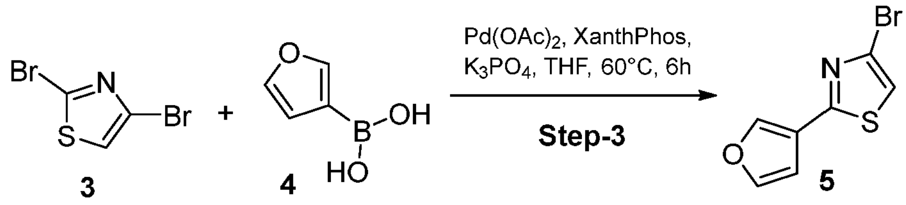


To a degassed mixture of 2,4-dibromothiazole **3** (250 mg, 1.0 mmol) and furan-3-ylboronic acid **4** (116.3 mg, 1.0 mmol) in THF (5 mL) under argon atmosphere was added $K_3PO_4$ (440.7 mg, 2.0 mmol). After stirring for 5 min added $Pd(OAc)_2$ (23.3 mg, 0.1 mmol) followed by XanthPhos (60.06 mg, 0.1 mmol) in sealed tube under argon atmosphere at room temperature. The reaction mixture was stirred at 60°C for 4h. After completion of reaction (checked by TLC, 5% EtOAc : Hexane) the reaction mixture was cooled to room temperature and the solvent was removed under reduced pressure. Crude solid obtained was purified by column chromatography (100-200mesh silica using 0.5 to 1% EtOAc: Hexane as an eluent) to afford 4-bromo-2-(furan-3-yl)thiazole **5** as a light brown solid (Yield- 200 mg, 84.1%).

**LCMS-Condition-:** $[M+H]^+$ =231.7 $R_t$ =1.84

**Step 4: Synthesis of 4-(6-chlorobenzofuran-2-yl)-2-(furan-3-yl)thiazole (VPCFTE-73)**

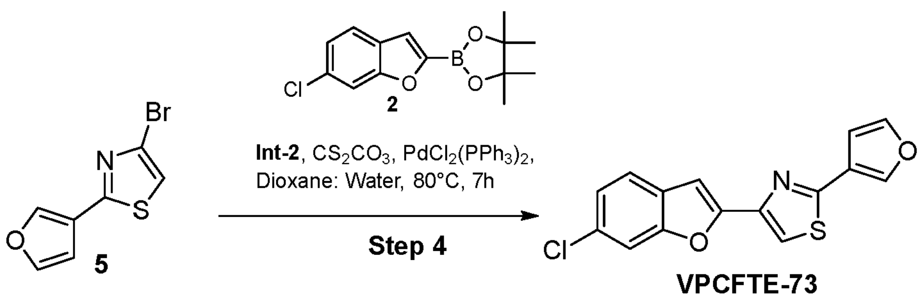


To a degassed mixture of 4-bromo-2-(furan-3-yl)thiazole **5** (100mg, 0.44 mmol) and 2-(6-chlorobenzofuran-2-yl)-4,4,5,5-tetramethyl-1,3,2-dioxaborolane **Int-2** (157.9mg, 0.57 mmol) in Dioxane: Water (10mL : 3mL) was added $CS_2CO_3$ (284.6mg, 0.87 mmol) followed by $PdCl_2(PPh_3)_2$ (30.6mg, 0.04 mmol) in sealed tube under argon atmosphere at room temperature. Stirred the reaction mixture at 80°C for 7h. After completion of reaction (checked by TLC, 5% EtOAc: Hexane) cooled to room temperature, solvent was removed under reduced pressure. Crude solid obtained was dissolved in ethyl acetate (40 mL) and washed with water (2×20 mL). Organic layer was separated, dried over anhydrous $Na_2SO_4$ and concentrated under reduced pressure. Crude product obtained was purified by column chromatography (100-200 mesh silica using 1-2% EtOAc: Hexane as an eluent) followed by Prep-HPLC purification to afford **VPCFTE73** as an off white solid (Yield- 12 mg, 9.1%).

**HPLC:** 99.15% **LCMS-Condition-1:** [M+H]+ = 301.90; $R_t$ = 2.32 min.

$^1$**H NMR** (400 MHz, $CDCl_3$) δ 7.66 (s, 1H), 7.57 (s, 1H), 7.53 - 7.56 (m, 2H), 7.24-7.25 (m, 2H), 7.13 (d, *J*=3.42 Hz, 1H), 6.59 (dd, *J*=3.18, 1.71 Hz, 1H).

<u>**VPCFTE68 (VPC-18-11)**</u>

**2-(furan-2-yl)-4-(6-methylbenzofuran-2-yl)thiazole**

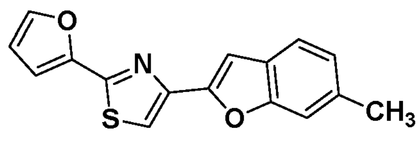

**VPCFTE-68**

**General Synthetic Scheme:**

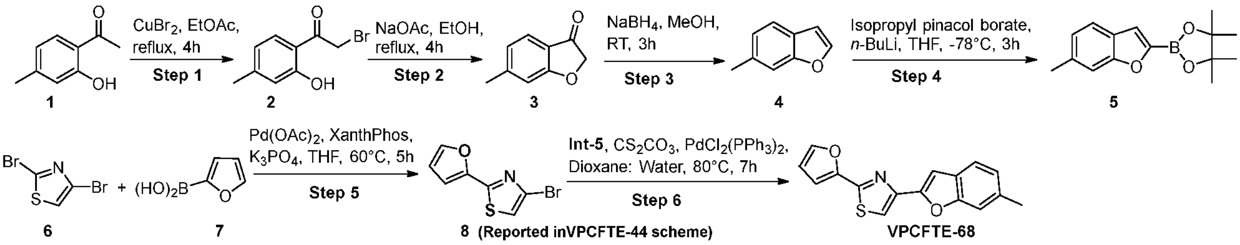


**Note:** Synthesis of **8** reported in **VPCFTE-44** scheme

**Step-1: Synthesis of 2-bromo-1-(2-hydroxy-4-methylphenyl)ethan-1-one (2):**

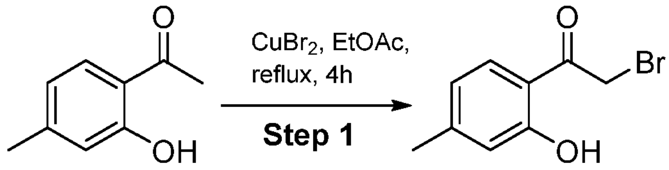


**1** **2**

To a stirred solution of 1-(2-hydroxy-4-methylphenyl) ethan-1-one **1** (1 g, 6.66 mmol) in EtOAc (30mL) was added $CuBr_2$ (2.23g, 10 mmol) at room temperature. Reaction mixture (RM) was allowed to stir at 8o°C for 4h. After completion, (checked by TLC, 5% EtOAc: Hexane) RM was cooled to room temperature, diluted with EtOAc (~50mL) and filtered through celite bed. Filtrate was then washed with water (2×30mL). Organic layer was separated, dried over anhydrous $Na_2SO_4$ and evaporated under reduced pressure. Crude product thus obtained, (brown oil) was submitted for next step without further purification. (Yield-1.4g crude)

**Step 2: Synthesis of 6-methylbenzofuran-3(2H)-one (3):**

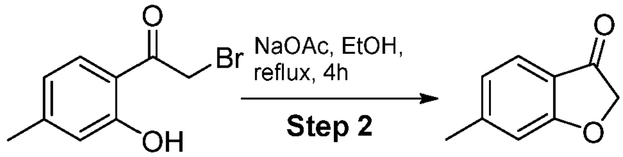


**2** **3**

To a stirred solution 2-bromo-1-(2-hydroxy-4-methylphenyl)ethan-1-one **2** (1.4 g, 1.0 mmol) in EtOH (30 mL) was added NaOAc (1.51g, 3.0 mmol) at room temperature and reaction mixture (RM) stired at 8o°C for 4h. After completion, RM was (checked by TLC, 10% EtOAc: Hexane), cooled to RT and Solvent was removed under reduced pressure to obtain crude product. Crude product was taken into EtOAc (somL) and washed with water (2×30mL). Organic layer was separated, dried over anhydrous $Na_2SO_4$ and conc. under reduced pressure. Crude product was then purified by column chromatography using 100-200 mesh silica using 3-4% EtoAc: Hexane as an eluent to afford 0.3g (0.33 yield) of **3** as an yellow solid.

**LCMS condition:** $[M+H]^+$ =148.90 $R_t$ =1.51

**Step 3: Synthesis of 6-methylbenzofuran (4):**

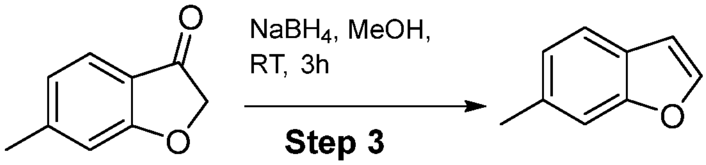


3 4

To 6-methylbenzofuran-3(2H)-one **3** (300mg, 1.0mmol) in methanol (6 mL) $NaBH_4$ (191.6mg, 2.5mmol) was added in portion at room temperature (RT). Reaction mixture (RM) was allowed to stir for 3h at RT. After completion (checked by TLC, 10% EtOAc: Hexane), RM was quenched with acetone (3 mL), followed by 3N HCl (3 mL) and stirred for another 1h. The resulting RM was extracted with EtOAc (2 × 25 mL), and combined organic layer was washed with water (2×10 mL), dried over anhydrous $Na_2SO_4$ and evaporated under reduced pressure. Crude product thus obtained was purified by column chromatography using 100-200 mesh silica using 2-4% EtOAc: Hexane as an eluent to afford 200mg (74.7% yield) of **4** as a light pink oil.

**$^1$H NMR** (400 MHz, $CDCl_3$) δ 7.55 (d, *J*=2 Hz, 1H), 7.48 (d, *J*=8 Hz, 1H), 7.32 (s, 1H), 7.07 (d, *J*=7.6 Hz, 1H), 6.72 (s, 1H), 2.48 (s, 3H)

**Step 4: Synthesis of 4,4,5,5-tetramethyl-2-(6-methylbenzofuran-2-yl)-1,3,2-dioxaborolane (5)**:

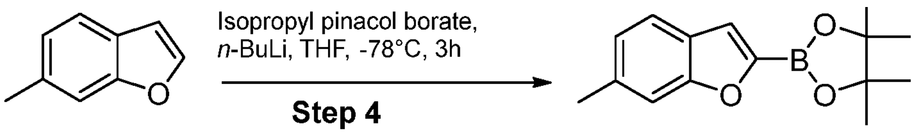


4 5

To a stirred solution 6-methylbenzofuran **4** (200 mg, 1.51 mmol) in THF (5 mL) was added *n*-BuLi 2.5M in hexane (0.72 mL, 1.82mmol) at -78°C and stirred for 1.5h. Isopropyl pinacol borate (545 mg, 2.0mmol) was then added to RM in dropwise manner while maintaining temperature at -78°C. The reaction mixture (RM) was allowed to stir further for 1.5h at -78°C. After completion, (checked by TLC, 20% EtOAc: Hexane), RM was quenched by sat.$NH_4Cl$ (20 mL) and extracted using ethyl acetate (3×20 mL). Combined organic layer was then washed with water (20 mL), dried over anhydrous $Na_2SO_4$ and evaporated under reduced pressure. Crude product thus obtained was triturated using pentane (2×20mL) to afford 350 mg (89.5% yield) of **5** as a pink solid. It was carried forward to next step without further purification.

**Step 6: Synthesis of 2-(furan-2-yl)-4-(6-methylbenzofuran-2-yl)thiazole (VPCFTE-68):**

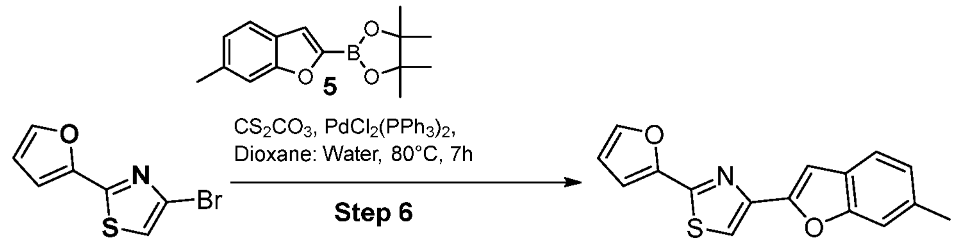


**8** (Reported inVPCFTE-44 scheme) **VPCFTE-68**

To a degassed mixture of 4-bromo-2-(furan-2-yl)thiazole **8** (150mg, 0.66 mmol) and 4,4,5,5-tetramethyl-2-(6-methylbenzofuran-2-yl)-1,3,2-dioxaborolane **Int-5** (219.8mg, 0.85 mmol) in Dioxane: Water (15mL: 4.5mL) was added $CS_2CO_3$ (426.9mg, 1.31 mmol) followed by $PdCl_2(PPh_3)_2$ (46mg, 0.07mmol) in sealed tube under argon atmosphere at room temperature. Stirred the reaction mixture (RM) at 80°C for 7h. After completion, RM (checked by TLC, 5% EtOAc :Hexane) was cooled to room temperature, solvent was removed under reduced pressure. Crude solid obtained was dissolved ethyl acetate (50 mL) and washed with water (2×25 mL). The organic layer thus separated was dried over anhydrous $Na_2SO_4$ and evaporated under reduced pressure. Resulting Crude product was purified by column chromatography using 100-200 mesh silica using 1-2% EtOAc: Hexane as an eluent to afford 78 mg (42.3% yield) of **VPCFTE-68** as pale yellow solid.

**HPLC:** 96.55% **LCMS-Condition-1:** [M+H]+ = 281.90; $R_t$ = 2.28 min.

$^1$H NMR (400 MHz, DMSO-$d_6$) δ 8.09 (s, 1H), 7.94-7.96 (m, 1H), 7.58 (d, *J*=8.31 Hz, 1H), 7.46 (s, 1H), 7.28 (s, 1H), 7.23 (d, *J*=3.42 Hz, 1H), 7.12 (d, *J*=7.82 Hz, 1H), 6.76 (dd, *J*=3.18, 1.71 Hz, 1H), 2.45 (s, 3H).

**VPCFTE105 (VPC-18-133)**

**2-(6-chlorobenzofuran-2-yl)-4-(1H-pyrrol-2-yl)pyrimidine**

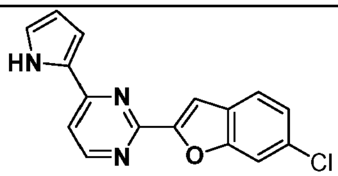

**VPCFTE-105**

**General Synthetic Scheme:**

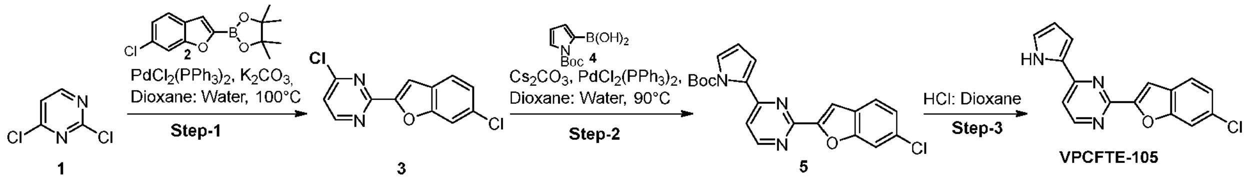


Note: Synthesis of 1,2,3 reported in VPCFTE-120 scheme

Note: Synthesis of **1, 2, 3** (Step 1) reported in VPCFTE-120 scheme

**Step 2: Synthesis of tert-butyl 2-(2-(6-chlorobenzofuran-2-yl)pyrimidin-4-yl)-1H-pyrrole-1-carboxylate**

To a degassed mixture of 4-chloro-2-(6-chlorobenzofuran-2-yl)pyrimidine **3** (0.2 g, 0.75 mmol) and **Int-4** (0.17 g, 0.79 mmol) in Dioxane: Water (20mL: 6mL) was added $Cs_2CO_3$ (0.49 g, 1.51 mmol) followed by addition of $PdCl_2(PPh_3)_2$ (0.053 g, 0.07 mmol) in sealed tube under argon atmosphere at room temperature. And the reaction mixture stirred at 90°C for 12h. After completion (checked by TLC 20% EtOAc: Hexane); RM was cooled to room temperature,

solvent was removed under reduced pressure. Crude solid thus obtained was purified by column chromatography using 100-200 mesh silica using 4-5% EtOAc: Hexane as an eluent to afford tert-butyl 2-(2-(6-chlorobenzofuran-2-yl)pyrimidin-4-yl)-1H-pyrrole-1-carboxylate **(Int-5)**as white solid (Yield- 0.2g, 66.8%).

**LCMS Condition:** $[M+H]^+$ =396.35 $R_t$=2.42 min

**Step 3: Synthesis of 2-(6-chlorobenzofuran-2-yl)-4-(1H-pyrrol-2-yl)pyrimidine (VPCFTE-105):**

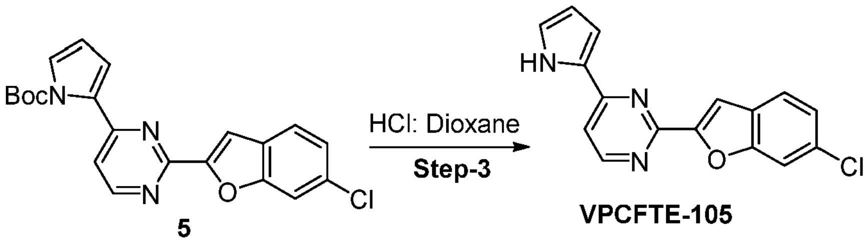


**5** **VPCFTE-105**

To a stirred solution of tert-butyl 2-(2-(6-chlorobenzofuran-2-yl)pyrimidin-4-yl)-1H-pyrrole-1-carboxylate **5** (200 mg, 0.51mmol) in 1,4 Dioxane (2.0 mL) was added 4 M HCl in Dioxane (2 ml) at 0°C, resulting reaction mixture was stirred for 12 hr. at RT. After completion, as checked by TLC ( 20% EtOAC:Hexane)); solvent was evaporated under reduced pressure and crude product was neutralized using aqueous solution of $NaHCO_3$, solid precipitate thus obtained was filtered and dried in vacuum. Crude product was purified by column chromatography using 100-200 mesh silica using 6-7% EtOAc: Hexane as an eluent to afford product as a light grey colored semisolid which was triturated using pentane (2 × 10mL) afford 80 mg (53.5% yield) of **VPCFTE-105** as an light grey colored solid.

**HPLC:** 99.34% **LCMS-Condition-1:** [M+H]+ = 295.90; $R_t$ = 2.12 min.

**$^1$H NMR** (400 MHz, DMSO-$d_6$) δ 11.75 (brs, 1H), 8.84 (d, *J*=4.89 Hz, 1H), 8.06 (s, 1H), 7.93 (s, 1H), 7.86 (d, *J*=8.31 Hz, 1H), 7.58 (d, *J*=4.89 Hz, 1H), 7.42 (dd, *J*=8.31, 1.47 Hz, 1H), 7.00-7.06 (m, 2H), 6.24 (d, *J*=3.42 Hz, 1H).

**VPCFTE72 (VPC-18-104)**

**2-(furan-3-yl)-4-(6-(trifluoromethyl)benzofuran-2-yl)thiazole**

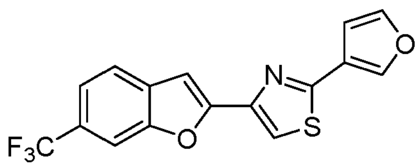

**VPCFTE-72**

**General Synthetic Scheme:**

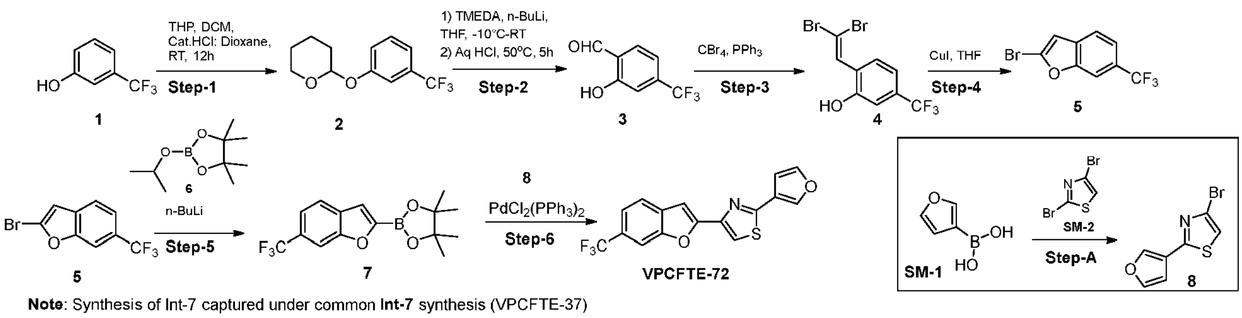


**Note**: Synthesis of Int-7 captured under common **Int-7** synthesis (VPCFTE-37)

Note: Synthesis of **Int-7** captured under common Int-7 synthesis **(steps 1-5)** (VPCFTE-37)

**Step 6: Synthesis of 2-(furan-3-yl)-4-(6-(trifluoromethyl)benzofuran-2-yl)thiazole**

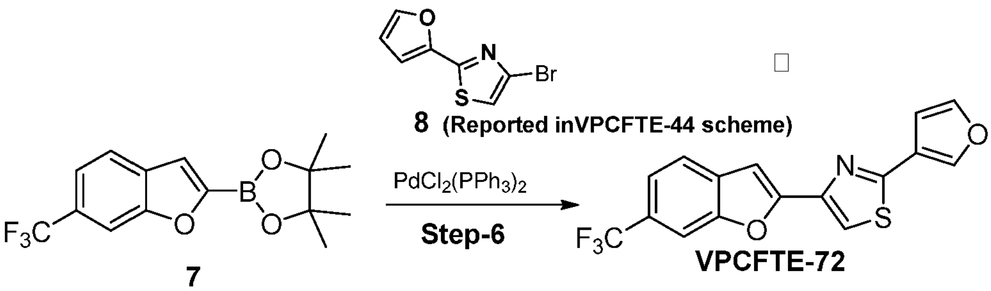


To a degassed mixture of 4,4,5,5-tetramethyl-2-(6-(trifluoromethyl)benzofuran-2-yl)-1,3,2-dioxaborolane 7 (177.2mg, 0.57 mmol) and 4-bromo-2-(furan-2-yl)thiazole **8** (100mg, 0.44 mmol) in Dioxane: Water (10mL: 3mL) was added $CS_2CO_3$ (284.6mg, 0.87 mmol) followed by $PdCl_2(PPh_3)_2$ (30.6mg, 0.04 mmol) in sealed tube under argon atmosphere at room temperature. The reaction mixture was stirred at 8o°C for 7h. After completion, reaction mixture (checked by TLC, 5% EtOAc :Hexane) cooled to room temperature, solvent was removed under reduced pressure. Crude solid thus obtained was dissolved ethyl acetate (40 mL) and washed with water (2×20 mL). Organic layer was separated, dried over anhydrous $Na_2SO_4$ and evaporated under reduced pressure. Crude product thus obtained was purified by column chromatography using 100-200 mesh silica using 1-2% EtOAc: Hexane as an eluent followed by Prep-HPLC purification to afford **VPCFTE72** as an off white solid (Yield- 15 mg, 10.2%).

**HPLC:** 99.36% **LCMS-Condition-1:** [M+H]+ = 335.9; $R_t$ = 2.32 min.

**$^1$H NMR** (400 MHz, $CDCl_3$) δ 7.81 (s, 1H), 7.71 - 7.75 (m, 2H), 7.58 (s, 1H), 7.53 (d, *J*=8.31 Hz, 1H), 7.33 (s, 1H), 7.14 (d, *J*=3.42 Hz, 1H), 6.60 (dd, *J*=3.18, 1.71 Hz, 1H).

**<u>VPCFTE95 (VPC-18-127)</u>**

**4-(6-chlorobenzofuran-2-yl)-2-(1H-pyrrol-2-yl)oxazole**

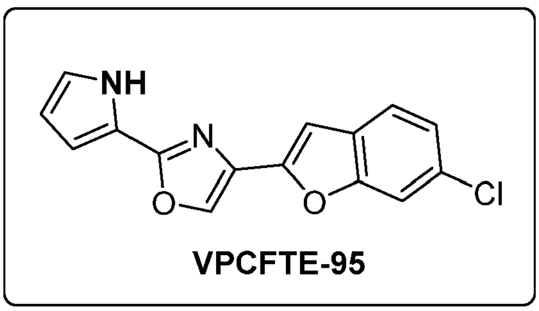

**General Synthetic Scheme:**

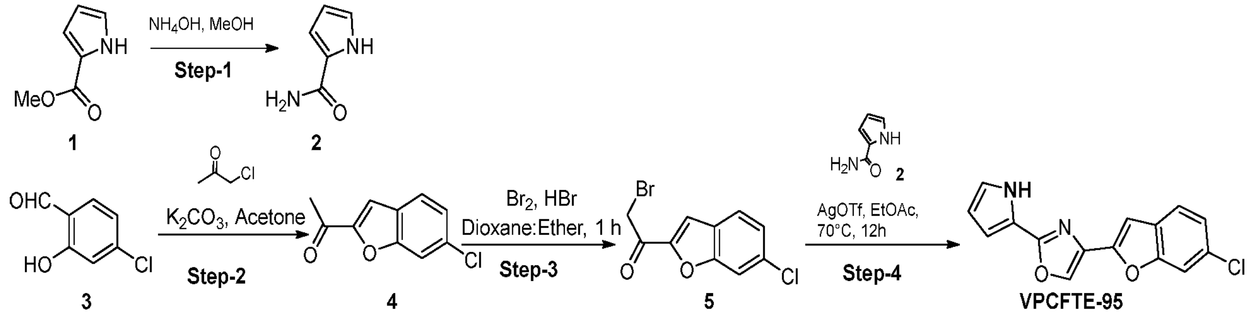


**Step 1: Synthesis of 1H-pyrrole-2-carboxamide (2):**

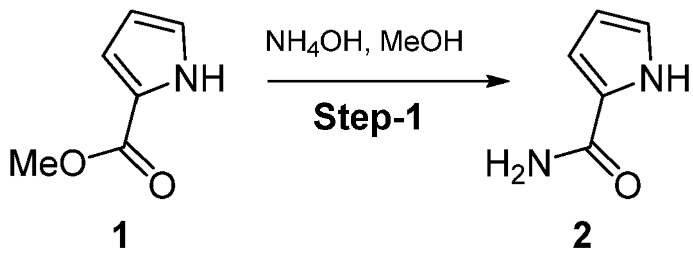


To a degassed mixture of methyl 1H-pyrrole-2-carboxylate **1** (0.5 g, 4.0 mmol) in MeOH (10 mL) was added $NH_4OH$ (20 mL) at RT and stirred for 12 h. After completion of reaction (checked by TLC, 10% EtOAc :Hexane), Reaction mass was concentrate under reduced pressure, diluted with cold water and extracted with ethyl acetate (3 × 50 mL). The Organic layer was concentrate under reduced pressure. Crude solid thus obtained was purified by column chromatography using 100-200 mesh silica using 10-15% EtOAc: Hexane as an eluent to afford 0.3 g (75% yield) of **2** as an off white solid.

**Step 2: Synthesis of 1-(6-chlorobenzofuran-2-yl)ethan-1-one (4):**

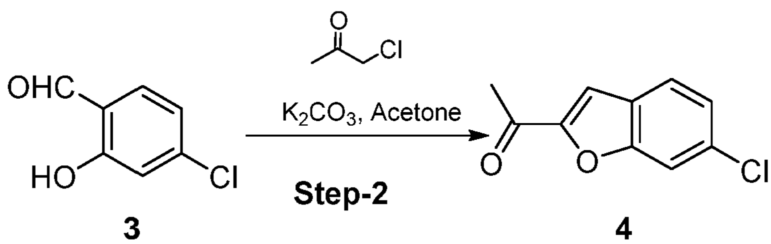


To a degassed mixture of 4-chloro-2-hydroxybenzaldehyde **3** (10 g, 64.10 mmol) in acetone (100 mL) was added chloro acetone (7.09 g, 76.60 mmol), $K_2CO_3$ (13.21 g, 95.72 mmol) at RT. The reaction mixture then stirred at 60 °C for 7 h. After completion (checked by TLC, 10% EtOAc :Hexane), reaction mixture was concentrate under reduced pressure, diluted cold water and extracted with ethyl acetate (3 × 50 mL). The Organic layer was evaporated under reduced pressure. Crude solid thus obtained was purified by column chromatography using 100-200 mesh silica using 2-4% EtOAc: Hexane as an eluent to afford 9.0 g (72.5% yield) of **4** as an off white solid.

**LCMS condition:** $[M+H]^+$ =195.0 $R_t$=1.79min.

**Step 3: Synthesis of 2-bromo-1-(6-chlorobenzofuran-2-yl)ethan-1-one (5):**

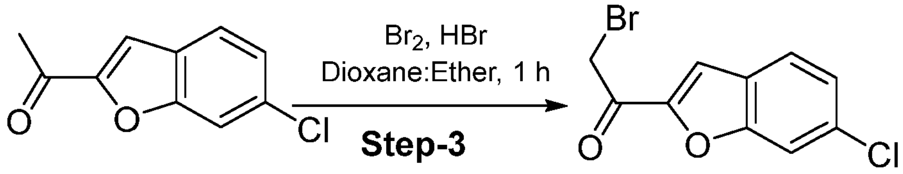


**4** **5**

To a degassed mixture of 1-(6-chlorobenzofuran-2-yl)ethan-1-one **4** (2.4 g, 12.37 mmol) in diethyl ether (10 mL) was added Bromine(1.85 g, 13.60 mmol) and HBr in AcOH (1.0 mL) at 0 °C and stirred for 30 min at RT. After completion (checked by TLC, 5% EtOAc :Hexane), the reaction mass was concentrate under reduced pressure, diluted with cold water and extracted with ethyl acetate (3 × 30 mL). Organic layer was evaporated under reduced pressure. Crude solid thus obtained was purified by column chromatography using 100-200 mesh silica using 2-3% EtOAc: Hexane as an eluent to afford 3.0 g (89.2% yield ) of **5** as an off white solid.

LCMS condition: $[M+H]^+$ =274.90 $R_t$ =2.21.

**Step 4: Synthesis of 4-(6-chlorobenzofuran-2-yl)-2-(1H-pyrrol-2-yl)oxazole (VPCFTE-95)**

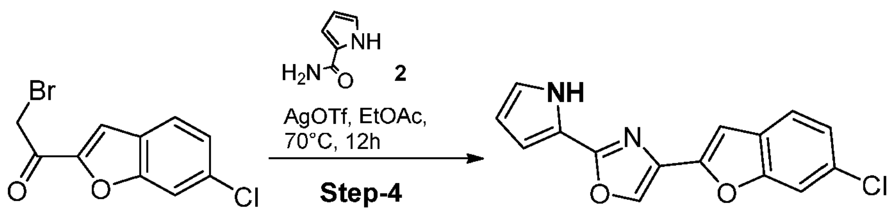


**5** **VPCFTE-95**

To a degassed mixture of 2-bromo-1-(6-chlorobenzofuran-2-yl)ethan-1-one **5** (0.2 g, 0.74 mmol.) and 2-(methylamino)acrylamide **2** (0.2 g, 1.82 mmol.) in ethyl acetate (10 mL) was added AgOTf (0.47 g, 1.84 mmol.) in sealed tube under argon atmosphere at room temperature. The reaction mixture stirred at 70°C for 7 h. After completion (checked by TLC 20% EtOAc :Hexane) reaction mixture was cooled to room temperature, solvent was removed under reduced pressure. Crude solid thus obtained was purified by column chromatography using 100-200 mesh silica using 15-20% EtOAc: Hexane followed by PREP HPLC to afford 0.03g (15% yield) of **VPCFTE-95** as an off white solid.

**HPLC:** 99.87% **LCMS-Condition-1:** [M+H]+ = 284.90; $R_t$ = 2.10 min.

$^1$**H NMR** (400 MHz, DMSO-$d_6$) δ 12.07 (brs, 1H), 8.61 (s, 1H), 7.80 (s, 1H), 7.71 (d, *J*=8.80 Hz, 1H), 7.34 (dd, *J*=8.31, 1.47 Hz, 1H), 7.21 (s, 1H), 7.00-7.02 (m, 1H), 6.80-6.82 (m, 1H), 6.23-6.24 (m, 1H).

**VPC-18-142**

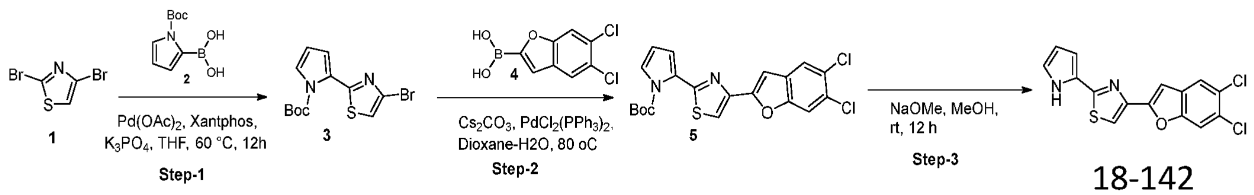


18-142

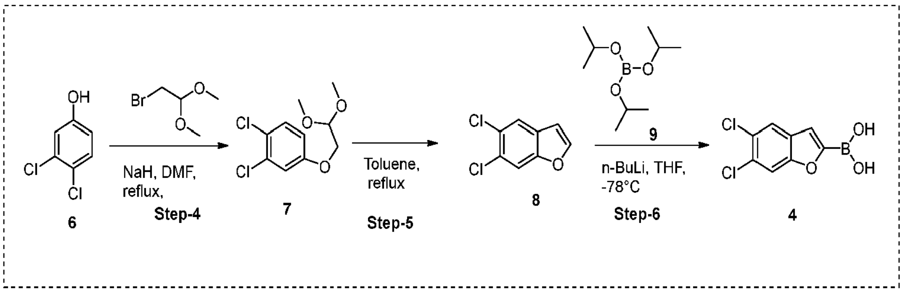


## VPC-18-149

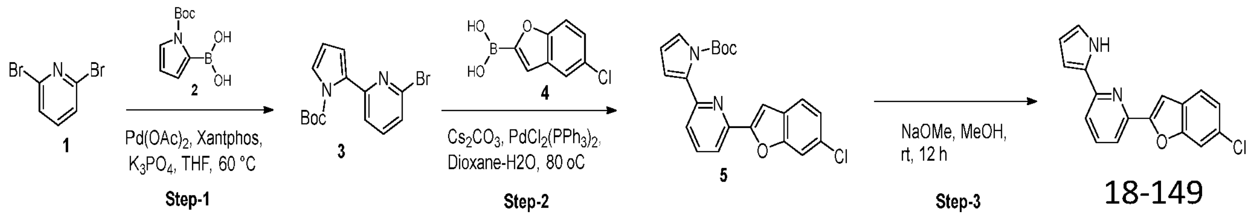


18-149

## VPC-18-152

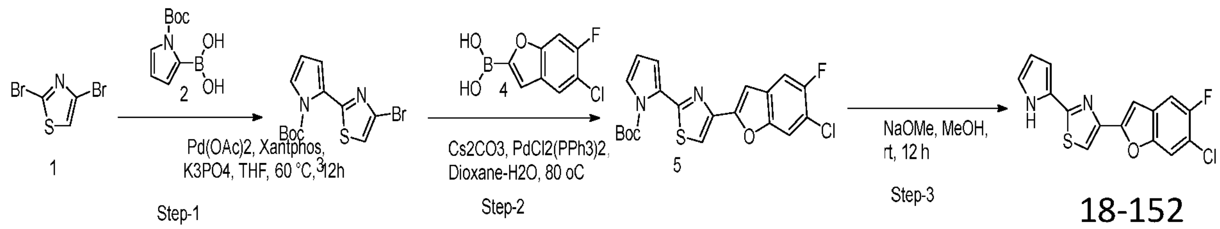


18-152

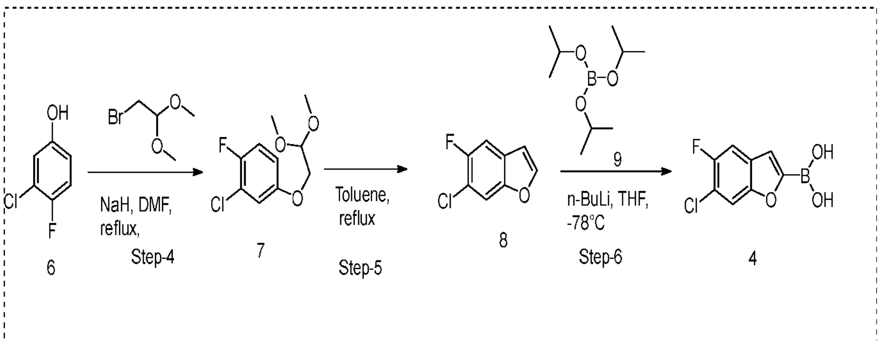


## VPC-18-155

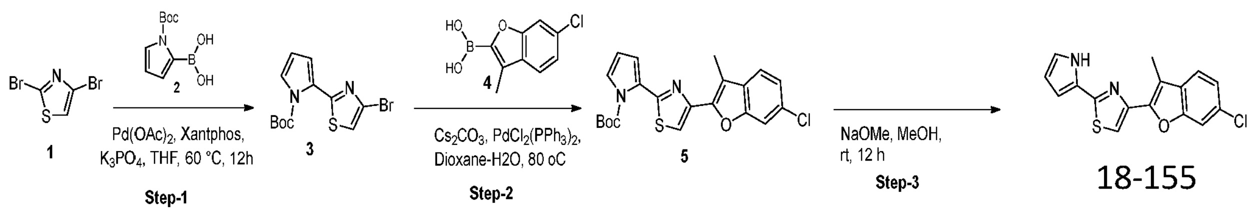


18-155

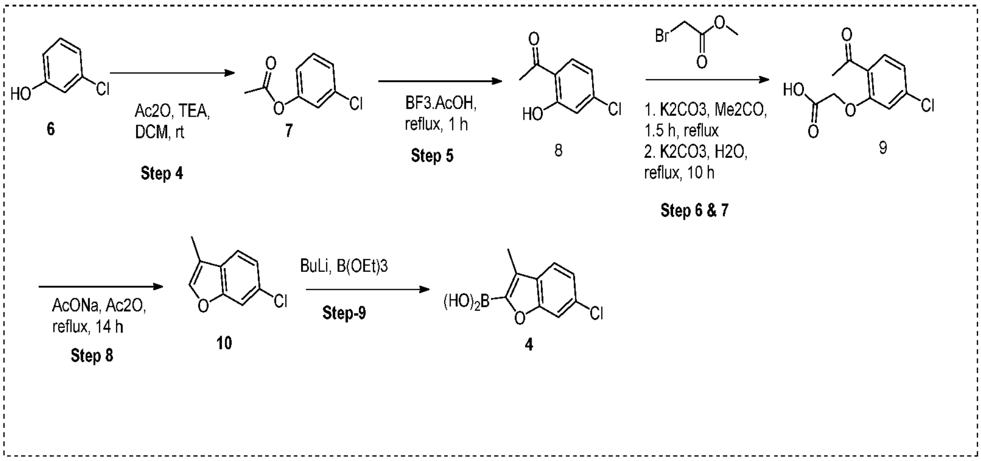


## VPC-18-163

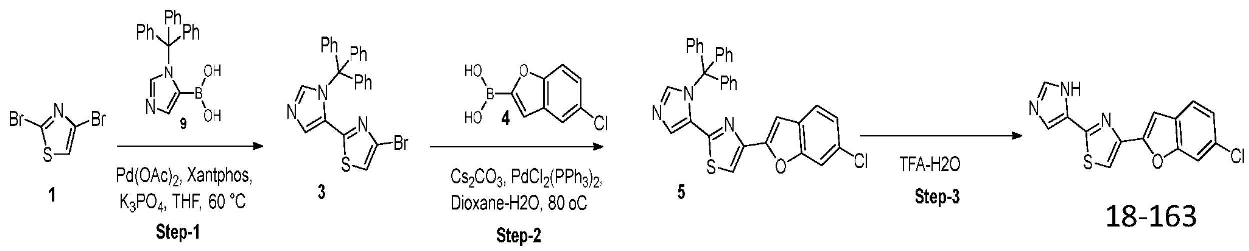


18-163

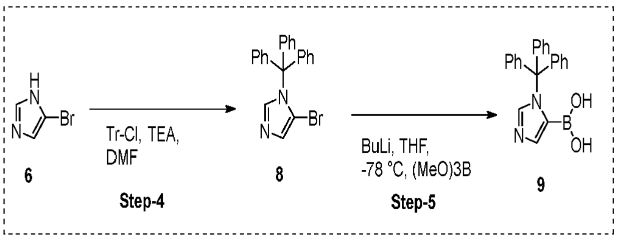


## VPC-18-164

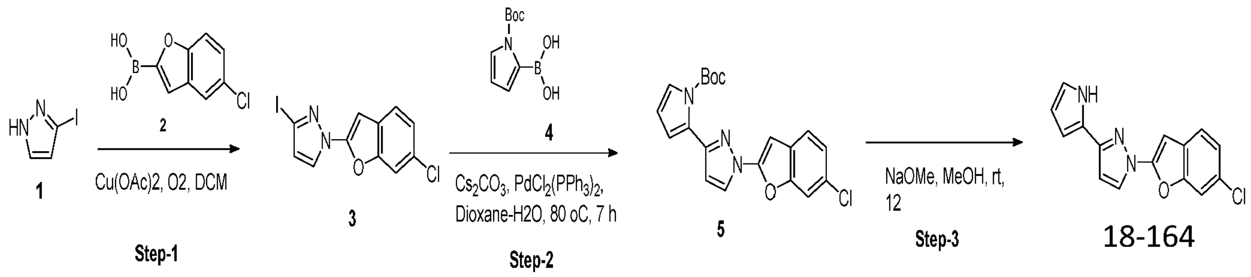


## Analytical methods:

**$^1$H** and $^{13}$C NMR spectra (COSY, $^1$H/$^{13}$C 2D-correlations) were recorded with Bruker Avance III™ 400 MHz. Processing of the spectra was performed with MestRec™ software and data are reported as follows: chemical shifts (δ) in parts per million, coupling constants (J) in hertz (Hz). The high-resolution mass spectra were recorded in positive ion-mode with an ESI ion source on an Agilent™ Time-of-Flight LC/MS mass spectrometer. HPLC analyses and purity of >95% were performed by analytical reverse-phase HPLC with a Agilent™ instrument with variable detector using *column* Agilent Zorbax 4.6×5mm, 5um ; flow: 2.0 mL.min1, $H_2O$ (0.1 % FA)/$CH_3CN$ (0.1 % FA), gradient 2 → 98 % (6 min) and 98 % (0.3 min).

A person of skill in the art based on the general knowledge in the art and the information provided herein would be able to synthesize the compounds described herein or modify the compounds described herein.

## BRN2 Inhibitor Selection

### Preparation of reagents

Preparation of potassium phosphate buffer, 50 mM (pH 7.4)

Potassium phosphate buffer (Kphos) was prepared by adding 0.647 g potassium phosphate monobasic ($KH_2PO_4$) and 3.527 g potassium phosphate dibasic ($KH_2PO_4$) to 400 mL of Milli-Q water. pH of the buffer was adjusted to 7.4 and volume was made up to 500 mL.

### Preparation of microsomes

Microsomes (20 mg/mL) were diluted in $KH_2PO_4$ buffer to prepare a concentration of 0.357 mg/mL.

Preparation of test compounds (BRN2 inhibitors)

Stock solutions of BRN2 inhibitors were prepared in DMSO at a concentration of 1 mM

Preparation of NADPH solution

A stock solution of 3.33 mM NADPH (3.33X) was prepared by dissolving appropriate amount of NADPH in $KH_2PO_4$ buffer.

### Assay Conditions

Total Incubation volume : 100 µL

Compound concentration : 1 µM

Protein Concentration : 0.25 mg/mL

NADPH : 1 mM

Final DMSO contain : 0.1%

Number of replicates : 2

Time points : 0, 5, 15, 30 and 60 min

**Assay**

An 1120 μL aliquot of Kphos buffer (50 mM, pH 7.4) containing liver microsomes (0.357 mg/mL) were added to individual 2 mL tubes (final concentration 0.25 mg/mL). Test compounds (1 mM) and positive controls were directly spiked into respective tubes to prepare a concentration of 1.428 μM (final concentration 1 μM). From the above mix, 70 μL was added to individual wells of 96 well reaction plates and pre-incubated in a 37 oC water bath for 5 min. All the reactions were initiated by adding 30 μL of 3.33 mM NADPH (final concentration 1 mM). Reactions without NADPH and buffer controls (minus NADPH) at 0 min and 60 min were also incubated to rule out non-NADPH metabolism or chemical instability in the incubation buffer. All reactions were terminated using 100 μL of ice-cold acetonitrile containing internal standard at 0, 5, 15, 30 and 60 min. The plates were centrifuged at 4000 RPM for 15 min and 100 μL aliquots were submitted for analysis by LC-MS/MS.

**Bio-Analysis**

Samples were monitored for parent compounds disappearance in MRM mode using LC-MS/MS.
The LC-MS/MS conditions and MRM chromatogram.

**Data Analysis**

The percent remaining of test compounds and positive control in each sample was determined by considering peak area ratio in the o minute sample as 100%. The Half-life of compounds in microsomes is calculated by formula: Half-life (t1/2) (min) = 0.693/k, where k is gradient of line determined from plot of peak area ratio (compound peak area / internal standard peak area) against time.
*In vitro* intrinsic clearance (CL'int) (units in mL/min/kg) was calculated using the formula.

$$CL'_{int} = \frac{0.693}{\text{in vitro T1/2}} \cdot \frac{\text{mL incubation}}{\text{mg microsomes}} \cdot \frac{\text{45 mg microsomes}}{\text{gm liver}} \cdot \frac{\text{liver weight in gm *}}{\text{Kg b.w}}$$

For liver microsomes, scaling factor used was 45 mg microsomal protein per gm liver. * Indicates liver weight (gm) which varies species wise. For mice the liver weights are 90 gm.

**Cell-based testing**

**Step 1: Reporter Activity**

*This assay quantifies inhibition of BRN2 transcriptional activity.*

- $42D^{ENZR}$ cells stably expressing BRN2-luciferase reporter are plated in a 12-well Corning™ plates at 100,000 cells per well.
- Next day cells are treated with 10 and 30μM of compounds from a stock of 10mM
- 24 hours later, cells are washed twice with PBS and lysed in 1x Passive Lysis Buffer (Promega™) for 15 minutes.
- Plates are frozen at -8o°C and thawed before lysed cells are collected.
- Cell lysate is spun at 10,000 rpm for 10 minutes
- Supernatant is plated in Flat White Bottom plates and bioluminescence is measured using Tecan M200 Luminometer™.

- Bioluminescence readings are normalized to protein concentration of the cell lysate.

Compounds with greater than 75% inhibition at 10 μM with a dose response showing higher inhibition at 30μM pass this criterion. Decreased expression of luciferase protein is validated via western blot to confirm downregulation and avoid false positive molecules that inhibit luciferase protein activity and not BRN2-drive luciferase expression. Molecules are prioritized by their inhibition potency to measure direct interaction with BRN2 in Step 2.

**Step 2: Drug Affinity Responsive Target Stability (DARTS) assay**

*This in cell assay provides a yes/no answer to direct interaction between BRN2 inhibitor and protein.*

**1)** Prepare and place the following on ice:

- Pronase
- TNC 10X
- - 500mM Tris-HCl (pH 8.0)
- - 500 mM NaCl, 100 mM CaCl,
- DMSO
- Drug
- 10mL NP40 + Protease inhibitor + Phosphatase inhibitor solution (can store in 4°C and use for up to 4 weeks):
- - 2mL NP40 5X
- - 8mL $ddH_2O$
- - 1 PhosphoSTOP™ pill
- - 200uL protease inhibitor 50X

**2)** Remove the growth medium from the cell plate ($42D^{ENZR}$) and add 10 mL of cold PBS/vanadate.

**3)** Scrape the cells. Remove the PBS-cell solution and add in a falcon tube.

**4)** Name a new 1.5 ml Eppendorf tube and put on ice to pre-chill.

**5)** Centrifuge the cells at 1500 for 5 minutes at 4°C. Remove supernatant and re-suspend the cells in 6oouL NP40 lysis buffer by pipetting up and down. If the cell pellet is small use less volume, for example 300 μl)

6) Transfer the lysing cells into the pre-chilled Eppendorf tube. Incubate on ice for 20 minutes. If not continuing the DARTS on the same day, you can put the lysate in -30 and work on it later.

**Preparing Cell Lysates for DARTS:**

**7)** Centrifuge the tube at maximum speed for 25-30 minutes at 4°C.

**8)** Discard the pellet (either transfer the supernatant in a new tube or just take out the pellet).

**9)** Measure protein concentration of the lysates using the BCA.

*Optimal protein concentration for DARTS is between 4-6 μg/μL. A little higher or lower works too (roughly 3 to 8).

**10)** Split the lysates into two samples by transferring half of the liquid into each of two tubes. Warm the lysates to room temperature.

**Incubate Protein Lysates with Small Molecule:**

**11)** Add into one tube 3 μL DMSO, and into the other tube 3 μL small molecule with 10mM final concentration.

**12)** Mix the samples immediately by gently flicking the tubes, spin the tubes for the drops, and allow them to incubate at room temperature for 1 hour. 45 mins in, start preparing the Pronase™.

**13)** Thaw one aliquot of 10 mg/mL Pronase™ (protease) and place on ice. (to make new Pronase™ stock 10mg/ml)

**14**) Thaw one aliquot of TNC 10X, dilute to 1X (add 100 μL to 900 μL $H_2O$). Don't Freeze and thaw 10X TNC.

**15)** Dilute Pronase™ to 1.25 mg/mL by mixing 12.5 μL Pronase™ with 87.5 μL cold **1**× TNC Buffer, which will serve as the **1**:100 Pronase™ stock solution.

**16)** Dilute the 1:100 Pronase™ solution serially by mixing with **1X** TNC to create 1:100, 1:500, 1:1000, 1:5000, and 1:10000 Pronase™ stock solutions. MIX every tube well by flicking before making the next solution.

1:500....... Take 20 μ L of 1:100 and add Soul of 1X TNC

1:1000...... Take 50 μL of 1:500 and add soul of 1X TNC

1:5000...... Take 20 μL of 1:1000 and add Soul of 1X TNC

1:10000.... Take 50 μL of 1:5000 and add soul of 1X TNC

**Performing Proteolysis:**

**17**) Prepare 5 aliquots from each of the tubes, each with 30uL, and save the remaining of each sample to be used as a control sample (if previously you used less than 600 μl of NP40 in step 5, you might want to omit some pronase dilutions).

**18)** Add 2uL of 1:100 pronase to one drug-treated aliquot and to one aliquot of DMSO sample. Mix them well by flicking and then incubate at room temperature. C ontinue adding 2uL of the corresponding pronase stock solution into each of the remaining aliquots.

**19)** After 30 minutes, add 10uL of 4X sample buffer into each sample. Heat at 100°C for 5 minutes. Spin down to collect the drops.

**20)** The samples are ready for Western blot gel running. You can run them now, or freeze to run later (in that case, after thawing you have to vortex and spin down, then run). For primary antibody, use your protein of interest (here BRN2 in Rb) and loading control proteins like GAPDH, actin, or Vinculin (better if its Ms antibody).

Molecules displaying positive binding progress to Step 3.

**Step 3: Cell Proliferation**

*This step selects BRN2 inhibitors to inhibit proliferation of BRN2 expressing 42D$^{ENZR}$ cells while being completely ineffective against 16D$^{CRPC}$ cells, which do not express BRN2.*

1. Plate 42D$^{ENZR}$ (~7000 cells per well) and 16D$^{CRPC}$ (~5000 cells per well) cells in 96 well plate with a volume of 100μL per well.

2. Next day 1 mL aliquots of media are combined with 3× the desired concentration of compounds. The aliquots are vortexed and 50μL of each 3× concentration is added to the 100μL of media in each well to give the 1× desired concentration.

The following are the desired concentrations:
0, 10nM, 100nM, 500nM, 1μM, 2.5μM, 5μM, 10μM, 15μM, 20μM, 30μM.

3. Approximately 72 hours later, 17 μL of 10% Glutaraldehyde is added to each well and plates are placed on a shaker for 20-30 minutes.

4. Media is washed off the fixed cells by slow submersion in a container of water. Then the water is removed by tapping the plate upside down.

5. 75 μL of Crystal Violet™ solution is added into the 96 well plates and placed on a shaker for ~30 minutes.

6. Media is washed off the fixed cells by repeated slow submersion in a container of water and the plates are allowed to dry over-night.

7. Next day, 75 μL of Sorensen Solution is accurately pipetted into each well. The plates placed on a shaker for ~30 minutes.

8. Absorbance is measured in the plate at 590 nm.

Compounds that show specificity towards 42D$^{ENZR}$ cells vs 16D$^{CRPC}$ cells progress to step 4.

**Step 4: Suppression of target genes**

*This step evaluates the ability of BRN2 inhibitors to suppress expression of BRN2 target genes NCAM1 and SOX2, as well as NEPC marker CHGA.*
1. 42D$^{ENZR}$ cells are plated in 10cm cell culture plates at 1,000,000 cells per plate.
2. The next day, cells are treated with compounds at 0, 1, 5 and 10 μM.
3. Approximately 48 hours later, cells are washed once with PBS and mRNA is extracted using PureLink RNA Mini Kit™ (Thermo Fisher™) as per manufacturer's instructions.
4. cDNA is prepared using SuperScript IV™ (Thermo Fisher™) as per manufacturer's instructions.
5. Using Taqman™ Probe master mix (Roche™) and Taqman™ Primers (Thermo Fisher™) q-PCR is conducted on the samples according to the following protocol:
For each sample prepare a small Eppendorf™ tube containing:

| | | | |
|---|---|---|---|
| 2X Taqman™ Mix | 5 μl | ×5 | 62 μl |
| cDNA from RT reaction - sample | 1 μl | × | μl |
| DEPC water | 1 μl | × | μl |
| **Final volume in each well** | **7 μl** | | |

For each gene (e.g. probe and rRNA) prepare MasterMix:

| | 1 sample | | replicates |
|---|---|---|---|
| 20X primer mix | 0.5 μl | 5 | μl |
| $H_2O$ | 2.5 μl | | μl |

**Put in each well 3.0 μl**

Plates are run on ABI ViiA7 machine with standard Taqman™ protocol. Cycle times are analyzed via ΔΔCT method. Relative expression of target genes NCAM1 and SOX2 as well as CHGA are quantified compared to control. Compounds clear this final selection step if the expression of these genes is reduced in a dose dependent manner.

## EXAMPLES

### EXAMPLE 1 - Cell-Based Testing

In **TABLE 1** compounds were tested at 10μM and 30μM for BRN2 inhibition. Some compounds were also tested to determine their specificity to 42D and not 16D cells as shown in for ZINC compounds in **TABLE 1** and for all others in **TABLE 2.**

**TABLE 1: Structural and experimental data for the BRN2 interactors.**

| **VPC-ID** (specificity) | **STRUCTURE** | **% BRN2 Inhibition (10μM)** | **% BRN2 Inhibition (30μM)** | **Met Stab % (60 mins HLM/MLM)** | **T1/2 in (mins HLM /MLM)** |
|---|---|---|---|---|---|
| **VPC-18 (42D not 16D)** | 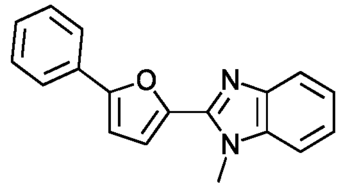 ZINC00452417 | 97.62 | 99.43 | | |
| **VPC-18-13 (specific)** | 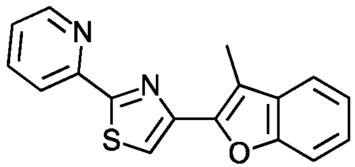 ZINC07845371 | 98.29 | 99.93 | | |
| **VPC-18-14 (specific)** | 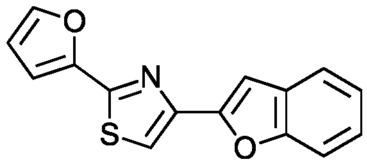 ZINC25204472 | 95.94 | 96.58 | | |
| **VPC-18-18 (specific - affects 42D but not 16D)** | 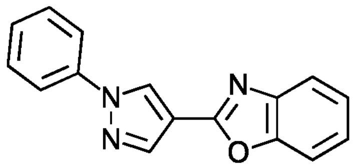 ZINC1398346 | 82.84 | 87.01 | | |
| | Non-Specific but active below | | | | |
| **VPC-18-74** | 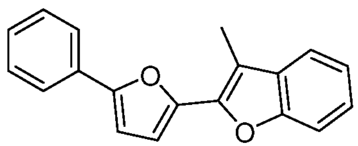 | 96.7 | 99.03 | 2 | 11 |

(continued)

| | Non-Specific but active below | | | | |
|---|---|---|---|---|---|
| **VPC-18-111** | 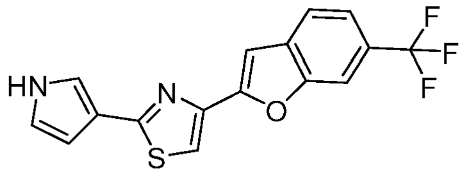 | 96.23 | 98.6 | 38 | 48 |
| **VPC-18-113** | 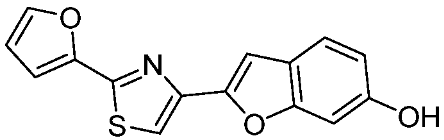 | 94 | 94 | 3 | 12 |
| **VPC-18-121** | 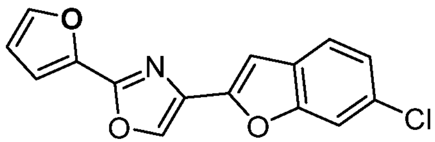 | 92.7 | 93.5 | 0 | 8 |
| **VPC-18-114** | 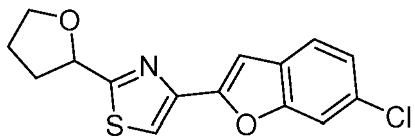 | 92 | 95 | 14 | 25 |
| **VPC-18-143** | 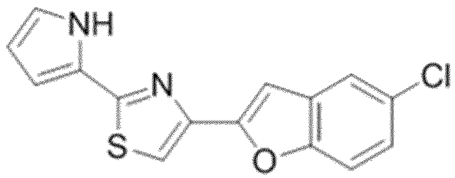 | 90.87 | 94.39 | 7 | 17 |
| **VPC-18-106** | 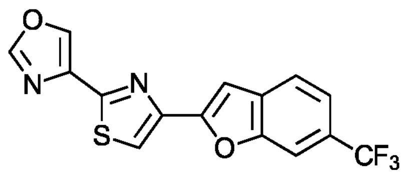 | 90.58 | 90 | 7 | 16 |

(continued)

| | Non-Specific but active below | | | | |
|---|---|---|---|---|---|
| **VPC-18-120** | 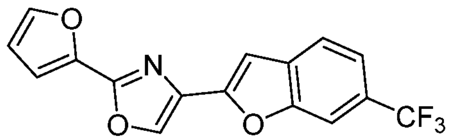 | 88.9 | 88.8 | 6 | 13 |
| **VPC-18-103** | 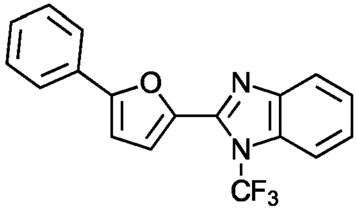 | 88.48 | 95.35 | 0 | 9 |
| **VPC-18-144** | 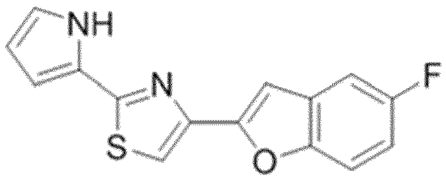 | 86.97 | 98.18 | 1 | 11 |
| **VPC-18-87** | 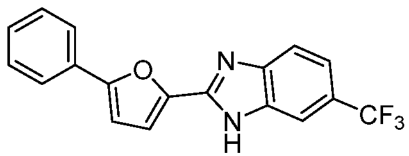 | 85 | 87 | 37 | 40 |
| **VPC-18-98** | 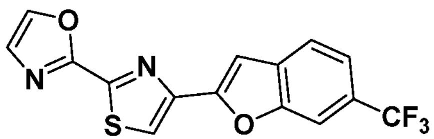 | 84 | 86 | 53 | >60 |
| **VPC-18-32** | 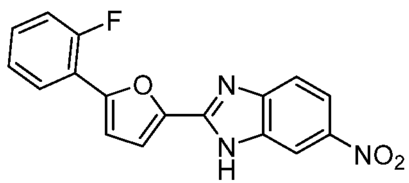 | 83.65 | 95.48 | 20 / 4 | 26 / 12 |

(continued)

| | Non-Specific but active below | | | | |
|---|---|---|---|---|---|
| **VPC-18-96** | 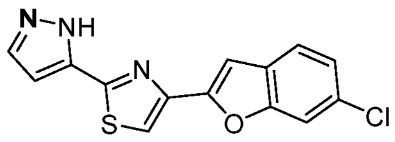 | 83.52 | 92.53 | 35 | 45 |
| **VPC-7-44** | 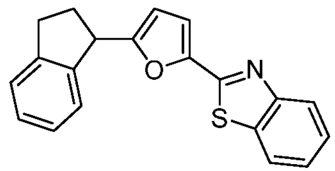 | 83.00 | 93.00 | 62 / 44 | >60 / 52 |
| **VPC-18-92** | 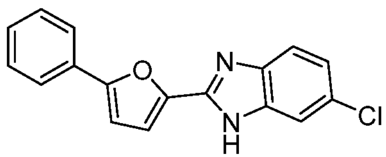 | 83 | 98.4 | 0 | 6 |
| **VPC-18-109** | 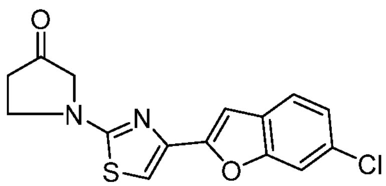 | 81.69 | 91.48 | 4 | 13 |
| **VPC-18-73** | 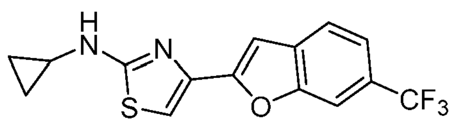 | 78.8 | 95.7 | 2 | 11 |
| **VPC-18-53** | 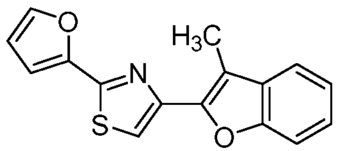 | 78.06 | 84.47 | 39 / 2 | 42 / 12 |

(continued)

| | Non-Specific but active below | | | | |
|---|---|---|---|---|---|
| **VPC-18-42** | 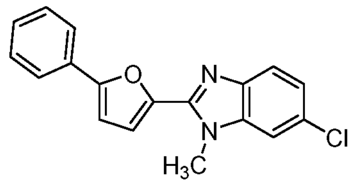 | 77.35 | 76.77 | 15 / 1 | 23 / 11 |
| **VPC-18-88** | 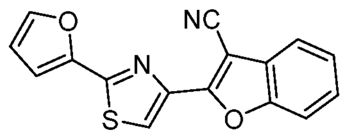 | 76 | 96 | 0 | 9 |
| **VPC-18-156** | 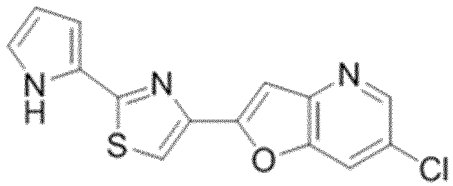 | | | 1 | 9 |
| **VPC-18-163** | 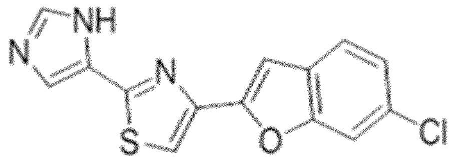 | | | 19 | 25 |
| **VPC-18-94-14** | 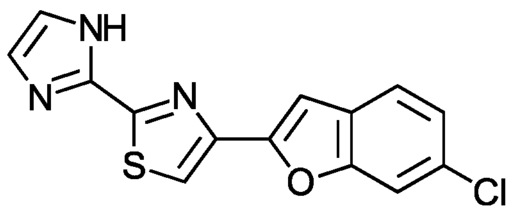 | | | | |

(continued)

| | | | | | |
|---|---|---|---|---|---|
| | Non-Specific but active below | | | | |
| **VPC-18-164** | 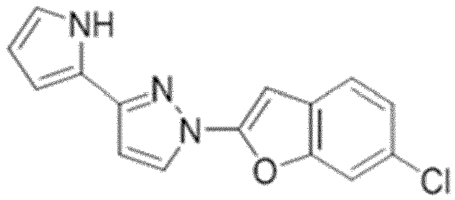 | | | 2 | 12 |
| **VPC-18-165** | 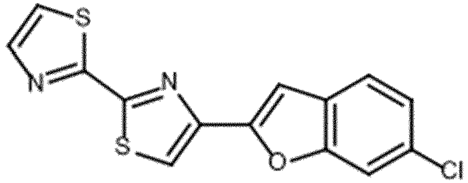 | | | 6 | 16 |
| **VPC-18-166** | 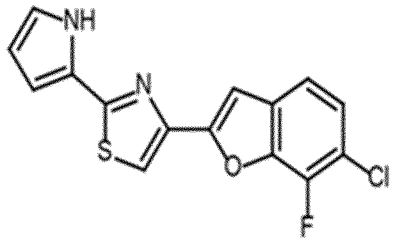 | | | 1 | 10 |
| **VPC-18-118** | 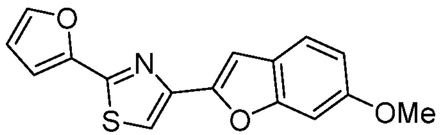 | 74 | 92.5 | 1 | 9 |
| **VPC-18-159** | 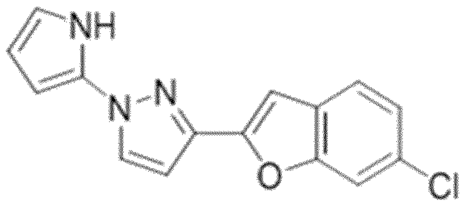 | | | 0 | NC |

(continued)

| | Non-Specific but active below | | | | |
|---|---|---|---|---|---|
| **VPC-18-63** | 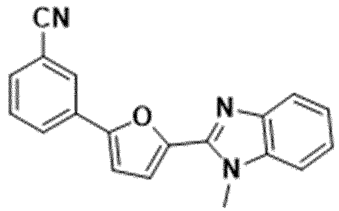 | 72 | 94 | 0 | NC |
| **VPC-18-75** | 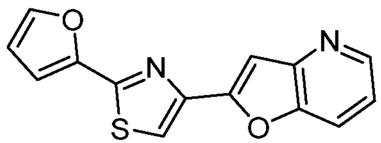 | 71.9 | 91.9 | 0 | NC |
| **VPC-18-91** | 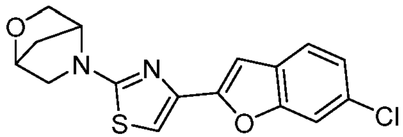 | 70 | 96 | 2 | 11 |
| **VPC-18-31** | 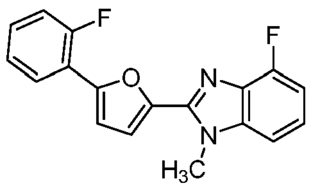 | 67.92 | 77.85 | 17 / 0 | 2 / NC |
| **VPC-18-89** | 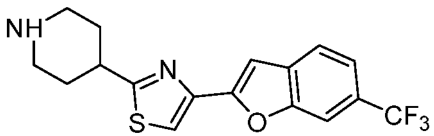 | 65 | 98 | 65 | >60 |

(continued)

| | Non-Specific but active below | | | | |
|---|---|---|---|---|---|
| **VPC-18-41** | 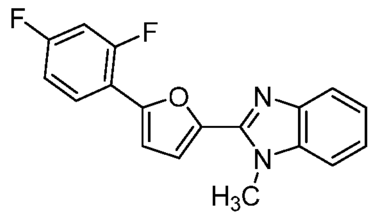 | 57.53 | 91.11 | 0 / 0 | NC |
| **VPC-18-36** | 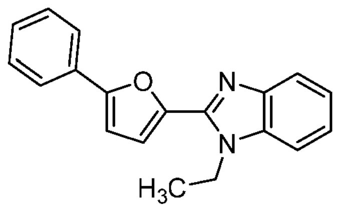 | 57.51 | 93.44 | 15 / 2 | 22 / 13 |
| **VPC-18-35** | 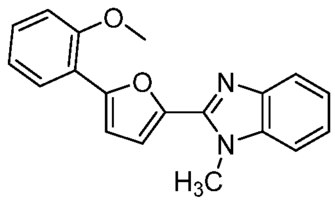 | 51.57 | 95.75 | 4 / 0 | 13 / 5 |
| **VPC-18-72** | 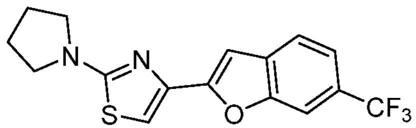 | 45.9 | 62.4 | 8 | 18 |
| **VPC-18-78** | 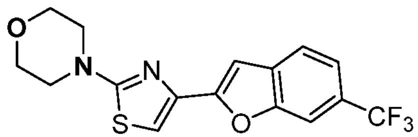 | 43.6 | 79.6 | 25 | 30 |

(continued)

| | Non-Specific but active below | | | | |
|---|---|---|---|---|---|
| **VPC-18-80** | 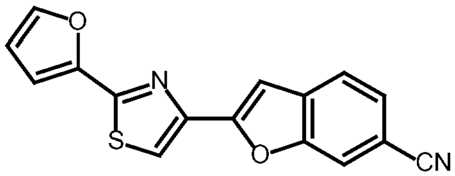 | 42 | 44 | 0 | NC |
| **VPC-18-47** | 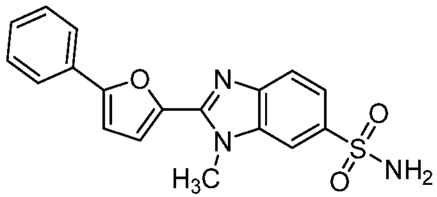 | 39.82 | 82.08 | 54 / 53 | >60 / >60 |
| **VPC-18-107** | 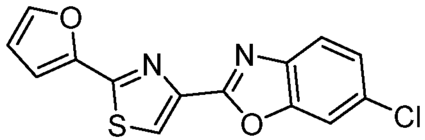 | 36.90 | 66.9 | 7 | 8 |
| **VPC-18-86** | 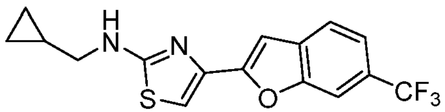 | 32 | 92 | 5 | 13 |
| **VPC-18-99** | 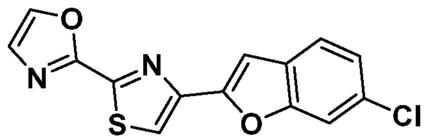 | 29.3 | 39.2 | 28 | 36 |
| **VPC-18-102** | 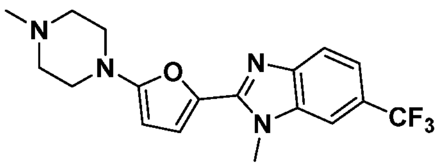 | 22.55 | 55.45 | 0 | 7 |

(continued)

| | Non-Specific but active below | | | | |
|---|---|---|---|---|---|
| **VPC-18-33** | 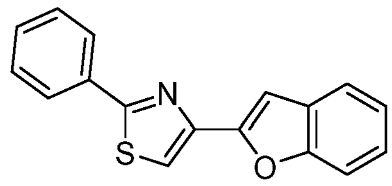 | 22.39 | 57.24 | 30 / 1 | 36 / 10 |
| **VPC-18-77** | 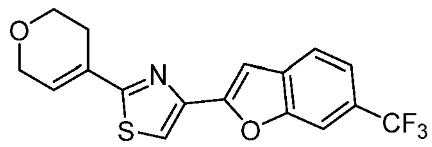 | 11.3 | 63.4 | 24 | 29 |
| **VPC-18-82** | 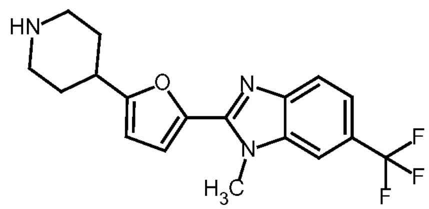 | 11 | 50 | 100 | >60 |
| **VPC-18-100** | 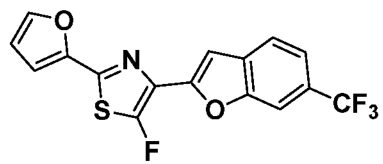 | 8.8 | 56.3 | 1 | 10 |
| **VPC-18-153** | 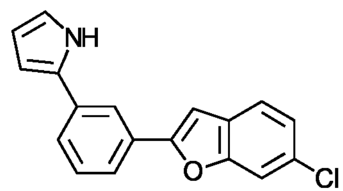 | | | 2 | 11 |

(continued)

| | | | | | |
|---|---|---|---|---|---|
| | Non-Specific but active below | | | | |
| **VPC-18-45** | 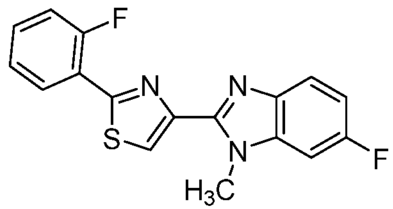 | 7.53 | 47.25 | 35 / 22 | 42 / 32 |
| **VPC-7-43** | 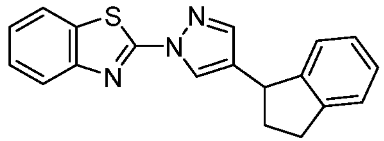 | 2 | 41 | 20/5 | 27 / 14 |
| **VPC-18-93** | 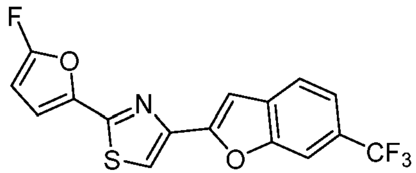 | -9.9 | 30.2 | 26 | 34 |
| **VPC-18-48** | 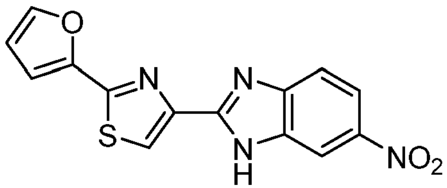 | -6.56 | 91.19 | 9 / 2 | 17 / 10 |
| | **THE REFERENCE COMPOUNDS BELOW ARE INACTIVE** | | | | |
| **VPC-18-130** | 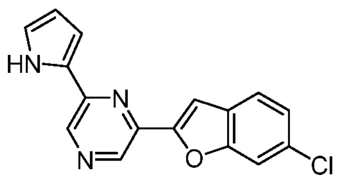 | Not tested | Not tested | 1 | 9 |

(continued)

| | THE REFERENCE COMPOUNDS BELOW ARE INACTIVE | | | | |
|---|---|---|---|---|---|
| **VPC-18-15** | 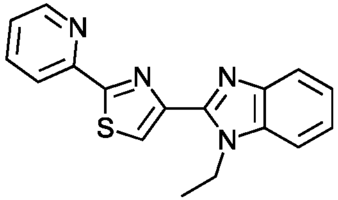 ZINC89873082 | insoluble | insoluble | | |
| **VPC-18-37** | 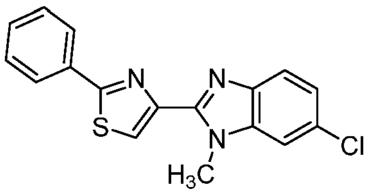 | -14.46 | -58.72 | | |
| **VPC-18-30** | 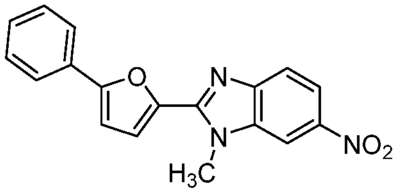 | 2.63 | 17.37 | | |
| **VPC-18-61** | 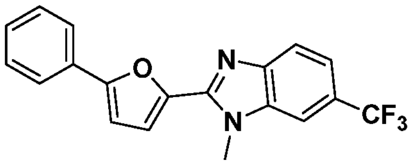 | -97 | -92 | | |
| **VPC-18-34** | 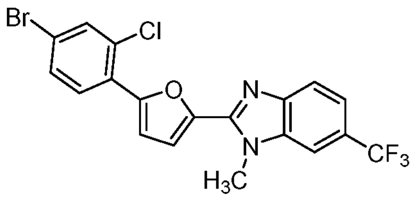 | -0.86 | 46.68 | | |

(continued)

| | THE REFERENCE COMPOUNDS BELOW ARE INACTIVE | | | | |
|---|---|---|---|---|---|
| **VPC-18-38** | 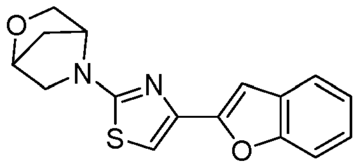 | -51.25 | 66.95 | | |
| **VPC-18-39** | 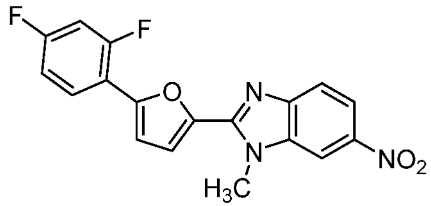 | -149.63 | -63.07 | | |
| **VPC-18-46** | 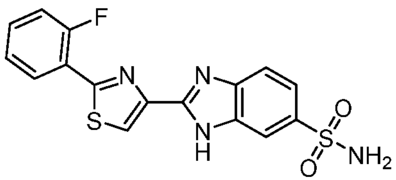 | -69.59 | -158.12 | | |
| **VPC-18-40** | 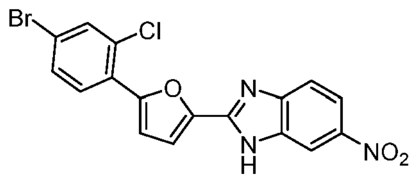 | -32.67 | -84.07 | | |
| **VPC-18-52** | 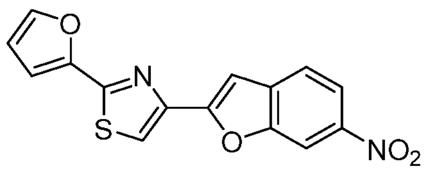 | -15.80 | -46.05 | | |

(continued)

| | THE REFERENCE COMPOUNDS BELOW ARE INACTIVE | | | | |
|---|---|---|---|---|---|
| **VPC-18-50** | 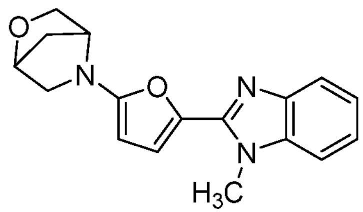 | -26.59 | 27.53 | | |
| **VPC-18-55** | 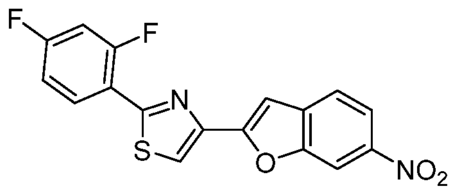 | -9.94 | -53.99 | | |
| **VPC-18-56** | 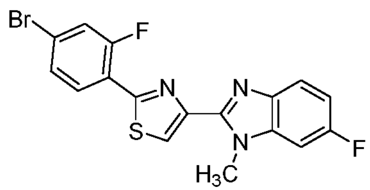 | INACTIVE | INACTIVE | | |
| **VPC-18-58** | 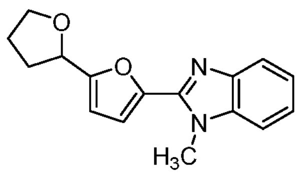 | INACTIVE | INACTIVE | | |
| **VPC-18-59** | 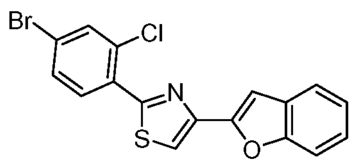 | INACTIVE | INACTIVE | | |

(continued)

| | THE REFERENCE COMPOUNDS BELOW ARE INACTIVE | | | | |
|---|---|---|---|---|---|
| **VPC-18-43** | 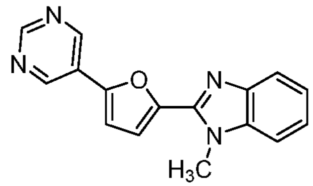 | -124 | -83 | | |
| **VPC-18-44** | 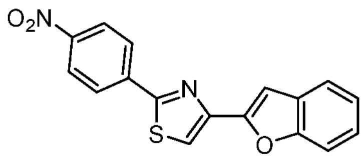 | -45 | -104 | | |
| **VPC-18-49** | 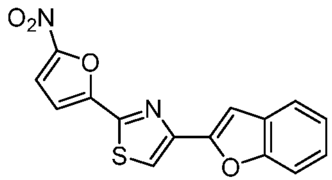 | -48 | -21 | | |
| **VPC-18-54** | 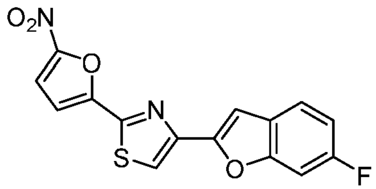 | -60 | -45 | | |
| **VPC-18-57** | 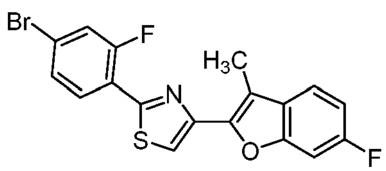 | INACTIVE | INACTIVE | | |

(continued)

| | THE REFERENCE COMPOUNDS BELOW ARE INACTIVE | | | | |
|---|---|---|---|---|---|
| VPC-18-60 | 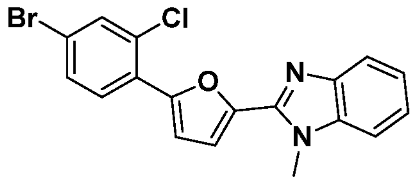 | 13 | -37 | | |
| VPC-18-62 | 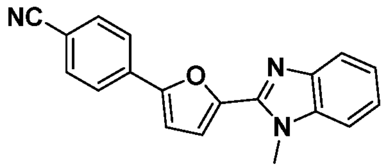 | -62 | -110 | | |
| VPC-18-64 | 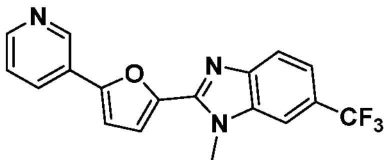 | -30 | -59 | | |
| VPC-18-65 | 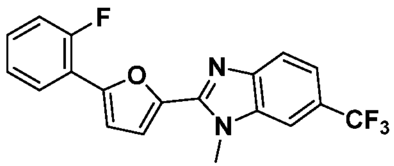 | -52 | -3 | | |
| VPC-18-67 | 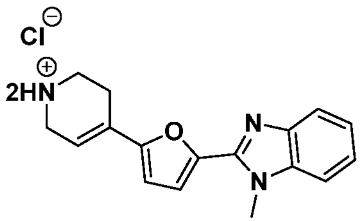 | -38 | 8 | | |

(continued)

| | THE REFERENCE COMPOUNDS BELOW ARE INACTIVE | | | | |
|---|---|---|---|---|---|
| **VPC-18-68** | 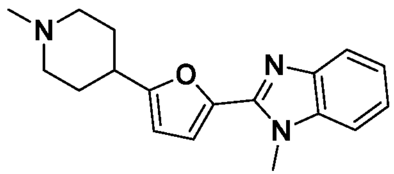 | 11 | 14 | | |
| **VPC-18-69** | 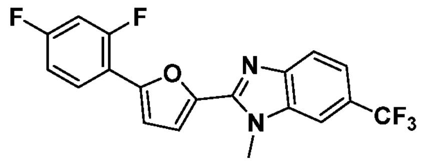 | -8 | 12 | | |
| **VPC-18-71** | 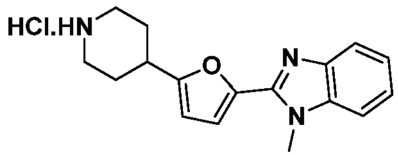 | -22 | -34 | | |
| **VPC-18-79** | 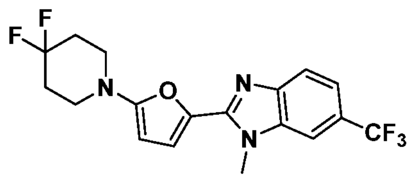 | -12.3 | 2.4 | | |
| **VPC-18-83** | 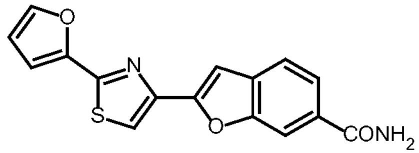 | -40 | -10 | | |
| **VPC-18-70** | 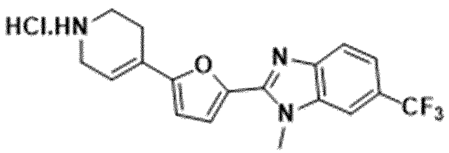 | 74 | 100 (cell death) | | |

(continued)

| | THE REFERENCE COMPOUNDS BELOW ARE INACTIVE | | | | |
|---|---|---|---|---|---|
| **VPC-18-95** | 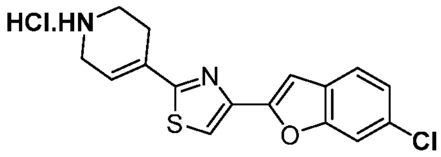 | 79.75 | 99.96 (cell death) | | |
| **VPC-18-97** | 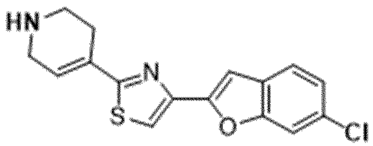 | 65 | 99.95 (cell death) | | |
| **VPC-18-84** | 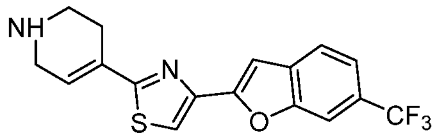 | cell death | cell death | | |
| **VPC-18-85** | 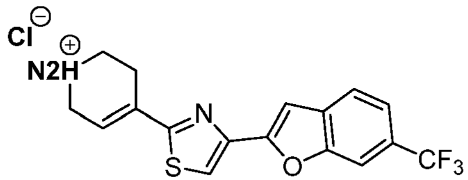 | cell death | cell death | | |
| **VPC-18-101** | 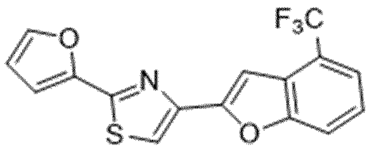 | -81 | -43 | | |
| **VPC-18-119** | 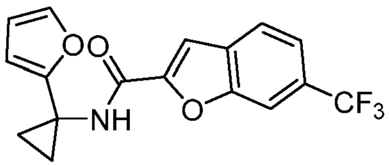 | 69.9 | -25 | | |

In **TABLES** 1 and **2,** when a single value is mentioned in columns Met Stab% and T1/2, it refers to compound's stability in MLM and when two values are given they represent HLM/MLM. There is a distinction in **TABLES 1** and **2** difference between "Active" and "Specific" or "Specificity". Compounds are first tested to quantify inhibition of BRN2 transcriptional activity in a luciferase assay to determine "Activity" at 10μM and 30μM concentrations. Compounds described as "inactive" (i.e. no inhibition of BRN2 transcriptional activity, low inhibition, activation of BRN2 transcription or cell death) were not further tested. "Active compounds", were further tested for "Specificity" on two cell lines (i.e. 42D cells, which express BRN2 and 16D cells, which do not express any BRN2). Generally, compounds having low inhibitory activity were compounds with less than about ~30% or activation at either concentration (i.e. 10μM or 30μM). As used herein "cell death" refers to compounds having severe toxicity at a low dose. Furthermore, these compounds lacked specificity and had profound off-target effects. Where data is not available "na" is inserted. Where data has yet to be calculated the notation "nc" is used.

**TABLE 2: List of BRN2 Compounds with Specificity that had Inhibition of BRN2 at 10μM and 30μM**

| **VPC-ID** (specificity) | **STRUCTURE** | **% BRN2 Inhibition (10μM)** | **% BRN2 Inhibition (30μM)** | **Met Stab % (60 mins HLM/MLM)** | **T1/2 in (mins HLM /MLM)** |
|---|---|---|---|---|---|
| **VPC-18-81 (specific to 42D)** | 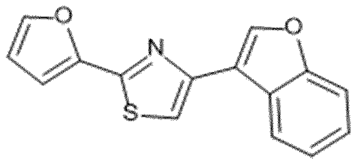 | 97 | 99 | 2 | 11 |
| **VPC-18-112 (specificity not tested)** | 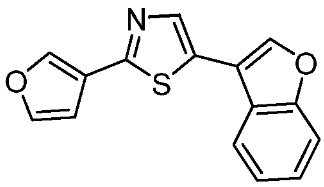 | 41.8 | 81.57 | 1 | 9 |
| **VPC-18-76 (specific)** | 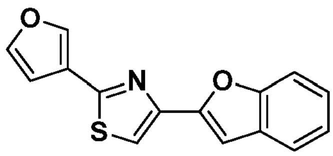 | 96.33 | 94.91 | 1 | 9 |
| **VPC-18-66 (specific - no effect on LNCaP and 16D)** | 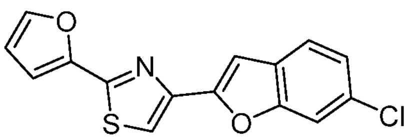 | 82 | 94 | 31 | 42 |
| **VPC-18-90 (specific)** | 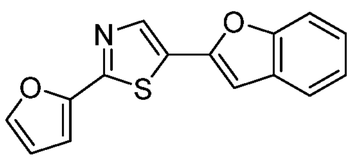 | 80 | 92 | 0 | 8 |
| **VPC-18-94 (specific - no effect on LNCaP and 16D)** | 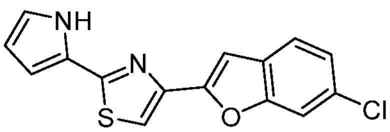 | 79.6 | 86.94 | 21 | 27 |

(continued)

| VPC-ID (specificity) | STRUCTURE | % BRN2 Inhibition (10μM) | % BRN2 Inhibition (30μM) | Met Stab % (60 mins HLM/MLM) | T1/2 in (mins HLM /MLM) |
|---|---|---|---|---|---|
| **VPC-18-51 (specific 42D not 16D) (BLI and DARTS binding)** | 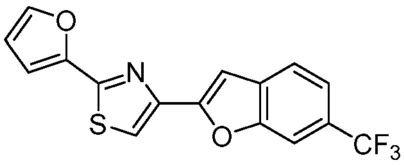 | 62.47 | 71.36 | 42 / 7 | 45 / 16 |
| **VPC-18-104 (specific 42D not 16D)** | 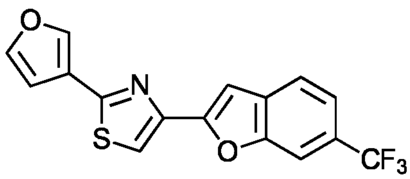 | 87.1 | 94.25 | 1 | 10 |
| **VPC-18-105 (specific 42D not 16D)** | 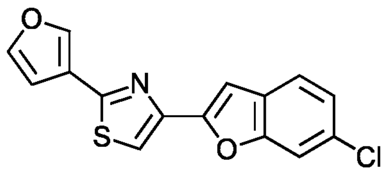 | 79.5 | 92 | 1 | 10 |
| **VPC-18-133 (specific)** | 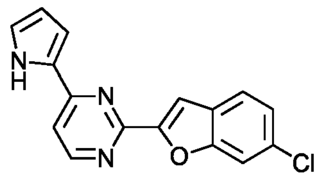 | na | na | 1 | 8 |
| **VPC-18-127 (specific)** | 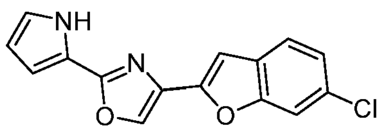 | na | na | 16 | 22 |
| **VPC-18-139 (specific)** | 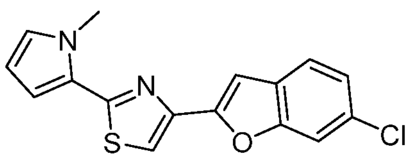 | na | na | 23 | 30 |
| **VPC-18-140 (specific)** | 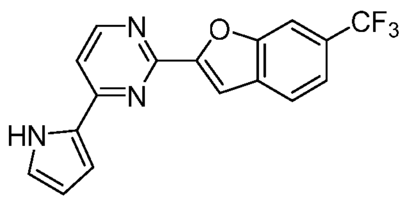 | na | na | 2 | 10 |
| **VPC-18-141 (specific)** | 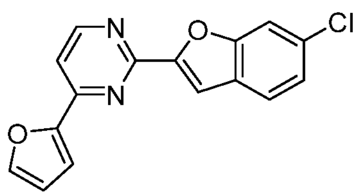 | na | na | 3 | 13 |

(continued)

| **VPC-ID** (specificity) | **STRUCTURE** | **% BRN2 Inhibition (10μM)** | **% BRN2 Inhibition (30μM)** | **Met Stab % (60 mins HLM/MLM)** | **T1/2 in (mins HLM /MLM)** |
|---|---|---|---|---|---|
| **VPC-18-108 (specific)** | 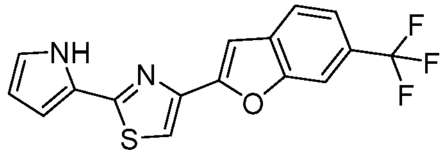 | 84.36 | 92.58 | 15 | 31 |
| **VPC-18-115 (specific)** | 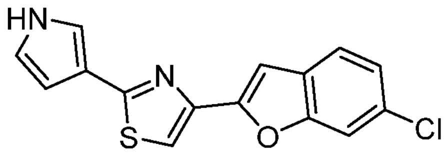 | 98 | 99 | 18 | 22 |
| **VPC-18-110 (specific)** | 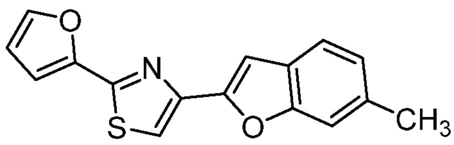 | 93.49 | 98.6 | 1 | 10 |
| **VPC-18-142 (42D IC50 is 2.5μM not 16D) (BLI and DARTS binding)** | 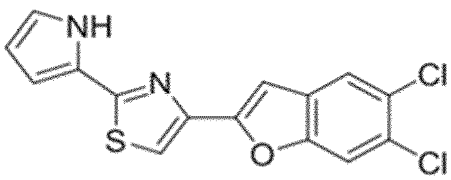 | 29.13 | 53.64 | 17 | 26 |
| **VPC-18-152 (42D IC50 is 5μM not 16D)** | 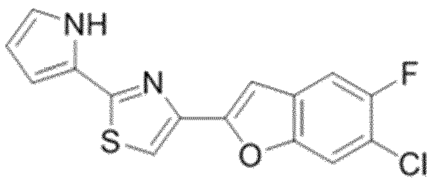 | | | 13 | 21 |
| **VPC-18-149 (42D IC50 is 1μM not 16D)** | 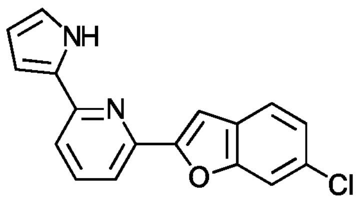 | 94.63 | 96.25 | 21 | 30 |
| **VPC-18-155 (42D IC50 is 10μM not 16D)** | 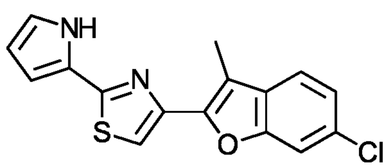 | | | 24 | 31 |
| **VPC-18 (42D not 16D)** | 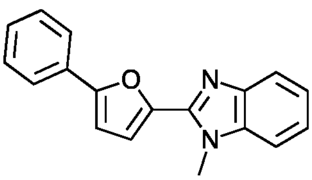 ZINC00452417 | 97.62 | 99.43 | | |

(continued)

| VPC-ID (specificity) | STRUCTURE | % BRN2 Inhibition (10μM) | % BRN2 Inhibition (30μM) | Met Stab % (60 mins HLM/MLM) | T1/2 in (mins HLM /MLM) |
|---|---|---|---|---|---|
| **VPC-18-13 (specific)** | 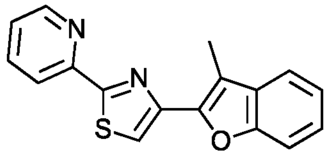 ZINC07845371 | 98.29 | 99.93 | | |
| **VPC-18-14 (specific)** | 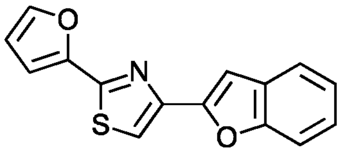 ZINC25204472 | 95.94 | 96.58 | | |
| **VPC-18-18 (specific - affects 42D but not 16D)** | 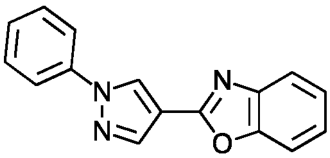 ZINC1398346 | 82.84 | 87.01 | | |

Although various embodiments of the invention are disclosed herein, many adaptations and modifications may be made within the scope of the invention as defined by the claims in accordance with the common general knowledge of those skilled in this art. Numeric ranges are inclusive of the numbers defining the range. The word "comprising" is used herein as an open-ended term, substantially equivalent to the phrase "including, but not limited to", and the word "comprises" has a corresponding meaning. As used herein, the singular forms "a", "an" and "the" include plural referents unless the context clearly dictates otherwise. Thus, for example, reference to "a thing" includes more than one such thing. Citation of references herein is not an admission that such references are prior art to an embodiment of the present invention.

## Claims

**1.** A compound having the structure of Formula I:

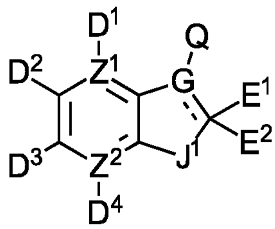

**I**

wherein,

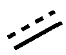

is a double bond;
Q is absent;

G is selected from N, C(H) and $C(CH_3)$ ;
$J^1$ is selected from N(H), $N(CH_3)$, $N(CH_2CH_3)$, $N(CF_3)$, $C(H)(CF_3)$, S and O;
$Z^1$ is C or N;
$Z^2$ is C or N;
alternatively, $Z^1$ is N, when $D^1$ is absent;
alternatively, $Z^2$ is N, when $D^4$ is absent;
$E^2$ is absent;
$E^1$ is $L^1$-$R^1$;
$L^1$ is selected from

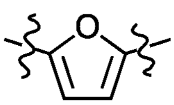, 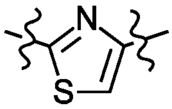, 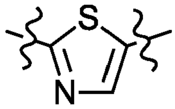, 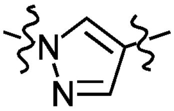,

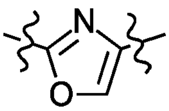, 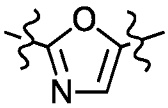, 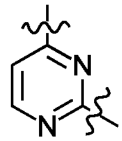, 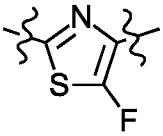, 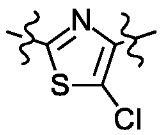, 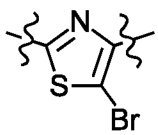, 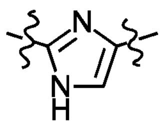,

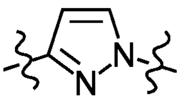, and 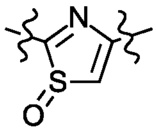 ;

$R^1$ is selected from

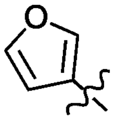, 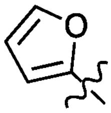, 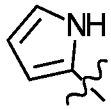, 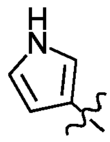, 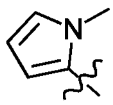, 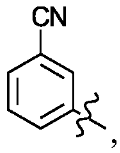,

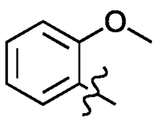, 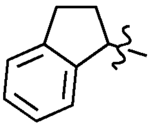, 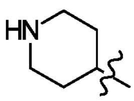, 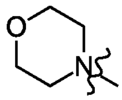, 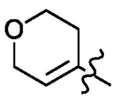, 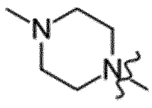, 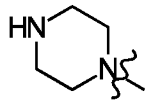, 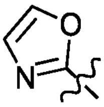,

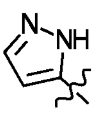, 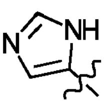, 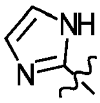, 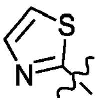, 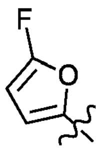, 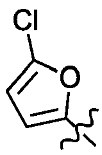, 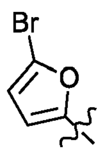, 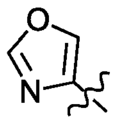, 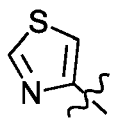, 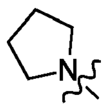,

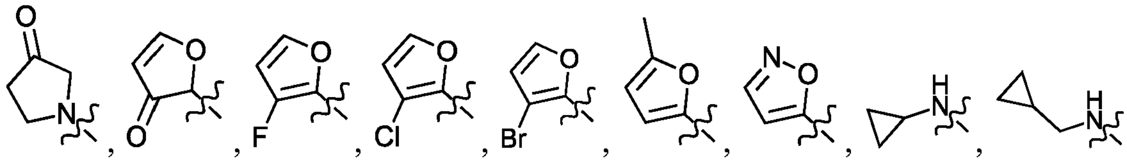 , , , , , , , , ,

and

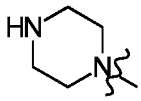;

alternatively, $R^1$ is

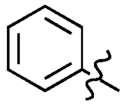

provided that $J^1$ is selected from N(H), $N(CH_2CH_3)$, $N(CF_3)$, $C(H)(CF_3)$, S and O;
alternatively, $R^1$ is

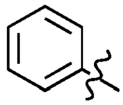

provided that $J^1$ is $N(CH_3)$, $L^1$ is selected from

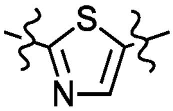

, 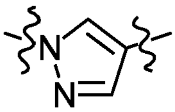 , 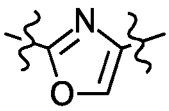 , 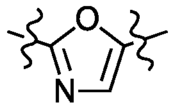 and 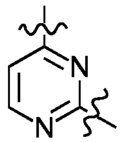

and $D^3$ is selected from H, Br, F, Cl, $NO_2$, $CF_3$,

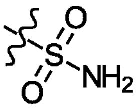,

OMe, $CH_3$ and OH;
alternatively, $R^1$ is

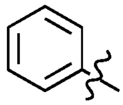

provided that $J^1$ is $N(CH_3)$, $L^1$ is

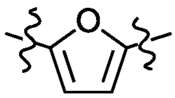

and D3 is selected from Br, F, Cl,

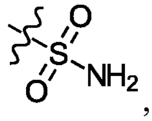

OMe, $CH_3$ and OH;
alternatively, $R^1$ is

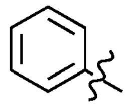

provided that $J^1$ is $N(CH_3)$, $L^1$ is

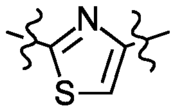

and D3 is selected from $NO_2$, $CF_3$,

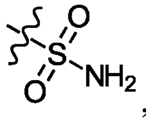

,OMe, $CH_3$ and OH;
alternatively, $R^1$ is

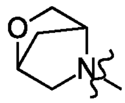

provided that $D^3$ is selected from Br, F, Cl and $NO_2$;
alternatively, $R^1$ is

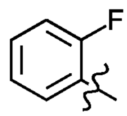

provided that $D^3$ is selected from H, Br, F, Cl and $NO_2$;
alternatively, $R^1$ is

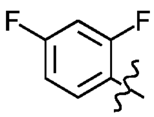

provided that $D^3$ is H;
alternatively, $R^1$ is

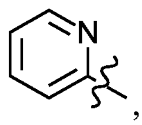

provided that $J^1$ is selected from N(H), $N(CH_3)$ and O;
alternatively, $R^1$ is

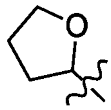

provided that $D^3$ is selected from H, Br, F, Cl, $CF_3$ and $NO_2$;
$D^1$ is selected from H, Br, F, Cl and OH;
$D^2$ is selected from H, Br, F and Cl;
$D^3$ is selected from H, Br, F, Cl, $CF_3$,

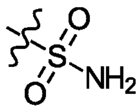,

OMe, $CH_3$ and OH;
alternatively, $D^3$ is $NO_2$, provided that when $R^1$ is

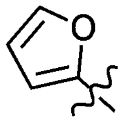

and when $J^1$ is O, $D^3$ is selected from H, Br, F, Cl, $CF_3$ and

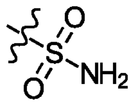;

$D^4$ is selected from H, Br, F and Cl;
provided that the compound is not

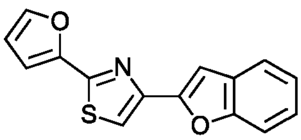, 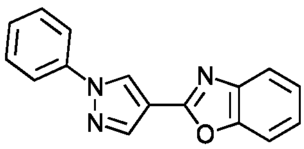,

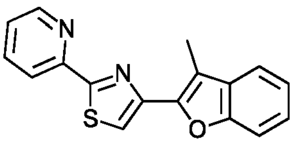, 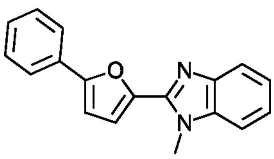, 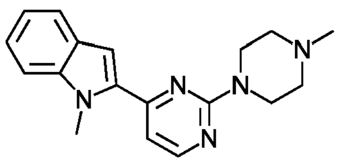,

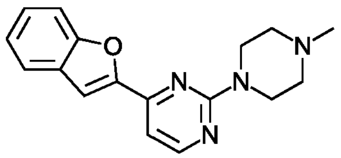, 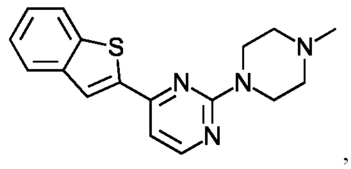, 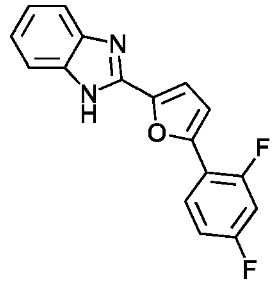,

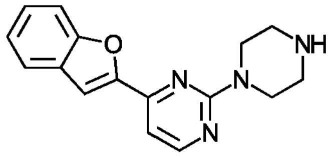, 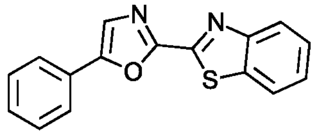, 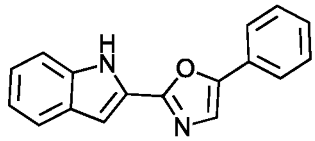,

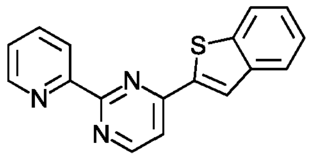, 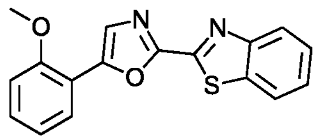, 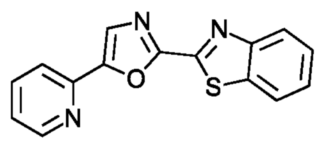,

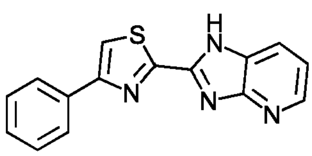, 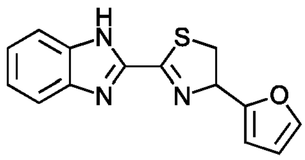, 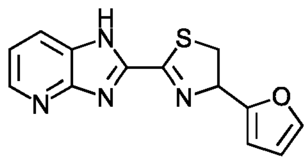,

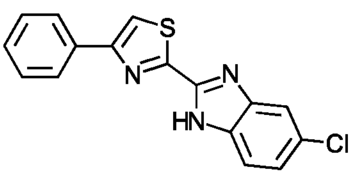, 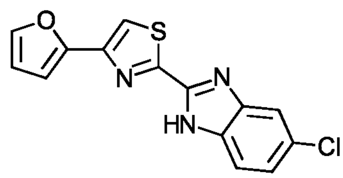, 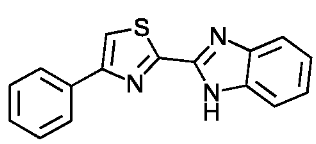,

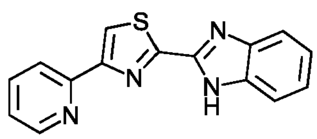, 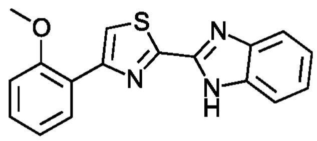, 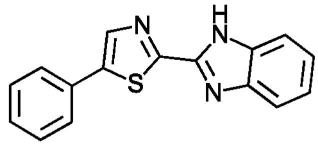,

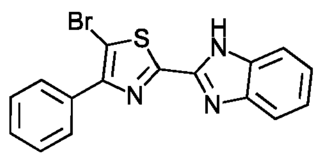, 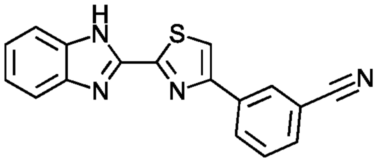, 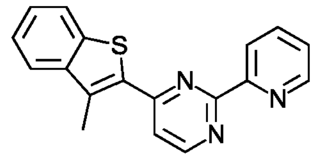,

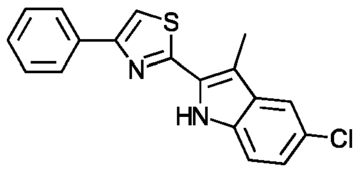, 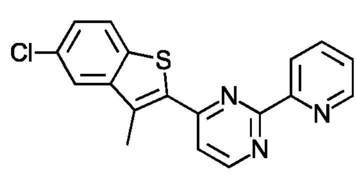, 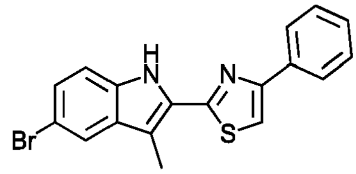,

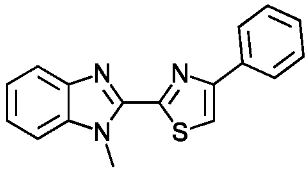, 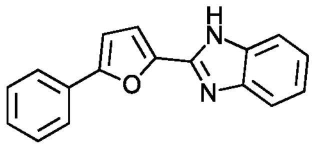 or 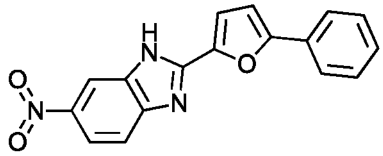 .

2. The compound of claim 1, wherein

L[1] is selected from

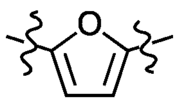, 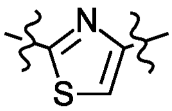, 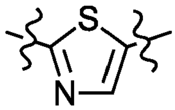, 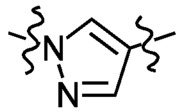,

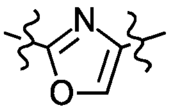, 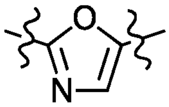, 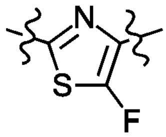, 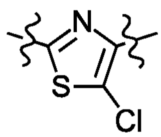, 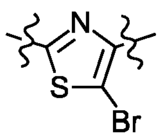, 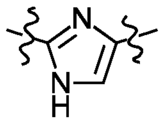,

and

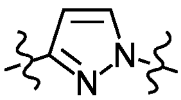;

R[1] is selected from

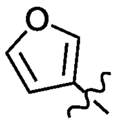, 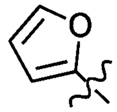, 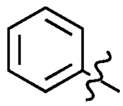, 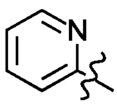, 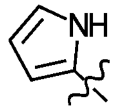, 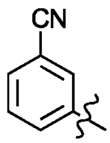,

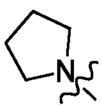, 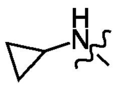, 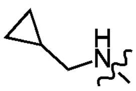, 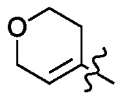, 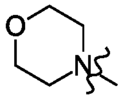, 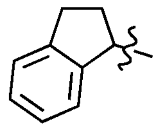, 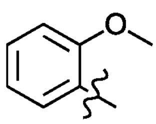, 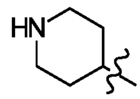,

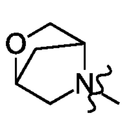, 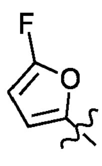, 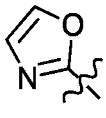, 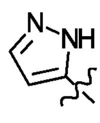, 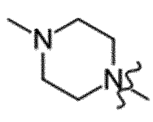 and 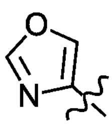;

$D^1$ is selected from H, Br, F and Cl;
$D^2$ is selected from H, Br, F and Cl;
$D^3$ is selected from H, Br, F, Cl, $NO_2$, $CF_3$, OMe, $CH_3$, OH and

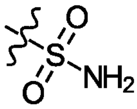;

and
$D^4$ is selected from H, Br, F and Cl;
or wherein
$L^1$ is selected from

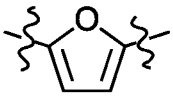, 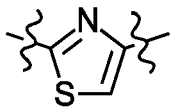, 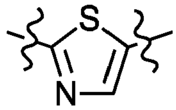 and 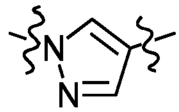;

$R^1$ is selected from

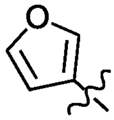, 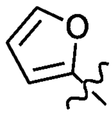, 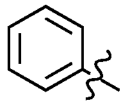, 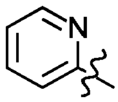 and 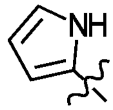;

$D^1$ is selected from H, Br, F and Cl;
$D^2$ is selected from H, Br, F and Cl;
D3 is selected from H, Br, F, Cl and $CF_3$; and
$D^4$ is selected from H, Br, F and Cl;
or wherein
$L^1$ is selected from

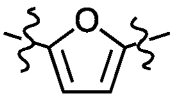, 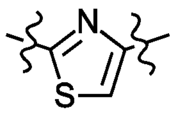, 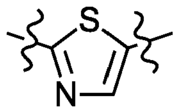 and 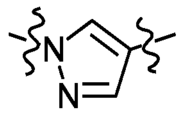;

$R^1$ is selected from

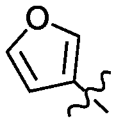, 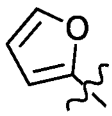, 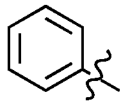, 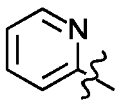 and 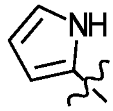;

$D^1$ is H;
$D^2$ is H;
$D^3$ is selected from H, Br, F, Cl and $CF_3$; and
D4 is H.

**3.** The compound of claim 1 or 2, wherein $R^1$ is

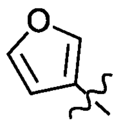, 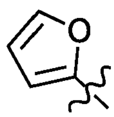, 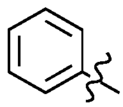, 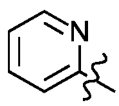,

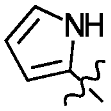, 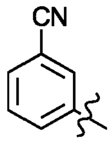, 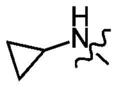, 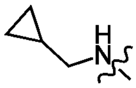, 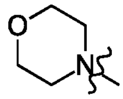, 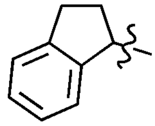, 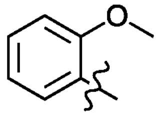, 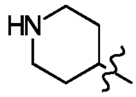,

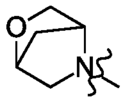, 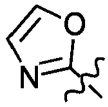, 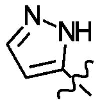 and 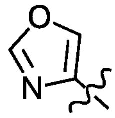.

**4.** The compound of any one of claims 1-3, wherein $D^1$ is H; $D^2$ is H; $D^3$ is selected from H, Br, F, Cl and $CF_3$; and $D^4$ is H.

**5.** The compound of claim 1, wherein $R^1$ is

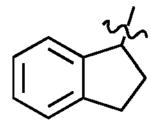

and $L^1$ is selected from

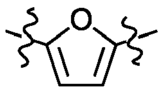

and

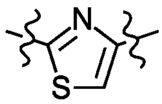;

or wherein G is selected from N, C(H) and $C(CH_3)$; and $J^1$ is selected from N(H), $N(CH_3)$, $N(CH_2CH_3)$, S and O;
or wherein
$L^1$ is selected from

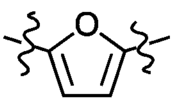, 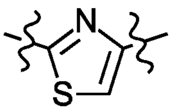, 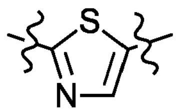 and 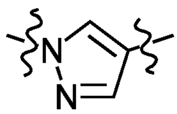;

$R^1$ is selected from

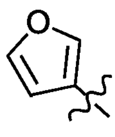, 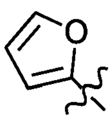, 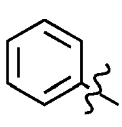, 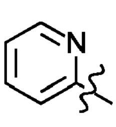 and 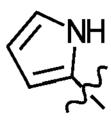;

$D^1$ is selected from H, Br, F and Cl;
$D^2$ is selected from H, Br, F and Cl;
D3 is selected from H, Br, F, Cl and $CF_3$; and
$D^4$ is selected from H, Br, F and Cl;
or wherein G is selected from N, C(H) and $C(CH_3)$;
$J^1$ is selected from N(H), $N(CH_3)$, $N(CH_2CH_3)$, S and O;
$L^1$ is selected from

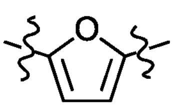, 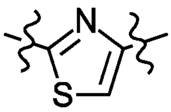, 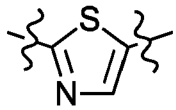 and 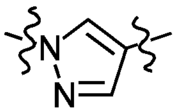;

$R^1$ is selected from

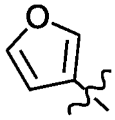, 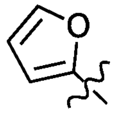, 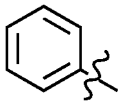, 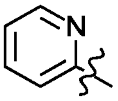 and 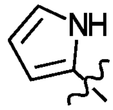;

$D^1$ is H;
$D^2$ is H;
$D^3$ is selected from H, Br, F, Cl and $CF_3$; and
$D^4$ is H.

**6.** The compound of any one of claims 1-5, wherein the compound is

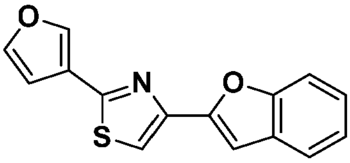, 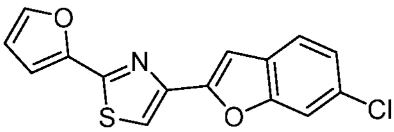, 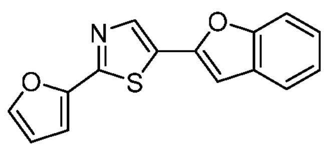,

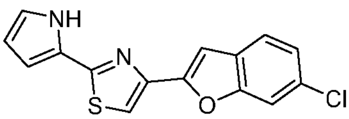, 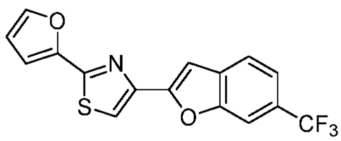 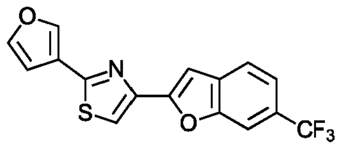,

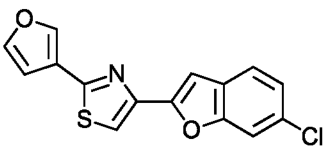, 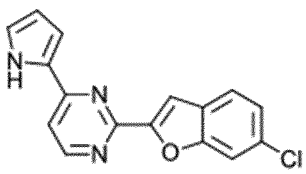, 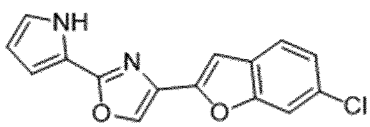,

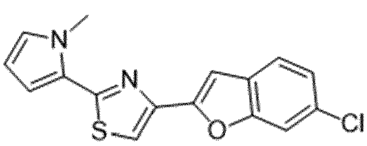, 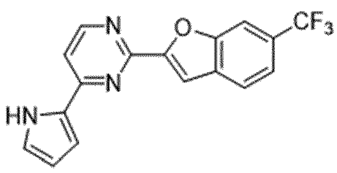, 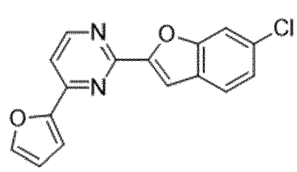,

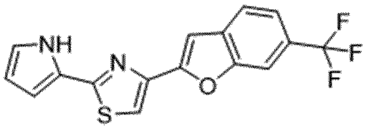, 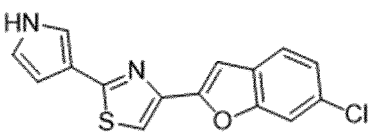, 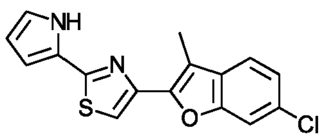,

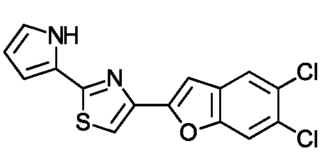, 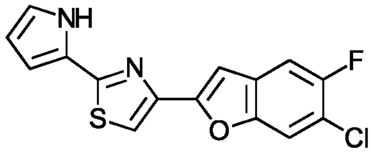 or 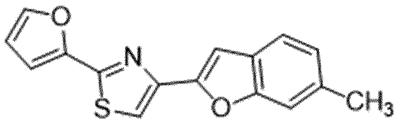.

**7.** The compound of claim 1, wherein the compound has the structure of Formula II:

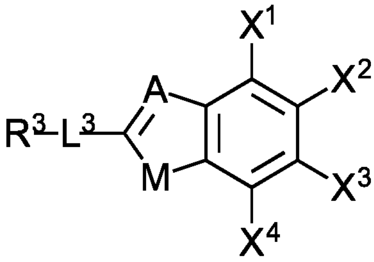

**II**

wherein,

A is selected from N, C(H) and $C(CH_3)$;
M is selected from N(H), $N(CF_3)$, $C(H)(CF_3)$, S and O;
$X^1$ is selected from H, Br, F and Cl;
$X^2$ is selected from H, Br, F and Cl;
$X^3$ is selected from H, Br, F, Cl, $CF_3$,

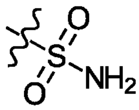

and OH;
$X^4$ is selected from H, Br, F and Cl;
$L^3$ is selected from

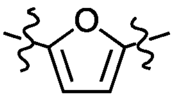, 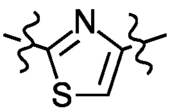, 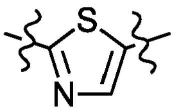, 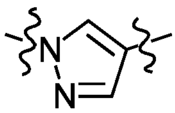,

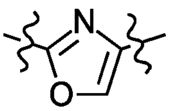, 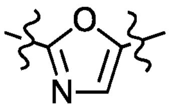 and 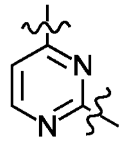;

$R^3$ is selected from

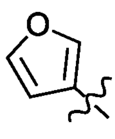, 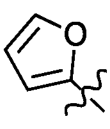, 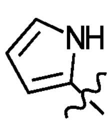, 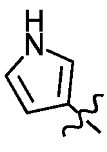 and 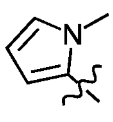.

**8.** A compound having the structure of Formula III:

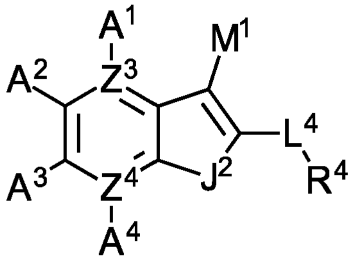

III

wherein,

$J^2$ is selected from $CH_2$, $CH(CH_3)$, N(H), $N(CH_3)$, $N(CH_2CH_3)$, $N(CF_3)$, $C(H)(CF_3)$, Sand O;
$M^1$ is selected from H and $CH_3$;
$Z^3$ is C;
$Z^4$ is C;
alternatively, $Z^3$ is N, when $A^1$ is absent;
alternatively, $Z^4$ is N, when $A^4$ is absent;
$L^4$ is selected from

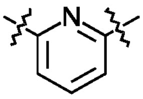 and 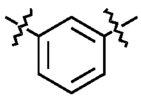 ;

R4 is selected from

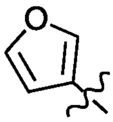, 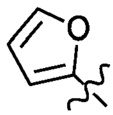, 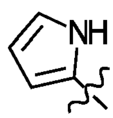, 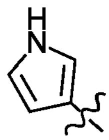, 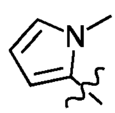, 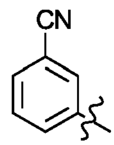,

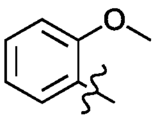, 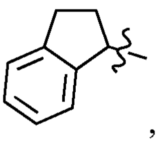, 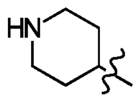, 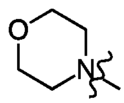, 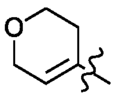, 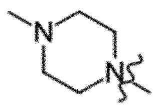, 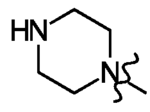, 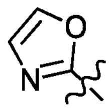,

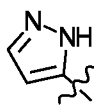, 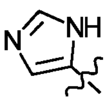, 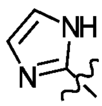, 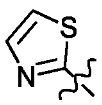, 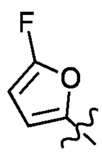, 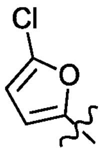, 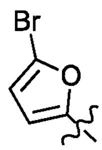, 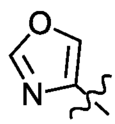, 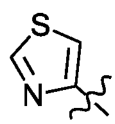, 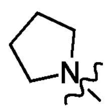,

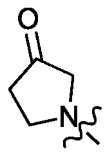, 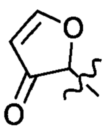, 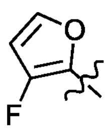, 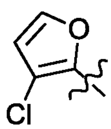, 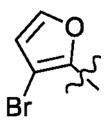, 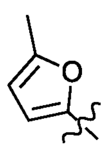, 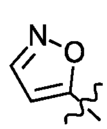, 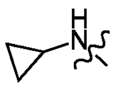, 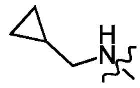

and

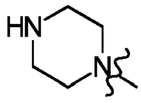;

$A^1$ is selected from H, Br, F, Cl and OH;
$A^2$ is selected from H, Br, F and Cl;
$A^3$ is selected from H, Br, F, Cl, $CF_3$,

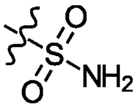,

OMe, $CH_3$, $NO_2$ and OH; and
$A^4$ is selected from H, Br, F and Cl.

**9.** The compound of claim 8, wherein $R^4$ is selected from

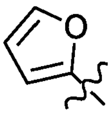, 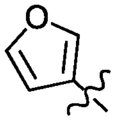, 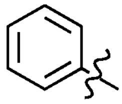,

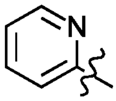, 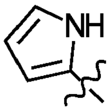, 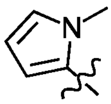 and 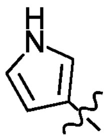.

**10.** The compound of claim 8 or 9, wherein $R^4$ is selected from

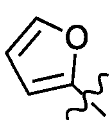, 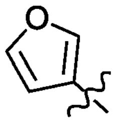, 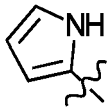

, 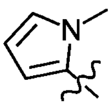 and 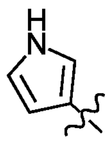.

**11.** The compound of any one of claims 8-10, wherein

$A^1$ is selected from H, Br, F, Cl and OH;
$A^2$ is selected from H, Br, F and Cl;
$A^3$ is selected from H, Br, F, Cl, $CF_3$, and $CH_3$; and
$A^4$ is selected from H, Br, F and Cl.

**12.** The compound of any one of claims 8-11, wherein $J^2$ is selected from $CH_2$, $CH(CH_3)$, N(H), $N(CH_3)$, S and O;

or wherein $J^2$ is selected from $CH_2$, $CH(CH_3)$, N(H), S and O; or
wherein $J^2$ is O.

**13.** The compound of any one of claims 8-12, wherein $L^4$ is

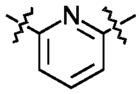.

**14.** A compound having the structure of Formula I:

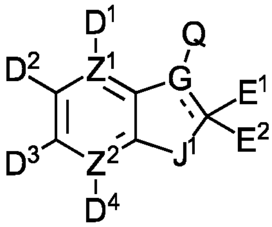

I

wherein,

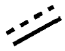

is a double bond;
Q is absent;
G is selected from N, C(H) and $C(CH_3)$ ;
$J^1$ is selected from N(H), $N(CH_3)$, $N(CH_2CH_3)$, $N(CF_3)$, $C(H)(CF_3)$, S and O;
$Z^1$ is C or N;
$Z^2$ is C or N;
alternatively, $Z^1$ is N, when $D^1$ is absent;
alternatively, $Z^2$ is N, when $D^4$ is absent;
$E^2$ is absent;
$E^1$ is $L^1$-$R^1$;
$L^1$ is selected from

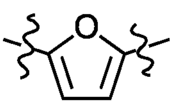, 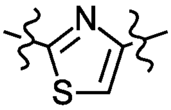, 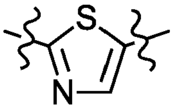, 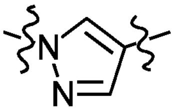,

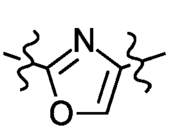, 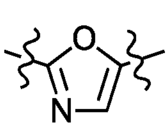, 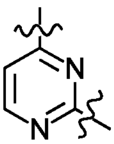, 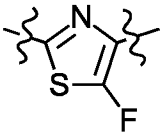, 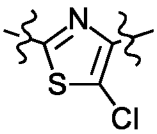, 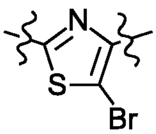, 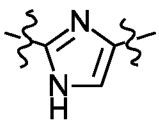,

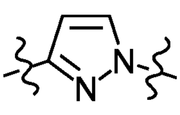, and 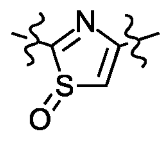 ;

$R^1$ is selected from

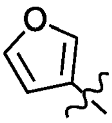, 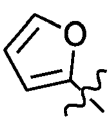, 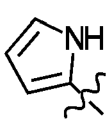, 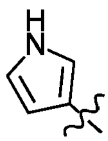, 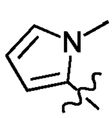, 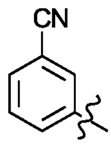,

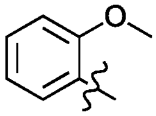, 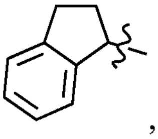, 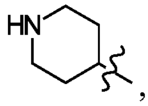, 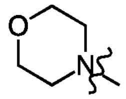, 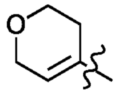, 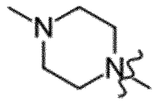, 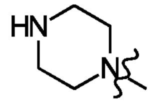, 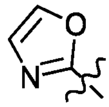,

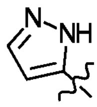, 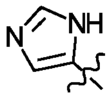, 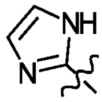, 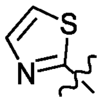, 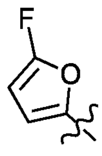, 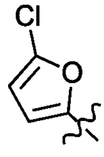, 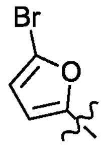, 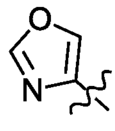, 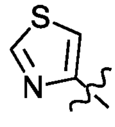, 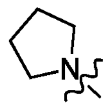,

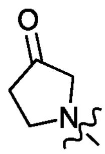, 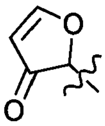, 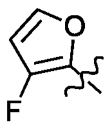, 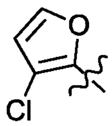, 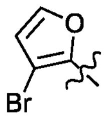, 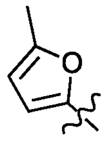, 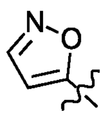, 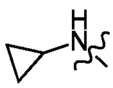, 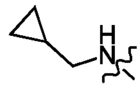

and

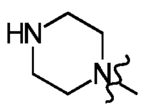;

alternatively, $R^1$ is

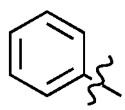

provided that $J^1$ is selected from N(H), $N(CH_2CH_3)$, $N(CF_3)$, $C(H)(CF_3)$, S and O; alternatively, $R^1$ is

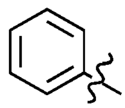

provided that $J^1$ is $N(CH_3)$, $L^1$ is selected from

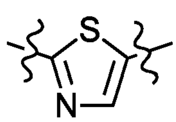

, 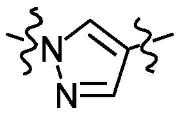, 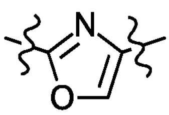, 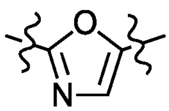 and 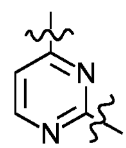

and $D^3$ is selected from H, Br, F, Cl, $NO_2$, $CF_3$,

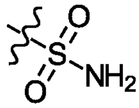,

OMe, $CH_3$ and OH;
alternatively, $R^1$ is

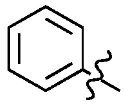

provided that $J^1$ is $N(CH_3)$, $L^1$ is

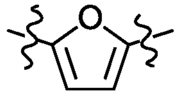

and $D^3$ is selected from Br, F, Cl,

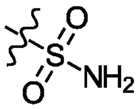,

OMe, $CH_3$ and OH;
alternatively, $R^1$ is

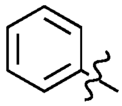

provided that $J^1$ is $N(CH_3)$, $L^1$ is

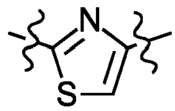

and D3 is selected from $NO_2$, $CF_3$,

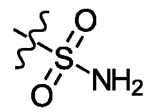,

OMe, $CH_3$ and OH;
alternatively, $R^1$ is

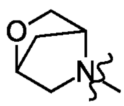

provided that $D^3$ is selected from Br, F, Cl and $NO_2$;

alternatively, $R^1$ is

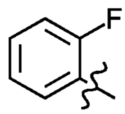

provided that $D^3$ is selected from H, Br, F, Cl and $NO_2$;
alternatively, $R^1$ is

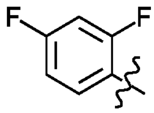

provided that $D^3$ is H;
alternatively, $R^1$ is

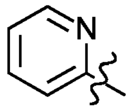

,

provided that $J^1$ is selected from N(H), $N(CH_3)$ and O;
alternatively, $R^1$ is

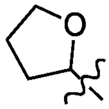

provided that $D^3$ is selected from H, Br, F, Cl, $CF_3$ and $NO_2$;
$D^1$ is selected from H, Br, F, Cl and OH;
$D^2$ is selected from H, Br, F and Cl;
$D^3$ is selected from H, Br, F, Cl, $CF_3$,

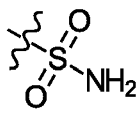

,

OMe, $CH_3$ and OH;
alternatively, $D^3$ is $NO_2$, provided that when $R^1$ is

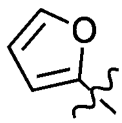

and when $J^1$ is O, $D^3$ is selected from H, Br, F, Cl, $CF_3$ and

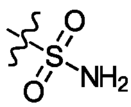

;

$D^4$ is selected from H, Br, F and Cl;
or a compound of any one of claims 7-13,

for use in the treatment of cancer.

**15.** The compound for use according to claim 14, wherein the cancer is a BRN2 expressing cancer selected from the following cancers:

prostate cancer (such as Neuroendocrine Prostate Cancer (NEPC); Prostate Adenocarcinoma; castration resistant prostate cancer (CRPC); androgen receptor pathway inhibitor (ARPI) resistant prostate cancer; enzalutamide (ENZ)-resistant ($ENZ^R$); and Abiraterone (Abi)-resistant ($ABI_R$));
lung cancer (such as small cell lung cancer (SCLC) or lung adenocarcinoma);
bladder cancer (such as small cell bladder cancer (SCBC));
sarcoma (such as Ewing's sarcoma);
glioma (such as glioblastoma multiforme); and
melanoma; or
a cancer selected from the following: bladder cancer; cholangiocarcinoma; colorectal cancer; diffuse large B-cell lymphoma (DLBC); liver cancer; ovarian cancer; thymoma;
thyroid cancer; clear cell renal cell carcinoma (CCRCC); chromophobe renal cell carcinoma (ChRCC); prostate cancer; breast cancer; uterine cancer; pancreatic cancer;
cervical cancer; uveal melanoma; acute myeloid leukemia (AML); head and neck cancer;
small cell lung cancer (SCLC); lung adenocarcinoma sarcoma; mesothelioma; adenoid cystic carcinoma (ACC); sarcoma; testicular germ cell cancer; uterine cancer;
pheochromocytoma and paraganglioma (PCPG); melanoma; glioma; glioblastoma multiforme; T-cell Acute Lymphoblastic Leukemia; T-cell Lymphoma, medulloblastoma;
and neuroblastoma.

## Patentansprüche

**1.** Eine Verbindung, aufweisend eine Struktur der Formel I:

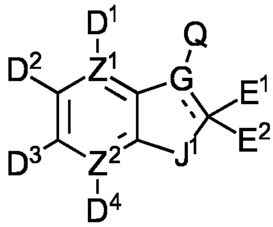

I,

wobei

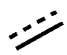

eine Doppelbindung ist;
Q abwesend ist;
G ausgewählt ist aus N, C(H) und $C(CH_3)$;
$J^1$ ausgewählt ist aus N(H), $N(CH_3)$, $N(CH_2CH_3)$, $N(CF_3)$, $C(H)(CF_3)$, S und O;
$Z^1$ C oder N ist;
$Z^2$ C oder N ist;
alternativ $Z^1$ N ist, wenn $D^1$ abwesend ist;
alternativ $Z^2$ N ist, wenn $D^4$ abwesend ist;
$E^2$ abwesend ist;
$E^1$ $L^1$-$R^1$ ist;
$L^1$ ausgewählt ist aus

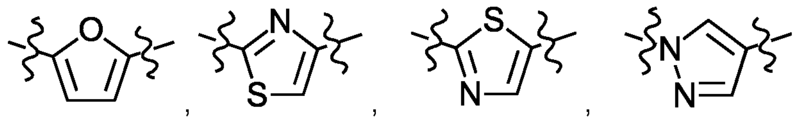, , , ,

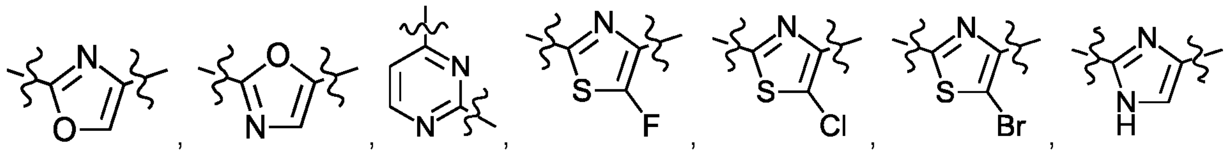, , , , , , ,

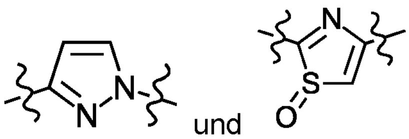 und ;

$R^1$ ausgewählt ist aus

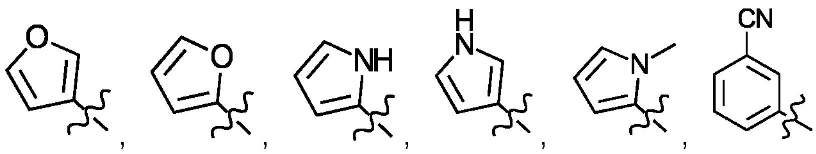, , , , , ,

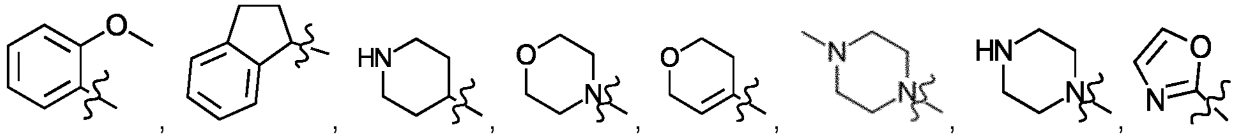, , , , , , , ,

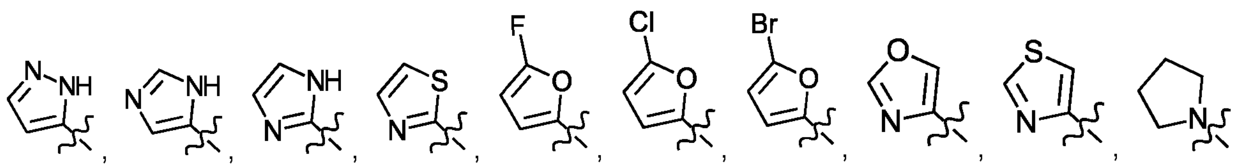, , , , , , , , , ,

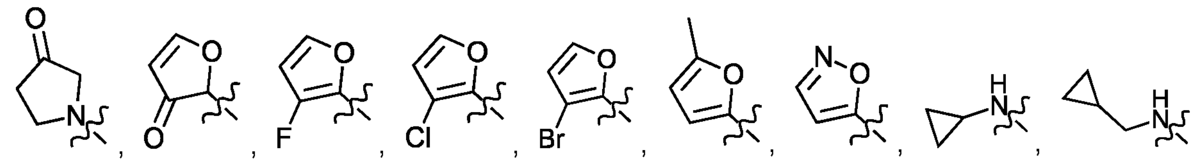, , , , , , , ,

und

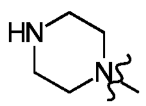;

alternativ $R^1$

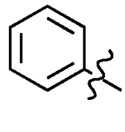

ist, sofern $J^1$ ausgewählt ist aus N(H), $N(CH_2CH_3)$, $N(CF_3)$, $C(H)(CF_3)$, S und O; alternativ $R^1$

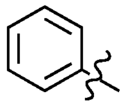

ist, sofern $J^1$ $N(CH_3)$ ist, $L^1$ ausgewählt ist aus

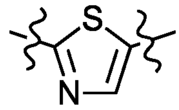,

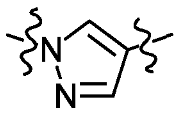, 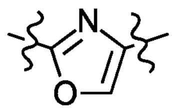, 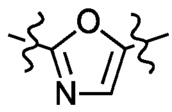 und 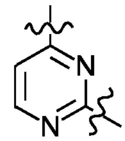

und $D^3$ ausgewählt ist aus H, Br, F, Cl, $NO_2$, $CF_3$,

, 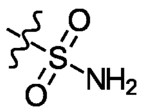,

OMe, $CH_3$ und OH;
alternativ $R^1$

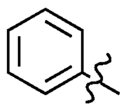

ist, sofern $J^1$ $N(CH_3)$ ist, $L^1$

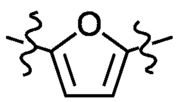

ist und $D^3$ ausgewählt ist aus Br, F, Cl,

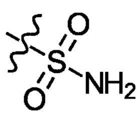,

OMe, $CH_3$ und OH;
alternativ $R^1$

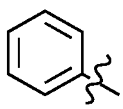

ist, sofern $J^1$ $N(CH_3)$ ist, $L^1$

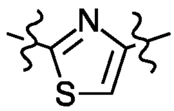

ist und $D^3$ ausgewählt ist aus $NO_2$, $CF_3$,

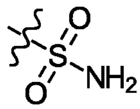

,

OMe, $CH_3$ und OH;
alternativ $R^1$

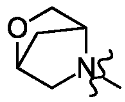

ist, sofern $D^3$ ausgewählt ist aus Br, F, Cl und $NO_2$;
alternativ $R^1$

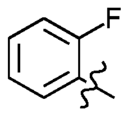

ist, sofern $D^3$ ausgewählt ist aus H, Br, F, Cl und $NO_2$;
alternativ $R^1$

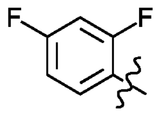

ist, sofern $D^3$ H ist;
alternativ $R^1$

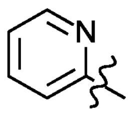

ist, sofern $J^1$ ausgewählt ist aus N(H), $N(CH_3)$ und O;
alternativ $R^1$

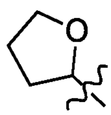

ist, sofern $D^3$ ausgewählt ist aus H, Br, F, Cl, $CF_3$ und $NO_2$;
$D^1$ ausgewählt ist aus H, Br, F, Cl und OH;
$D^2$ ausgewählt ist aus H, Br, F und Cl;
$D^3$ ausgewählt ist aus H, Br, F, Cl, $CF_3$

, 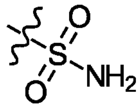 ,

OMe, $CH_3$ und OH;
alternativ $D^3$ $NO_2$ ist, sofern, wenn $R^1$

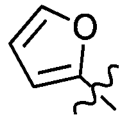

ist und wenn $J^1$ O ist, $D^3$ ausgewählt ist aus H, Br, F, Cl, $CF_3$ und

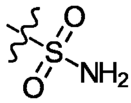 ;

$D^4$ ausgewählt ist aus H, Br, F und Cl;
sofern diese Verbindung nicht

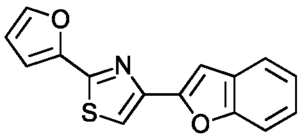 , 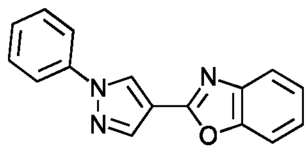 ,

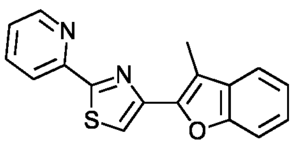 , 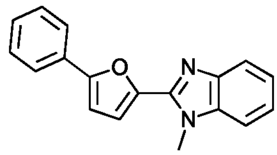 , 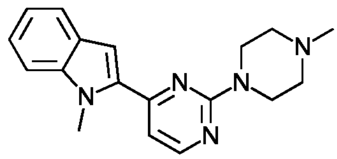 ,

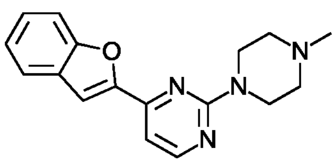 , 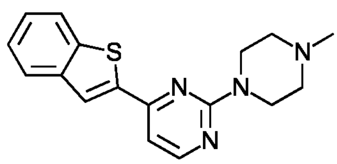 , 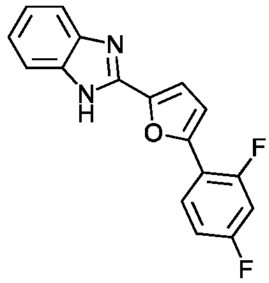 ,

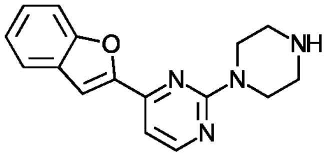 , 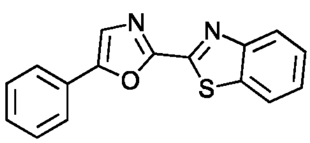 , 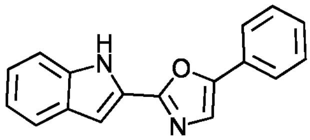 ,

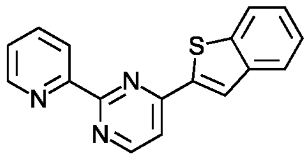,
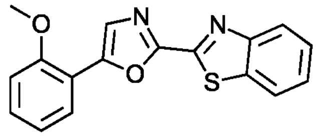,
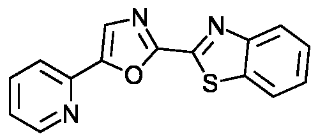,

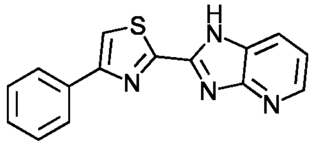,
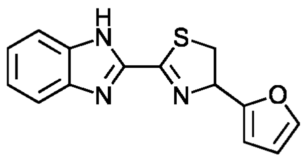,
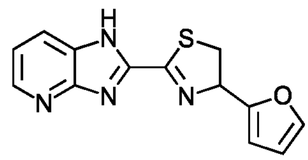,

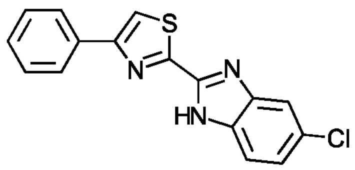,
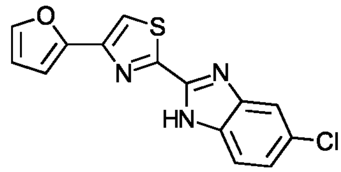,
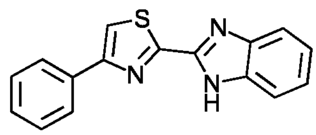,

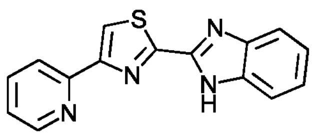,
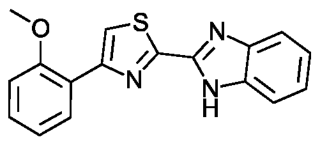,
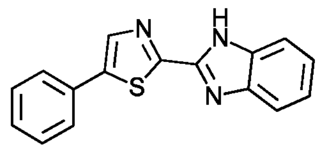,

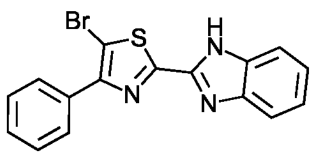,
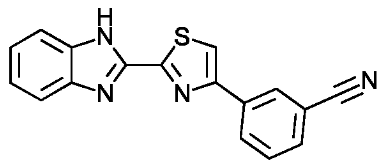,
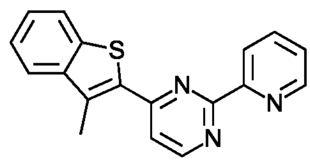,

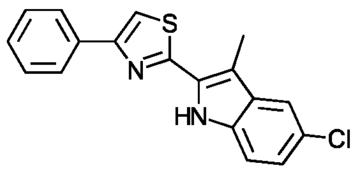,
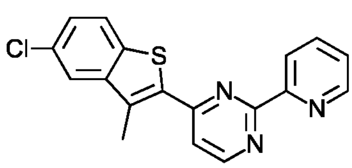,
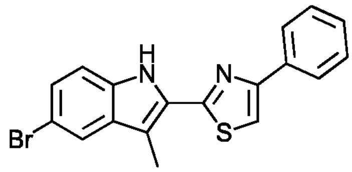,

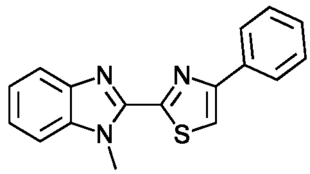,
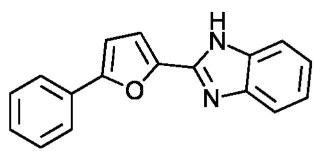 oder
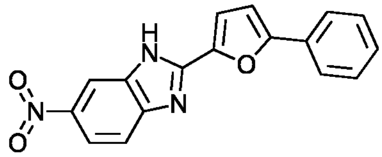

ist.

**2.** Die Verbindung nach Anspruch 1, wobei

$L^1$ ausgewählt ist aus

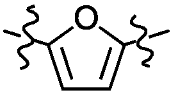,
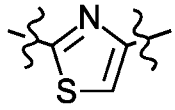,
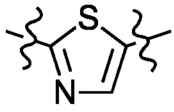,
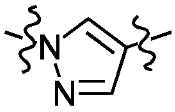,

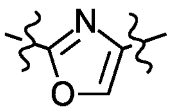, 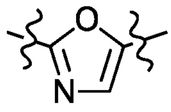, 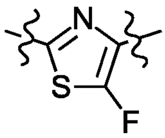, 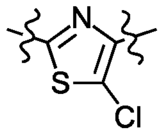, 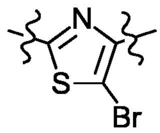, 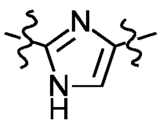

und

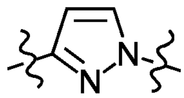;

$R^1$ ausgewählt ist aus

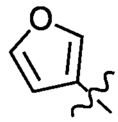, 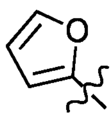, 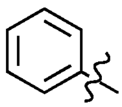, 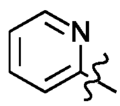, 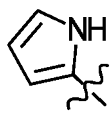, 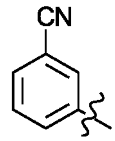,

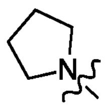, 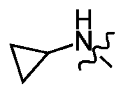, 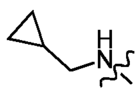, 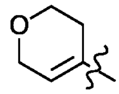, 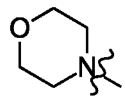, 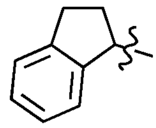, 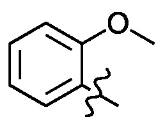, 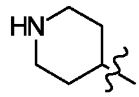,

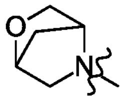, 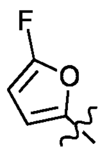, 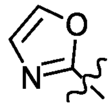, 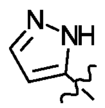, 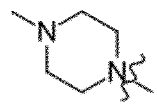 und 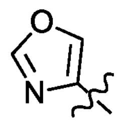;

$D^1$ ausgewählt ist aus H, Br, F und Cl;
$D^2$ ausgewählt ist aus H, Br, F und Cl;
$D^3$ ausgewählt ist aus H, Br, F, Cl, $NO_2$, $CF_3$, OMe, $CH_3$, OH und

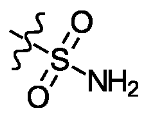;

und
$D^4$ ausgewählt ist aus H, Br, F und Cl;
oder wobei
$L^1$ ausgewählt ist aus

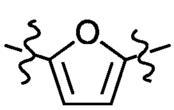, 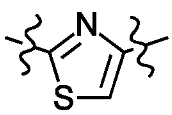, 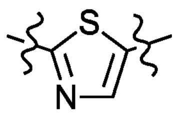 und 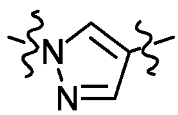;

$R^1$ ausgewählt ist aus

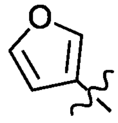, 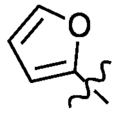, 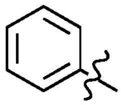, 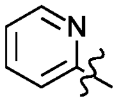 und 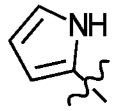;

$D^1$ ausgewählt ist aus H, Br, F und Cl;
$D^2$ ausgewählt ist aus H, Br, F und Cl;
$D^3$ ausgewählt ist aus H, Br, F, Cl und $CF_3$; und
$D^4$ ausgewählt ist aus H, Br, F und Cl;
oder wobei
$L^1$ ausgewählt ist aus

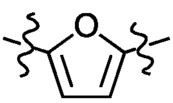, 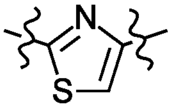, 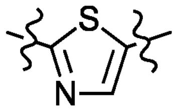 und 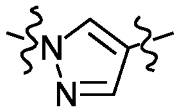;

$R^1$ ausgewählt ist aus

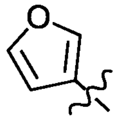, 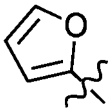, 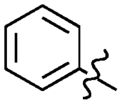, 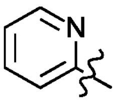 und 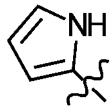;

$D^1$ H ist;
$D^2$ H ist;
$D^3$ ausgewählt ist aus H, Br, F, Cl und $CF_3$; und
$D^4$ H ist.

**3.** Die Verbindung nach Anspruch 1 oder 2, wobei $R^1$

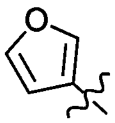, 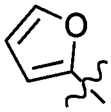, 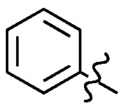,

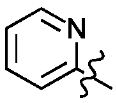, 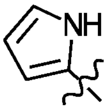, 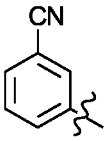, 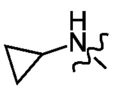, 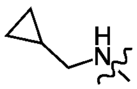, 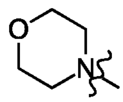, 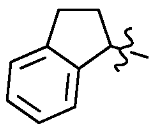, 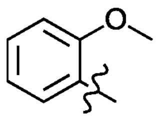,

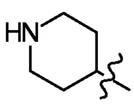, 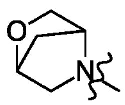, 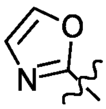, 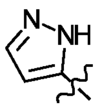 und 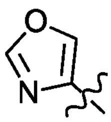 ist.

**4.** Die Verbindung nach einem der Ansprüche 1-3, wobei $D^1$ H ist; $D^2$ H ist; $D^3$ ausgewählt ist aus H, Br, F, Cl und $CF_3$; und $D^4$ H ist.

**5.** Die Verbindung nach Anspruch 1, wobei $R^1$

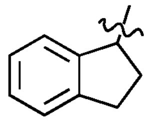

ist und $L^1$ ausgewählt ist aus

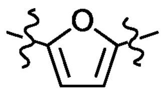 und 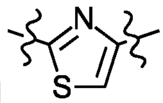 ;

oder wobei G ausgewählt ist aus N, C(H) und $C(CH_3)$; und $J^1$ ausgewählt ist aus N(H), $N(CH_3)$, $N(CH_2CH_3)$, S und O;
oder wobei
$L^1$ ausgewählt ist aus

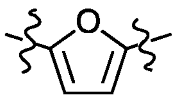, 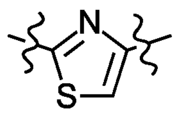, 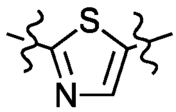 und 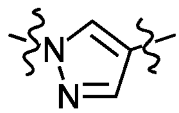 ;

$R^1$ ausgewählt ist aus

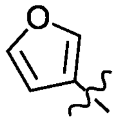, 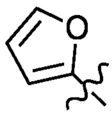, 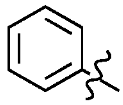, 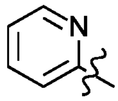 und 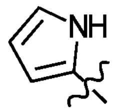 ;

$D^1$ ausgewählt ist aus H, Br, F und Cl;
$D^2$ ausgewählt ist aus H, Br, F und Cl;
$D^3$ ausgewählt ist aus H, Br, F, Cl und $CF_3$; und
$D^4$ ausgewählt ist aus H, Br, F und Cl;
oder wobei G ausgewählt ist aus N, C(H) und $C(CH_3)$;
$J^1$ ausgewählt ist aus N(H), $N(CH_3)$, $N(CH_2CH_3)$, S und O;
$L^1$ ausgewählt ist aus

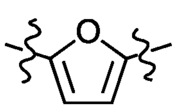, 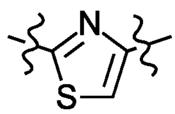, 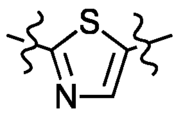 und 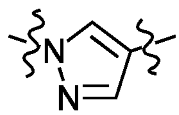 ;

$R^1$ ausgewählt ist aus

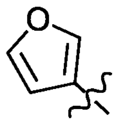, 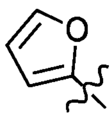, 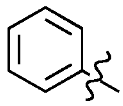, 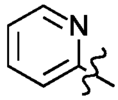 und 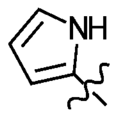 ;

$D^1$ H ist;
$D^2$ H ist;
$D^3$ ausgewählt ist aus H, Br, F, Cl und $CF_3$; und
$D^4$ H ist.

**6.** Die Verbindung nach einem der Ansprüche 1-5, wobei die Verbindung

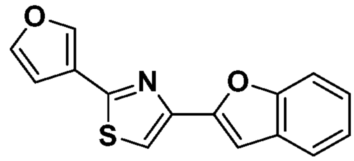, 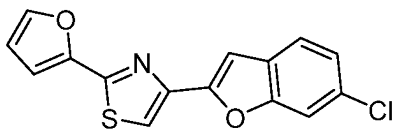, 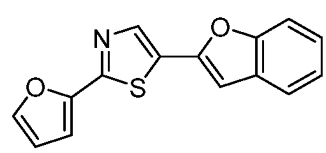,

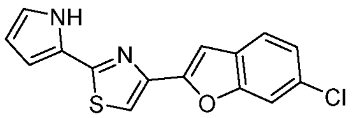, 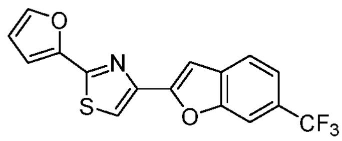 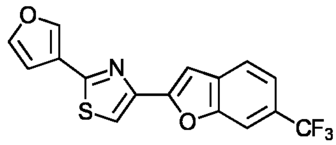,

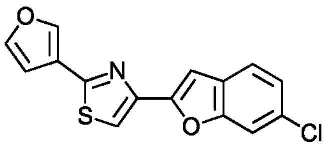, 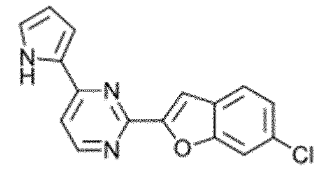, 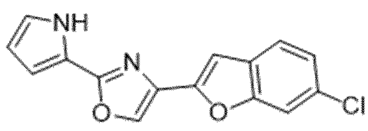,

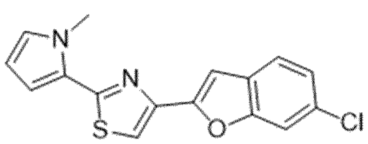, 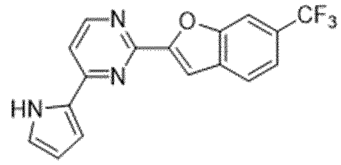, 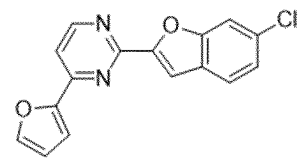,

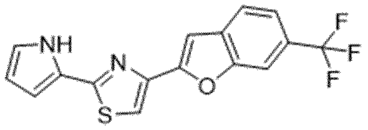, 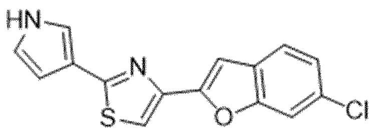, 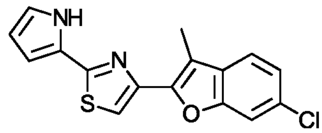,

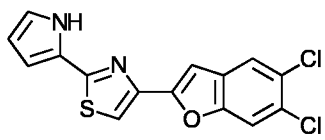, 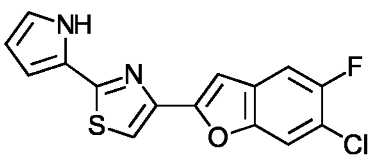 oder 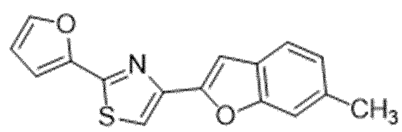 ist.

**7.** Die Verbindung nach Anspruch 1, wobei die Verbindung eine Struktur der Formel II aufweist:

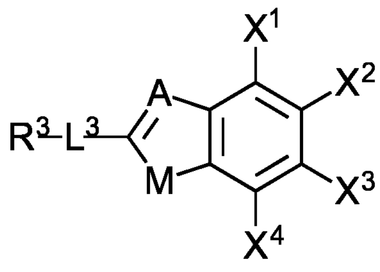

II

wobei

A ausgewählt ist aus N, C(H) und C($CH_3$);
M ausgewählt ist aus N(H), N($CF_3$), C(H)($CF_3$), S und O;
$X^1$ ausgewählt ist aus H, Br, F und Cl;
$X^2$ ausgewählt ist aus H, Br, F und Cl;

$X^3$ ausgewählt ist aus H, Br, F, Cl, $CF_3$,

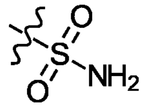

und OH;
$X^4$ ausgewählt ist aus H, Br, F und Cl;
$L^3$ ausgewählt ist aus

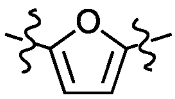, 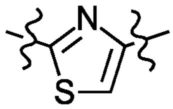, 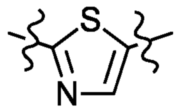, 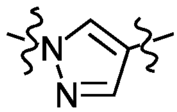,

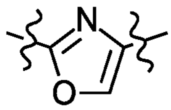, 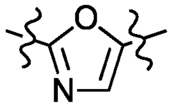 und 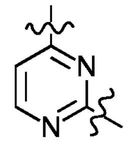;

$R^3$ ausgewählt ist aus

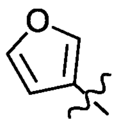, 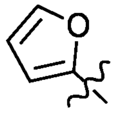, 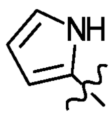, 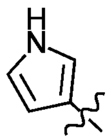 und 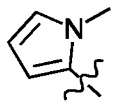.

**8.** Eine Verbindung, aufweisend eine Struktur der Formel III:

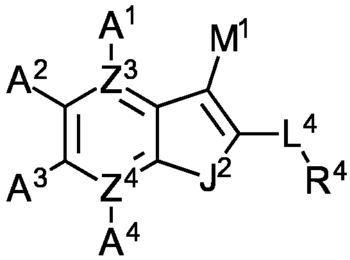

III

wobei

$J^2$ ausgewählt ist aus $CH_2$, $CH(CH_3)$, N(H), $N(CH_3)$, $N(CH_2CH_3)$, $N(CF_3)$, $C(H)(CF_3)$, S und O;
$M^1$ ausgewählt ist aus H und $CH_3$;
$Z^3$ C ist;
$Z^4$ C ist;
alternativ $Z^3$ N ist, wenn $A^1$ abwesend ist;
alternativ $Z^4$ N ist, wenn $A^4$ abwesend ist;
$L^4$ ausgewählt ist aus

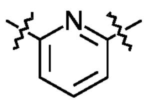 und 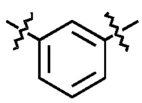;

$R^4$ ausgewählt ist aus

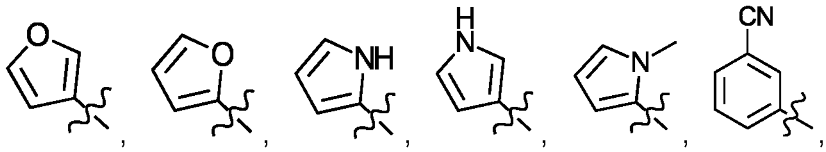

,

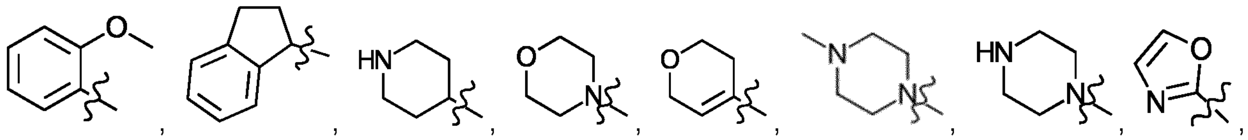

,

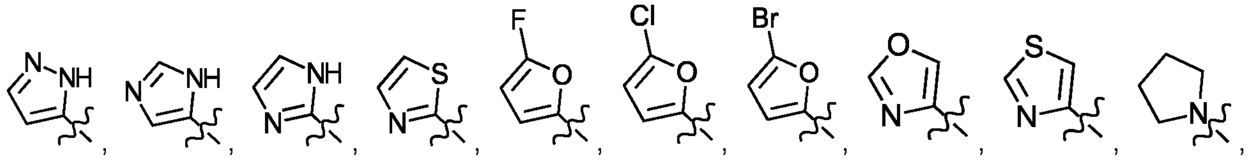

,

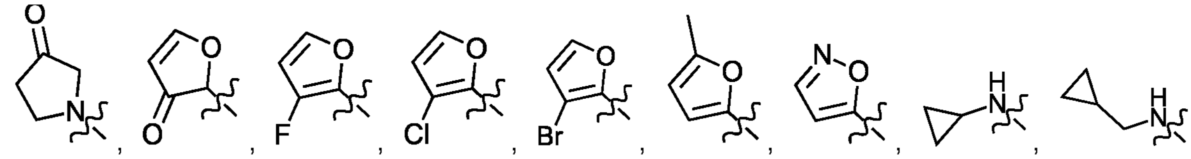

,

und

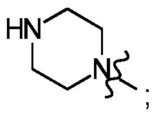

;

$A^1$ ausgewählt ist aus H, Br, F, Cl und OH;
$A^2$ ausgewählt ist aus H, Br, F und Cl;
$A^3$ ausgewählt ist aus H, Br, F, Cl, $CF_3$,

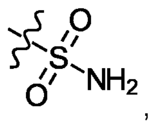

,

OMe, $CH_3$, $NO_2$ und OH; und
$A^4$ ausgewählt ist aus H, Br, F und Cl.

**9.** Die Verbindung nach Anspruch 8, wobei $R^4$ ausgewählt ist aus

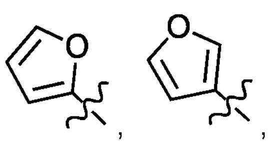

,

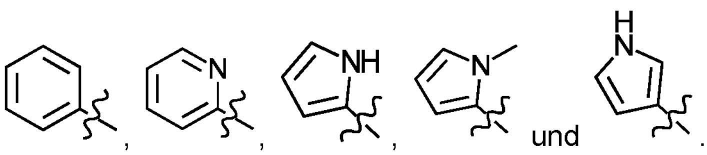

, und .

**10.** Die Verbindung nach Anspruch 8 oder 9, wobei $R^4$ ausgewählt ist aus

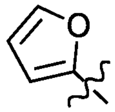,
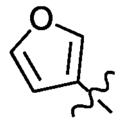,

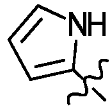,
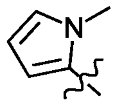 und
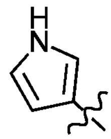.

**11.** Die Verbindung nach einem der Ansprüche 8-10, wobei

$A^1$ ausgewählt ist aus H, Br, F, Cl und OH;
$A^2$ ausgewählt ist aus H, Br, F und Cl;
$A^3$ ausgewählt ist aus H, Br, F, Cl, $CF_3$ und $CH_3$; und
$A^4$ ausgewählt ist aus H, Br, F und Cl.

**12.** Die Verbindung nach einem der Ansprüche 8-11, wobei $J^2$ ausgewählt ist aus $CH_2$, $CH(CH_3)$, N(H), $N(CH_3)$, S und O;

oder wobei $J^2$ ausgewählt ist aus $CH_2$, $CH(CH_3)$, N(H), S und O; oder
wobei $J^2$ O ist.

**13.** Die Verbindung nach einem der Ansprüche 8-12, wobei $L^4$

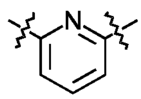

ist.

**14.** Eine Verbindung, aufweisend eine Struktur der Formel I:

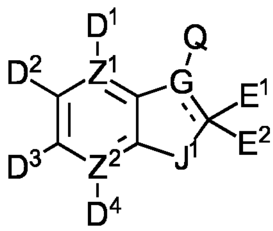

I,

wobei

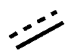

eine Doppelbindung ist;
Q abwesend ist;
G ausgewählt ist aus N, C(H) und $C(CH_3)$;
$J^1$ ausgewählt ist aus N(H), $N(CH_3)$, $N(CH_2CH_3)$, $N(CF_3)$, $C(H)(CF_3)$, S und O;
$Z^1$ C oder N ist;
$Z^2$ C oder N ist;
alternativ $Z^1$ N ist, wenn $D^1$ abwesend ist;

alternativ $Z^2$ N ist, wenn $D^4$ abwesend ist;
$E^2$ abwesend ist;
$E^1$ $L^1$-$R^1$ ist;
L' ausgewählt ist aus

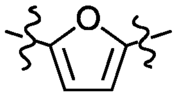, 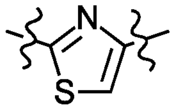, 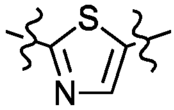, 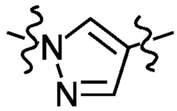,

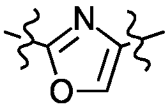, 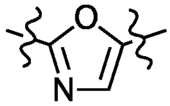, 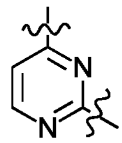, 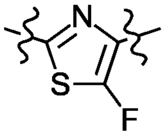, 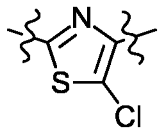, 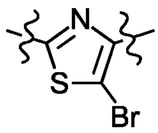, 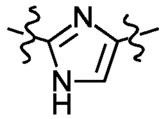,

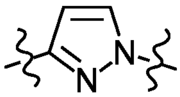 und 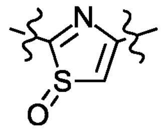;

$R^1$ ausgewählt ist aus

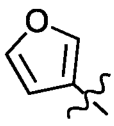, 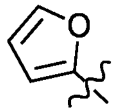, 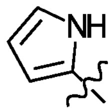, 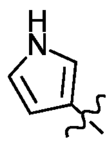, 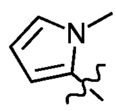, 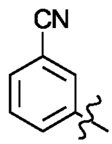,

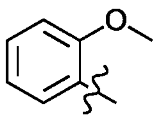, 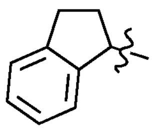, 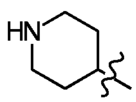, 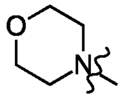, 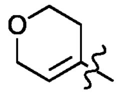, 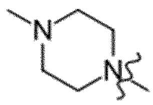, 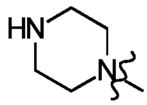, 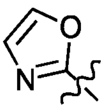,

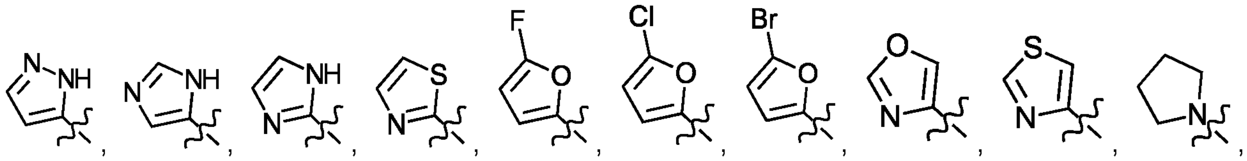

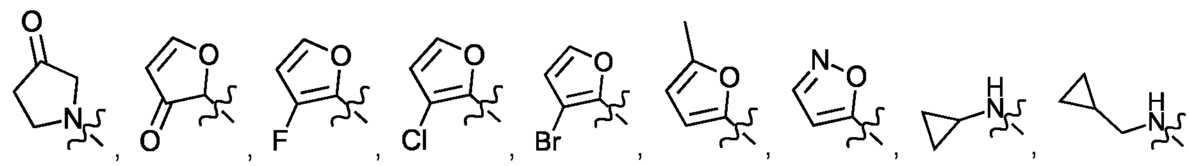

und

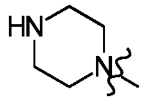;

alternativ $R^1$

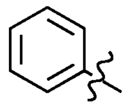

ist, sofern $J^1$ ausgewählt ist aus N(H), $N(CH_2CH_3)$, $N(CF_3)$, $C(H)(CF_3)$, S und O; alternativ $R^1$

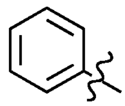

ist, sofern $J^1$ $N(CH_3)$ ist, $L^1$ ausgewählt ist aus

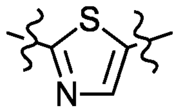

,

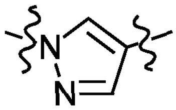
,
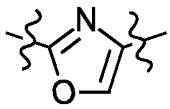
,
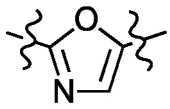
und
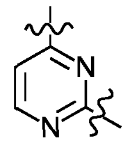

und $D^3$ ausgewählt ist aus H, Br, F, Cl, $NO_2$, $CF_3$,

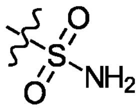

,

OMe, $CH_3$ und OH;
alternativ $R^1$

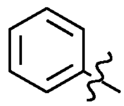

ist, sofern $J^1$ $N(CH_3)$ ist, $L^1$

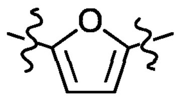

ist und $D^3$ ausgewählt ist aus Br, F, Cl,

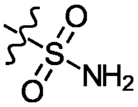

,

OMe, $CH_3$ und OH;
alternativ $R^1$

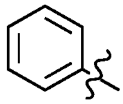

ist, sofern $J^1$ $N(CH_3)$ ist, L'

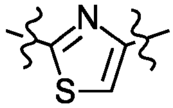

ist und $D^3$ ausgewählt ist aus $NO_2$, $CF_3$,

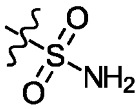

,

OMe, $CH_3$ und OH;
alternativ $R^1$

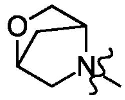

ist, sofern $D^3$ ausgewählt ist aus Br, F, Cl und $NO_2$;
alternativ $R^1$

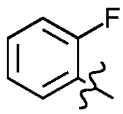

ist, sofern $D^3$ ausgewählt ist aus H, Br, F, Cl und $NO_2$;
alternativ $R^1$

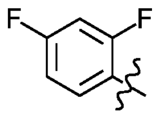

ist, sofern $D^3$ H ist;
alternativ $R^1$

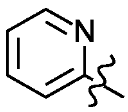

ist, sofern $J^1$ ausgewählt ist aus N(H), $N(CH_3)$ und O;
alternativ $R^1$

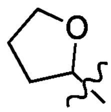

ist, sofern $D^3$ ausgewählt ist aus H, Br, F, Cl, $CF_3$ und $NO_2$;
$D^1$ ausgewählt ist aus H, Br, F, Cl und OH;
$D^2$ ausgewählt ist aus H, Br, F und Cl;
$D^3$ ausgewählt ist aus H, Br, F, Cl, $CF_3$,

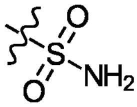

,

OMe, $CH_3$ und OH;
alternativ $D^3$ $NO_2$ ist, sofern, wenn $R^1$

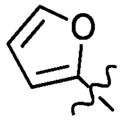

ist und wenn $J^1$ O ist, $D^3$ ausgewählt ist aus H, Br, F, Cl, $CF_3$ und

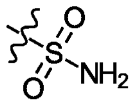

;

$D^4$ ausgewählt ist aus H, Br, F und Cl;
oder eine Verbindung nach einem der Ansprüche 7-13,
zur Verwendung bei der Behandlung von Krebs.

**15.** Die Verbindung zur Verwendung gemäß Anspruch 14, wobei der Krebs ein BRN2 exprimierender Krebs ist, ausgewählt aus den folgenden Krebsarten:

Prostatakrebs (wie z. B. neuroendokriner Prostatakrebs (NEPC); Adenokarzinom der Prostata; kastrationsresistenter Prostatakrebs (CRPC); Androgenrezeptor-Weg-Inhibitor (ARPI)-resistenter Prostatakrebs; Enzalutamid (ENZ)-resistenter ($ENZ^R$); und Abirateron (Abi)-resistenter ($ABI_R$));
Lungenkrebs (wie kleinzelliger Lungenkrebs (SCLC) oder Adenokarzinom der Lunge);
Blasenkrebs (wie kleinzelliger Blasenkrebs (SCBC));
Sarkom (wie z. B. Ewing-Sarkom);
Gliom (wie z. B. Glioblastoma multiforme); und
Melanom; oder
Krebs, ausgewählt aus den folgenden: Blasenkrebs; Cholangiokarzinom; Kolorektalkrebs; diffuses großzelliges B-Zell-Lymphom (DLBC); Leberkrebs; Eierstockkrebs; Thymom; Schilddrüsenkrebs; klarzelliges Nierenzellkarzinom (CCRCC); chromophobes Nierenzellkarzinom (ChRCC); Prostatakrebs; Brustkrebs; Gebärmutterkrebs; Bauchspeicheldrüsenkrebs; Gebärmutterhalskrebs; Aderhautmelanom; akute myeloische Leukämie (AML); Kopf- und Halskrebs; kleinzelliger Lungenkrebs (SCLC); Lungen-Adenokarzinom-Sarkom; Mesotheliom; adenoid-zystisches Karzinom (ACC); Sarkom; Keimzelltumor des Hodens; Gebärmutterkrebs; Phäochromozytom und Paragangliom (PCPG); Melanom; Gliom; Glioblastoma multiforme; Akute T-Zell-Lymphoblasten-Leukämie; T-Zell-Lymphom, Medulloblastom und Neuroblastom.

## Revendications

**1.** Composé ayant la structure de formule I :

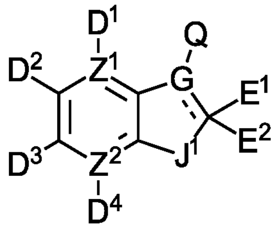

I

dans laquelle

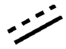

est une double liaison ;
Q est absent ;
G est choisi parmi N, C(H) et $C(CH_3)$ ;
$J^1$ est choisi parmi N(H), $N(CH_3)$, $N(CH_2CH_3)$, $N(CF_3)$, $C(H)(CF_3)$, S et O ;
$Z^1$ représente C ou N ;
$Z^2$ représente C ou N ;
en variante, $Z^1$ représente N, lorsque $D^1$ est absent ;
en variante, $Z^2$ représente N, lorsque $D^4$ est absent ;
$E^2$ est absent ;
$E^1$ représente $L^1$-$R^1$ ;
L' est choisi parmi

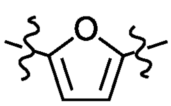 , 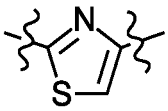 , 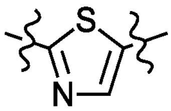 , 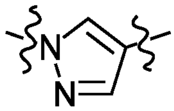 ,

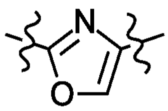 , 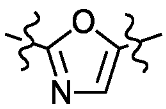 , 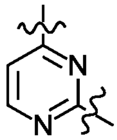 , 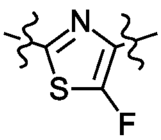 , 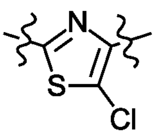 , 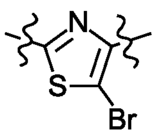 , 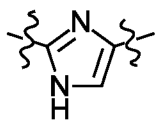

, 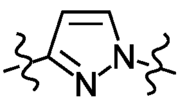 , et 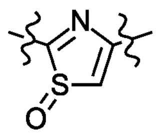 ;

$R^1$ est choisi parmi

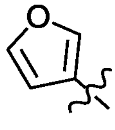 , 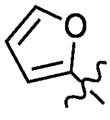 , 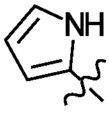 , 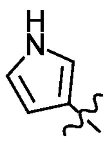 , 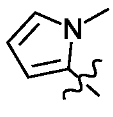 , 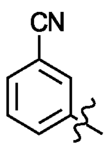 ,

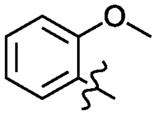 , 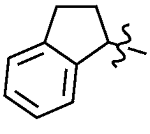 , 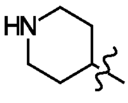 , 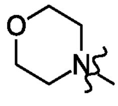 , 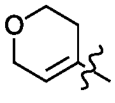 , 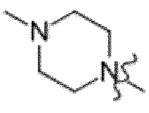 , 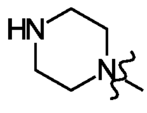 , 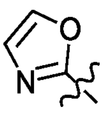 ,

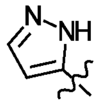, 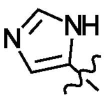, 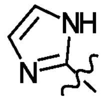, 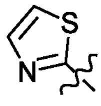, 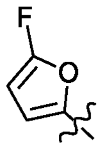, 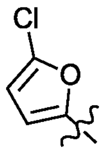, 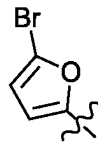, 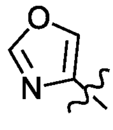, 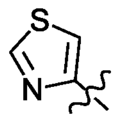, 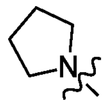,

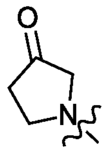, 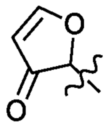, 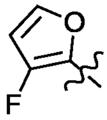, 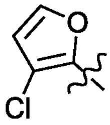, 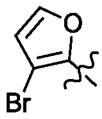, 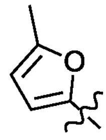, 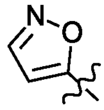, 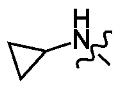, 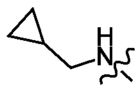

et

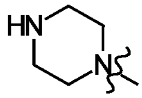 ;

en variante, $R^1$ représente

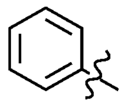

à condition que $J^1$ soit choisi parmi N(H), $N(CH_2CH_3)$, $N(CF_3)$, $C(H)(CF_3)$, S et O ; en variante, $R^1$ représente

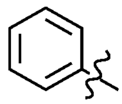

à condition que $J^1$ représente $N(CH_3)$, L' soit choisi parmi

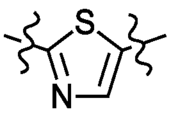, 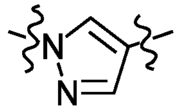, 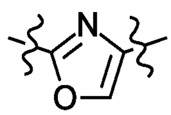, 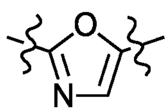 et 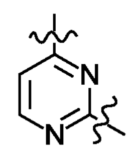

et $D^3$ soit choisi parmi H, Br, F, Cl, $NO_2$, $CF_3$, , OMe, $CH_3$ et OH ; en variante, $R^1$ représente

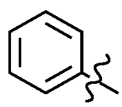

à condition que $J^1$ représente $N(CH_3)$, $L^1$ représente

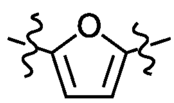

et $D^3$ soit choisi parmi Br, F, Cl,

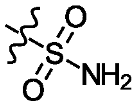,

OMe, $CH_3$ et OH ;
en variante, $R^1$ représente

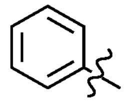

à condition que $J^1$ représente $N(CH_3)$, $L^1$ représente

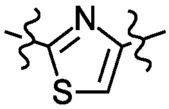

et $D^3$ soit choisi parmi $NO_2$, $CF_3$,

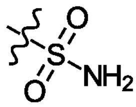,

OMe, $CH_3$ et OH ;
en variante, $R^1$ représente

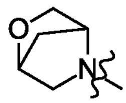

à condition que $D^3$ soit choisi parmi Br, F, Cl et NOz ;
en variante, $R^1$ représente

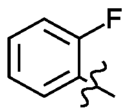

à condition que $D^3$ soit choisi parmi H, Br, F, Cl et NOz ;
en variante, $R^1$ représente

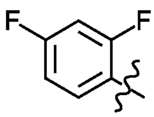

à condition que $D^3$ représente H ;
en variante, $R^1$ représente

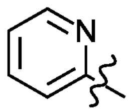,

à condition que $J^1$ soit choisi parmi N(H), N($CH_3$) et O ;
en variante, $R^1$ représente

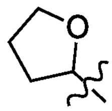

à condition que $D^3$ soit choisi parmi H, Br, F, Cl, $CF_3$ et $NO_2$ ;
$D^1$ est choisi parmi H, Br, F, Cl et OH ;
$D^2$ est choisi parmi H, Br, F et Cl ;
$D^3$ est choisi parmi H, Br, F, Cl, $CF_3$,

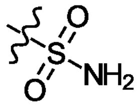,

OMe, $CH_3$ et OH ;
en variante, $D^3$ représente NOz, à condition que lorsque $R^1$ représente

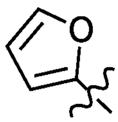

et lorsque $J^1$ représente O, $D^3$ soit choisi parmi H, Br, F, Cl, $CF_3$ et

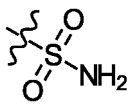;

$D^4$ est choisi parmi H, Br, F et Cl ;
à condition que le composé ne représente pas

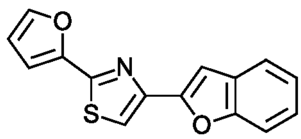,

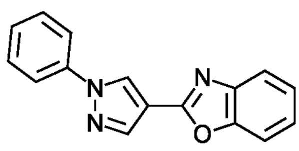, 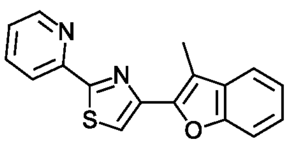, 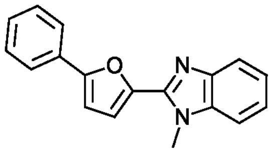, 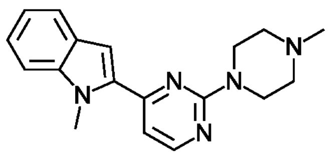,

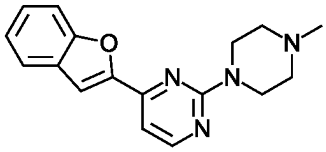,
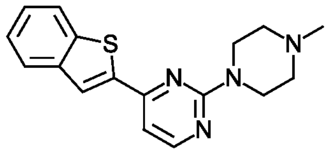,
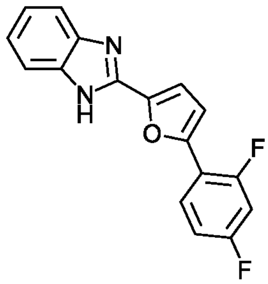,
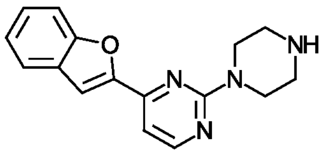,
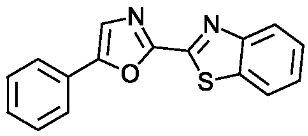,
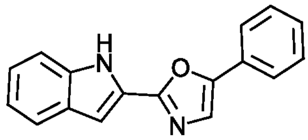,
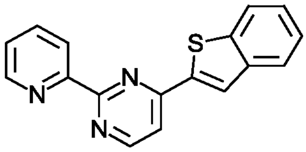,
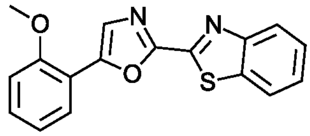,
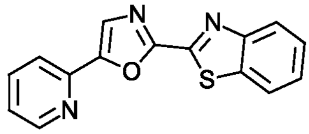,
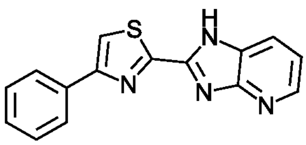,
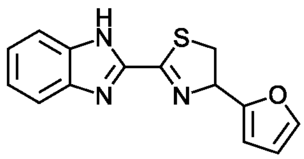,
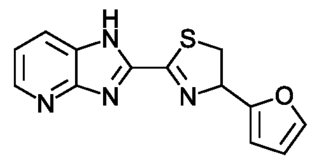,
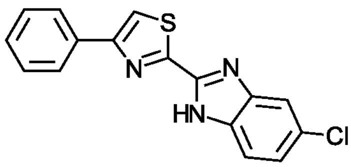,
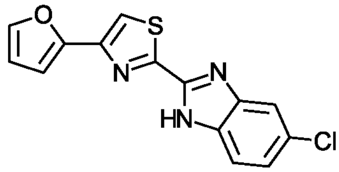,
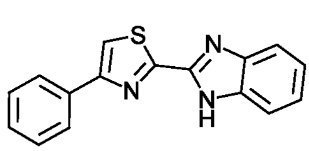,
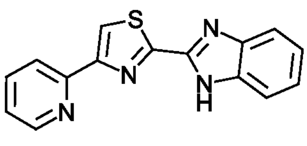,
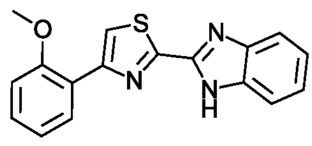,
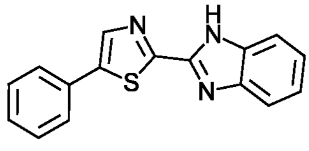,
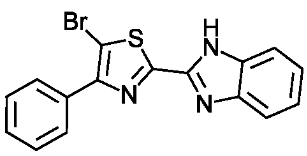,
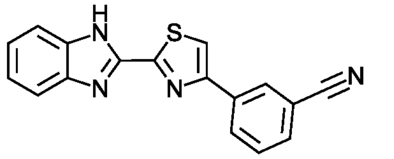,
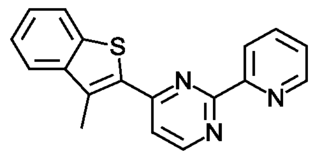,
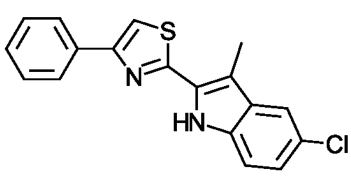,
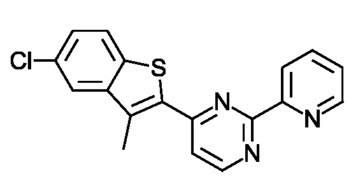,
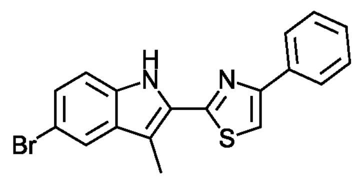,

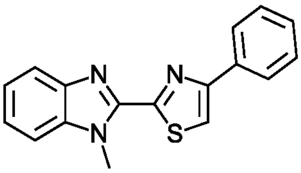, 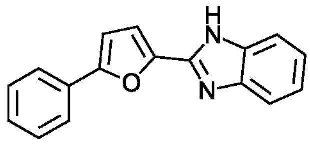 ou 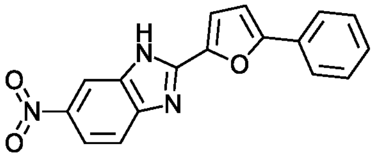.

**2.** Composé selon la revendication 1, dans lequel

L' est choisi parmi

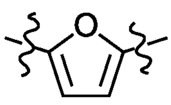, 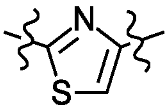, 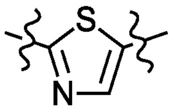, 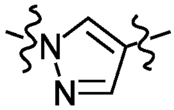,

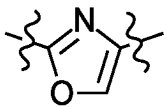, 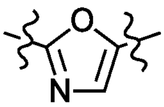, 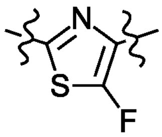, 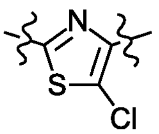, 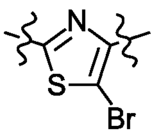, 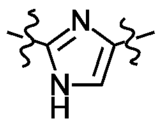,

et

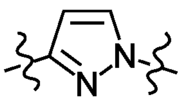;

$R^1$ est choisi parmi

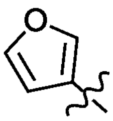, 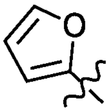, 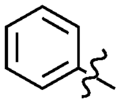, 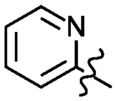, 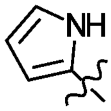, 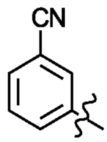,

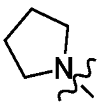, 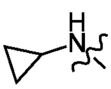, 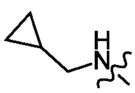, 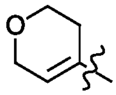, 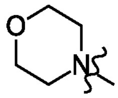, 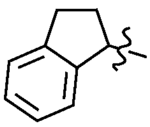, 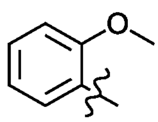, 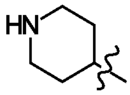,

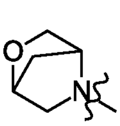, 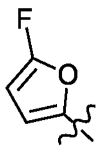, 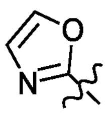, 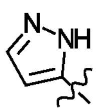, 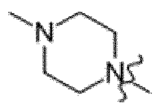 et 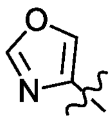;

$D^1$ est choisi parmi H, Br, F et Cl ;
$D^2$ est choisi parmi H, Br, F et Cl ;
$D^3$ est choisi parmi H, Br, F, Cl, $NO_2$, $CF_3$, OMe, $CH_3$, OH et

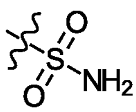;

et
$D^4$ est choisi parmi H, Br, F et Cl ;
ou dans lequel
$L^1$ est choisi parmi

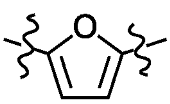, 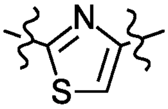, 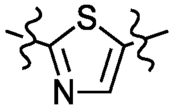 et 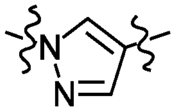 ;

$R^1$ est choisi parmi

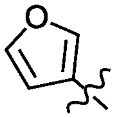, 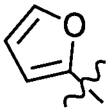, 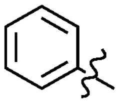, 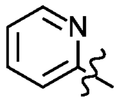 et 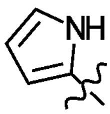 ;

$D^1$ est choisi parmi H, Br, F et Cl ;
$D^2$ est choisi parmi H, Br, F et Cl ;
$D^3$ est choisi parmi H, Br, F, Cl et $CF_3$ ; et
$D^4$ est choisi parmi H, Br, F et Cl ;
ou dans lequel
$L^1$ est choisi parmi

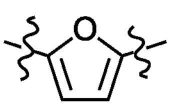, 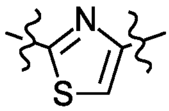, 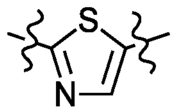 et 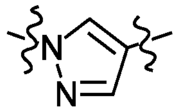 ;

$R^1$ est choisi parmi

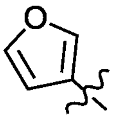, 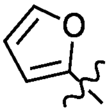, 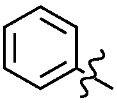, 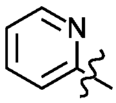 et 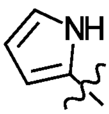 ;

$D^1$ représente H ;
$D^2$ représente H ;
$D^3$ est choisi parmi H, Br, F, Cl et $CF_3$ ; et
$D^4$ représente H.

**3.** Composé selon la revendication 1 ou 2, dans lequel $R^1$ représente

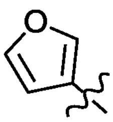, 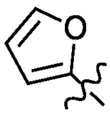,

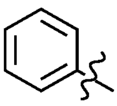, 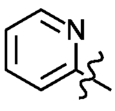, 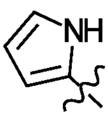, 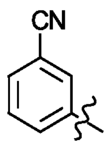, 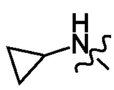, 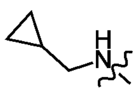, 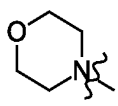, 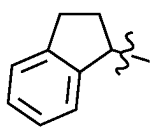,

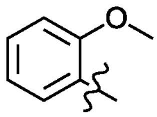 , 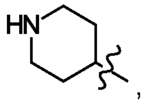 , 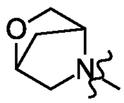 , 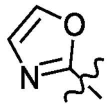 , 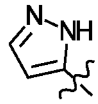 et 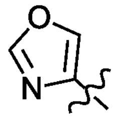 .

4. Composé selon l'une quelconque des revendications 1 à 3, dans lequel $D^1$ représente H ; $D^2$ représente H ; $D^3$ est choisi parmi H, Br, F, Cl et $CF_3$ ; et $D^4$ représente H.

5. Composé selon la revendication 1, dans lequel $R^1$ représente

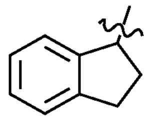

et L' est choisi parmi

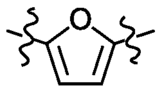 et 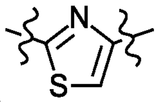 ;

ou dans lequel G est choisi parmi N, C(H) et $C(CH_3)$ ; et $J^1$ est choisi parmi N(H), $N(CH_3)$, $N(CH_2CH_3)$, S et O ; ou dans lequel
$L^1$ est choisi parmi

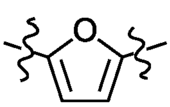 , 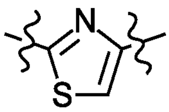 , 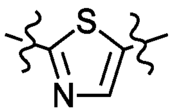 et 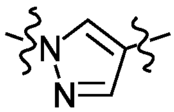 ;

$R^1$ est choisi parmi

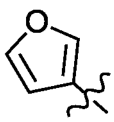 , 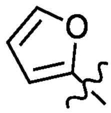 , 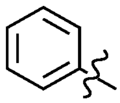 , 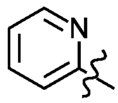 et 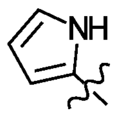 ;

$D^1$ est choisi parmi H, Br, F et Cl ;
$D^2$ est choisi parmi H, Br, F et Cl ;
$D^3$ est choisi parmi H, Br, F, Cl et $CF_3$ ; et
$D^4$ est choisi parmi H, Br, F et Cl ;
ou dans lequel G est choisi parmi N, C(H) et $C(CH_3)$ ;
$J^1$ est choisi parmi N(H), $N(CH_3)$, $N(CH_2CH_3)$, S et O ;
$L^1$ est choisi parmi

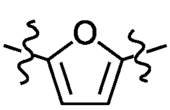 , 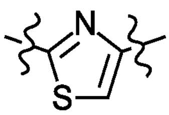 , 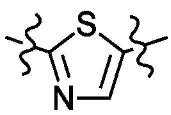 et 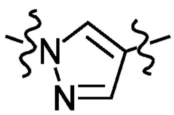 ;

$R^1$ est choisi parmi

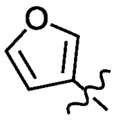, 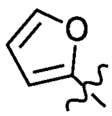, 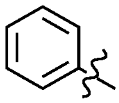, 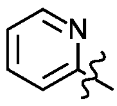 et 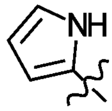 ;

$D^1$ représente H ;
$D^2$ représente H ;
$D^3$ est choisi parmi H, Br, F, Cl et $CF_3$ ; et
$D^4$ représente H.

**6.** Composé selon l'une quelconque des revendications 1 à 5, le composé étant

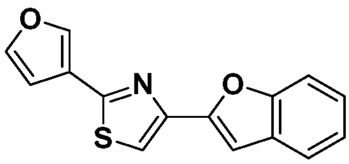, 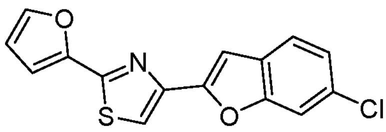, 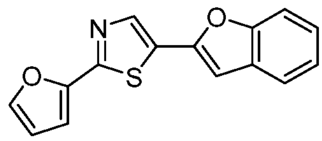,

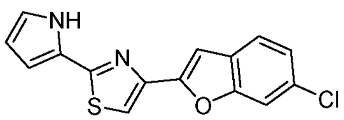, 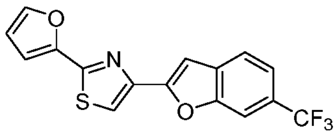 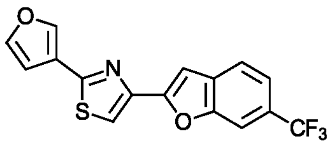,

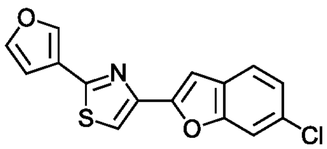, 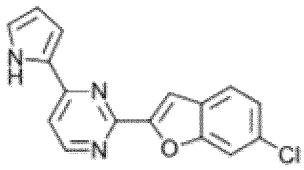, 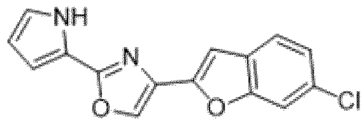,

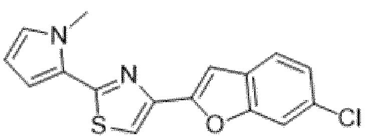, 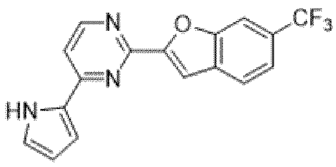, 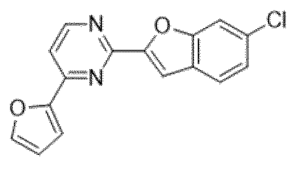,

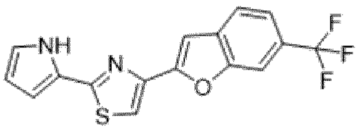, 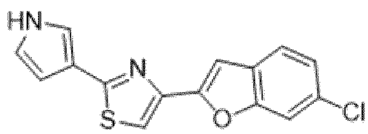, 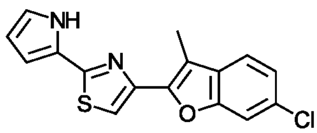,

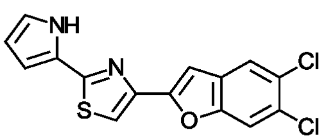, 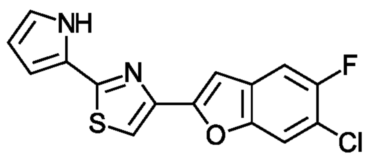 ou 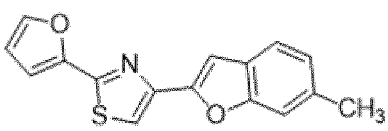.

**7.** Composé selon la revendication 1, le composé ayant la structure de formule II :

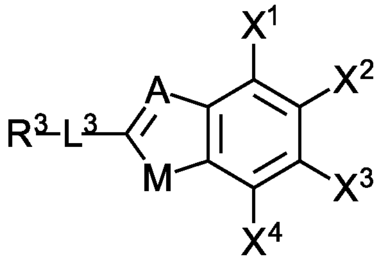

II

dans laquelle

A est choisi parmi N, C(H) et $C(CH_3)$ ;
M est choisi parmi N(H), $N(CF_3)$, $C(H)(CF_3)$, S et O ;
$X^1$ est choisi parmi H, Br, F et Cl ;
$X^2$ est choisi parmi H, Br, F et Cl ;
$X^3$ est choisi parmi H, Br, F, Cl, $CF_3$,

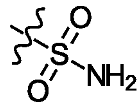

et OH ;
$X^4$ est choisi parmi H, Br, F et Cl ;
$L^3$ est choisi parmi

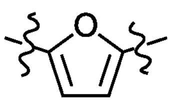, 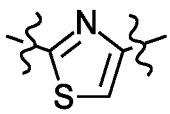, 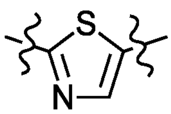, 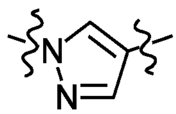,

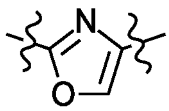, 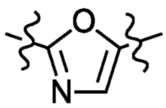 et 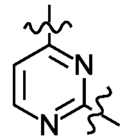 ;

$R^3$ est choisi parmi

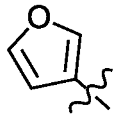, 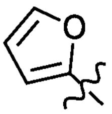, 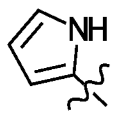, 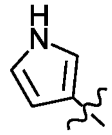 et 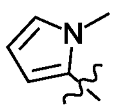.

**8.** Composé ayant la structure de formule III :

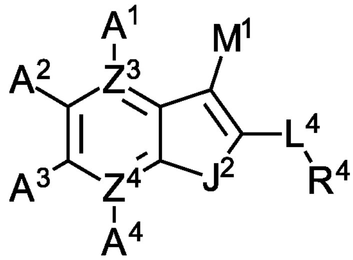

III

dans laquelle

$J^2$ est choisi parmi $CH_2$, $CH(CH_3)$, N(H), $N(CH_3)$, $N(CH_2CH_3)$, $N(CF_3)$, $C(H)(CF_3)$, S et O ;
$M^1$ est choisi parmi H et $CH_3$ ;
$Z^3$ représente C ;
$Z^4$ représente C ;
en variante, $Z^3$ représente N, lorsque $A^1$ est absent ;
en variante, $Z^4$ représente N, lorsque $A^4$ est absent ;
$L^4$ est choisi parmi

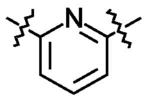 et 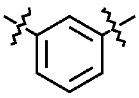 ;

$R^4$ est choisi parmi

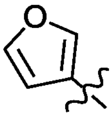, 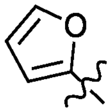, 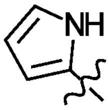, 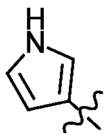, 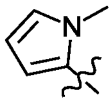, 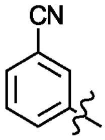,

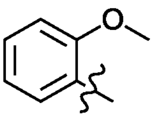, 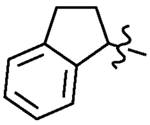, 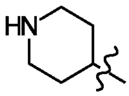, 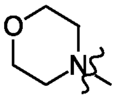, 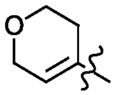, 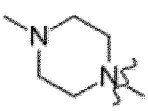, 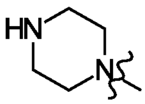, 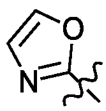,

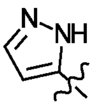, 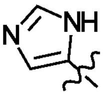, 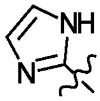, 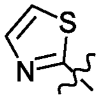, 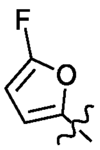, 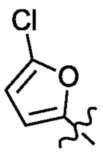, 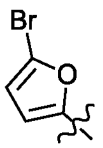, 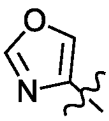, 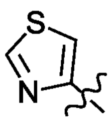, 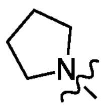,

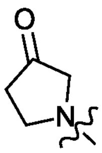, 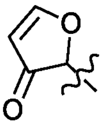, 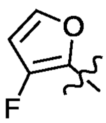, 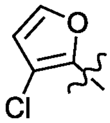, 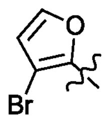, 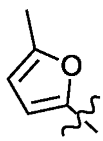, 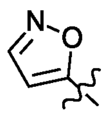, 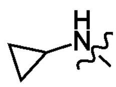, 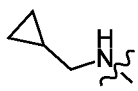

et

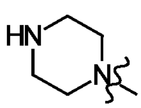 ;

$A^1$ est choisi parmi H, Br, F, Cl et OH ;
$A^2$ est choisi parmi H, Br, F et Cl ;
$A^3$ est choisi parmi H, Br, F, Cl, $CF_3$,

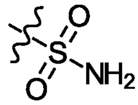,

OMe, $CH_3$, $NO_2$ et OH ;
$A^4$ est choisi parmi H, Br, F et Cl.

**9.** Composé selon la revendication 8, dans lequel $R^4$ est choisi parmi

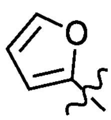, 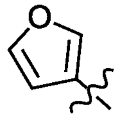,

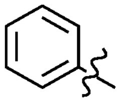, 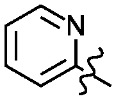, 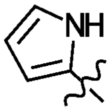, 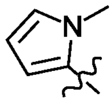 et 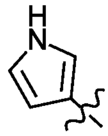.

**10.** Composé selon la revendication 8 ou 9, dans lequel $R^4$ est choisi parmi

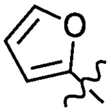,

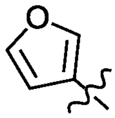, 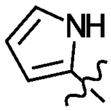, 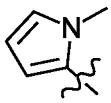 et 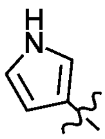.

**11.** Composé selon l'une quelconque des revendications 8 à 10, dans lequel

$A^1$ est choisi parmi H, Br, F, Cl et OH ;
$A^2$ est choisi parmi H, Br, F et Cl ;
$A^3$ est choisi parmi H, Br, F, Cl, $CF_3$, et $CH_3$; et
$A^4$ est choisi parmi H, Br, F et Cl.

**12.** Composé selon l'une quelconque des revendications 8 à 11, dans lequel $J^2$ est choisi parmi $CH_2$, $CH(CH_3)$, N(H), $N(CH_3)$, S et O;

ou dans lequel $J^2$ est choisi parmi $CH_2$, $CH(CH_3)$, N(H), S et O; ou
ou dans lequel $J^2$ représente O.

**13.** Composé selon l'une quelconque des revendications 8 à 12, dans lequel $L^4$ représente

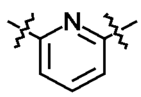.

**14.** Composé ayant la structure de formule I :

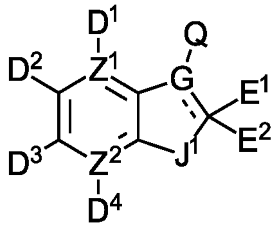

I

dans laquelle

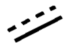

est une double liaison ;
Q est absent ;
G est choisi parmi N, C(H) et $C(CH_3)$ ;
$J^1$ est choisi parmi N(H), $N(CH_3)$, $N(CH_2CH_3)$, $N(CF_3)$, $C(H)(CF_3)$, S et O ;
$Z^1$ représente C ou N ;
$Z^2$ représente C ou N ;
en variante, $Z^1$ représente N, lorsque $D^1$ est absent ;
en variante, $Z^2$ représente N, lorsque $D^4$ est absent ;
$E^2$ est absent ;
$E^1$ représente $L^1$-$R^1$ ;
L' est choisi parmi

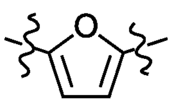 , 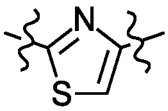 , 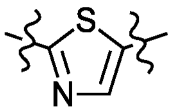 , 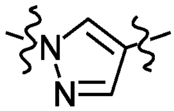 ,

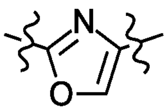 , 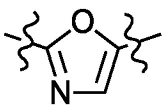 , 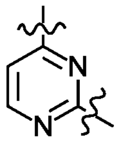 , 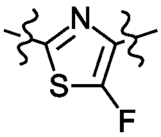 , 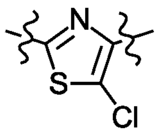 , 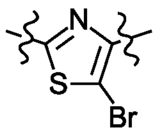 , 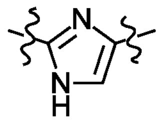

, 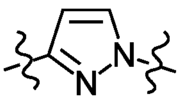 , et 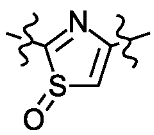 ;

$R^1$ est choisi parmi

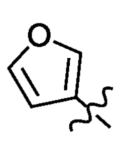 , 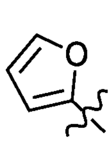 , 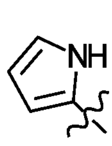 , 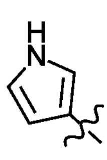 , 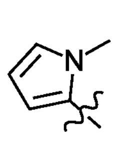 , 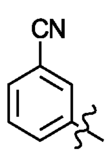 ,

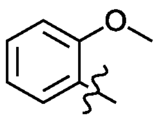 , 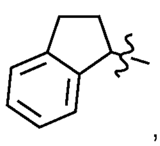 , 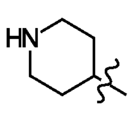 , 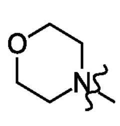 , 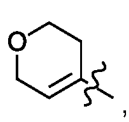 , 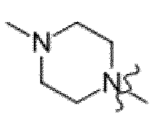 , 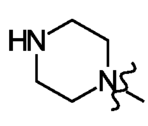 , 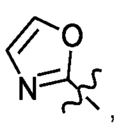 ,

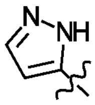, 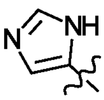, 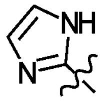, 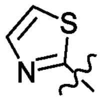, 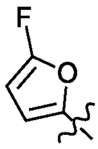, 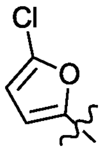, 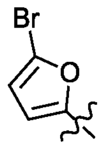, 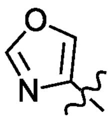, 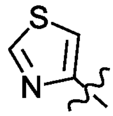, 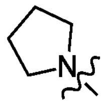,

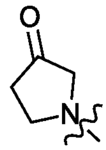, 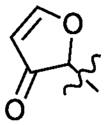, 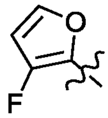, 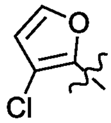, 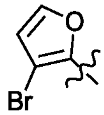, 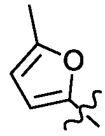, 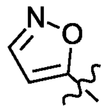, 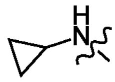, 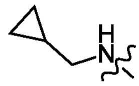 et

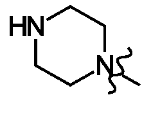 ;

en variante, $R^1$ représente

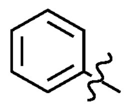

à condition que $J^1$ soit choisi parmi N(H), $N(CH_2CH_3)$, $N(CF_3)$, $C(H)(CF_3)$, S et O ;
en variante, $R^1$ représente

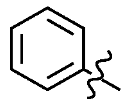

à condition que $J^1$ représente $N(CH_3)$, $L^1$ soit choisi parmi

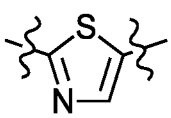, 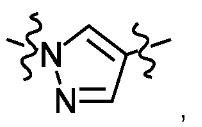, 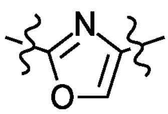, 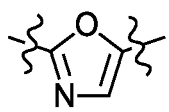 et 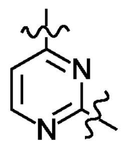

et $D^3$ soit choisi parmi H, Br, F, Cl, $NO_2$, $CF_3$,

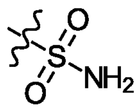,

OMe, $CH_3$ et OH ;
en variante, $R^1$ représente

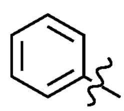

à condition que $J^1$ représente $N(CH_3)$, $L^1$ représente

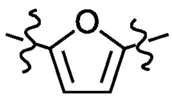

et $D^3$ soit choisi parmi Br, F, Cl,

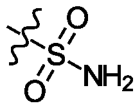

,

OMe, $CH_3$ et OH ;
en variante, $R^1$ représente

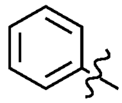

à condition que $J^1$ représente $N(CH_3)$, $L^1$ représente

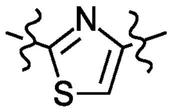

et $D^3$ soit choisi parmi $NO_2$, $CF_3$,

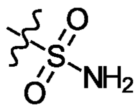

,

OMe, $CH_3$ et OH ;
en variante, $R^1$ représente

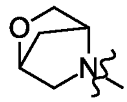

à condition que $D^3$ soit choisi parmi Br, F, Cl et NOz ;
en variante, $R^1$ représente

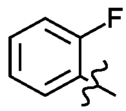

à condition que $D^3$ soit choisi parmi H, Br, F, Cl et NOz ;
en variante, $R^1$ représente

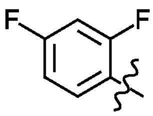

à condition que $D^3$ représente H ;
en variante, $R^1$ représente

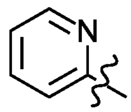
,

à condition que $J^1$ soit choisi parmi N(H), $N(CH_3)$ et O ;
en variante, $R^1$ représente

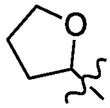

à condition que $D^3$ soit choisi parmi H, Br, F, Cl, $CF_3$ et $NO_2$ ;
$D^1$ est choisi parmi H, Br, F, Cl et OH ;
$D^2$ est choisi parmi H, Br, F et Cl ;
$D^3$ est choisi parmi H, Br, F, Cl, $CF_3$,

, 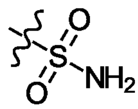 ,

OMe, $CH_3$ et OH ;
en variante, $D^3$ représente NOz, à condition que lorsque $R^1$ représente

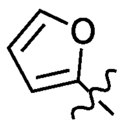

et lorsque $J^1$ représente O, $D^3$ soit choisi parmi H, Br, F, Cl, $CF_3$ et

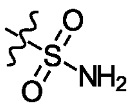
;

$D^4$ est choisi parmi H, Br, F et Cl ;
ou un composé selon l'une quelconque des revendications 7 à 13,
pour une utilisation dans le traitement du cancer.

**15.** Composé pour une utilisation selon la revendication 14, où le cancer est un cancer avec expression de BRN2 choisi parmi les cancers suivants :

cancer de la prostate (tel que le cancer neuroendocrine de la prostate (NEPC), l'adénocarcinome de la prostate, le cancer de la prostate résistant à la castration (CPRC), le cancer de la prostate résistant aux inhibiteurs de la voie du récepteur des androgènes (IPRA), résistant à l'enzalutamide (ENZ) ($ENZ^R$) ; et résistant à l'abiratérone (Abi) ($ABI_R$)) ;
cancer du poumon (tel que le cancer du poumon à petites cellules (CPPC) ou l'adénocarcinome du poumon) ;
cancer de la vessie (tel que le cancer de la vessie à petites cellules (CPCV)) ;
sarcome (tel que le sarcome d'Ewing) ;
gliome (tel que le glioblastome multiforme) ; et
mélanome ; ou
un cancer choisi parmi les suivants : cancer de la vessie ; cholangiocarcinome ; cancer colorectal ; lymphome

diffus à grandes cellules B (DLBC) ; cancer du foie ; cancer de l'ovaire ; thymome ; cancer de la thyroïde ; carcinome rénal à cellules claires (ccRCC) ; carcinome rénal chromophobe (ChRCC) ; cancer de la prostate ; cancer du sein ; cancer de l'utérus ; cancer du pancréas ; cancer du col de l'utérus ; mélanome uvéal ; leucémie myéloïde aiguë (LMA) ; cancer de la tête et du cou ; cancer du poumon à petites cellules (CPPC) ; sarcome adénocarcinome pulmonaire ; mésothéliome ; carcinome adénoïde kystique (CAK) ; sarcome ; cancer des cellules germinales testiculaires ; cancer de l'utérus ; phéochromocytome et paragangliome (PHEO/PGL) ; mélanome ; gliome ; glioblastome multiforme ; leucémie lymphoblastique aiguë à lymphocytes T ; lymphome à lymphocytes T, médulloblastome ; et neuroblastome.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015120543 A


### Non-patent literature cited in the description


- **BISHOP et al.** *Cancer Discovery,* 2017, vol. 7 (1), 54-71
- **XU et al.** *Chemical Biology & Drug Design,* 2018, vol. 91, 172-180
- **IRWIN, J. et al.** Abstracts of Papers. Am. Chem. Soc, 2005, vol. 230, U1009
- **BERGE S. M. et al.** *J. Pharm. Sci.,* 1977, vol. 66 (1), 1-19
- Remington: the Science & Practice of Pharmacy by Alfonso Gennaro. Lippencott Williams & Wilkins, 2000
- Comparative Protein Structure Modeling Using Modeller. **B. WEBB ; A. SALI.** Current Protocols in Bioinformatics. John Wiley & Sons, Inc, 2016, vol. 54, 5.6.1-5.6.37
- **DAVID CASE ; THOMAS CHEATHAM, III ; TOM DARDEN ; HOLGER GOHLKE ; RAY LUO ; KENNETH MERZ, JR. ; ALEXEY ONUFRIEV ; CARLOS SIMMERLING ; BING WANG ; ROBERT J. WOODS.** The Amber Biomolecular Simulation Programs. *J Comput Chem.,* December 2005, vol. 26 (16), 1668-1688
- **TEAGUE STERLING ; JOHN J. IRWIN.** ZINC 15 - Ligand Discovery for Everyone. *J Chem Inf Model.,* 2015, vol. 55 (11), 2324-2337
- **FRIESNER RA1 ; BANKS JL ; MURPHY RB ; HALGREN TA ; KLICIC JJ ; MAINZ DT ; REPASKY MP ; KNOLL EH ; SHELLEY M, PERRY JK ; SHAW DE.** Glide: a new approach for rapid, accurate docking and scoring. 1. *Method and assessment of docking accuracy. J Med Chem.,* 25 March 2004, vol. 47 (7), 1739-49
- **STEFANO FORLI ; RUTH HUEY ; MICHAEL E. PIQUE ; MICHEL SANNER ; DAVID S. GOODSELL ; ARTHUR J. OLSON.** Computational protein-ligand docking and virtual drug screening with the AutoDock suite. *Nat Protoc.,* 2016, vol. 11 (5), 905-919